# EUROPEAN PATENT SPECIFICATION

(11) **EP 1 781 796 B1**
(45) Date of publication and mention of the grant of the patent: **11.09.2013**
(21) Application number: 05769842.5
(22) Date of filing: 07.07.2005
(51) Int. Cl.: C12N 15/90, C12N 5/10, C12N 15/85, A01K 67/027

(54) **TARGETED TRANSGENESIS OF SHORT HAIRPIN RNA EXPRESSION CASSETTES USING RECOMBINASE MEDIATED CASSETTE EXCHANGE**
GEZIELTE TRANSGENESE KURZER HAARNADEL-RNA-EXPRESSIONSKASSETTEN MITTELS REKOMBINASE-VERMITTELTEM KASSETTENAUSTAUSCH
TRANSGENESE CIBLEE DE CASSETTES D'EXPRESSION D'ARN COURT EN EPINGLE A CHEVEUX UTILISANT UN ECHANGE DE CASSETTES INDUIT PAR LA RECOMBINASE

(30) Priority: 07.07.2004 EP 04103229
(43) Date of publication of application: 09.05.2007
(73) Proprietor: TaconicArtemis GmbH, 51063 Köln (DE)
(72) Inventor: SEIBLER, Jost, 50733 Köln (DE); SCHWENK, Frieder, 50999 Köln (DE); KERN, Heidrun, 40227 Düsseldorf (DE)
(74) Representative: von Kreisler Selting Werner
(86) International application number: PCT/EP2005/053245
(87) International publication number: WO 2006/003215

(56) References cited:
- EP-A- 0 939 120
- WO-A-2004/035782
- SEIBLER J. ET AL.: "Single copy shRNA configuration for ubiquitous gene knockdown in mice." NUCL. ACIDS RES., vol. 33, no. 7, 14 April 2005 (2005-04-14), page E67, XP002361032 cited in the application
- BRANKO ZEVNIK ET AL: "Speeding up conditional gene targeting: RNAi & ES mouse technology" ORAL PRESENTATION AT THE 2ND WORKSHOP ON INNOVATIVE MOUSE MODELS, 17 June 2004 (2004-06-17), XP002361033 LEIDEN UNIVERSITY MEDICAL CENTER, THE NETHERLANDS.
- SEIBLER J ET AL: "Rapid generation of inducible mouse mutants" NUCLEIC ACIDS RESEARCH, OXFORD UNIVERSITY PRESS, SURREY, GB, vol. 31, no. 4, 15 February 2003 (2003-02-15), pages E12-1, XP002300497 ISSN: 0305-1048 cited in the application
- WUNDERLICH F.T.: "Generation of inducible Cre systems for conditional gene inactivation in mice (Dissertation)." March 2004 (2004-03), UNIVERSITÄT KÖLN , KÖLN, GERMANY , XP002361035 the whole document
- BODE J. ET AL.: "The transgenicist's toolbox: Novel methods for the targeted modification of eukaryotic genomes.", BIOL CHEM, vol. 381, 2000, pages 801-813, XP001021315,

### SEQUENCE LISTING

<110> ARTEMIS Pharmaceuticals GmbH
<120> Targeted Transgenesis of Short Hairpin RNA Expression Cassettes Using Recombinase Mediated Cassette Exchange
<130> 050876wo/JH
<140>
   <141>
<160> 219
<170> PatentIn Ver. 2.1
<210> 1
   <211> 23
   <212> DNA
   <213> Artificial Sequence
<220>
   <223> Description of Artificial Sequence: primer Rscreen1s
<400> 1
   gacaggacag tgcttgttta agg 23
<210> 2
   <211> 23
   <212> DNA
   <213> Artificial Sequence
<220>
   <223> Description of Artificial Sequence: primer Rscreen1as
<400> 2
   tgactacaca atattgctcg cac 23
<210> 3
   <211> 13139
   <212> DNA
   <213> Mus musculus
<220>
   <223> Description: Rosa26 locus
<400> 3
   aagcttctca cgtagcaacc agagctccag agccagcagc tgctgccgcc ttgtatactc 60
   actcctgtga tccaacacag gagcaacctt ttctttaccc cacccccact tcttaacaca 120
   cttttttttg gggggggggg gggaacaagt gctccatgct ggaaggattg gaactatgct 180
   tttagaaagg aacaatccta aggtcacttt taaattgagg tctttgattt gaaaatcaac 240
   aaataccaaa ttccaaatat tcgttttaat taaaccagca atgtggatat aagcattaag 300
   ttttagtttt aaaaaggtca attttccaaa cattcagcaa tcatatttaa atttacagct 360
   aggaacaaga gccttgggtc atgtcctacc aaagaacata actcaatatt ctacacatga 420
   caatctgaat aaccttaaag cctctaatcc cataacaggc cacaaatttt ggacagagaa 480
   ctaatgatcc tcctgagaaa actggaagaa atccagggaa aagaaattcc tgtgtcctcc 540
   aaactcagaa atctctaatt atgtcagtat tctctgcttt agtcctaggt cagattgcac 600
   acatctaaaa taacctctta aagttttcct cctagcgacc taaaccatta ttaatatcaa 660
   attaaccatc aaaacacttt cctctcaata tgctgcacac aaacctcctc ctggaacctc 720
   ctccatctgg atcctcccca atcaaaagta taggtattta acatataagc aaggaagtaa 780
   tgtaaacatg accttggtca caaatatgtc atctaaaaac aatttagtca aggtatggag 840
   gaaattcgag aacctgaatc tttttaagta ttttgagcac aggaacaatt ggcaaaagga 900
   atccaggtat agacaaaacc cagagcccag agctctgggc gaaaaatgag ttgctggtga 960
   agacgttaca caagtaacat gagaaagcag aaaatgcagg tcatccacgc acccctgacc 1020
   caggccagca gggcgggctg cagcatcagt acacaggaga aagatcctta ttcctaagaa 1080
   tgagaaaggc aaaggcgccc gatagaataa attagcatag aaggggcttt cccaggagtt 1140
   aaaactttcc ttctgagcga ttacctacta aaaccagggc ttttgcccac taccatttac 1200
   ctaggatctt ggcttgcacg gattcatagg ggcatatccc tccccctctt ctttagagtc 1260
   gttcttaaaa gatcgctctc cacgccctag gcagggaaaa cgacaaaatc tggctcaatt 1320
   ccaggctaga accctacaaa ttcaacaggg atatcgcaag gatactgggg catacgccac 1380
   agggagtcca agaatgtgag gtgggggtgg cgaaggtaat gtctttggtg tgggaaaagc 1440
   agcagccatc tgagatagga actggaaaac cagaggagag gcgttcagga agattatgga 1500
   ggggaggact gggcccccac gagcgaccag agttgtcaca aggccgcaag aacaggggag 1560
   gtggggggct cagggacaga aaaaaaagta tgtgtatttt gagagcaggg ttgggaggcc 1620
   tctcctgaaa agggtataaa cgtggagtag gcaataccca ggcaaaaagg ggagaccaga 1680
   gtagggggag gggaagagtc ctgacccagg gaagacatta aaaaggtagt ggggtcgact 1740
   agatgaagga gagcctttct ctctgggcaa gagcggtgca atggtgtgta aaggtagctg 1800
   agaagacgaa aagggcaagc atcttcctgc taccaggctg gggaggccca ggcccacgac 1860
   cccgaggaga gggaacgcag ggagactgag gtgacccttc tttcccccgg ggcccggtcg 1920
   tgtggttcgg tgtctctttt ctgttggacc cttaccttga cccaggcgct gccggggcct 1980
   gggcccgggc tgcggcgcac ggcactcccg ggaggcagcg agactcgagt taggcccaac 2040
   gcggcgccac ggcgtttcct ggccgggaat ggcccgtacc cgtgaggtgg gggtgggggg 2100
   cagaaaaggc ggagcgagcc cgagcgggga gggggagggc caggggcgga gggggccggc 2160
   actactgtgt tggcggactg gcgggactag ggctgcgtga gtctctgagc gcaggcgggc 2220
   ggcggccgcc cctcccccgg cggcggcagc ggcggcagcg gcggcagctc actcagcccg 2280
   ctgcccgagc ggaaacgcca ctgaccgcac ggggattccc agtgccggcg ccaggggcac 2340
   gcgggacacg ccccctcccg ccgcgccatt ggcctctccg cccaccgccc cacacttatt 2400
   ggccggtgcg ccgccaatca gcggaggctg ccggggccgc ctaaagaaga ggctgtgctt 2460
   tggggctccg gctcctcaga gagcctcggc taggtagggg atcgggactc tggcgggagg 2520
   gcggcttggt gcgtttgcgg ggatgggcgg ccgcggcagg ccctccgagc gtggtggagc 2580
   cgttctgtga gacagccggg tacgagtcgt gacgctggaa ggggcaagcg ggtggtgggc 2640
   aggaatgcgg tccgccctgc agcaaccgga gggggaggga gaagggagcg gaaaagtctc 2700
   caccggacgc ggccatggct cggggggggg ggggcagcgg aggagcgctt ccggccgacg 2760
   tctcgtcgct gattggcttc ttttcctccc gccgtgtgtg aaaacacaaa tggcgtgttt 2820
   tggttggcgt aaggcgcctg tcagttaacg gcagccggag tgcgcagccg ccggcagcct 2880
   cgctctgccc actgggtggg gcgggaggta ggtggggtga ggcgagctgg acgtgcgggc 2940
   gcggtcggcc tctggcgggg cgggggaggg gagggagggt cagcgaaagt agctcgcgcg 3000
   cgagcggccg cccaccctcc ccttcctctg ggggagtcgt tttacccgcc gccggccggg 3060
   cctcgtcgtc tgattggctc tcggggccca gaaaactggc ccttgccatt ggctcgtgtt 3120
   cgtgcaagtt gagtccatcc gccggccagc gggggcggcg aggaggcgct cccaggttcc 3180
   ggccctcccc tcggccccgc gccgcagagt ctggccgcgc gcccctgcgc aacgtggcag 3240
   gaagcgcgcg ctgggggcgg ggacgggcag tagggctgag cggctgcggg gcgggtgcaa 3300
   gcacgtttcc gacttgagtt gcctcaagag gggcgtgctg agccagacct ccatcgcgca 3360
   ctccggggag tggagggaag gagcgagggc tcagttgggc tgttttggag gcaggaagca 3420
   cttgctctcc caaagtcgct ctgagttgtt atcagtaagg gagctgcagt ggagtaggcg 3480
   gggagaaggc cgcacccttc tccggagggg ggaggggagt gttgcaatac ctttctggga 3540
   gttctctgct gcctcctggc ttctgaggac cgccctgggc ctgggagaat cccttccccc 3600
   tcttccctcg tgatctgcaa ctccagtctt tctagaagat gggcgggagt cttctgggca 3660
   ggcttaaagg ctaacctggt gtgtgggcgt tgtcctgcag gggaattgaa caggtgtaaa 3720
   attggaggga caagacttcc cacagatttt cggttttgtc gggaagtttt ttaatagggg 3780
   caaataagga aaatgggagg ataggtagtc atctggggtt ttatgcagca aaactacagg 3840
   ttattattgc ttgtgatccg cctcggagta ttttccatcg aggtagatta aagacatgct 3900
   cacccgagtt ttatactctc ctgcttgaga tccttactac agtatgaaat tacagtgtcg 3960
   cgagttagac tatgtaagca gaattttaat catttttaaa gagcccagta cttcatatcc 4020
   atttctcccg ctccttctgc agccttatca aaaggtattt tagaacactc attttagccc 4080
   cattttcatt tattatactg gcttatccaa cccctagaca gagcattggc attttccctt 4140
   tcctgatctt agaagtctga tgactcatga aaccagacag attagttaca tacaccacaa 4200
   atcgaggctg tagctggggc ctcaacactg cagttctttt ataactcctt agtacacttt 4260
   ttgttgatcc tttgccttga tccttaattt tcagtgtcta tcacctctcc cgtcagtggt 4320
   gttccacatt tgggcctatt ctcagtccag ggagttttac aacaatagat gtattgagaa 4380
   tccaacctaa agcttaactt tccactccca tgaatgcctc tctccttttt ctccatttat 4440
   aaactgagct attaaccatt aatggttcca ggtggatgtc tcctccccat attacctgat 4500
   gtatcttaca tattgccagg ctgatatttt aagacattaa aaggtatatt tcattattga 4560
   gccacatggt attgattact gcttactaaa attttgtcat tgtacacatc tgtaaaaggt 4620
   ggttcctttt ggaatgcaaa gttcaggtgt ttgttgtctt tcctgaccta aggtcttgtg 4680
   agcttgtatt ttttctattt aagcagtgct ttctcttgga ctggcttgac tcatggcatt 4740
   ctacacgtta ttgctggtct aaatgtgatt ttgccaagct tcttcaggac ctataatttt 4800
   gcttgacttg tagccaaaca caagtaaaat gattaagcaa caaatgtatt tgtgaagctt 4860
   ggtttttagg ttgttgtgtt gtgtgtgctt gtgctctata ataatactat ccaggggctg 4920
   gagaggtggc tcggagttca agagcacaga ctgctcttcc agaagtcctg agttcaattc 4980
   ccagcaacca catggtggct cacaaccatc tgtaatggga tctgatgccc tcttctggtg 5040
   tgtctgaaga ccacaagtgt attcacatta aataaataaa tcctccttct tcttcttttt 5100
   ttttttttta aagagaatac tgtctccagt agaatttact gaagtaatga aatactttgt 5160
   gtttgttcca atatggtagc caataatcaa attactcttt aagcactgga aatgttacca 5220
   aggaactaat ttttatttga agtgtaactg tggacagagg agccataact gcagacttgt 5280
   gggatacaga agaccaatgc agactttaat gtcttttctc ttacactaag caataaagaa 5340
   ataaaaattg aacttctagt atcctatttg tttaaactgc tagctttact taacttttgt 5400
   gcttcatcta tacaaagctg aaagctaagt ctgcagccat tactaaacat gaaagcaagt 5460
   aatgataatt ttggatttca aaaatgtagg gccagagttt agccagccag tggtggtgct 5520
   tgcctttatg cctttaatcc cagcactctg gaggcagaga caggcagatc tctgagtttg 5580
   agcccagcct ggtctacaca tcaagttcta tctaggatag ccaggaatac acacagaaac 5640
   cctgttgggg aggggggctc tgagatttca taaaattata attgaagcat tccctaatga 5700
   gccactatgg atgtggctaa atccgtctac ctttctgatg agatttgggt attatttttt 5760
   ctgtctctgc tgttggttgg gtcttttgac actgtgggct ttctttaaag cctccttcct 5820
   gccatgtggt ctcttgtttg ctactaactt cccatggctt aaatggcatg gctttttgcc 5880
   ttctaagggc agctgctgag atttgcagcc tgatttccag ggtggggttg ggaaatcttt 5940
   caaacactaa aattgtcctt taattttttt tttaaaaaat gggttatata ataaacctca 6000
   taaaatagtt atgaggagtg aggtggacta atattaaatg agtccctccc ctataaaaga 6060
   gctattaagg ctttttgtct tatacttaac ttttttttta aatgtggtat ctttagaacc 6120
   aagggtctta gagttttagt atacagaaac tgttgcatcg cttaatcaga ttttctagtt 6180
   tcaaatccag agaatccaaa ttcttcacag ccaaagtcaa attaagaatt tctgactttt 6240
   aatgttaatt tgcttactgt gaatataaaa atgatagctt ttcctgaggc agggtctcac 6300
   tatgtatctc tgcctgatct gcaacaagat atgtagacta aagttctgcc tgcttttgtc 6360
   tcctgaatac taaggttaaa atgtagtaat acttttggaa cttgcaggtc agattctttt 6420
   ataggggaca cactaaggga gcttgggtga tagttggtaa aatgtgtttc aagtgatgaa 6480
   aacttgaatt attatcaccg caacctactt tttaaaaaaa aaagccaggc ctgttagagc 6540
   atgcttaagg gatccctagg acttgctgag cacacaagag tagttacttg gcaggctcct 6600
   ggtgagagca tatttcaaaa aacaaggcag acaaccaaga aactacagtt aaggttacct 6660
   gtctttaaac catctgcata tacacaggga tattaaaata ttccaaataa tatttcattc 6720
   aagttttccc ccatcaaatt gggacatgga tttctccggt gaataggcag agttggaaac 6780
   taaacaaatg ttggttttgt gatttgtgaa attgttttca agtgatagtt aaagcccatg 6840
   agatacagaa caaagctgct atttcgaggt ctcttggttt atactcagaa gcacttcttt 6900
   gggtttccct gcactatcct gatcatgtgc taggcctacc ttaggctgat tgttgttcaa 6960
   ataaacttaa gtttcctgtc aggtgatgtc atatgatttc atatatcaag gcaaaacatg 7020
   ttatatatgt taaacatttg tacttaatgt gaaagttagg tctttgtggg tttgattttt 7080
   aattttcaaa acctgagcta aataagtcat ttttacatgt cttacatttg gtggaattgt 7140
   ataattgtgg tttgcaggca agactctctg acctagtaac cctacctata gagcactttg 7200
   ctgggtcaca agtctaggag tcaagcattt caccttgaag ttgagacgtt ttgttagtgt 7260
   atactagttt atatgttgga ggacatgttt atccagaaga tattcaggac tatttttgac 7320
   tgggctaagg aattgattct gattagcact gttagtgagc attgagtggc ctttaggctt 7380
   gaattggagt cacttgtata tctcaaataa tgctggcctt ttttaaaaag cccttgttct 7440
   ttatcaccct gttttctaca taatttttgt tcaaagaaat acttgtttgg atctcctttt 7500
   gacaacaata gcatgttttc aagccatatt ttttttcctt tttttttttt tttttggttt 7560
   ttcgagacag ggtttctctg tatagccctg gctgtcctgg aactcacttt gtagaccagg 7620
   ctggcctcga actcagaaat ccgcctgcct ctgcctcctg agtgccggga ttaaaggcgt 7680
   gcaccaccac gcctggctaa gttggatatt ttgttatata actataacca atactaactc 7740
   cactgggtgg atttttaatt cagtcagtag tcttaagtgg tctttattgg cccttcatta 7800
   aaatctactg ttcactctaa cagaggctgt tggtactagt ggcacttaag caacttccta 7860
   cggatatact agcagattaa gggtcaggga tagaaactag tctagcgttt tgtataccta 7920
   ccagctttat actaccttgt tctgatagaa atatttcagg acatctagag tgtactataa 7980
   ggttgatggt aagcttataa ggaacttgaa agtggagtaa ctactccatt tctctgaggg 8040
   gagaattaaa atttttgacc aagtgttgtt gagccactga gaatggtctc agaacataac 8100
   ttcttaagga accttcccag attgccctca acactgcacc acatttggtc ctgcttgaac 8160
   attgccatgg ctcttaaagt cttaattaag aatattaatt gtgtaattat tgtttttcct 8220
   cctttagatc attccttgag gacaggacag tgcttgttta aggctatatt tctgctgtct 8280
   gagcagcaac aggtcttcga gatcaacatg atgttcataa tcccaagatg ttgccattta 8340
   tgttctcaga agcaagcaga ggcatgatgg tcagtgacag taatgtcact gtgttaaatg 8400
   ttgctatgca gtttggattt ttctaatgta gtgtaggtag aacatatgtg ttctgtatga 8460
   attaaactct taagttacac cttgtataat ccatgcaatg tgttatgcaa ttaccatttt 8520
   aagtattgta gctttctttg tatgtgagga taaaggtgtt tgtcataaaa tgttttgaac 8580
   atttccccaa agttccaaat tataaaacca caacgttaga acttatttat gaacaatggt 8640
   tgtagtttca tgcttttaaa atgcttaatt attcaattaa caccgtttgt gttataatat 8700
   atataaaact gacatgtaga agtgtttgtc cagaacattt cttaaatgta tactgtcttt 8760
   agagagttta atatagcatg tcttttgcaa catactaact tttgtgttgg tgcgagcaat 8820
   attgtgtagt cattttgaaa ggagtcattt caatgagtgt cagattgttt tgaatgttat 8880
   tgaacatttt aaatgcagac ttgttcgtgt tttagaaagc aaaactgtca gaagctttga 8940
   actagaaatt aaaaagctga agtatttcag aagggaaata agctacttgc tgtattagtt 9000
   gaaggaaagt gtaatagctt agaaaattta aaaccatata gttgtcattg ctgaatatct 9060
   ggcagatgaa aagaaatact cagtggttct tttgagcaat ataacagctt gttatattaa 9120
   aaatttcccc cacagatata aactctaatc tataactcat aaatgttaca aatggatgaa 9180
   gcttacaaat gtggcttgac ttgtcactgt gcttgtttta gttatgtgaa agtttggcaa 9240
   taaacctatg tcctaaatag tcaaactgtg gaatgacttt ttaatctatt ggtttgtcta 9300
   gaacagttat gttgccattt gccctaatgg tgaaagaaaa agtggggagt gccttggcac 9360
   tgttcatttg tggtgtgaac caaagagggg ggcatgcact tacacttcaa acatcctttt 9420
   gaaagactga caagtttggg tcttcacagt tggaattggg catccctttt gtcagggagg 9480
   gagggaggga gggaggctgg cttgttatgc tgacaagtgt gattaaattc aaactttgag 9540
   gtaagttgga ggaacttgta cattgttagg agtgtgacaa tttggactct taatgatttg 9600
   gtcatacaaa atgaacctag accaacttct ggaagatgta tataataact ccatgttaca 9660
   ttgatttcac ctgactaata cttatccctt atcaattaaa tacagaagat gccagccatc 9720
   tgggcctttt aacccagaaa tttagtttca aactcctagg ttagtgttct cactgagcta 9780
   catcctgatc tagtcctgaa aataggacca ccatcacccc caaaaaaatc tcaaataaga 9840
   tttatgctag tgtttcaaaa ttttaggaat aggtaagatt agaaagtttt aaattttgag 9900
   aaatggcttc tctagaaaga tgtacatagt gaacactgaa tggctcctaa agagcctaga 9960
   aaactggtac tgagcacaca ggactgagag gtctttcttg aaaagcatgt attgctttac 10020
   gtgggtcaca gaaggcaggc aggaagaact tgggctgaaa ctggtgtctt aagtggctaa 10080
   catcttcaca actgatgagc aagaacttta tcctgatgca aaaaccatcc aaacaaacta 10140
   agtgaaaggt ggcaatggat cccaggctgc tctagaggag gacttgactt ctcatcccat 10200
   cacccacacc agatagctca tagactgcca attaacacca gcttctagcc tccacaggca 10260
   cctgcactgg tacacataat ttcacacaaa cacagtaaga agccttccac ctggcatggt 10320
   attgcttatc tttagttccc aacacttggg aggcagaggc cagccagggc tatgtgacaa 10380
   aaaccttgtc tagaggagaa acttcatagc ttatttccta ttcacgtaac caggttagca 10440
   aaatttacca gccagagatg aagctaacag tgtccactat atttgtagtg ttttaagtca 10500
   attttttaaa tatacttaat agaattaaag ctatggtgaa ccaagtacaa acctggtgta 10560
   ttaacttgag aacttagcat aaaaagtagt tcatttgttc agtaaatatt aaatgcttac 10620
   tggcaaagat tatgtcagga acttggtaaa tggtgatgaa acaatcatag ttgtacatct 10680
   tggttctgtg atcaccttgg tttgaggtaa aagtggttcc tttgatcaag gatggaattt 10740
   taagtttata ttcaatcaat aatgtattat tttgtgattg caaaattgcc tatctagggt 10800
   ataaaacctt taaaaatttc ataataccag ttcattctcc agttactaat tccaaaaagc 10860
   cactgactat ggtgccaatg tggattctgt tctcaaagga aggattgtct gtgcccttta 10920
   ttctaataga aacatcacac tgaaaatcta agctgaaaga agccagactt tcctaaataa 10980
   ataactttcc ataaagctca aacaaggatt acttttagga ggcactgtta aggaactgat 11040
   aagtaatgag gttacttata taatgatagt cccacaagac tatctgagga aaaatcagta 11100
   caactcgaaa acagaacaac cagctaggca ggaataacag ggctcccaag tcaggaggtc 11160
   tatccaacac ccttttctgt tgagggcccc agacctacat attgtataca aacagggagg 11220
   tgggtgattt taactctcct gaggtacctt ggtaaatctt tgtcctgagt aagcagtaca 11280
   gtgtacagtt tacattttca tttaaagata cattagctcc ctctaccccc taagactgac 11340
   aggcactttg ggggtgggga gggctttgga aaataacgct tccatacact aaaagagaaa 11400
   tttctttaat taggcttgtt ggttccatac atctactggt gtttctacta cttagtaata 11460
   ttataatagt cacacaagca tctttgctct gtttaggttg tatatttatt ttaaggcaga 11520
   tgataaaact gtagatctta agggatgctt ctgcttctga gatgatacaa agaatttaga 11580
   ccataaaaca gtaggttgca caagcaatag aatatggcct aaagtgttct gacacttaga 11640
   agccaagcag tgtaggcttc ttaagaaata ccattacaat caccttgcta gaaatcaagc 11700
   attctggagt ggtcaagcag tgtaacctgt actgtaagtt acttttctgc tatttttctc 11760
   ccaaagcaag ttctttatgc tgatatttcc agtgttagga actacaaata ttaataagtt 11820
   gtcttcactc ttttctttac caaggagggt ctcttccttc atcttgatct gaaggatgaa 11880
   caaaggcttg agcagtgcgc tttagaagat aaactgcagc atgaaggccc ccgatgttca 11940
   cccagactac atggaccttt cgccacacat gtcccattcc agataaggcc tggcacacac 12000
   aaaaaacata agtcattagg ctaccagtct gattctaaaa caacctaaaa tcttcccact 12060
   taaatgctat gggtggtggg ttggaaagtt gactcagaaa atcacttgct gtttttagag 12120
   aggatctggg ttcagtttct gatacattgt ggcttacaac tataactcca gttctagggg 12180
   gtccatccaa catcctcttc tgttgagggc accaaataaa tgtattgtgt acaaacaggg 12240
   aggtgagtga tttaactctc gtgtatagta ccttggtaaa acatttcttg tcctgagtaa 12300
   gcagtacagc tctgcctgtc cctggtctac agacacggct catttcccga aggcaagctg 12360
   gatagagatt ccaatttctc ttcttggatc ccatcctata aaagaaggtc aagtttaatc 12420
   tattgcaaaa ggtaaatagg tagtttctta catgagacaa gaacaaatct taggtgtgaa 12480
   gcagtcatct tttacaggcc agagcctcta ttctatgcca atgaaggaaa ctgttagtcc 12540
   agtgttatag agttagtcca gtgtatagtt ttctatcaga acactttttt tttaaacaac 12600
   tgcaacttag cttattgaag acaaaccacg agtagaaatc tgtccaagaa gcaagtgctt 12660
   ctcagcctac aatgtggaat aggaccatgt aatggtacag tgagtgaaat gaattatggc 12720
   atgtttttct gactgagaag acagtacaat aaaaggtaaa ctcatggtat ttatttaaaa 12780
   agaatccaat ttctaccttt ttccaaatgg catatctgtt acaataatat ccacagaagc 12840
   agttctcagt gggaggttgc agatatccca ctgaacagca tcaatgggca aaccccaggt 12900
   tgtttttctg tggagacaaa ggtaagatat ttcaatatat tttcccaagc taatgagatg 12960
   gctcagcaaa taatggtact ggccattaag tctcatgacc tgagcttgat cctcagggac 13020
   catgtggtac aaggagagac ctaaatcctt cagttggact tcaatcttct accctcatgt 13080
   ccacacacaa ataaatacaa taaaaaacat tctgcagtcg aatttctaaa agggcgaat  13139
<210> 4
   <211> 1073
   <212> DNA
   <213> Mus musculus
<220>
   <223> Description: 5' arm for Rosa26
<400> 4
   caggccctcc gagcgtggtg gagccgttct gtgagacagc cgggtacgag tcgtgacgct 60
   ggaaggggca agcgggtggt gggcaggaat gcggtccgcc ctgcagcaac cggaggggga 120
   gggagaaggg agcggaaaag tctccaccgg acgcggccat ggctcggggg ggggggggca 180
   gcggaggagc gcttccggcc gacgtctcgt cgctgattgg cttcttttcc tcccgccgtg 240
   tgtgaaaaca caaatggcgt gttttggttg gcgtaaggcg cctgtcagtt aacggcagcc 300
   ggagtgcgca gccgccggca gcctcgctct gcccactggg tggggcggga ggtaggtggg 360
   gtgaggcgag ctggacgtgc gggcgcggtc ggcctctggc ggggcggggg aggggaggga 420
   gggtcagcga aagtagctcg cgcgcgagcg gccgcccacc ctccccttcc tctgggggag 480
   tcgttttacc cgccgccggc cgggcctcgt cgtctgattg gctctcgggg cccagaaaac 540
   tggcccttgc cattggctcg tgttcgtgca agttgagtcc atccgccggc cagcgggggc 600
   ggcgaggagg cgctcccagg ttccggccct cccctcggcc ccgcgccgca gagtctggcc 660
   gcgcgcccct gcgcaacgtg gcaggaagcg cgcgctgggg gcggggacgg gcagtagggc 720
   tgagcggctg cggggcgggt gcaagcacgt ttccgacttg agttgcctca agaggggcgt 780
   gctgagccag acctccatcg cgcactccgg ggagtggagg gaaggagcga gggctcagtt 840
   gggctgtttt ggaggcagga agcacttgct ctcccaaagt cgctctgagt tgttatcagt 900
   aagggagctg cagtggagta ggcggggaga aggccgcacc cttctccgga ggggggaggg  960
   gagtgttgca atacctttct gggagttctc tgctgcctcc tggcttctga ggaccgccct 1020
   gggcctggga gaatcccttc cccctcttcc ctcgtgatct gcaactccag tct        1073
<210> 5
   <211> 4333
   <212> DNA
   <213> Mus musculus
<220>
   <223> Description: 3' arm for Rosa26
<400> 5
   tagaagatgg gcgggagtct tctgggcagg cttaaaggct aacctggtgt gtgggcgttg 60
   tcctgcaggg gaattgaaca ggtgtaaaat tggagggaca agacttccca cagattttcg 120
   gttttgtcgg gaagtttttt aatagggcca aataaggaaa atgggaggat aggtagtcat 180
   ctggggtttt atgcagcaaa actacaggtt attattgctt gtgatccgcc tcggagtatt 240
   ttccatcgag gtagattaaa gacatgctca cccgagtttt atactctcct gcttgagatc 300
   cttactacag tatgaaatta cagtgtcgcg agttagacta tgtaagcaga attttaatca 360
   tttttaaaga gcccagtact tcatatccat ttctcccgct ccttctgcag ccttatcaaa 420
   aggtatttta gaacactcat tttagcccca ttttcattta ttatactggc ttatccaacc 480
   cctagacaga gcattggcat tttccctttc ctgatcttag aagtctgatg actcatgaaa 540
   ccagacagat tagttacata caccacaaat cgaggctgta gctggggcct caacactgca 600
   gttcttttat aactccttag tacacttttt gttgatcctt tgccttgatc cttaattttc 660
   agtgtctatc acctctcccg tcagtggtgt tccacatttg ggcctattct cagtccaggg 720
   agttttacaa caatagatgt attgagaatc caacctaaag cttaactttc cactcccatg 780
   aatgcctctc tccttttctt ccatttataa actgagctat taaccattaa tggttccagg 840
   tggatgtctc ctccccatat tacctgatgt atcttacata ttgccaggct gatattttaa 900
   gacattaaaa ggtatatttc attattgagc cacatggtat tgattactgc ttactaaaat 960
   tttgtcattg tacacatctg taaaaggtgg ttccttttgg aatgcaaagt tcaggtgttt 1020
   gttgtctttc ctgacctaag gtcttgtgag cttgtatttt ttctatttaa gcagtgcttt 1080
   ctcttggact ggcttgactc atggcattct acacgttatt gctggtctaa atgtgatttt 1140
   gccaagcttc ttcaggacct ataattttgc ttgacttgta gccaaacaca agtaaaatga 1200
   ttaagcaaca aatgtatttg tgaagcttgg tttttaggtt gttgtgttgt gtgtgcttgt 1260
   gctctataat aatactatcc aggggctgga gaggtggctc ggagttcaag agcacagact 1320
   gctcttccag aagtcctgag ttcaattccc agcaaccaca tggtggctca caaccatctg 1380
   taatgggatc tgatgccctc ttctggtgtg tctgaagacc acaagtgtat tcacattaaa 1440
   taaataaatc ctccttcttc ttcttttttt tttttttaaa gagaatactg tctccagtag 1500
   aatttactga agtaatgaaa tactttgtgt ttgttccaat atggtagcca ataatcaaat 1560
   tactctttaa gcactggaaa tgttaccaag gaactaattt ttatttgaag tgtaactgtg 1620
   gacagaggag ccataactgc agacttgtgg gatacagaag accaatgcag actttaatgt 1680
   cttttctctt acactaagca ataaagaaat aaaaattgaa cttctagtat cctatttgtt 1740
   taaactgcta gctttactta acttttgtgc ttcatctata caaagctgaa agctaagtct 1800
   gcagccatta ctaaacatga aagcaagtaa tgataatttt ggatttcaaa aatgtagggc 1860
   cagagtttag ccagccagtg gtggtgcttg cctttatgcc tttaatccca gcactctgga 1920
   ggcagagaca ggcagatctc tgagtttgag cccagcctgg tctacacatc aagttctatc 1980
   taggatagcc aggaatacac acagaaaccc tgttggggag gggggctctg agatttcata 2040
   aaattataat tgaagcattc cctaatgagc cactatggat gtggctaaat ccgtctacct 2100
   ttctgatgag atttgggtat tattttttct gtctctgctg ttggttgggt cttttgacac 2160
   tgtgggcttt ctttaaagcc tccttcctgc catgtggtct cttgtttgct actaacttcc 2220
   catggcttaa atggcatggc tttttgcctt ctaagggcag ctgctgagat ttgcagcctg 2280
   atttccaggg tggggttggg aaatctttca aacactaaaa ttgtccttta attttttttt 2340
   taaaaaatgg gttatataat aaacctcata aaatagttat gaggagtgag gtggactaat 2400
   attaaatgag tccctcccct ataaaagagc tattaaggct ttttgtctta tacttaactt 2460
   tttttttaaa tgtggtatct ttagaaccaa gggtcttaga gttttagtat acagaaactg 2520
   ttgcatcgct taatcagatt ttctagtttc aaatccagag aatccaaatt cttcacagcc 2580
   aaagtcaaat taagaatttc tgacttttaa tgttaatttg cttactgtga atataaaaat 2640
   gatagctttt cctgaggcag ggtctcacta tgtatctctg cctgatctgc aacaagatat 2700
   gtagactaaa gttctgcctg cttttgtctc ctgaatacta aggttaaaat gtagtaatac 2760
   ttttggaact tgcaggtcag attcttttat aggggacaca ctaagggagc ttgggtgata 2820
   gttggtaaaa tgtgtttcaa gtgatgaaaa cttgaattat tatcaccgca acctactttt 2880
   taaaaaaaaa agccaggcct gttagagcat gcttaaggga tccctaggac ttgctgagca 2940
   cacaagagta gttacttggc aggctcctgg tgagagcata tttcaaaaaa caaggcagac 3000
   aaccaagaaa ctacagttaa ggttacctgt ctttaaacca tctgcatata cacagggata 3060
   ttaaaatatt ccaaataata tttcattcaa gttttccccc atcaaattgg gacatggatt 3120
   tctccggtga ataggcagag ttggaaacta aacaaatgtt ggttttgtga tttgtgaaat 3180
   tgttttcaag tgatagttaa agcccatgag atacagaaca aagctgctat ttcgaggtct 3240
   cttggtttat actcagaagc acttctttgg gtttccctgc actatcctga tcatgtgcta 3300
   ggcctacctt aggctgattg ttgttcaaat aaacttaagt ttcctgtcag gtgatgtcat 3360
   atgatttcat atatcaaggc aaaacatgtt atatatgtta aacatttgta cttaatgtga 3420
   aagttaggtc tttgtgggtt tgatttttaa ttttcaaaac ctgagctaaa taagtcattt 3480
   ttacatgtct tacatttggt ggaattgtat aattgtggtt tgcaggcaag actctctgac 3540
   ctagtaaccc tacctataga gcactttgct gggtcacaag tctaggagtc aagcatttca 3600
   ccttgaagtt gagacgtttt gttagtgtat actagtttat atgttggagg acatgtttat 3660
   ccagaagata ttcaggacta tttttgactg ggctaaggaa ttgattctga ttagcactgt 3720
   tagtgagcat tgagtggcct ttaggcttga attggagtca cttgtatatc tcaaataatg 3780
   ctggcctttt ttaaaaagcc cttgttcttt atcaccctgt tttctacata atttttgttc 3840
   aaagaaatac ttgtttggat ctccttttga caacaatagc atgttttcaa gccatatttt 3900
   ttttcctttt tttttttttt tttggttttt cgagacaggg tttctctgta tagccctggc 3960
   tgtcctggaa ctcactttgt agaccaggct ggcctcgaac tcagaaatcc gcctgcctct 4020
   gcctcctgag tgccgggatt aaaggcgtgc accaccacgc ctggctaagt tggatatttt 4080
   gttatataac tataaccaat actaactcca ctgggtggat ttttaattca gtcagtagtc 4140
   ttaagtggtc tttattggcc cttcattaaa atctactgtt cactctaaca gaggctgttg 4200
   gtactagtgg cacttaagca acttcctacg gatatactag cagattaagg gtcagggata 4260
   gaaactagtc tagcgttttg tatacctacc agctttatac taccttgttc tgatagaaat 4320
   atttcaggac atc                                                    4333
<210> 6
   <211> 6039
   <212> DNA
   <213> Artificial Sequence
<220>
   <223> Description of Artificial Sequence: Insert CAGGAS-creER
<400> 6
   attgattatt gactagttat taatagtaat caattacggg gtcattagtt catagcccat 60
   atatggagtt ccgcgttaca taacttacgg taaatggccc gcctggctga ccgcccaacg 120
   acccccgccc attgacgtca ataatgacgt atgttcccat agtaacgcca atagggactt 180
   tccattgacg tcaatgggtg gactatttac ggtaaactgc ccacttggca gtacatcaag 240
   tgtatcatat gccaagtacg ccccctattg acgtcaatga cggtaaatgg cccgcctggc 300
   attatgccca gtacatgacc ttatgggact ttcctacttg gcagtacatc tacgtattag 360
   tcatcgctat taccatgggt cgaggtgagc cccacgttct gcttcactct ccccatctcc 420
   cccccctccc cacccccaat tttgtattta tttatttttt aattattttg tgcagcgatg 480
   ggggcggggg gggggggggc gcgcgccagg cggggcgggg cggggcgagg ggcggggcgg 540
   ggcgaggcgg agaggtgcgg cggcagccaa tcagagcggc gcgctccgaa agtttccttt 600
   tatggcgagg cggcggcggc ggcggcccta taaaaagcga agcgcgcggc gggcgggagt 660
   cgctgcgttg ccttcgcccc gtgccccgct ccgcgccgcc tcgcgccgcc cgccccggct 720
   ctgactgacc gcgttactcc cacaggtgag cgggcgggac ggcccttctc ctccgggctg 780
   taattagcgc ttggtttaat gacggctcgt ttcttttctg tggctgcgtg aaagccttaa 840
   agggctccgg gagggccctt tgtgcggggg ggagcggctc ggggggtgcg tgcgtgtgtg 900
   tgtgcgtggg gagcgccgcg tgcggcccgc gctgcccggc ggctgtgagc gctgcgggcg 960
   cggcgcgggg ctttgtgcgc tccgcgtgtg cgcgagggga gcgcggccgg gggcggtgcc 1020
   ccgcggtgcg gggggggtgc gaggggaaca aaggctgcgt gcggggtgtg tgcgtggggg 1080
   ggtgagcagg gggtgtgggc gcggcggtcg ggctgtaacc cccccctgca cccccctccc 1140
   cgagttgctg agcacggccc ggcttcgggt gcggggctcc gtgcggggcg tggcgcgggg 1200
   ctcgccgtgc cgggcggggg gtggcggcag gtgggggtgc cgggcggggc ggggccgcct 1260
   cgggccgggg agggctcggg ggaggggcgc ggcggccccg gagcgccggc ggctgtcgag 1320
   gcgcggcgag ccgcagccat tgccttttat ggtaatcgtg cgagagggcg cagggacttc 1380
   ctttgtccca aatctggcgg agccgaaatc tgggaggcgc cgccgcaccc cctctagcgg 1440
   gcgcgggcga agcggtgcgg cgccggcagg aaggaaatgg gcggggaggg ccttcgtgcg 1500
   tcgccgcgcc gccgtcccct tctccatctc cagcctcggg gctgccgcag ggggacggct 1560
   gccttcgggg gggacggggc agggcggggt tcggcttctg gcgtgtgacc ggcggctcta 1620
   gaagcgttgg ggtgagtact ccctctcaaa agcgggcatg acttctgcgc taagattgtc 1680
   agtttccaaa aacgaggagg atttgatatt cacctggccc gcggtgatgc ctttgagggt 1740
   ggccgcgtcc atctggtcag aaaagacaat ctttttgttg tcaagcttga ggtgtggcag 1800
   gcttgagatc tggccataca cttgagtgac attgacatcc actttgcctt tctctccaca 1860
   ggtgtccact cccagggcgg cctccggagc gatcgccggt ccgcctaggc tagccggccg 1920
   cgtcgaccat gtccaattta ctgaccgtac accaaaattt gcctgcatta ccggtcgatg 1980
   caacgagtga tgaggttcgc aagaacctga tggacatgtt cagggatcgc caggcgtttt 2040
   ctgagcatac ctggaaaatg cttctgtccg tttgccggtc gtgggcggca tggtgcaagt 2100
   tgaataaccg gaaatggttt cccgcagaac ctgaagatgt tcgcgattat cttctatatc 2160
   ttcaggcgcg cggtctggca gtaaaaacta tccagcaaca tttgggccag ctaaacatgc 2220
   ttcatcgtcg gtccgggctg ccacgaccaa gtgacagcaa tgctgtttca ctggttatgc 2280
   ggcggatccg aaaagaaaac gttgatgccg gtgaacgtgc aaaacaggct ctagcgttcg 2340
   aacgcactga tttcgaccag gttcgttcac tcatggaaaa tagcgatcgc tgccaggata 2400
   tacgtaatct ggcatttctg gggattgctt ataacaccct gttacgtata gccgaaattg 2460
   ccaggatcag ggttaaagat atctcacgta ctgacggtgg gagaatgtta atccatattg 2520
   gcagaacgaa aacgctggtt agcaccgcag gtgtagagaa ggcacttagc ctgggggtaa 2580
   ctaaactggt cgagcgatgg atttccgtct ctggtgtagc tgatgatccg aataactacc 2640
   tgttttgccg ggtcagaaaa aatggtgttg ccgcgccatc tgccaccagc cagctatcaa 2700
   ctcgcgccct ggaagggatt tttgaagcaa ctcatcgatt gatttacggc gctaaggatg 2760
   actctggtca gagatacctg gcctggtctg gacacagtgc ccgtgtcgga gccgcgcgag 2820
   atatggcccg cgctggagtt tcaataccgg agatcatgca agctggtggc tggaccaatg 2880
   taaatattgt catgaactat atccgtaacc tggatagtga aacaggggca atggtgcgcc 2940
   tgctggaaga tggcgattct gctggagaca tgagagctgc caacctttgg ccaagcccgc 3000
   tcatgatcaa acgctctaag aagaacagcc tggccttgtc cctgacggcc gaccagatgg 3060
   tcagtgcctt gttggatgct gagcccccca tactctattc cgagtatgat cctaccagac 3120
   ccttcagtga agcttcgatg atgggcttac tgaccaacct ggcagacagg gagctggttc 3180
   acatgatcaa ctgggcgaag agggtgccag gctttgtgga tttgaccctc catgatcagg 3240
   tccaccttct agaatgtgcc tggctagaga tcctgatgat tggtctcgtc tggcgctcca 3300
   tggagcaccc agtgaagcta ctgtttgctc ctaacttgct cttggacagg aaccagggaa 3360
   aatgtgtaga gggcatggtg gagatcttcg acatgctgct ggctacatca tctcggttcc 3420
   gcatgatgaa tctgcaggga gaggagtttg tgtgcctcaa atctattatt ttgcttaatt 3480
   ctggagtgta cacatttctg tccagcaccc tgaagtctct ggaagagaag gaccatatcc 3540
   accgagtcct ggacaagatc acagacactt tgatccacct gatggccaag gcaggcctga 3600
   ccctgcagca gcagcaccag cggctggccc agctcctcct catcctctcc cacatcaggc 3660
   acatgagtaa caaaggcatg gagcatctgt acagcatgaa gtgcaagaac gtggtgcccc 3720
   tctatgacct gctgctggag gcggccgacg cccaccgcct acatgcgccc actagccgtg 3780
   gaggggcatc cgtggaggag acggaccaaa gccacttggc cactgcgggc tctacttcat 3840
   cgcattcctt gcaaaagtat tacatcacgg gggaggcaga gggtttccct gccacagtct 3900
   gagcggccga ccggttcgag atccaggcgc ggatcaataa aagatcatta ttttcaatag 3960
   atctgtgtgt tggttttttg tgtgccttgg gggaggggga ggccagaatg aggcgcggcc 4020
   aagggggagg gggaggccag aatgaccttg gggggagggg aggccagaat gaccttgggg 4080
   gagggggagg ccagaatgag gcgcgccggt aaccgaagtt cctatacttt ctagagaata 4140
   ggaacttcgg aataggaact tcttaggtca attctaccgg gtaggggagg cgcttttccc 4200
   aaggcagtct ggagcatgcg ctttagcagc cccgctgggc acttggcgct acacaagtgg 4260
   cctctggcct cgcacacatt ccacatccac cggtaggcgc caaccggctc cgttctttgg 4320
   tggccccttc gcgccacctt ctactcctcc cctagtcagg aagttccccc ccgccccgca 4380
   gctcgcgtcg tgcaggacgt gacaaatgga agtagcacgt ctcactagtc tcgtgcagat 4440
   ggacagcacc gctgagcaat ggaagcgggt aggcctttgg ggcagcggcc aatagcagct 4500
   ttgctccttc gctttctggg ctcagaggct gggaaggggt gggtccgggg gcgggctcag 4560
   gggcgggctc aggggcgggg cgggcgcccg aaggtcctcc ggaggcccgg cattctgcac 4620
   gcttcaaaag cgcacgtctg ccgcgctgtt ctcctcttcc tcatctccgg gcctttcgac 4680
   ctgcagccaa tatgggatcg gccattgaac aagatggatt gcacgcaggt tctccggccg 4740
   cttgggtgga gaggctattc ggctatgact gggcacaaca gacaatcggc tgctctgatg 4800
   ccgccgtgtt ccggctgtca gcgcaggggc gcccggttct ttttgtcaag accgacctgt 4860
   ccggtgccct gaatgaactg caggacgagg cagcgcggct atcgtggctg gccacgacgg 4920
   gcgttccttg cgcagctgtg ctcgacgttg tcactgaagc gggaagggac tggctgctat 4980
   tgggcgaagt gccggggcag gatctcctgt catctcacct tgctcctgcc gagaaagtat 5040
   ccatcatggc tgatgcaatg cggcggctgc atacgcttga tccggctacc tgcccattcg 5100
   accaccaagc gaaacatcgc atcgagcgag cacgtactcg gatggaagcc ggtcttgtcg 5160
   atcaggatga tctggacgaa gagcatcagg ggctcgcgcc agccgaactg ttcgccaggc 5220
   tcaaggcgcg catgcccgac ggcgaggatc tcgtcgtgac ccatggcgat gcctgcttgc 5280
   cgaatatcat ggtggaaaat ggccgctttt ctggattcat cgactgtggc cggctgggtg 5340
   tggcggaccg ctatcaggac atagcgttgg ctacccgtga tattgctgaa gagcttggcg 5400
   gcgaatgggc tgaccgcttc ctcgtgcttt acggtatcgc cgctcccgat tcgcagcgca 5460
   tcgccttcta tcgccttctt gacgagttct tctgagggga tcgatccgct gtaagtctgc 5520
   agaaattgat gatctattaa acaataaaga tgtccactaa aatggaagtt tttcctgtca 5580
   tactttgtta agaagggtga gaacagagta cctacatttt gaatggaagg attggagcta 5640
   cgggggtggg ggtggggtgg gattagataa atgcctgctc tttactgaag gctctttact 5700
   attgctttat gataatgttt catagttgga tatcataatt taaacaagca aaaccaaatt 5760
   aagggccagc tcattcctcc cactcatgat ctatagatct atagatctct cgtgggatca 5820
   ttgtttttct cttgattccc actttgtggt tctaagtact gtggtttcca aatgtgtcag 5880
   tttcatagcc tgaagaacga gatcagcagc ctctgttcca catacacttc attctcagta 5940
   ttgttttgcc aagttctaat tccatcagaa gctgactcta gatcccgcgc cgaagttcct 6000
   atactttcta gagaatagga acttcggaat aggaacttc                        6039
<210> 7
   <211> 14411
   <212> DNA
   <213> Artificial Sequence
<220>
<223> Description of Artificial Sequence: targeting vector for Rosa26 locus with a CAGGS-creER insert
<400> 7
   tttgagtgag ctgataccgc tcgccgcagc cgaacgaccg agcgcagcga gtcagtgagc 60
   gaggaagcgg aagagcgccc aatacgcaaa ccgcctctcc ccgcgcgttg gccgattcat 120
   taatgcagct ggcacgacag gtttcccgac tggaaagcgg gcagtgagcg caacgcaatt 180
   aatgtgagtt agctcactca ttaggcaccc caggctttac actttatgct tccggctcgt 240
   atgttgtgtg gaattgtgag cggataacaa tttcacacag gaaacagcta tgaccatgat 300
   tacgccaagc gcgcaattaa ccctcactaa agggaacaaa agctgtcgag atctagatat 360
   cgatggccat agagttacgc tagggataac agggtaatat agccgcggca ggccctccga 420
   gcgtggtgga gccgttctgt gagacagccg ggtacgagtc gtgacgctgg aaggggcaag 480
   cgggtggtgg gcaggaatgc ggtccgccct gcagcaaccg gagggggagg gagaagggag 540
   cggaaaagtc tccaccggac gcggccatgg ctcggggggg ggggggcagc ggaggagcgc 600
   ttccggccga cgtctcgtcg ctgattggct tcttttcctc ccgccgtgtg tgaaaacaca 660
   aatggcgtgt tttggttggc gtaaggcgcc tgtcagttaa cggcagccgg agtgcgcagc 720
   cgccggcagc ctcgctctgc ccactgggtg gggcgggagg taggtggggt gaggcgagct 780
   ggacgtgcgg gcgcggtcgg cctctggcgg ggcgggggag gggagggagg gtcagcgaaa 840
   gtagctcgcg cgcgagcggc cgcccaccct ccccttcctc tgggggagtc gttttacccg 900
   ccgccggccg ggcctcgtcg tctgattggc tctcggggcc cagaaaactg gcccttgcca 960
   ttggctcgtg ttcgtgcaag ttgagtccat ccgccggcca gcgggggcgg cgaggaggcg 1020
   ctcccaggtt ccggccctcc cctcggcccc gcgccgcaga gtctggccgc gcgcccctgc 1080
   gcaacgtggc aggaagcgcg cgctgggggc ggggacgggc agtagggctg agcggctgcg 1140
   gggcgggtgc aagcacgttt ccgacttgag ttgcctcaag aggggcgtgc tgagccagac 1200
   ctccatcgcg cactccgggg agtggaggga aggagcgagg gctcagttgg gctgttttgg 1260
   aggcaggaag cacttgctct cccaaagtcg ctctgagttg ttatcagtaa gggagctgca 1320
   gtggagtagg cggggagaag gccgcaccct tctccggagg ggggagggga gtgttgcaat 1380
   acctttctgg gagttctctg ctgcctcctg gcttctgagg accgccctgg gcctgggaga 1440
   atcccttccc cctcttccct cgtgatctgc aactccagtc tttctaggta accgatatcc 1500
   ctgcaggttt tcgacattga ttattgacta gttattaata gtaatcaatt acggggtcat 1560
   tagttcatag cccatatatg gagttccgcg ttacataact tacggtaaat ggcccgcctg 1620
   gctgaccgcc caacgacccc cgcccattga cgtcaataat gacgtatgtt cccatagtaa 1680
   cgccaatagg gactttccat tgacgtcaat gggtggacta tttacggtaa actgcccact 1740
   tggcagtaca tcaagtgtat catatgccaa gtacgccccc tattgacgtc aatgacggta 1800
   aatggcccgc ctggcattat gcccagtaca tgaccttatg ggactttcct acttggcagt 1860
   acatctacgt attagtcatc gctattacca tgggtcgagg tgagccccac gttctgcttc 1920
   actctcccca tctccccccc ctccccaccc ccaattttgt atttatttat tttttaatta 1980
   ttttgtgcag cgatgggggc gggggggggg ggggcgcgcg ccaggcgggg cggggcgggg 2040
   cgaggggcgg ggcggggcga ggcggagagg tgcggcggca gccaatcaga gcggcgcgct 2100
   ccgaaagttt ccttttatgg cgaggcggcg gcggcggcgg ccctataaaa agcgaagcgc 2160
   gcggcgggcg ggagtcgctg cgttgccttc gccccgtgcc ccgctccgcg ccgcctcgcg 2220
   ccgcccgccc cggctctgac tgaccgcgtt actcccacag gtgagcgggc gggacggccc 2280
   ttctcctccg ggctgtaatt agcgcttggt ttaatgacgg ctcgtttctt ttctgtggct 2340
   gcgtgaaagc cttaaagggc tccgggaggg ccctttgtgc gggggggagc ggctcggggg 2400
   gtgcgtgcgt gtgtgtgtgc gtggggagcg ccgcgtgcgg cccgcgctgc ccggcggctg 2460
   tgagcgctgc gggcgcggcg cggggctttg tgcgctccgc gtgtgcgcga ggggagcgcg 2520
   gccgggggcg gtgccccgcg gtgcgggggg gctgcgaggg gaacaaaggc tgcgtgcggg 2580
   gtgtgtgcgt gggggggtga gcagggggtg tgggcgcggc ggtcgggctg taaccccccc 2640
   ctgcaccccc ctccccgagt tgctgagcac ggcccggctt cgggtgcggg gctccgtgcg 2700
   gggcgtggcg cggggctcgc cgtgccgggc ggggggtggc ggcaggtggg ggtgccgggc 2760
   ggggcggggc cgcctcgggc cggggagggc tcgggggagg ggcgcggcgg ccccggagcg 2820
   ccggcggctg tcgaggcgcg gcgagccgca gccattgcct tttatggtaa tcgtgcgaga 2880
   gggcgcaggg acttcctttg tcccaaatct ggcggagccg aaatctggga ggcgccgccg 2940
   caccccctct agcgggcgcg ggcgaagcgg tgcggcgccg gcaggaagga aatgggcggg 3000
   gagggccttc gtgcgtcgcc gcgccgccgt ccccttctcc atctccagcc tcggggctgc 3060
   cgcaggggga cggctgcctt cgggggggac ggggcagggc ggggttcggc ttctggcgtg 3120
   tgaccggcgg ctctagaagc gttggggtga gtactccctc tcaaaagcgg gcatgacttc 3180
   tgcgctaaga ttgtcagttt ccaaaaacga ggaggatttg atattcacct ggcccgcggt 3240
   gatgcctttg agggtggccg cgtccatctg gtcagaaaag acaatctttt tgttgtcaag 3300
   cttgaggtgt ggcaggcttg agatctggcc atacacttga gtgacattga catccacttt 3360
   gcctttctct ccacaggtgt ccactcccag ggcggcctcc ggagcgatcg ccggtccgcc 3420
   taggctagcc ggccgcgtcg accatgtcca atttactgac cgtacaccaa aatttgcctg 3480
   cattaccggt cgatgcaacg agtgatgagg ttcgcaagaa cctgatggac atgttcaggg 3540
   atcgccaggc gttttctgag catacctgga aaatgcttct gtccgtttgc cggtcgtggg 3600
   cggcatggtg caagttgaat aaccggaaat ggtttcccgc agaacctgaa gatgttcgcg 3660
   attatcttct atatcttcag gcgcgcggtc tggcagtaaa aactatccag caacatttgg 3720
   gccagctaaa catgcttcat cgtcggtccg ggctgccacg accaagtgac agcaatgctg 3780
   tttcactggt tatgcggcgg atccgaaaag aaaacgttga tgccggtgaa cgtgcaaaac 3840
   aggctctagc gttcgaacgc actgatttcg accaggttcg ttcactcatg gaaaatagcg 3900
   atcgctgcca ggatatacgt aatctggcat ttctggggat tgcttataac accctgttac 3960
   gtatagccga aattgccagg atcagggtta aagatatctc acgtactgac ggtgggagaa 4020
   tgttaatcca tattggcaga acgaaaacgc tggttagcac cgcaggtgta gagaaggcac 4080
   ttagcctggg ggtaactaaa ctggtcgagc gatggatttc cgtctctggt gtagctgatg 4140
   atccgaataa ctacctgttt tgccgggtca gaaaaaatgg tgttgccgcg ccatctgcca 4200
   ccagccagct atcaactcgc gccctggaag ggatttttga agcaactcat cgattgattt 4260
   acggcgctaa ggatgactct ggtcagagat acctggcctg gtctggacac agtgcccgtg 4320
   tcggagccgc gcgagatatg gcccgcgctg gagtttcaat accggagatc atgcaagctg 4380
   gtggctggac caatgtaaat attgtcatga actatatccg taacctggat agtgaaacag 4440
   gggcaatggt gcgcctgctg gaagatggcg attctgctgg agacatgaga gctgccaacc 4500
   tttggccaag cccgctcatg atcaaacgct ctaagaagaa cagcctggcc ttgtccctga 4560
   cggccgacca gatggtcagt gccttgttgg atgctgagcc ccccatactc tattccgagt 4620
   atgatcctac cagacccttc agtgaagctt cgatgatggg cttactgacc aacctggcag 4680
   acagggagct ggttcacatg atcaactggg cgaagagggt gccaggcttt gtggatttga 4740
   ccctccatga tcaggtccac cttctagaat gtgcctggct agagatcctg atgattggtc 4800
   tcgtctggcg ctccatggag cacccagtga agctactgtt tgctcctaac ttgctcttgg 4860
   acaggaacca gggaaaatgt gtagagggca tggtggagat cttcgacatg ctgctggcta 4920
   catcatctcg gttccgcatg atgaatctgc agggagagga gtttgtgtgc ctcaaatcta 4980
   ttattttgct taattctgga gtgtacacat ttctgtccag caccctgaag tctctggaag 5040
   agaaggacca tatccaccga gtcctggaca agatcacaga cactttgatc cacctgatgg 5100
   ccaaggcagg cctgaccctg cagcagcagc accagcggct ggcccagctc ctcctcatcc 5160
   tctcccacat caggcacatg agtaacaaag gcatggagca tctgtacagc atgaagtgca 5220
   agaacgtggt gcccctctat gacctgctgc tggaggcggc cgacgcccac cgcctacatg 5280
   cgcccactag ccgtggaggg gcatccgtgg aggagacgga ccaaagccac ttggccactg 5340
   cgggctctac ttcatcgcat tccttgcaaa agtattacat cacgggggag gcagagggtt 5400
   tccctgccac agtctgagcg gccgaccggt tcgagatcca ggcgcggatc aataaaagat 5460
   cattattttc aatagatctg tgtgttggtt ttttgtgtgc cttgggggag gggaggccca 5520
   gaatgaggcg cggccaaggg ggagggggag gccagaatga ccttggggga gggggaggcc 5580
   agaatgacct tgggggaggg ggaggccaga atgaggcgcg ccggtaaccg aagttcctat 5640
   actttctaga gaataggaac ttcggaatag gaacttctta ggtcaattct accgggtagg 5700
   ggaggcgctt ttcccaaggc agtctggagc atgcgcttta gcagccccgc tgggcacttg 5760
   gcgctacaca agtggcctct ggcctcgcac acattccaca tccaccggta ggcgccaacc 5820
   ggctccgttc tttggtggcc ccttcgcgcc accttctact cctcccctag tcaggaagtt 5880
   cccccccgcc ccgcagctcg cgtcgtgcag gacgtgacaa atggaagtag cacgtctcac 5940
   tagtctcgtg cagatggaca gcaccgctga gcaatggaag cgggtaggcc tttggggcag 6000
   cggccaatag cagctttgct ccttcgcttt ctgggctcag aggctgggaa ggggtgggtc 6060
   cgggggcggg ctcaggggcg ggctcagggg cggggcgggc gcccgaaggt cctccggagg 6120
   cccggcattc tgcacgcttc aaaagcgcac gtctgccgcg ctgttctcct cttcctcatc 6180
   tccgggcctt tcgacctgca gccaatatgg gatcggccat tgaacaagat ggattgcacg 6240
   caggttctcc ggccgcttgg gtggagaggc tattcggcta tgactgggca caacagacaa 6300
   tcggctgctc tgatgccgcc gtgttccggc tgtcagcgca ggggcgcccg gttctttttg 6360
   tcaagaccga cctgtccggt gccctgaatg aactgcagga cgaggcagcg cggctatcgt 6420
   ggctggccac gacgggcgtt ccttgcgcag ctgtgctcga cgttgtcact gaagcgggaa 6480
   gggactggct gctattgggc gaagtgccgg ggcaggatct cctgtcatct caccttgctc 6540
   ctgccgagaa agtatccatc atggctgatg caatgcggcg gctgcatacg cttgatccgg 6600
   ctacctgccc attcgaccac caagcgaaac atcgcatcga gcgagcacgt actcggatgg 6660
   aagccggtct tgtcgatcag gatgatctgg acgaagagca tcaggggctc gcgccagccg 6720
   aactgttcgc caggctcaag gcgcgcatgc ccgacggcga ggatctcgtc gtgacccatg 6780
   gcgatgcctg cttgccgaat atcatggtgg aaaatggccg cttttctgga ttcatcgact 6840
   gtggccggct gggtgtggcg gaccgctatc aggacatagc gttggctacc cgtgatattg 6900
   ctgaagagct tggcggcgaa tgggctgacc gcttcctcgt gctttacggt atcgccgctc 6960
   ccgattcgca gcgcatcgcc ttctatcgcc ttcttgacga gttcttctga ggggatcgat 7020
   ccgctgtaag tctgcagaaa ttgatgatct attaaacaat aaagatgtcc actaaaatgg 7080
   aagtttttcc tgtcatactt tgttaagaag ggtgagaaca gagtacctac attttgaatg 7140
   gaaggattgg agctacgggg gtgggggtgg ggtgggatta gataaatgcc tgctctttac 7200
   tgaaggctct ttactattgc tttatgataa tgtttcatag ttggatatca taatttaaac 7260
   aagcaaaacc aaattaaggg ccagctcatt cctcccactc atgatctata gatctataga 7320
   tctctcgtgg gatcattgtt tttctcttga ttcccacttt gtggttctaa gtactgtggt 7380
   ttccaaatgt gtcagtttca tagcctgaag aacgagatca gcagcctctg ttccacatac 7440
   acttcattct cagtattgtt ttgccaagtt ctaattccat cagaagctga ctctagatcc 7500
   cgcgccgaag ttcctatact ttctagagaa taggaacttc ggaataggaa cttcaagctt 7560
   aagcgctaga agatgggcgg gagtcttctg ggcaggctta aaggctaacc tggtgtgtgg 7620
   gcgttgtcct gcaggggaat tgaacaggtg taaaattgga gggacaagac ttcccacaga 7680
   ttttcggttt tgtcgggaag ttttttaata ggggcaaata aggaaaatgg gaggataggt 7740
   agtcatctgg ggttttatgc agcaaaacta caggttatta ttgcttgtga tccgcctcgg 7800
   agtattttcc atcgaggtag attaaagaca tgctcacccg agttttatac tctcctgctt 7860
   gagatcctta ctacagtatg aaattacagt gtcgcgagtt agactatgta agcagaattt 7920
   taatcatttt taaagagccc agtacttcat atccatttct cccgctcctt ctgcagcctt 7980
   atcaaaaggt attttagaac actcatttta gccccatttt catttattat actggcttat 8040
   ccaaccccta gacagagcat tggcattttc cctttcctga tcttagaagt ctgatgactc 8100
   atgaaaccag acagattagt tacatacacc acaaatcgag gctgtagctg gggcctcaac 8160
   actgcagttc ttttataact ccttagtaca ctttttgttg atcctttgcc ttgatcctta 8220
   attttcagtg tctatcacct ctcccgtcag tggtgttcca catttgggcc tattctcagt 8280
   ccagggagtt ttacaacaat agatgtattg agaatccaac ctaaagctta actttccact 8340
   cccatgaatg cctctctcct ttttctccat ttataaactg agctattaac cattaatggt 8400
   tccaggtgga tgtctcctcc ccatattacc tgatgtatct tacatattgc caggctgata 8460
   ttttaagaca ttaaaaggta tatttcatta ttgagccaca tggtattgat tactgcttac 8520
   taaaattttg tcattgtaca catctgtaaa aggtggttcc ttttggaatg caaagttcag 8580
   gtgtttgttg tctttcctga cctaaggtct tgtgagcttg tattttttct atttaagcag 8640
   tgctttctct tggactggct tgactcatgg cattctacac gttattgctg gtctaaatgt 8700
   gattttgcca agcttcttca ggacctataa ttttgcttga cttgtagcca aacacaagta 8760
   aaatgattaa gcaacaaatg tatttgtgaa gcttggtttt taggttgttg tgttgtgtgt 8820
   gcttgtgctc tataataata ctatccaggg gctggagagg tggctcggag ttcaagagca 8880
   cagactgctc ttccagaagt cctgagttca attcccagca accacatggt ggctcacaac 8940
   catctgtaat gggatctgat gccctcttct ggtgtgtctg aagaccacaa gtgtattcac 9000
   attaaataaa taaatcctcc ttcttcttct tttttttttt tttaaagaga atactgtctc 9060
   cagtagaatt tactgaagta atgaaatact ttgtgtttgt tccaatatgg tagccaataa 9120
   tcaaattact ctttaagcac tggaaatgtt accaaggaac taatttttat ttgaagtgta 9180
   actgtggaca gaggagccat aactgcagac ttgtgggata cagaagacca atgcagactt 9240
   taatgtcttt tctcttacac taagcaataa agaaataaaa attgaacttc tagtatccta 9300
   tttgtttaaa ctgctagctt tacttaactt ttgtgcttca tctatacaaa gctgaaagct 9360
   aagtctgcag ccattactaa acatgaaagc aagtaatgat aattttggat ttcaaaaatg 9420
   tagggccagg gtttagccag ccagtggtgg tgcttgcctt tatgccttta atcccagcac 9480
   tctggaggca gagacaggca gatctctgag tttgagccca gcctggtcta cacatcaagt 9540
   tctatctagg atagccagga atacacacag aaaccctgtt ggggaggggg gctctgagat 9600
   ttcataaaat tataattgaa gcattcccta atgagccact atggatgtgg ctaaatccgt 9660
   ctacctttct gatgagattt gggtattatt ttttctgtct ctgctgttgg ttgggtcttt 9720
   tgacactgtg ggctttcttt aaagcctcct tcctgccatg tggtctcttg tttgctacta 9780
   acttcccatg gcttaaatgg catggctttt tgccttctaa gggcagctgc tgagatttgc 9840
   agcctgattt ccagggtggg gttgggaaat ctttcaaaca ctaaaattgt cctttaattt 9900
   tttttttaaa aaatgggtta tataataaac ctcataaaat agttatgagg agtgaggtgg 9960
   actaatatta aatgagtccc tcccctataa aagagctatt aaggcttttt gtcttatact 10020
   taactttttt tttaaatgtg gtatctttag aaccaagggt cttagagttt tagtatacag 10080
   aaactgttgc atcgcttaat cagattttct agtttcaaat ccagagaatc caaattcttc 10140
   acagccaaag tcaaattaag aatttctgac ttttaatgtt aatttgctta ctgtgaatat 10200
   aaaaatgata gcttttcctg aggcagggtc tcactatgta tctctgcctg atctgcaaca 10260
   agatatgtag actaaagttc tgcctgcttt tgtctcctga atactaaggt taaaatgtag 10320
   taatactttt ggaacttgca ggtcagattc ttttataggg gacacactaa gggagcttgg 10380
   gtgatagttg gtaaaatgtg tttcaagtga tgaaaacttg aattattatc accgcaacct 10440
   actttttaaa aaaaaaagcc aggcctgtta gagcatgctt aagggatccc taggacttgc 10500
   tgagcacaca agagtagtta cttggcaggc tcctggtgag agcatatttc aaaaaacaag 10560
   gcagacaacc aagaaactac agttaaggtt acctgtcttt aaaccatctg catatacaca 10620
   gggatattaa aatattccaa ataatatttc attcaagttt tcccccatca aattgggaca 10680
   tggatttctc cggtgaatag gcagagttgg aaactaaaca aatgttggtt ttgtgatttg 10740
   tgaaattgtt ttcaagtgat agttaaagcc catgagatac agaacaaagc tgctatttcg 10800
   aggtctcttg gtttatactc agaagcactt ctttgggttt ccctgcacta tcctgatcat 10860
   gtgctaggcc taccttaggc tgattgttgt tcaaataaac ttaagtttcc tgtcaggtga 10920
   tgtcatatga tttcatatat caaggcaaaa catgttatat atgttaaaca tttgtactta 10980
   atgtgaaagt taggtctttg tgggtttgat ttttaatttt caaaacctga gctaaataag 11040
   tcatttttac atgtcttaca tttggtggaa ttgtataatt gtggtttgca ggcaagactc 11100
   tctgacctag taaccctacc tatagagcac tttgctgggt cacaagtcta ggagtcaagc 11160
   atttcacctt gaagttgaga cgttttgtta gtgtatacta gtttatatgt tggaggacat 11220
   gtttatccag aagatattca ggactatttt tgactgggct aaggaattga ttctgattag 11280
   cactgttagt gagcattgag tggcctttag gcttgaattg gagtcacttg tatatctcaa 11340
   ataatgctgg ccttttttaa aaagcccttg ttctttatca ccctgttttc tacataattt 11400
   ttgttcaaag aaatacttgt ttggatctcc ttttgacaac aatagcatgt tttcaagcca 11460
   tatttttttt cctttttttt tttttttttg gtttttcgag acagggtttc tctgtatagc 11520
   cctggctgtc ctggaactca ctttgtagac caggctggcc tcgaactcag aaatccgcct 11580
   gcctctgcct cctgagtgcc gggattaaag gcgtgcacca ccacgcctgg ctaagttgga 11640
   tattttgtta tataactata accaatacta actccactgg gtggattttt aattcagtca 11700
   gtagtcttaa gtggtcttta ttggcccttc attaaaatct actgttcact ctaacagagg 11760
   ctgttggtac tagtggcact taagcaactt cctacggata tactagcaga ttaagggtca 11820
   gggatagaaa ctagtctagc gttttgtata cctaccagct ttatactacc ttgttctgat 11880
   agaaatattt caggacatct agcacccaat tcgccctata gtgagtcgta ttacaattca 11940
   ctggccgtcg ttttacaacg tcgtgactgg gaaaaccctg gcgttaccca acttaatcgc 12000
   cttgcagcac atcccccttt cgccagctgg cgtaatagcg aagaggcccg caccgatcgc 12060
   ccttcccaac agttgcgcag cctgaatggc gaatgggacg cgccctgtag cggcgcatta 12120
   agcgcggcgg gtgtggtggt tacgcgcagc gtgaccgcta cacttgccag cgccctagcg 12180
   cccgctcctt tcgctttctt cccttccttt ctcgccacgt tcgccggctt tccccgtcaa 12240
   gctctaaatc gggggctccc tttagggttc cgatttagtg ctttacggca cctcgacccc 12300
   aaaaaacttg attagggtga tggttcacgt agtgggccat cgccctgata gacggttttt 12360
   cgccctttga cgttggagtc cacgttcttt aatagtggac tcttgttcca aactggaaca 12420
   acactcaacc ctatctcggt ctattctttt gatttataag ggattttgcc gatttcggcc 12480
   tattggttaa aaaatgagct gatttaacaa aaatttaacg cgaattttaa caaaatatta 12540
   acgcttacaa tttaggtggc acttttcggg gaaatgtgcg cggaacccct atttgtttat 12600
   ttttctaaat acattcaaat atgtatccgc tcatgagaca ataaccctga taaatgcttc 12660
   aataatattg aaaaaggaag agtatgagta ttcaacattt ccgtgtcgcc cttattccct 12720
   tttttgcggc attttgcctt cctgtttttg ctcacccaga aacgctggtg aaagtaaaag 12780
   atgctgaaga tcagttgggt gcacgagtgg gttacatcga actggatctc aacagcggta 12840
   agatccttga gagttttcgc cccgaagaac gttttccaat gatgagcact tttaaagttc 12900
   tgctatgtgg cgcggtatta tcccgtattg acgccgggca agagcaactc ggtcgccgca 12960
   tacactattc tcagaatgac ttggttgagt actcaccagt cacagaaaag catcttacgg 13020
   atggcatgac agtaagagaa ttatgcagtg ctgccataac catgagtgat aacactgcgg 13080
   ccaacttact tctgacaacg atcggaggac cgaaggagct aaccgctttt ttgcacaaca 13140
   tgggggatca tgtaactcgc cttgatcgtt gggaaccgga gctgaatgaa gccataccaa 13200
   acgacgagcg tgacaccacg atgcctgtag caatggcaac aacgttgcgc aaactattaa 13260
   ctggcgaact acttactcta gcttcccggc aacaattaat agactggatg gaggcggata 13320
   aagttgcagg accacttctg cgctcggccc ttccggctgg ctggtttatt gctgataaat 13380
   ctggagccgg tgagcgtggg tctcgcggta tcattgcagc actggggcca gatggtaagc 13440
   cctcccgtat cgtagttatc tacacgacgg ggagtcaggc aactatggat gaacgaaata 13500
   gacagatcgc tgagataggt gcctcactga ttaagcattg gtaactgtca gaccaagttt 13560
   actcatatat actttagatt gatttaaaac ttcattttta atttaaaagg atctaggtga 13620
   agatcctttt tgataatctc atgaccaaaa tcccttaacg tgagttttcg ttccactgag 13680
   cgtcagaccc cgtagaaaag atcaaaggat cttcttgaga tccttttttt ctgcgcgtaa 13740
   tctgctgctt gcaaacaaaa aaaccaccgc taccagcggt ggtttgtttg ccggatcaag 13800
   agctaccaac tctttttccg aaggtaactg gcttcagcag agcgcagata ccaaatactg 13860
   tccttctagt gtagccgtag ttaggccacc acttcaagaa ctctgtagca ccgcctacat 13920
   acctcgctct gctaatcctg ttaccagtgg ctgctgccag tggcgataag tcgtgtctta 13980
   ccgggttgga ctcaagacga tagttaccgg ataaggcgca gcggtcgggc tgaacggggg 14040
   gttcgtgcac acagcccagc ttggagcgaa cgacctacac cgaactgaga tacctacagc 14100
   gtgagctatg agaaagcgcc acgcttcccg aagggagaaa ggcggacagg tatccggtaa 14160
   gcggcagggt cggaacagga gagcgcacga gggagcttcc agggggaaac gcctggtatc 14220
   tttatagtcc tgtcgggttt cgccacctct gacttgagcg tcgatttttg tgatgctcgt 14280
   caggggggcg gagcctatgg aaaaacgcca gcaacgcggc ctttttacgg ttcctggcct 14340
   tttgctggcc ttttgctcac atgttctttc ctgcgttatc ccctgattct gtggataacc 14400
   gtattaccgc c                                                      14411
<210> 8
   <211> 18866
   <212> DNA
   <213> Artificial Sequence
<220>
   <223> Description of Artificial Sequence: FABP-Cre targeting vector
<400> 8
   tcgagacatt cctgcgcatc gtgcagtgcc gctctgtgga gggcagctgt ggcttctagc 60
   tgcccgggtg gcatccctgt gacccctccc cagtgcctct cctggccctg gaagttgcca 120
   ctccagtgcc caccagcctt gtcctaataa aattaagttg catcattttg tctgactagg 180
   tgtccttcta taatattatg gggtggaggg gggtggtatg gagcaagggg caagttggga 240
   agacaacctg tagggcctgc ggggtctatt gggaaccaag ctggagtgca gtggcacaat 300
   cttggctcac tgcaatctcc gcctcctggg ttcaagcgat tctcctgcct cagcctcccg 360
   agttgttggg attccaggca tgcatgacca ggctcagcta atttttgttt ttttggtaga 420
   gacggggttt caccatattg gccaggctgg tctccaactc ctaatctcag gtgatctacc 480
   caccttggcc tcccaaattg ctgggattac aggcgtgaac cactgctccc ttccctgtcc 540
   ttctgatttt aaaataacta taccagcagg aggacgtcca gacacagcat aggctacctg 600
   gccatgccca accggtggga catttgagtt gcttgcttgg cactgtcctc tcatgcgttg 660
   ggtccactca gtagatgcct gttgaattaa gcttgatatc gaattctcag aatacaaaac 720
   agctttaggg actgataaaa tatatgtaaa attatgtttg tataataata gaaaaattga 780
   gaaataaaca taaggctacg tgaaaaggtt ttgagttcaa aggtattttt ctccacgtaa 840
   tgcacatata catgtgtgtt tatatatgtg catatgtata tgtagctaca tgtgtataaa 900
   tatattacat atacaagtgt gcacatgtat aaacacatac atatgtacac ataggtatat 960
   atgcatgtat gcattgctag agatgtgatt cacatgtctg ttgcacatat atacatctgt 1020
   caacatacat ttcatgtatg cactcttatt tcatgagcgg tgataagaca ccaaaaatgc 1080
   accatttaca gagagctttg cccttgattg gactcactaa tgtttgctga attagaacaa 1140
   acttctgcct tgcccattct gatttaaatt agaacaaact tctgccttgc ccattctgat 1200
   ttttatcgtt agaacaaact tctgccttgc ccattctgat ttttatcgtt agaacaaact 1260
   tctgccttgc ccattctgat ttttatcgtt agaacaaact tctgccttgc ccattctgat 1320
   tttgattttt atcgttgacc attgctctca ggagttaatg tttgatcctg gccataaaga 1380
   aatcgacaat cactgaccta tggcctatat ttgaggagga agaagcccct tataaaatag 1440
   ccaacagtgg gtggcctggc agacagagct gttgtggtca gggggatcaa ataggccggc 1500
   ctaggcgcgt aagcgttggg gtgagtactc cctctcaaaa gcgggcatga cttctgcgct 1560
   aagattgtca gtttccaaaa acgaggagga tttgatattc acctggcccg cggtgatgcc 1620
   tttgagggtg gccgcgtcca tctggtcaga aaagacaatc tttttgttgt caagcttgag 1680
   gtgtggcagg cttgagatct ggccatacac ttgagtgaca ttgacatcca ctttgccttt 1740
   ctctccacag gtgtccactc ccagggcggc ctcgaccatg cccaagaaga agaggaaggt 1800
   gtccaattta ctgaccgtac accaaaattt gcctgcatta ccggtcgatg caacgagtga 1860
   tgaggttcgc aagaacctga tggacatgtt cagggatcgc caggcgtttt ctgagcatac 1920
   ctggaaaatg cttctgtccg tttgccggtc gtgggcggca tggtgcaagt tgaataaccg 1980
   gaaatggttt cccgcagaac ctgaagatgt tcgcgattat cttctatatc ttcaggcgcg 2040
   cggtctggca gtaaaaacta tccagcaaca tttgggccag ctaaacatgc ttcatcgtcg 2100
   gtccgggctg ccacgaccaa gtgacagcaa tgctgtttca ctggttatgc ggcggatccg 2160
   aaaagaaaac gttgatgccg gtgaacgtgc aaaacaggct ctagcgttcg aacgcactga 2220
   tttcgaccag gttcgttcac tcatggaaaa tagcgatcgc tgccaggata tacgtaatct 2280
   ggcatttctg ggattgcttt ataacaccct gttacgtata gccgaaattg ccaggatcag 2340
   ggttaaagat atctcacgta ctgacggtgg gagaatgtta atccatattg gcagaacgaa 2400
   aacgctggtt agcaccgcag gtgtagagaa ggcacttagc ctgggggtaa ctaaactggt 2460
   cgagcgatgg atttccgtct ctggtgtagc tgatgatccg aataactacc tgttttgccg 2520
   ggtcagaaaa aatggtgttg ccgcgccatc tgccaccagc cagctatcaa ctcgcgccct 2580
   ggaagggatt tttgaagcaa ctcatcgatt gatttacggc gctaaggatg actctggtca 2640
   gagatacctg gcctggtctg gacacagtgc ccgtgtcgga gccgcgcgag atatggcccg 2700
   cgctggagtt tcaataccgg agatcatgca agctggtggc tggaccaatg taaatattgt 2760
   catgaactat atccgtaacc tggatagtga aacaggggca atggtgcgcc tgctggaaga 2820
   tggcgattag ccattaacgc gtaaatgatt gcagatccac tagttctagg gccgcgtcga 2880
   cctcgagatc caggcgcgga tcaataaaag atcattattt tcaatagatc tgtgtgttgg 2940
   ttttttgtgt gccttggggg agggggaggc cagaatgagg cgcggccaag gggagggggg 3000
   aggccagaat gaccttgggg gagggggagg ccagaatgac cttgggggag ggggaggcca 3060
   gaatgaggcg cgccggtaac cgaagttcct atactttcta gagaatagga acttcggaat 3120
   aggaacttct taggtcaatt ctaccgggta ggggaggcgc ttttcccaag gcagtctgga 3180
   gcatgcgctt tagcagcccc gctgggcact tggcgctaca caagtggcct ctggcctcgc 3240
   acacattcca catccaccgg taggcgccaa ccggctccgt tctttggtgg ccccttcgcg 3300
   ccaccttcta ctcctcccct agtcaggaag ttcccccccg ccccgcagct cgcgtcgtgc 3360
   aggacgtgac aaatggaagt agcacgtctc actagtctcg tgcagatgga cagcaccgct 3420
   gagcaatgga agcgggtagg cctttggggc agcggccaat agcagctttg ctccttcgct 3480
   ttctgggctc agaggctggg aaggggtggg tccgggggcg ggctcagggg cgggctcagg 3540
   ggcggggcgg gcgcccgaag gtcctccgga ggcccggcat tctgcacgct tcaaaagcgc 3600
   acgtctgccg cgctgttctc ctcttcctca tctccgggcc tttcgacctg cagccaatat 3660
   gggatcggcc attgaacaag atggattgca cgcaggttct ccggccgctt gggtggagag 3720
   gctattcggc tatgactggg cacaacagac aatcggctgc tctgatgccg ccgtgttccg 3780
   gctgtcagcg caggggcgcc cggttctttt tgtcaagacc gacctgtccg gtgccctgaa 3840
   tgaactgcag gacgaggcag cgcggctatc gtggctggcc acgacgggcg ttccttgcgc 3900
   agctgtgctc gacgttgtca ctgaagcggg aagggactgg ctgctattgg gcgaagtgcc 3960
   ggggcaggat ctcctgtcat ctcaccttgc tcctgccgag aaagtatcca tcatggctga 4020
   tgcaatgcgg cggctgcata cgcttgatcc ggctacctgc ccattcgacc accaagcgaa 4080
   acatcgcatc gagcgagcac gtactcggat ggaagccggt cttgtcgatc aggatgatct 4140
   ggacgaagag catcaggggc tcgcgccagc cgaactgttc gccaggctca aggcgcgcat 4200
   gcccgacggc gaggatctcg tcgtgaccca tggcgatgcc tgcttgccga atatcatggt 4260
   ggaaaatggc cgcttttctg gattcatcga ctgtggccgg ctgggtgtgg cggaccgcta 4320
   tcaggacata gcgttggcta cccgtgatat tgctgaagag cttggcggcg aatgggctga 4380
   ccgcttcctc gtgctttacg gtatcgccgc tcccgattcg cagcgcatcg ccttctatcg 4440
   ccttcttgac gagttcttct gaggggatcg atccgctgta agtctgcaga aattgatgat 4500
   ctattaaaca ataaagatgt ccactaaaat ggaagttttt cctgtcatac tttgttaaga 4560
   agggtgagaa cagagtacct acattttgaa tggaaggatt ggagctacgg gggtgggggt 4620
   ggggtgggat tagataaatg cctgctcttt actgaaggct ctttactatt gctttatgat 4680
   aatgtttcat agttggatat cataatttaa acaagcaaaa ccaaattaag ggccagctca 4740
   ttcctcccac tcatgatcta tagatctata gatctctcgt gggatcattg tttttctctt 4800
   gattcccact ttgtggttct aagtactgtg gtttccaaat gtgtcagttt catagcctga 4860
   agaacgagat cagcagcctc tgttccacat acacttcatt ctcagtattg ttttgccaag 4920
   ttctaattcc atcagaagct gactctagat cccgcgccga agttcctata ctttctagag 4980
   aataggaact tcggaatagg aacttcaagc ttaagcgcta gaagatgggc gggagtcttc 5040
   tgggcaggct taaaggctaa cctggtgtgt gggcgttgtc ctgcagggga attgaacagg 5100
   tgtaaaattg gagggacaag acttcccaca gattttcggt tttgtcggga agttttttaa 5160
   taggggcaaa taaggaaaat gggaggatag gtagtcatct ggggttttat gcagcaaaac 5220
   tacaggttat tattgcttgt gatccgcctc ggagtatttt ccatcgaggt agattaaaga 5280
   catgctcacc cgagttttat actctcctgc ttgagatcct tactacagta tgaaattaca 5340
   gtgtcgcgag ttagactatg taagcagaat tttaatcatt tttaaagagc ccagtacttc 5400
   atatccattt ctcccgctcc ttctgcagcc ttatcaaaag gtattttaga acactcattt 5460
   tagccccatt ttcatttatt atactggctt atccaacccc tagacagagc attggcattt 5520
   tccctttcct gatcttagaa gtctgatgac tcatgaaacc agacagatta gttacataca 5580
   ccacaaatcg aggctgtagc tggggcctca acactgcagt tcttttataa ctccttagta 5640
   cactttttgt tgatcctttg ccttgatcct taattttcag tgtctatcac ctctcccgtc 5700
   agtggtgttc cacatttggg cctattctca gtccagggag ttttacaaca atagatgtat 5760
   tgagaatcca acctaaagct taactttcca ctcccatgaa tgcctctctc ctttttctcc 5820
   atttataaac tgagctatta accattaatg gttccaggtg gatgtctcct ccccatatta 5880
   cctgatgtat cttacatatt gccaggctga tattttaaga cattaaaagg tatatttcat 5940
   tattgagcca catggtattg attactgctt actaaaattt tgtcattgta cacatctgta 6000
   aaaggtggtt ccttttggaa tgcaaagttc aggtgtttgt tgtctttcct gacctaaggt 6060
   cttgtgagct tgtatttttt ctatttaagc agtgctttct cttggactgg cttgactcat 6120
   ggcattctac acgttattgc tggtctaaat gtgattttgc caagcttctt caggacctat 6180
   aattttgctt gacttgtagc caaacacaag taaaatgatt aagcaacaaa tgtatttgtg 6240
   aagcttggtt tttaggttgt tgtgttgtgt gtgcttgtgc tctataataa tactatccag 6300
   gggctggaga ggtggctcgg agttcaagag cacagactgc tcttccagaa gtcctgagtt 6360
   caattcccag caaccacatg gtggctcaca accatctgta atgggatctg atgccctctt 6420
   ctggtgtgtc tgaagaccac aagtgtattc acattaaata aataaatcct ccttcttctt 6480
   cttttttttt tttttaaaga gaatactgtc tccagtagaa tttactgaag taatgaaata 6540
   ctttgtgttt gttccaatat ggtagccaat aatcaaatta ctctttaagc actggaaatg 6600
   ttaccaagga actaattttt atttgaagtg taactgtgga cagaggagcc ataactgcag 6660
   acttgtggga tacagaagac caatgcagac tttaatgtct tttctcttac actaagcaat 6720
   aaagaaataa aaattgaact tctagtatcc tatttgttta aactgctagc tttacttaac 6780
   ttttgtgctt catctataca aagctgaaag ctaagtctgc agccattact aaacatgaaa 6840
   gcaagtaatg ataattttgg atttcaaaaa tgtagggcca gagtttagcc agccagtggt 6900
   ggtgcttgcc tttatgcctt taatcccagc actctggagg cagagacagg cagatctctg 6960
   agtttgagcc cagcctggtc tacacatcaa gttctatcta ggatagccag gaatacacac 7020
   agaaaccctg ttggggaggg gggctctgag atttcataaa attataattg aagcattccc 7080
   taatgagcca ctatggatgt ggctaaatcc gtctaccttt ctgatgagat ttgggtatta 7140
   ttttttctgt ctctgctgtt ggttgggtct tttgacactg tgggctttct ttaaagcctc 7200
   cttcctgcca tgtggtctct tgtttgctac taacttccca tggcttaaat ggcatggctt 7260
   tttgccttct aagggcagct gctgagattt gcagcctgat ttccagggtg gggttgggaa 7320
   atctttcaaa cactaaaatt gtcctttaat ttttttttta aaaaatgggt tatataataa 7380
   acctcataaa atagttatga ggagtgaggt ggactaatat taaatgagtc cctcccctat 7440
   aaaagagcta ttaaggcttt ttgtcttata cttaactttt tttttaaatg tggtatcttt 7500
   agaaccaagg gtcttagagt tttagtatac agaaactgtt gcatcgctta atcagatttt 7560
   ctagtttcaa atccagagaa tccaaattct tcacagccaa agtcaaatta agaatttctg 7620
   acttttaatg ttaatttgct tactgtgaat ataaaaatga tagcttttcc tgaggcaggg 7680
   tctcactatg tatctctgcc tgatctgcaa caagatatgt agactaaagt tctgcctgct 7740
   tttgtctcct gaatactaag gttaaaatgt agtaatactt ttggaacttg caggtcagat 7800
   tcttttatag gggacacact aagggagctt gggtgatagt tggtaaaatg tgtttcaagt 7860
   gatgaaaact tgaattatta tcaccgcaac ctacttttta aaaaaaaaag ccaggcctgt 7920
   tagagcatgc ttaagggatc cctaggactt gctgagcaca caagagtagt tacttggcag 7980
   gctcctggtg agagcatatt tcaaaaaaca aggcagacaa ccaagaaact acagttaagg 8040
   ttacctgtct ttaaaccatc tgcatataca cagggatatt aaaatattcc aaataatatt 8100
   tcattcaagt tttcccccat caaattggga catggatttc tccggtgaat aggcagagtt 8160
   ggaaactaaa caaatgttgg ttttgtgatt tgtgaaattg ttttcaagtg atagttaaag 8220
   cccatgagat acagaacaaa gctgctattt cgaggtctct tggtttatac tcagaagcac 8280
   ttctttgggt ttccctgcac tatcctgatc atgtgctagg cctaccttag gctgattgtt 8340
   gttcaaataa acttaagttt cctgtcaggt gatgtcatat gatttcatat atcaaggcaa 8400
   aacatgttat atatgttaaa catttgtact taatgtgaaa gttaggtctt tgtgggtttg 8460
   atttttaatt ttcaaaacct gagctaaata agtcattttt acatgtctta catttggtgg 8520
   aattgtataa ttgtggtttg caggcaagac tctctgacct agtaacccta cctatagagc 8580
   actttgctgg gtcacaagtc taggagtcaa gcatttcacc ttgaagttga gacgttttgt 8640
   tagtgtatac tagtttatat gttggaggac atgtttatcc agaagatatt caggactatt 8700
   tttgactggg ctaaggaatt gattctgatt agcactgtta gtgagcattg agtggccttt 8760
   aggcttgaat tggagtcact tgtatatctc aaataatgct ggcctttttt aaaaagccct 8820
   tgttctttat caccctgttt tctacataat ttttgttcaa agaaatactt gtttggatct 8880
   ccttttgaca acaatagcat gttttcaagc catatttttt ttcctttttt tttttttttt 8940
   tggtttttcg agacagggtt tctctgtata gccctggctg tcctggaact cactttgtag 9000
   accaggctgg cctcgaactc agaaatccgc ctgcctctgc ctcctgagtg ccgggattaa 9060
   aggcgtgcac caccacgcct ggctaagttg gatattttgt tatataacta taaccaatac 9120
   taactccact gggtggattt ttaattcagt cagtagtctt aagtggtctt tattggccct 9180
   tcattaaaat ctactgttca ctctaacaga ggctgttggt actagtggca cttaagcaac 9240
   ttcctacgga tatactagca gattaagggt cagggataga aactagtcta gcgttttgta 9300
   tacctaccag ctttatacta ccttgttctg atagaaatat ttcaggacat ctagagtgta 9360
   ctataaggtt gatggtaagc ttataaggaa cttgaaagtg gagtaactac tccatttctc 9420
   tgaggggaga attaaaattt ttgaccaagt gttgttgagc cactgagaat ggtctcagaa 9480
   cataacttct taaggaacct tcccagattg ccctcaacac tgcaccacat ttggtcctgc 9540
   ttgaacattg ccatggctct taaagtctta attaagaata ttaattgtgt aattattgtt 9600
   tttcctcctt tagatcattc cttgaggaca ggacagtgct tgtttaaggc tatatttctg 9660
   ctgtctgagc agcaacaggt cttcgagatc aacatgatgt tcataatccc aagatgttgc 9720
   catttatgtt ctcagaagca agcagaggca tgatggtcag tgacagtaat gtcactgtgt 9780
   taaatgttgc tatgcagttt ggatttttct aatgtagtgt aggtagaaca tatgtgttct 9840
   gtatgaatta aactcttaag ttacaccttg tataatccat gcaatgtgtt atgcaattac 9900
   cattttaagt attgtagctt tctttgtatg tgaggataaa ggtgtttgtc ataaaatgtt 9960
   ttgaacattt ccccaaagtt ccaaattata aaaccacaac gttagaactt atttatgaac 10020
   aatggttgta gtttcatgct tttaaaatgc ttaattattc aattaacacc gtttgtgtta 10080
   taatatatat aaaactgaca tgtagaagtg tttgtccaga acatttctta aatgtatact 10140
   gtctttagag agtttaatat agcatgtctt ttgcaacata ctaacttttg tgttggtgcg 10200
   agcaatattg tgtagtcatt ttgaaaggag tcatttcaat gagtgtcaga ttgttttgaa 10260
   tgttattgaa cattttaaat gcagacttgt tcgtgtttta gaaagcaaaa ctgtcagaag 10320
   ctttgaacta gaaattaaaa agctgaagta tttcagaagg gaaataagct acttgctgta 10380
   ttagttgaag gaaagtgtaa tagcttagaa aatttaaaac catatagttg tcattgctga 10440
   atatctggca gatgaaaaga aatactcagt ggttcttttg agcaatataa cagcttgtta 10500
   tattaaaaat tttccccaca gatataaact ctaatctata actcataaat gttacaaatg 10560
   gatgaagctt acaaatgtgg cttgacttgt cactgtgctt gttttagtta tgtgaaagtt 10620
   tggcaataaa cctatgtcct aaatagtcaa actgtggaat gactttttaa tctattggtt 10680
   tgtctagaac agttatgttg ccatttgccc taatggtgaa agaaaaagtg gggagtgcct 10740
   tggcactgtt catttgtggt gtgaaccaaa gaggggggca tgcacttaca cttcaaacat 10800
   ccttttgaaa gactgacaag tttgggtctt cacagttgga attgggcatc ccttttgtca 10860
   gggagggagg gagggaggga ggctggcttg ttatgctgac aagtgtgatt aaattcaaac 10920
   tttgaggtaa gttggaggaa cttgtacatt gttaggagtg tgacaatttg gactcttaat 10980
   gatttggtca tacaaaatga acctagacca acttctggaa gatgtatata ataactccat 11040
   gttacattga tttcacctga ctaatactta tcccttatca attaaataca gaagatgcca 11100
   gccatctggg ccttttaacc cagaaattta gtttcaaact cctaggttag tgttctcact 11160
   gagctacatc ctgatctagt cctgaaaata ggaccaccat cacccccaaa aaaatctcaa 11220
   ataagattta tgctagtgtt tcaaaatttt aggaataggt aagattagaa agttttaaat 11280
   tttgagaaat ggcttctcta gaaagatgta catagtgaac actgaatggc tcctaaagag 11340
   cctagaaaac tggtactgag cacacaggac tgagaggtct ttcttgaaaa gcatgtattg 11400
   ctttacgtgg gtcacagaag gcaggcagga agaacttggg ctgaaactgg tgtcttaagt 11460
   ggctaacatc ttcacaactg atgagcaaga actttatcct gatgcaaaaa ccatccaaac 11520
   aaactaagtg aaaggtggca atggatccca ggctgctcta gaggaggact tgacttctca 11580
   tcccatcacc cacaccagat agctcataga ctgccaatta acaccagctt ctagcctcca 11640
   caggcacctg cactggtaca cataatttca cacaaacaca gtaagaagcc ttccacctgg 11700
   catggtattg cttatcttta gttcccaaca cttgggaggc agaggccagc cagggctatg 11760
   tgacaaaaac cttgtctaga ggagaaactt catagcttat ttcctattca cgtaaccagg 11820
   ttagcaaaat ttaccagcca gagatgaagc taacagtgtc cactatattt gtagtgtttt 11880
   aagtcaattt tttaaatata cttaatagaa ttaaagctat ggtgaaccaa gtacaaacct 11940
   ggtgtattaa cttgagaact tagcataaaa agtagttcat ttgttcagta aatattaaat 12000
   gcttactggc aaagattatg tcaggaactt ggtaaatggt gatgaaacaa tcatagttgt 12060
   acatcttggt tctgtgatca ccttggtttg aggtaaaagt ggttcctttg atcaaggatg 12120
   gaattttaag tttatattca atcaataatg tattattttg tgattgcaaa attgcctatc 12180
   tagggtataa aacctttaaa aatttcataa taccagttca ttctccagtt actaattcca 12240
   aaaagccact gactatggtg ccaatgtgga ttctgttctc aaaggaagga ttgtctgtgc 12300
   cctttattct aatagaaaca tcacactgaa aatctaagct gaaagaagcc agactttcct 12360
   aaataaataa ctttccataa agctcaaaca aggattactt ttaggaggca ctgttaagga 12420
   actgataagt aatgaggtta cttatataat gatagtccca caagactatc tgaggaaaaa 12480
   tcagtacaac tcgaaaacag aacaaccagc taggcaggaa taacagggct cccaagtcag 12540
   gaggtctatc caacaccctt ttctgttgag ggccccagac ctacatattg tatacaaaca 12600
   gggaggtggg tgattttaac tctcctgagg taccttggta aatctttgtc ctgagtaagc 12660
   agtacagtgt acagtttaca ttttcattta aagatacatt agctccctct accccctaag 12720
   actgacaggc actttggggg tggggagggc tttggaaaat aacgcttcca tacactaaaa 12780
   gagaaatttc tttaattagg cttgttggtt ccatacatct actggtgttt ctactactta 12840
   gtaatattat aatagtcaca caagcatctt tgctctgttt aggttgtata tttattttaa 12900
   ggcagatgat aaaactgtag atcttaaggg atgcttctgc ttctgagatg atacaaagaa 12960
   tttagaccat aaaacagtag gttgcacaag caatagaata tggcctaaag tgttctgaca 13020
   cttagaagcc aagcagtgta ggcttcttaa gaaataccat tacaatcacc ttgctagaaa 13080
   tcaagcattc tggagtggtc aagcagtgta acctgtactg taagttactt ttctgctatt 13140
   tttctcccaa agcaagttct ttatgctgat atttccagtg ttaggaacta caaatattaa 13200
   taagttgtct tcactctttt ctttaccaag gagggtctct tccttcatct tgatctgaag 13260
   gatgaacaaa ggcttgagca gtgcgcttta gaagataaac tgcagcatga aggcccccga 13320
   tgttcaccca gactacatgg acctttcgcc acacatgtcc cattccagat aaggcctggc 13380
   acacacaaaa aacataagtc attaggctac cagtctgatt ctaaaacaac ctaaaatctt 13440
   cccacttaaa tgctatgggt ggtgggttgg aaagttgact cagaaaatca cttgctgttt 13500
   ttagagagga tctgggttca gtttctgata cattgtggct tacaactata actccagttc 13560
   tagggggtcc atccaacatc ctcttctgtt gagggcacca aataaatgta ttgtgtacaa 13620
   acagggaggt gagtgattta actctcgtgt atagtacctt ggtaaaacat ttcttgtcct 13680
   gagtaagcag tacagctctg cctgtccctg gtctacagac acggctcatt tcccgaaggc 13740
   aagctggata gagattccaa tttctcttct tggatcccat cctataaaag aaggtcaagt 13800
   ttaatctatt gcaaaaggta aataggtagt ttcttacatg agacaagaac aaatcttagg 13860
   tgtgaagcag tcatctttta caggccagag cctctattct atgccaatga aggaaactgt 13920
   tagtccagtg ttatagagtt agtccagtgt atagttttct atcagaacac ttttttttta 13980
   aacaactgca acttagctta ttgaagacaa accacgagta gaaatctgtc caagaagcaa 14040
   gtgcttctca gcctacaatg tggaatagga ccatgtaatg gtacagtgag tgaaatgaat 14100
   tatggcatgt ttttctgact gagaagacag tacaataaaa ggtaaactca tggtatttat 14160
   ttaaaaagaa tccaatttct acctttttcc aaatggcata tctgttacaa taatatccac 14220
   agaagcagtt ctcagtggga ggttgcagat gacctcgagg gggggcccgg tacccagctt 14280
   ttgttccctt tagtgagggt taattgcgcg cttggcgtaa tcatggtcat agctgtttcc 14340
   tgtgtgaaat tgttatccgc tcacaattcc acacaacata cgagccggaa gcataaagtg 14400
   taaagcctgg ggtgcctaat gagtgagcta actcacatta attgcgttgc gctcactgcc 14460
   cgctttccag tcgggaaacc tgtcgtgcca gctgcattaa tgaatcggcc aacgcgcggg 14520
   gagaggcggt ttgcgtattg ggcgctcttc cgctccaatt cgccctatag tgagtcgtat 14580
   tacaattcac tggccgtcgt tttacaacgt cgtgactggg aaaaccctgg cgttacccaa 14640
   cttaatcgcc ttgcagcaca tccccctttc gccagctggc gtaatagcga agaggcccgc 14700
   accgatcgcc cttcccaaca gttgcgcagc ctgaatggcg aatgggacgc gccctgtagc 14760
   ggcgcattaa gcgcggcggg tgtggtggtt acgcgcagcg tgaccgctac acttgccagc 14820
   gccctagcgc ccgctccttt cgctttcttc ccttcctttc tcgccacgtt cgccggcttt 14880
   ccccgtcaag ctctaaatcg ggggctccct ttagggttcc gatttagtgc tttacggcac 14940
   ctcgacccca aaaaacttga ttagggtgat ggttcacgta gtgggccatc gccctgatag 15000
   acggtttttc gccctttgac gttggagtcc acgttcttta atagtggact cttgttccaa 15060
   actggaacaa cactcaaccc tatctcggtc tattcttttg atttataagg gattttgccg 15120
   atttcggcct attggttaaa aaatgagctg atttaacaaa aatttaacgc gaattttaac 15180
   aaaatattaa cgcttacaat ttaggtggca cttttcgggg aaatgtgcgc ggaaccccta 15240
   tttgtttatt tttctaaata cattcaaata tgtatccgct catgagacaa taaccctgat 15300
   aaatgcttca ataatattga aaaaggaaga gtatgagtat tcaacatttc cgtgtcgccc 15360
   ttattccctt ttttgcggca ttttgccttc ctgtttttgc tcacccagaa acgctggtga 15420
   aagtaaaaga tgctgaagat cagttgggtg cacgagtggg ttacatcgaa ctggatctca 15480
   acagcggtaa gatccttgag agttttcgcc ccgaagaacg ttttccaatg atgagcactt 15540
   ttaaagttct gctatgtggc gcggtattat cccgtattga cgccgggcaa gagcaactcg 15600
   gtcgccgcat acactattct cagaatgact tggttgagta ctcaccagtc acagaaaagc 15660
   atcttacgga tggcatgaca gtaagagaat tatgcagtgc tgccataacc atgagtgata 15720
   acactgcggc caacttactt ctgacaacga tcggaggacc gaaggagcta accgcttttt 15780
   tgcacaacat gggggatcat gtaactcgcc ttgatcgttg ggaaccggag ctgaatgaag 15840
   ccataccaaa cgacgagcgt gacaccacga tgcctgtagc aatggcaaca acgttgcgca 15900
   aactattaac tggcgaacta cttactctag cttcccggca acaattaata gactggatgg 15960
   aggcggataa agttgcagga ccacttctgc gctcggccct tccggctggc tggtttattg 16020
   ctgataaatc tggagccggt gagcgtgggt ctcgcggtat cattgcagca ctggggccag 16080
   atggtaagcc ctcccgtatc gtagttatct acacgacggg gagtcaggca actatggatg 16140
   aacgaaatag acagatcgct gagataggtg cctcactgat taagcattgg taactgtcag 16200
   accaagttta ctcatatata ctttagattg atttaaaact tcatttttaa tttaaaagga 16260
   tctaggtgaa gatccttttt gataatctca tgaccaaaat cccttaacgt gagttttcgt 16320
   tccactgagc gtcagacccc gtagaaaaga tcaaaggatc ttcttgagat cctttttttc 16380
   tgcgcgtaat ctgctgcttg caaacaaaaa aaccaccgct accagcggtg gtttgtttgc 16440
   cggatcaaga gctaccaact ctttttccga aggtaactgg cttcagcaga gcgcagatac 16500
   caaatactgt ccttctagtg tagccgtagt taggccacca cttcaagaac tctgtagcac 16560
   cgcctacata cctcgctctg ctaatcctgt taccagtggc tgctgccagt ggcgataagt 16620
   cgtgtcttac cgggttggac tcaagacgat agttaccgga taaggcgcag cggtcgggct 16680
   gaacgggggg ttcgtgcaca cagcccagct tggagcgaac gacctacacc gaactgagat 16740
   acctacagcg tgagctatga gaaagcgcca cgcttcccga agggagaaag gcggacaggt 16800
   atccggtaag cggcagggtc ggaacaggag agcgcacgag ggagcttcca gggggaaacg 16860
   cctggtatct ttatagtcct gtcgggtttc gccacctctg acttgagcgt cgatttttgt 16920
   gatgctcgtc aggggggcgg agcctatgga aaaacgccag caacgcggcc tttttacggt 16980
   tcctggcctt ttgctggcct tttgctcaca tgttctttcc tgcgttatcc cctgattctg 17040
   tggataaccg tattaccgcc tttgagtgag ctgataccgc tcgccgcagc cgaacgaccg 17100
   agcgcagcga gtcagtgagc gaggaagcgg aagagcgccc aatacgcaaa ccgcctctcc 17160
   ccgcgcgttg gccgattcat taatgcagct ggcacgacag gtttcccgac tggaaagcgg 17220
   gcagtgagcg caacgcaatt aatgtgagtt agctcactca ttaggcaccc caggctttac 17280
   actttatgct tccggctcgt atgttgtgtg gaattgtgag cggataacaa tttcacacag 17340
   gaaacagcta tgaccatgat tacgccaagc gcgcaattaa ccctcactaa agggaacaaa 17400
   agctgtcgag atctagatat cgatggccat agagttacgc tagggataac agggtaatat 17460
   agccgcggca ggccctccga gcgtggtgga gccgttctgt gagacagccg ggtacgagtc 17520
   gtgacgctgg aaggggcaag cgggtggtgg gcaggaatgc ggtccgccct gcagcaaccg 17580
   gagggggagg gagaagggag cggaaaagtc tccaccggac gcggccatgg ctcggggggg 17640
   ggggggcagc ggaggagcgc ttccggccga cgtctcgtcg ctgattggct tcttttcctc 17700
   ccgccgtgtg tgaaaacaca aatggcgtgt tttggttggc gtaaggcgcc tgtcagttaa 17760
   cggcagccgg agtgcgcagc cgccggcagc ctcgctctgc ccactgggtg gggcgggagg 17820
   taggtggggt gaggcgagct ggacgtgcgg gcgcggtcgg cctctggcgg ggcgggggag 17880
   gggagggagg gtcagcgaaa gtagctcgcg cgcgagcggc cgcccaccct ccccttcctc 17940
   tgggggagtc gttttacccg ccgccggccg ggcctcgtcg tctgattggc tctcggggcc 18000
   cagaaaactg gcccttgcca ttggctcgtg ttcgtgcaag ttgagtccat ccgccggcca 18060
   gcgggggcgg cgaggaggcg ctcccaggtt ccggccctcc cctcggcccc gcgccgcaga 18120
   gtctggccgc gcgcccctgc gcaacgtggc aggaagcgcg cgctgggggc ggggacgggc 18180
   agtagggctg agcggctgcg ggcgggtgcc aagcacgttt ccgacttgag ttgcctcaag 18240
   aggggcgtgc tgagccagac ctccatcgcg cactccgggg agtggaggga aggagcgagg 18300
   gctcagttgg gctgttttgg aggcaggaag cacttgctct cccaaagtcg ctctgagttg 18360
   ttatcagtaa gggagctgca gtggagtagg cggggagaag gccgcaccct tctccggagg 18420
   ggggagggga gtgttgcaat acctttctgg gagttctctg ctgcctcctg gcttctgagg 18480
   accgccctgg gcctgggaga atcccttccc cctcttccct cgtgatctgc aactccagtc 18540
   tttctaggta accgatatcc ctgcaggggt gacctgcacg tctagggcgc agtagtccag 18600
   ggtttccttg atgatgtcat acttatcctg tccctttttt ttccacagct cgcggttgag 18660
   gacaaactct tcgcggtctt tccagtactc ctgcaggtga ctgactgagt cgacttaatt 18720
   aaggccatag cggccatttc tagacttaga tccccgcggg atcttcaagc agacctacag 18780
   caagttcgac acaaactcac acaacgatga cgcactactc aagaactacg ggctgctcta 18840
   ctgcttcagg aaggacatgg acaagg                                      18866
<210> 9
   <211> 21730
   <212> DNA
   <213> Artificial Sequence
<220>
   <223> Description of Artificial Sequence: Cre substrate reporter
<400> 9
   taaagggaac aaaagctgtc gagatctaga tatcgatggc catagagtta cgctagggat 60
   aacagggtaa tatagccgcg gtcaggccct ccgagcgtgg tggagccgtt ctgtgagaca 120
   gccggatcgg caggccctcc gagcgtggtg gagccgttct gtgagacagc cgggtacgag 180
   tcgtgacgct ggaaggggca agcgggtggt gggcaggaat gcggtccgcc ctgcagcaac 240
   cggaggggga gggagaaggg agcggaaaag tctccaccgg acgcggccat ggctcggggg 300
   ggggggggca gcggaggagc gcttccggcc gacgtctcgt cgctgattgg cttcttttcc 360
   tcccgccgtg tgtgaaaaca caaatggcgt gttttggttg gcgtaaggcg cctgtcagtt 420
   aacggcagcc ggagtgcgca gccgccggca gcctcgctct gcccactggg tggggcggga 480
   ggtaggtggg gtgaggcgag ctggacgtgc gggcgcggtc ggcctctggc ggggcggggg 540
   aggggaggga gggtcagcga aagtagctcg cgcgcgagcg gccgcccacc ctccccttcc 600
   tctgggggag tcgttttacc cgccgccggc cgggcctcgt cgtctgattg gctctcgggg 660
   cccagaaaac tggcccttgc cattggctcg tgttcgtgca agttgagtcc atccgccggc 720
   cagcgggggc ggcgaggagg cgctcccagg ttccggccct cccctcggcc ccgcgccgca 780
   gagtctggcc gcgcgcccct gcgcaacgtg gcaggaagcg cgcgctgggg gcggggacgg 840
   gcagtagggc tgagcggctg cggggcgggt gcaagcacgt ttccgacttg agttgcctca 900
   agaggggcgt gctgagccag acctccatcg cgcactccgg ggagtggagg gaaggagcga 960
   gggctcagtt gggctgtttt ggaggcagga agcacttgct ctcccaaagt cgctctgagt 1020
   tgttatcagt aagggagctg cagtggagta ggcggggaga aggccgcacc cttctccgga 1080
   ggggggaggg gagtgttgca atacctttct gggagttctc tgctgcctcc tggcttctga 1140
   ggaccgccct gggcctggga gaatcccttc cccctcttcc ctcgtgatct gcaactccag 1200
   tctttcctag gtaaccgata tccctgcagg ttttcgacat tgattattga ctagttatta 1260
   atagtaatca attacggggt cattagttca tagcccatat atggagttcc gcgttacata 1320
   acttacggta aatggcccgc ctggctgacc gcccaacgac ccccgcccat tgacgtcaat 1380
   aatgacgtat gttcccatag taacgccaat agggactttc cattgacgtc aatgggtgga 1440
   ctatttacgg taaactgccc acttggcagt acatcaagtg tatcatatgc caagtacgcc 1500
   ccctattgac gtcaatgacg gtaaatggcc cgcctggcat tatgcccagt acatgacctt 1560
   atgggacttt cctacttggc agtacatcta cgtattagtc atcgctatta ccatgggtcg 1620
   aggtgagccc cacgttctgc ttcactctcc ccatctcccc cccctcccca cccccaattt 1680
   tgtatttatt tattttttaa ttattttgtg cagcgatggg ggcggggggg gggggggcgc 1740
   gcgccaggcg gggcggggcg gggcgagggg cggggcgggg cgaggcggag aggtgcggcg 1800
   gcagccaatc agagcggcgc gctccgaaag tttcctttta tggcgaggcg gcggcggcgg 1860
   cggccctata aaaagcgaag cgcgcggcgg gcgggagtcg ctgcgttgcc ttcgccccgt 1920
   gccccgctcc gcgccgcctc gcgccgcccg ccccggctct gactgaccgc gttactccca 1980
   caggtgagcg ggcgggacgg cccttctcct ccgggctgta attagcgctt ggtttaatga 2040
   cggctcgttt cttttctgtg gctgcgtgaa agccttaaag ggctccggga gggccctttg 2100
   tgcggggggg agcggctcgg ggggtgcgtg cgtgtgtgtg tgcgtgggga gcgccgcgtg 2160
   cggcccgcgc tgcccggcgg ctgtgagcgc tgcgggcgcg gcgcggggct ttgtgcgctc 2220
   cgcgtgtgcg cgaggggagc gcggccgggg gcggtgcccc gcggtgcggg ggggctgcga 2280
   ggggaacaaa ggctgcgtgc ggggtgtgtg cgtggggggg tgagcagggg gtgtgggcgc 2340
   ggcggtcggg ctgtaacccc cccctgcacc cccctccccg agttgctgag cacggcccgg 2400
   cttcgggtgc ggggctccgt gcggggcgtg gcgcggggct cgccgtgccg ggcggggggt 2460
   ggcggcaggt gggggtgccg ggcggggcgg ggccgcctcg ggccggggag ggctcggggg 2520
   aggggcgcgg cggccccgga gcgccggcgg ctgtcgaggc gcggcgagcc gcagccattg 2580
   ccttttatgg taatcgtgcg agagggcgca gggacttcct ttgtcccaaa tctggcggag 2640
   ccgaaatctg ggaggcgccg ccgcaccccc tctagcgggc gcgggcgaag cggtgcggcg 2700
   ccggcaggaa ggaaatgggc ggggagggcc ttcgtgcgtc gccgcgccgc cgtccccttc 2760
   tccatctcca gcctcggggc tgccgcaggg ggacggctgc cttcgggggg gacggggcag 2820
   ggcggggttc ggcttctggc gtgtgaccgg cggctctaga agcgttgggg tgagtactcc 2880
   ctctcaaaag cgggcatgac ttctgcgcta agattgtcag tttccaaaaa cgaggaggat 2940
   ttgatattca cctggcccgc ggtgatgcct ttgagggtgg ccgcgtccat ctggtcagaa 3000
   aagacaatct ttttgttgtc aagcttgagg tgtggcaggc ttgagatctg gccatacact 3060
   tgagtgacat tgacatccac tttgcctttc tctccacagg tgtccactcc cagggcggcc 3120
   tccggagcga tcgccggtcc gcctaggcta gccggccgcg tcgacttaat taaggccata 3180
   gcggccattt tcgacgacac tgcagagacc tacttcacta acaaccggta cagttcggtg 3240
   gagtacgcgc ccggggagcc caagggcacg ccctggcacc cgcaccgcgg cgaataactt 3300
   cgtatagcat acattatacg aagttataag ctcgatgaat tctaccgggt aggggaggcg 3360
   cttttcccaa ggcagtctgg agcatgcgct ttagcagccc cgctggcact tggcgctaca 3420
   caagtggcct ctggcctcgc acacattcca catccaccgg tagcgccaac cggctccgtt 3480
   ctttggtggc cccttcgcgc caccttctac tcctccccta gtcaggaagt tcccccccgc 3540
   cccgcagctc gcgtcgtgca ggacgtgaca aatggaagta gcacgtctca ctagtctcgt 3600
   gcagatggac agcaccgctg agcaatggaa gcgggtaggc ctttggggca gcggccaata 3660
   gcagctttgc tccttcgctt tctgggctca gaggctggga aggggtgggt ccgggggcgg 3720
   gctcaggggc gggctcaggg gcggggcggg cgcgaaggtc ctcccgaggc ccggcattct 3780
   cgcacgcttc aaaagcgcac gtctgccgcg ctgttctcct cttcctcatc tccgggcctt 3840
   tcgacgatcc agccgccacc atgaaaaagc ctgaactcac cgcgacgtct gtcgagaagt 3900
   ttctgatcga aaagttcgac agcgtctccg acctgatgca gctctcggag ggcgaagaat 3960
   ctcgtgcttt cagcttcgat gtaggagggc gtggatatgt cctgcgggta aatagctgcg 4020
   ccgatggttt ctacaaagat cgttatgttt atcggcactt tgcatcggcc gcgctcccga 4080
   ttccggaagt gcttgacatt ggggaattca gcgagagcct gacctattgc atctcccgcc 4140
   gtgcacaggg tgtcacgttg caagacctgc ctgaaaccga actgcccgct gttctgcagc 4200
   cggtcgcgga ggccatggat gcgatcgctg cggccgatct tagccagacg agcgggttcg 4260
   gcccattcgg accgcaagga atcggtcaat acactacatg gcgtgatttc atatgcgcga 4320
   ttgctgatcc ccatgtgtat cactggcaaa ctgtgatgga cgacaccgtc agtgcgtccg 4380
   tcgcgcaggc tctcgatgag ctgatgcttt gggccgagga ctgccccgaa gtccggcacc 4440
   tcgtgcacgc ggatttcggc tccaacaatg tcctgacgga caatggccgc ataacagcgg 4500
   tcattgactg gagcgaggcg atgttcgggg attcccaata cgaggtcgcc aacatcttct 4560
   tctggaggcc gtggttggct tgtatggagc agcagacgcg ctacttcgag cggaggcatc 4620
   cggagcttgc aggatcgccg cggctccggg cgtatatgct ccgcattggt cttgaccaac 4680
   tctatcagag cttggttgac ggcaatttcg atgatgcagc ttgggcgcag ggtcgatgcg 4740
   acgcaatcgt ccgatccgga gccgggactg tcgggcgtac acaaatcgcc cgcagaagcg 4800
   cggccgtctg gaccgatggc tgtgtagaag tactcgccga tagtggaaac cgacgcccca 4860
   gcactcgtcc gagggcaaag gaatagtcga tgcagaaatt gatgatctat taaacaataa 4920
   agatgtccac taaaatggaa gtttttcctg tcatactttg ttaagaaggg tgagaacaga 4980
   gtacctacat tttgaatgga aggattggag ctacgggggt gggggtgggg tgggattaga 5040
   taaatgcctg ctctttactg aaggctcttt actattgctt tatgataatg tttcatagtt 5100
   ggatatcata atttaaacaa gcaaaaccaa attaagggcc agctcattcc tcccactcat 5160
   gatctataga tctatagatc tctcgtggga tcattgtttt tctcttgatt cccactttgt 5220
   ggttctaagt actgtggttt ccaaatgtgt cagtttcata gcctgaagaa cgagatcagc 5280
   agcctctgtt ccacatacac ttcattctca gtattgtttt gccaagttct aattccatca 5340
   gaagcttcag ctgctcgact agaggatcat aatcagccat accacatttg tagaggtttt 5400
   acttgcttta aaaaacctcc cacacctccc cctgaacctg aaacataaaa tgaatgcaat 5460
   tgttgttgtt aacttgttta ttgcagctta taatggttac aaataaagca atagcatcac 5520
   aaatttcaca aataaagcat ttttttcact gcattctagt tgtggtttgt ccaaactcat 5580
   caatgtatct tatcatgtct ggatccctac gcccccaact gagagaactc aaaggttacc 5640
   ccagttgggg cactacggat ccataacttc gtatagcata cattatacga agttataccg 5700
   ggccaccggc gcgccatgcc taagaagaag aggaaggttg tcgcgagtag cttggcactg 5760
   gccgtcgttt tacaacgtcg tgactgggaa aaccctggcg ttacccaact taatcgcctt 5820
   gcagcacatc cccctttcgc cagctggcgt aatagcgaag aggcccgcac cgatcgccct 5880
   tcccaacagt tgcgcagcct gaatggcgaa tggcgctttg cctggtttcc ggcaccagaa 5940
   gcggtgccgg aaagctggct ggagtgcgat cttcctgagg ccgatactgt cgtcgtcccc 6000
   tcaaactggc agatgcacgg ttacgatgcg cccatctaca ccaacgtaac ctatcccatt 6060
   acggtcaatc cgccgtttgt tcccacggag aatccgacgg gttgttactc gctcacattt 6120
   aatgttgatg aaagctggct acaggaaggc cagacgcgaa ttatttttga tggcgttaac 6180
   tcggcgtttc atctgtggtg caacgggcgc tgggtcggtt acggccagga cagtcgtttg 6240
   ccgtctgaat ttgacctgag cgcattttta cgcgccggag aaaaccgcct cgcggtgatg 6300
   gtgctgcgtt ggagtgacgg cagttatctg gaagatcagg atatgtggcg gatgagcggc 6360
   attttccgtg acgtctcgtt gctgcataaa ccgactacac aaatcagcga tttccatgtt 6420
   gccactcgct ttaatgatga tttcagccgc gctgtactgg aggctgaagt tcagatgtgc 6480
   ggcgagttgc gtgactacct acgggtaaca gtttctttat ggcagggtga aacgcaggtc 6540
   gccagcggca ccgcgccttt cggcggtgaa attatcgatg agcgtggtgg ttatgccgat 6600
   cgcgtcacac tacgtctgaa cgtcgaaaac ccgaaactgt ggagcgccga aatcccgaat 6660
   ctctatcgtg cggtggttga actgcacacc gccgacggca cgctgattga agcagaagcc 6720
   tgcgatgtcg gtttccgcga ggtgcggatt gaaaatggtc tgctgctgct gaacggcaag 6780
   ccgttgctga ttcgaggcgt taaccgtcac gagcatcatc ctctgcatgg tcaggtcatg 6840
   gatgagcaga cgatggtgca ggatatcctg ctgatgaagc agaacaactt taacgccgtg 6900
   cgctgttcgc attatccgaa ccatccgctg tggtacacgc tgtgcgaccg ctacggcctg 6960
   tatgtggtgg atgaagccaa tattgaaacc cacggcatgg tgccaatgaa tcgtctgacc 7020
   gatgatccgc gctggctacc ggcgatgagc gaacgcgtaa cgcgaatggt gcagcgcgat 7080
   cgtaatcacc cgagtgtgat catctggtcg ctggggaatg aatcaggcca cggcgctaat 7140
   cacgacgcgc tgtatcgctg gatcaaatct gtcgatcctt cccgcccggt gcagtatgaa 7200
   ggcggcggag ccgacaccac ggccaccgat attatttgcc cgatgtacgc gcgcgtggat 7260
   gaagaccagc ccttcccggc tgtgccgaaa tggtccatca aaaaatggct ttcgctacct 7320
   ggagagacgc gcccgctgat cctttgcgaa tacgcccacg cgatgggtaa cagtcttggc 7380
   ggtttcgcta aatactggca ggcgtttcgt cagtatcccc gtttacaggg cggcttcgtc 7440
   tgggactggg tggatcagtc gctgattaaa tatgatgaaa acggcaaccc gtggtcggct 7500
   tacggcggtg attttggcga tacgccgaac gatcgccagt tctgtatgaa cggtctggtc 7560
   tttgccgacc gcacgccgca tccagcgctg acggaagcaa aacaccagca gcagtttttc 7620
   cagttccgtt tatccgggca aaccatcgaa gtgaccagcg aatacctgtt ccgtcatagc 7680
   gataacgagc tcctgcactg gatggtggcg ctggatggta agccgctggc aagcggtgaa 7740
   gtgcctctgg atgtcgctcc acaaggtaaa cagttgattg aactgcctga actaccgcag 7800
   ccggagagcg ccgggcaact ctggctcaca gtacgcgtag tgcaaccgaa cgcgaccgca 7860
   tggtcagaag ccgggcacat cagcgcctgg cagcagtggc gtctggcgga aaacctcagt 7920
   gtgacgctcc ccgccgcgtc ccacgccatc ccgcatctga ccaccagcga aatggatttt 7980
   tgcatcgagc tgggtaataa gcgttggcaa tttaaccgcc agtcaggctt tctttcacag 8040
   atgtggattg gcgataaaaa acaactgctg acgccgctgc gcgatcagtt cacccgtgca 8100
   ccgctggata acgacattgg cgtaagtgaa gcgacccgca ttgaccctaa cgcctgggtc 8160
   gaacgctgga aggcggcggg ccattaccag gccgaagcag cgttgttgca gtgcacggca 8220
   gatacacttg ctgatgcggt gctgattacg accgctcacg cgtggcagca tcaggggaaa 8280
   accttattta tcagccggaa aacctaccgg attgatggta gtggtcaaat ggcgattacc 8340
   gttgatgttg aagtggcgag cgatacaccg catccggcgc ggattggcct gaactgccag 8400
   ctggcgcagg tagcagagcg ggtaaactgg ctcggattag ggccgcaaga aaactatccc 8460
   gaccgcctta ctgccgcctg ttttgaccgc tgggatctgc cattgtcaga catgtatacc 8520
   ccgtacgtct tcccgagcga aaacggtctg cgctgcggga cgcgcgaatt gaattatggc 8580
   ccacaccagt ggcgcggcga cttccagttc aacatcagcc gctacagtca acagcaactg 8640
   atggaaacca gccatcgcca tctgctgcac gcggaagaag gcacatggct gaatatcgac 8700
   ggtttccata tggggattgg tggcgacgac tcctggagcc cgtcagtatc ggcggaattc 8760
   cagctgagcg ccggtcgcta ccattaccag ttggtctggt gtcaaaaata ataataaccg 8820
   ggcagggggg atctttgtga aggaacctta cttctgtggt gtgacataat tggacaaact 8880
   acctacagag atttaaagct ctaaggtaaa tataaaattt ttaagtgtat aatgtgttaa 8940
   actactgatt ctaattgttt gtgtatttta gattccaacc tatggaactg atgaatggga 9000
   gcagtggtgg aatgccagat ccagacatga taagatacat tgatgagttt ggacaaacca 9060
   caactagaat gcagtgaaaa aaatgcttta tttgtgaaat ttgtgatgct attgctttat 9120
   ttgtaaccat tataagctgc aataaacaag ttaacaacaa caattgcatt cattttatgt 9180
   ttcaggttca gggggaggtg tgggaggttt tttaaagcaa gtaaaacctc tacaaatgtg 9240
   gtatggctga ttatgatctg cggccaaatc ggccggccta ggcgcgccgg taaccgaagt 9300
   tcctatactt tctagagaat aggaacttcg gaataggaac ttcaagctta agcgctagca 9360
   ctagaagatg ggcgggagtc ttctgggcag gcttaaaggc taacctggtg tgtgggcgtt 9420
   gtcctgcagg ggaattgaac aggtgtaaaa ttggagggac aagacttccc acagattttc 9480
   ggttttgtcg ggaagttttt taataggggc aaataaggaa aatgggagga taggtagtca 9540
   tctggggttt tatgcagcaa aactacaggt tattattgct tgtgatccgc ctcggagtat 9600
   tttccatcga ggtagattaa agacatgctc acccgagttt tatactctcc tgcttgagat 9660
   ccttactaca gtatgaaatt acagtgtcgc gagttagact atgtaagcag aattttaatc 9720
   atttttaaag agcccagtac ttcatatcca tttctcccgc tccttctgca gccttatcaa 9780
   aaggtatttt agaacactca ttttagcccc attttcattt attatactgg cttatccaac 9840
   ccctagacag agcattggca ttttcccttt cctgatctta gaagtctgat gactcatgaa 9900
   accagacaga ttagttacat acaccacaaa tcgaggctgt agctggggcc tcaacactgc 9960
   agttctttta taactcctta gtacactttt tgttgatcct ttgccttgat ccttaatttt 10020
   cagtgtctat cacctctccc gtcagtggtg ttccacattt gggcctattc tcagtccagg 10080
   gagttttaca acaatagatg tattgagaat ccaacctaaa gcttaacttt ccactcccat 10140
   gaatgcctct ctcctttttc tccatttata aactgagcta ttaaccatta atggttccag 10200
   gtggatgtct cctccccata ttacctgatg tatcttacat attgccaggc tgatatttta 10260
   agacattaaa aggtatattt cattattgag ccacatggta ttgattactg cttactaaaa 10320
   ttttgtcatt gtacacatct gtaaaaggtg gttccttttg gaatgcaaag ttcaggtgtt 10380
   tgttgtcttt cctgacctaa ggtcttgtga gcttgtattt tttctattta agcagtgctt 10440
   tctcttggac tggcttgact catggcattc tacacgttat tgctggtcta aatgtgattt 10500
   tgccaagctt cttcaggacc tataattttg cttgacttgt agccaaacac aagtaaaatg 10560
   attaagcaac aaatgtattt gtgaagcttg gtttttaggt tgttgtgttg tgtgtgcttg 10620
   tgctctataa taatactatc caggggctgg agaggtggct cggagttcaa gagcacagac 10680
   tgctcttcca gaagtcctga gttcaattcc cagcaaccac atggtggctc acaaccatct 10740
   gtaatgggat ctgatgccct cttctggtgt gtctgaagac cacaagtgta ttcacattaa 10800
   ataaataaat cctccttctt cttctttttt ttttttttaa agagaatact gtctccagta 10860
   gaatttactg aagtaatgaa atactttgtg tttgttccaa tatggtagcc aataatcaaa 10920
   ttactcttta agcactggaa atgttaccaa ggaactaatt tttatttgaa gtgtaactgt 10980
   ggacagagga gccataactg cagacttgtg ggatacagaa gaccaatgca gactttaatg 11040
   tcttttctct tacactaagc aataaagaaa taaaaattga acttctagta tcctatttgt 11100
   ttaaactgct agctttactt aacttttgtg cttcatctat acaaagctga aagctaagtc 11160
   tgcagccatt actaaacatg aaagcaagta atgataattt tggatttcaa aaatgtaggg 11220
   ccagagttta gccagccagt ggtggtgctt gcctttatgc ctttaatccc agcactctgg 11280
   aggcagagac aggcagatct ctgagtttga gcccagcctg gtctacacat caagttctat 11340
   ctaggatagc caggaataca cacagaaacc ctgttgggga ggggggctct gagatttcat 11400
   aaaattataa ttgaagcatt ccctaatgag ccactatgga tgtggctaaa tccgtctacc 11460
   tttctgatga gatttgggta ttattttttc tgtctctgct gttggttggg tcttttgaca 11520
   ctgtgggctt tctttaaagc ctccttcctg ccatgtggtc tcttgtttgc tactaacttc 11580
   ccatggctta aatggcatgg ctttttgcct tctaagggca gctgctgaga tttgcagcct 11640
   gatttccagg gtggggttgg gaaatctttc aaacactaaa attgtccttt aatttttttt 11700
   ttaaaaaatg ggttatataa taaacctcat aaaatagtta tgaggagtga ggtggactaa 11760
   tattaaatga gtccctcccc tataaaagag ctattaaggc tttttgtctt atacttaact 11820
   ttttttttaa atgtggtatc tttagaacca agggtcttag agttttagta tacagaaact 11880
   gttgcatcgc ttaatcagat tttctagttt caaatccaga gaatccaaat tcttcacagc 11940
   caaagtcaaa ttaagaattt ctgactttta atgttaattt gcttactgtg aatataaaaa 12000
   tgatagcttt tcctgaggca gggtctcact atgtatctct gcctgatctg caacaagata 12060
   tgtagactaa agttctgcct gcttttgtct cctgaatact aaggttaaaa tgtagtaata 12120
   cttttggaac ttgcaggtca gattctttta taggggacac actaagggag cttgggtgat 12180
   agttggtaaa atgtgtttca agtgatgaaa acttgaatta ttatcaccgc aacctacttt 12240
   ttaaaaaaaa aagccaggcc tgttagagca tgcttaaggg atccctagga cttgctgagc 12300
   acacaagagt agttacttgg caggctcctg gtgagagcat atttcaaaaa acaaggcaga 12360
   caaccaagaa actacagtta aggttacctg tctttaaacc atctgcatat acacagggat 12420
   attaaaatat tccaaataat atttcattca agttttcccc catcaaattg ggacatggat 12480
   ttctccggtg aataggcaga gttggaaact aaacaaatgt tggttttgtg atttgtgaaa 12540
   ttgttttcaa gtgatagtta aagcccatga gatacagaac aaagctgcta tttcgaggtc 12600
   tcttggttta tactcagaag cacttctttg ggtttccctg cactatcctg atcatgtgct 12660
   aggcctacct taggctgatt gttgttcaaa taaacttaag tttcctgtca ggtgatgtca 12720
   tatgatttca tatatcaagg caaaacatgt tatatatgtt aaacatttgt acttaatgtg 12780
   aaagttaggt ctttgtgggt ttgattttta attttcaaaa cctgagctaa ataagtcatt 12840
   tttacatgtc ttacatttgg tggaattgta taattgtggt ttgcaggcaa gactctctga 12900
   cctagtaacc ctacctatag agcactttgc tgggtcacaa gtctaggagt caagcatttc 12960
   accttgaagt tgagacgttt tgttagtgta tactagttta tatgttggag gacatgttta 13020
   tccagaagat attcaggact atttttgact gggctaagga attgattctg attagcactg 13080
   ttagtgagca ttgagtggcc tttaggcttg aattggagtc acttgtatat ctcaaataat 13140
   gctggccttt tttaaaaagc ccttgttctt tatcaccctg ttttctacat aatttttgtt 13200
   caaagaaata cttgtttgga tctccttttg acaacaatag catgttttca agccatattt 13260
   tttttccttt tttttttttt ttttggtttt tcgagacagg gtttctctgt atagccctgg 13320
   ctgtcctgga actcactttg tagaccaggc tggcctcgaa ctcagaaatc cgcctgcctc 13380
   tgcctcctga gtgccgggat taaaggcgtg caccaccacg cctggctaag ttggatattt 13440
   tgttatataa ctataaccaa tactaactcc actgggtgga tttttaattc agtcagtagt 13500
   cttaagtggt ctttattggc ccttcattaa aatctactgt tcactctaac agaggctgtt 13560
   ggtactagtg gcacttaagc aacttcctac ggatatacta gcagattaag ggtcagggat 13620
   agaaactagt ctagcgtttt gtatacctac cagctttata ctaccttgtt ctgatagaaa 13680
   tatttcagga catctagagt gtactataag gttgatggta agcttataag gaacttgaaa 13740
   gtggagtaac tactccattt ctctgagggg agaattaaaa tttttgacca agtgttgttg 13800
   agccactgag aatggtctca gaacataact tcttaaggaa ccttcccaga ttgccctcaa 13860
   cactgcacca catttggtcc tgcttgaaca ttgccatggc tcttaaagtc ttaattaaga 13920
   atattaattg tgtaattatt gtttttcctc ctttagatca ttccttgagg acaggacagt 13980
   gcttgtttaa ggctatattt ctgctgtctg agcagcaaca ggtcttcgag atcaacatga 14040
   tgttcataat cccaagatgt tgccatttat gttctcagaa gcaagcagag gcatgatggt 14100
   cagtgacagt aatgtcactg tgttaaatgt tgctatgcag tttggatttt tctaatgtag 14160
   tgtaggtaga acatatgtgt tctgtatgaa ttaaactctt aagttacacc ttgtataatc 14220
   catgcaatgt gttatgcaat taccatttta agtattgtag ctttctttgt atgtgaggat 14280
   aaaggtgttt gtcataaaat gttttgaaca tttccccaaa gttccaaatt ataaaaccac 14340
   aacgttagaa cttatttatg aacaatggtt gtagtttcat gcttttaaaa tgcttaatta 14400
   ttcaattaac accgtttgtg ttataatata tataaaactg acatgtagaa gtgtttgtcc 14460
   agaacatttc ttaaatgtat actgtcttta gagagtttaa tatagcatgt cttttgcaac 14520
   atactaactt ttgtgttggt gcgagcaata ttgtgtagtc attttgaaag gagtcatttc 14580
   aatgagtgtc agattgtttt gaatgttatt gaacatttta aatgcagact tgttcgtgtt 14640
   ttagaaagca aaactgtcag aagctttgaa ctagaaatta aaaagctgaa gtatttcaga 14700
   agggaaataa gctacttgct gtattagttg aaggaaagtg taatagctta gaaaatttaa 14760
   aaccatatag ttgtcattgc tgaatatctg gcagatgaaa agaaatactc agtggttctt 14820
   ttgagcaata taacagcttg ttatattaaa aattttcccc acagatataa actctaatct 14880
   ataactcata aatgttacaa atggatgaag cttacaaatg tggcttgact tgtcactgtg 14940
   cttgttttag ttatgtgaaa gtttggcaat aaacctatgt cctaaatagt caaactgtgg 15000
   aatgactttt taatctattg gtttgtctag aacagttatg ttgccatttg ccctaatggt 15060
   gaaagaaaaa gtggggagtg ccttggcact gttcatttgt ggtgtgaacc aaagaggggg 15120
   gcatgcactt acacttcaaa catccttttg aaagactgac aagtttgggt cttcacagtt 15180
   ggaattgggc atcccttttg tcagggaggg agggagggag ggaggctggc ttgttatgct 15240
   gacaagtgtg attaaattca aactttgagg taagttggag gaacttgtac attgttagga 15300
   gtgtgacaat ttggactctt aatgatttgg tcatacaaaa tgaacctaga ccaacttctg 15360
   gaagatgtat ataataactc catgttacat tgatttcacc tgactaatac ttatccctta 15420
   tcaattaaat acagaagatg ccagccatct gggcctttta acccagaaat ttagtttcaa 15480
   actcctaggt tagtgttctc actgagctac atcctgatct agtcctgaaa ataggaccac 15540
   catcaccccc aaaaaaatct caaataagat ttatgctagt gtttcaaaat tttaggaata 15600
   ggtaagatta gaaagtttta aattttgaga aatggcttct ctagaaagat gtacatagtg 15660
   aacactgaat ggctcctaaa gagcctagaa aactggtact gagcacacag gactgagagg 15720
   tctttcttga aaagcatgta ttgctttacg tgggtcacag aaggcaggca ggaagaactt 15780
   gggctgaaac tggtgtctta agtggctaac atcttcacaa ctgatgagca agaactttat 15840
   cctgatgcaa aaaccatcca aacaaactaa gtgaaaggtg gcaatggatc ccaggctgct 15900
   ctagaggagg acttgacttc tcatcccatc acccacacca gatagctcat agactgccaa 15960
   ttaacaccag cttctagcct ccacaggcac ctgcactggt acacataatt tcacacaaac 16020
   acagtaagaa gccttccacc tggcatggta ttgcttatct ttagttccca acacttggga 16080
   ggcagaggcc agccagggct atgtgacaaa aaccttgtct agaggagaaa cttcatagct 16140
   tatttcctat tcacgtaacc aggttagcaa aatttaccag ccagagatga agctaacagt 16200
   gtccactata tttgtagtgt tttaagtcaa ttttttaaat atacttaata gaattaaagc 16260
   tatggtgaac caagtacaaa cctggtgtat taacttgaga acttagcata aaaagtagtt 16320
   catttgttca gtaaatatta aatgcttact ggcaaagatt atgtcaggaa cttggtaaat 16380
   ggtgatgaaa caatcatagt tgtacatctt ggttctgtga tcaccttggt ttgaggtaaa 16440
   agtggttcct ttgatcaagg atggaatttt aagtttatat tcaatcaata atgtattatt 16500
   ttgtgattgc aaaattgcct atctagggta taaaaccttt aaaaatttca taataccagt 16560
   tcattctcca gttactaatt ccaaaaagcc actgactatg gtgccaatgt ggattctgtt 16620
   ctcaaaggaa ggattgtctg tgccctttat tctaatagaa acatcacact gaaaatctaa 16680
   gctgaaagaa gccagacttt cctaaataaa taactttcca taaagctcaa acaaggatta 16740
   cttttaggag gcactgttaa ggaactgata agtaatgagg ttacttatat aatgatagtc 16800
   ccacaagact atctgaggaa aaatcagtac aactcgaaaa cagaacaacc agctaggcag 16860
   gaataacagg gctcccaagt caggaggtct atccaacacc cttttctgtt gagggcccca 16920
   gacctacata ttgtatacaa acagggaggt gggtgatttt aactctcctg aggtaccttg 16980
   gtaaatcttt gtcctgagta agcagtacag tgtacagttt acattttcat ttaaagatac 17040
   attagctccc tctaccccct aagactgaca ggcactttgg gggtggggag ggctttggaa 17100
   aataacgctt ccatacacta aaagagaaat ttctttaatt aggcttgttg gttccataca 17160
   tctactggtg tttctactac ttagtaatat tataatagtc acacaagcat ctttgctctg 17220
   tttaggttgt atatttattt taaggcagat gataaaactg tagatcttaa gggatgcttc 17280
   tgcttctgag atgatacaaa gaatttagac cataaaacag taggttgcac aagcaataga 17340
   atatggccta aagtgttctg acacttagaa gccaagcagt gtaggcttct taagaaatac 17400
   cattacaatc accttgctag aaatcaagca ttctggagtg gtcaagcagt gtaacctgta 17460
   ctgtaagtta cttttctgct atttttctcc caaagcaagt tctttatgct gatatttcca 17520
   gtgttaggaa ctacaaatat taataagttg tcttcactct tttctttacc aaggagggtc 17580
   tcttccttca tcttgatctg aaggatgaac aaaggcttga gcagtgcgct ttagaagata 17640
   aactgcagca tgaaggcccc cgatgttcac ccagactaca tggacctttc gccacacatg 17700
   tcccattcca gataaggcct ggcacacaca aaaaacataa gtcattaggc taccagtctg 17760
   attctaaaac aacctaaaat cttcccactt aaatgctatg ggtggtgggt tggaaagttg 17820
   actcagaaaa tcacttgctg tttttagaga ggatctgggt tcagtttctg atacattgtg 17880
   gcttacaact ataactccag ttctaggggg tccatccaac atcctcttct gttgagggca 17940
   ccaaataaat gtattgtgta caaacaggga ggtgagtgat ttaactctcg tgtatagtac 18000
   cttggtaaaa catttcttgt cctgagtaag cagtacagct ctgcctgtcc ctggtctaca 18060
   gacacggctc atttcccgaa ggcaagctgg atagagattc caatttctct tcttggatcc 18120
   catcctataa aagaaggtca agtttaatct attgcaaaag gtaaataggt agtttcttac 18180
   atgagacaag aacaaatctt aggtgtgaag cagtcatctt ttacaggcca gagcctctat 18240
   tctatgccaa tgaaggaaac tgttagtcca gtgttataga gttagtccag tgtatagttt 18300
   tctatcagaa cacttttttt ttaaacaact gcaacttagc ttattgaaga caaaccacga 18360
   gtagaaatct gtccaagaag caagtgcttc tcagcctaca atgtggaata ggaccatgta 18420
   atggtacagt gagtgaaatg aattatggca tgtttttctg actgagaaga cagtacaata 18480
   aaaggtaaac tcatggtatt tatttaaaaa gaatccaatt tctacctttt tccaaatggc 18540
   atatctgtta caataatatc cacagaagca gttctcagtg ggaggttgca gatgacctcg 18600
   agggggggcc cggtacccag cttttgttcc ctttagtgag ggttaattgc gcgcttggcg 18660
   taatcatggt catagctgtt tcctgtgtga aattgttatc cgctcacaat tccacacaac 18720
   atacgagccg gaagcataaa gtgtaaagcc tggggtgcct aatgagtgag ctaactcaca 18780
   ttaattgcgt tgcgctcact gcccgctttc cagtcgggaa acctgtcgtg ccagctgcat 18840
   taatgaatcg gccaacgcgc ggggagaggc ggtttgcgta ttgggcgctc ttccgctcca 18900
   attcgcccta tagtgagtcg tattacaatt cactggccgt cgttttacaa cgtcgtgact 18960
   gggaaaaccc tggcgttacc caacttaatc gccttgcagc acatccccct ttcgccagct 19020
   ggcgtaatag cgaagaggcc cgcaccgatc gcccttccca acagttgcgc agcctgaatg 19080
   gcgaatggga cgcgccctgt agcggcgcat taagcgcggc gggtgtggtg gttacgcgca 19140
   gcgtgaccgc tacacttgcc agcgccctag cgcccgctcc tttcgctttc ttcccttcct 19200
   ttctcgccac gttcgccggc tttccccgtc aagctctaaa tcgggggctc cctttagggt 19260
   tccgatttag tgctttacgg cacctcgacc ccaaaaaact tgattagggt gatggttcac 19320
   gtagtgggcc atcgccctga tagacggttt ttcgcccttt gacgttggag tccacgttct 19380
   ttaatagtgg actcttgttc caaactggaa caacactcaa ccctatctcg gtctattctt 19440
   ttgatttata agggattttg ccgatttcgg cctattggtt aaaaaatgag ctgatttaac 19500
   aaaaatttaa cgcgaatttt aacaaaatat taacgcttac aatttaggtg gcacttttcg 19560
   gggaaatgtg cgcggaaccc ctatttgttt atttttctaa atacattcaa atatgtatcc 19620
   gctcatgaga caataaccct gataaatgct tcaataatat tgaaaaagga agagtatgag 19680
   tattcaacat ttccgtgtcg cccttattcc cttttttgcg gcattttgcc ttcctgtttt 19740
   tgctcaccca gaaacgctgg tgaaagtaaa agatgctgaa gatcagttgg gtgcacgagt 19800
   gggttacatc gaactggatc tcaacagcgg taagatcctt gagagttttc gccccgaaga 19860
   acgttttcca atgatgagca cttttaaagt tctgctatgt ggcgcggtat tatcccgtat 19920
   tgacgccggg caagagcaac tcggtcgccg catacactat tctcagaatg acttggttga 19980
   gtactcacca gtcacagaaa agcatcttac ggatggcatg acagtaagag aattatgcag 20040
   tgctgccata accatgagtg ataacactgc ggccaactta cttctgacaa cgatcggagg 20100
   accgaaggag ctaaccgctt ttttgcacaa catgggggat catgtaactc gccttgatcg 20160
   ttgggaaccg gagctgaatg aagccatacc aaacgacgag cgtgacacca cgatgcctgt 20220
   agcaatggca acaacgttgc gcaaactatt aactggcgaa ctacttactc tagcttcccg 20280
   gcaacaatta atagactgga tggaggcgga taaagttgca ggaccacttc tgcgctcggc 20340
   ccttccggct ggctggttta ttgctgataa atctggagcc ggtgagcgtg ggtctcgcgg 20400
   tatcattgca gcactggggc cagatggtaa gccctcccgt atcgtagtta tctacacgac 20460
   ggggagtcag gcaactatgg atgaacgaaa tagacagatc gctgagatag gtgcctcact 20520
   gattaagcat tggtaactgt cagaccaagt ttactcatat atactttaga ttgatttaaa 20580
   acttcatttt taatttaaaa ggatctaggt gaagatcctt tttgataatc tcatgaccaa 20640
   aatcccttaa cgtgagtttt cgttccactg agcgtcagac cccgtagaaa agatcaaagg 20700
   atcttcttga gatccttttt ttctgcgcgt aatctgctgc ttgcaaacaa aaaaaccacc 20760
   gctaccagcg gtggtttgtt tgccggatca agagctacca actctttttc cgaaggtaac 20820
   tggcttcagc agagcgcaga taccaaatac tgtccttcta gtgtagccgt agttaggcca 20880
   ccacttcaag aactctgtag caccgcctac atacctcgct ctgctaatcc tgttaccagt 20940
   ggctgctgcc agtggcgata agtcgtgtct taccgggttg gactcaagac gatagttacc 21000
   ggataaggcg cagcggtcgg gctgaacggg gggttcgtgc acacagccca gcttggagcg 21060
   aacgacctac accgaactga gatacctaca gcgtgagcta tgagaaagcg ccacgcttcc 21120
   cgaagggaga aaggcggaca ggtatccggt aagcggcagg gtcggaacag gagagcgcac 21180
   gagggagctt ccagggggaa acgcctggta tctttatagt cctgtcgggt ttcgccacct 21240
   ctgacttgag cgtcgatttt tgtgatgctc gtcagggggg cggagcctat ggaaaaacgc 21300
   cagcaacgcg gcctttttac ggttcctggc cttttgctgg ccttttgctc acatgttctt 21360
   tcctgcgtta tcccctgatt ctgtggataa ccgtattacc gcctttgagt gagctgatac 21420
   cgctcgccgc agccgaacga ccgagcgcag cgagtcagtg agcgaggaag cggaagagcg 21480
   cccaatacgc aaaccgcctc tccccgcgcg ttggccgatt cattaatgca gctggcacga 21540
   caggtttccc gactggaaag cgggcagtga gcgcaacgca attaatgtga gttagctcac 21600
   tcattaggca ccccaggctt tacactttat gcttccggct cgtatgttgt gtggaattgt 21660
   gagcggataa caatttcaca caggaaacag ctatgaccat gattacgcca agcgcgcaat 21720
   taaccctcac                                                        21730
<210> 10
   <211> 10491
   <212> DNA
   <213> Artificial Sequence
<220>
   <223> Description of Artificial Sequence: basic Rosa targeting vector

<400> 10
   tttgagtgag ctgataccgc tcgccgcagc cgaacgaccg agcgcagcga gtcagtgagc 60
   gaggaagcgg aagagcgccc aatacgcaaa ccgcctctcc ccgcgcgttg gccgattcat 120
   taatgcagct ggcacgacag gtttcccgac tggaaagcgg gcagtgagcg caacgcaatt 180
   aatgtgagtt agctcactca ttaggcaccc caggctttac actttatgct tccggctcgt 240
   atgttgtgtg gaattgtgag cggataacaa tttcacacag gaaacagcta tgaccatgat 300
   tacgccaagc gcgcaattaa ccctcactaa agggaacaaa agctgtcgag atctagatat 360
   cgatggccat agagttacgc tagggataac agggtaatat agccgcggca ggccctccga 420
   gcgtggtgga gccgttctgt gagacagccg ggtacgagtc gtgacgctgg aaggggcaag 480
   cgggtggtgg gcaggaatgc ggtccgccct gcagcaaccg gagggggagg gagaagggag 540
   cggaaaagtc tccaccggac gcggccatgg ctcggggggg ggggggcagc ggaggagcgc 600
   ttccggccga cgtctcgtcg ctgattggct tcttttcctc ccgccgtgtg tgaaaacaca 660
   aatggcgtgt tttggttggc gtaaggcgcc tgtcagttaa cggcagccgg agtgcgcagc 720
   cgccggcagc ctcgctctgc ccactgggtg gggcgggagg taggtggggt gaggcgagct 780
   ggacgtgcgg gcgcggtcgg cctctggcgg ggcgggggag gggagggagg gtcagcgaaa 840
   gtagctcgcg cgcgagcggc cgcccaccct ccccttcctc tgggggagtc gttttacccg 900
   ccgccggccg ggcctcgtcg tctgattggc tctcggggcc cagaaaactg gcccttgcca 960
   ttggctcgtg ttcgtgcaag ttgagtccat ccgccggcca gcgggggcgg cgaggaggcg 1020
   ctcccaggtt ccggccctcc cctcggcccc gcgccgcaga gtctggccgc gcgcccctgc 1080
   gcaacgtggc aggaagcgcg cgctgggggc ggggacgggc agtagggctg agcggctgcg 1140
   gggcgggtgc aagcacgttt ccgacttgag ttgcctcaag aggggcgtgc tgagccagac 1200
   ctccatcgcg cactccgggg agtggaggga aggagcgagg gctcagttgg gctgttttgg 1260
   aggcaggaag cacttgctct cccaaagtcg ctctgagttg ttatcagtaa gggagctgca 1320
   gtggagtagg cggggagaag gccgcaccct tctccggagg ggggagggga gtgttgcaat 1380
   acctttctgg gagttctctg ctgcctcctg gcttctgagg accgccctgg gcctgggaga 1440
   atcccttccc cctcttccct cgtgatctgc aactccagtc tttctaggta accgatatcc 1500
   ctgcaggggt gacctgcacg tctagggcgc agtagtccag ggtttccttg atgatgtcat 1560
   acttatcctg tccctttttt ttccacagct cgcggttgag gacaaactct tcgcggtctt 1620
   tccagtactc ctgcaggtga ctgactgagt cgacttaatt aaggccatag cggccattta 1680
   aatcggccgg cctaggcgcg ccggtaaccg aagttcctat actttctaga gaataggaac 1740
   ttcggaatag gaacttctta ggtcaattct accgggtagg ggaggcgctt ttcccaaggc 1800
   agtctggagc atgcgcttta gcagccccgc tgggcacttg gcgctacaca agtggcctct 1860
   ggcctcgcac acattccaca tccaccggta ggcgccaacc ggctccgttc tttggtggcc 1920
   ccttcgcgcc accttctact cctcccctag tcaggaagtt cccccccgcc ccgcagctcg 1980
   cgtcgtgcag gacgtgacaa atggaagtag cacgtctcac tagtctcgtg cagatggaca 2040
   gcaccgctga gcaatggaag cgggtaggcc tttggggcag cggccaatag cagctttgct 2100
   ccttcgcttt ctgggctcag aggctgggaa ggggtgggtc cgggggcggg ctcaggggcg 2160
   ggctcagggg cggggcgggc gcccgaaggt cctccggagg cccggcattc tgcacgcttc 2220
   aaaagcgcac gtctgccgcg ctgttctcct cttcctcatc tccgggcctt tcgacctgca 2280
   gccaatatgg gatcggccat tgaacaagat ggattgcacg caggttctcc ggccgcttgg 2340
   gtggagaggc tattcggcta tgactgggca caacagacaa tcggctgctc tgatgccgcc 2400
   gtgttccggc tgtcagcgca ggggcgcccg gttctttttg tcaagaccga cctgtccggt 2460
   gccctgaatg aactgcagga cgaggcagcg cggctatcgt ggctggccac gacgggcgtt 2520
   ccttgcgcag ctgtgctcga cgttgtcact gaagcgggaa gggactggct gctattgggc 2580
   gaagtgccgg ggcaggatct cctgtcatct caccttgctc ctgccgagaa agtatccatc 2640
   atggctgatg caatgcggcg gctgcatacg cttgatccgg ctacctgccc attcgaccac 2700
   caagcgaaac atcgcatcga gcgagcacgt actcggatgg aagccggtct tgtcgatcag 2760
   gatgatctgg acgaagagca tcaggggctc gcgccagccg aactgttcgc caggctcaag 2820
   gcgcgcatgc ccgacggcga ggatctcgtc gtgacccatg gcgatgcctg cttgccgaat 2880
   atcatggtgg aaaatggccg cttttctgga ttcatcgact gtggccggct gggtgtggcg 2940
   gaccgctatc aggacatagc gttggctacc cgtgatattg ctgaagagct tggcggcgaa 3000
   tgggctgacc gcttcctcgt gctttacggt atcgccgctc ccgattcgca gcgcatcgcc 3060
   ttctatcgcc ttcttgacga gttcttctga ggggatcgat ccgctgtaag tctgcagaaa 3120
   ttgatgatct attaaacaat aaagatgtcc actaaaatgg aagtttttcc tgtcatactt 3180
   tgttaagaag ggtgagaaca gagtacctac attttgaatg gaaggattgg agctacgggg 3240
   gtgggggtgg ggtgggatta gataaatgcc tgctctttac tgaaggctct ttactattgc 3300
   tttatgataa tgtttcatag ttggatatca taatttaaac aagcaaaacc aaattaaggg 3360
   ccagctcatt cctcccactc atgatctata gatctataga tctctcgtgg gatcattgtt 3420
   tttctcttga ttcccacttt gtggttctaa gtactgtggt ttccaaatgt gtcagtttca 3480
   tagcctgaag aacgagatca gcagcctctg ttccacatac acttcattct cagtattgtt 3540
   ttgccaagtt ctaattccat cagaagctga ctctagatcc cgcgccgaag ttcctatact 3600
   ttctagagaa taggaacttc ggaataggaa cttcaagctt aagcgctaga agatgggcgg 3660
   gagtcttctg ggcaggctta aaggctaacc tggtgtgtgg gcgttgtcct gcaggggaat 3720
   tgaacaggtg taaaattgga gggacaagac ttcccacaga ttttcggttt tgtcgggaag 3780
   ttttttaata ggggcaaata aggaaaatgg gaggataggt agtcatctgg ggttttatgc 3840
   agcaaaacta caggttatta ttgcttgtga tccgcctcgg agtattttcc atcgaggtag 3900
   attaaagaca tgctcacccg agttttatac tctcctgctt gagatcctta ctacagtatg 3960
   aaattacagt gtcgcgagtt agactatgta agcagaattt taatcatttt taaagagccc 4020
   agtacttcat atccatttct cccgctcctt ctgcagcctt atcaaaaggt attttagaac 4080
   actcatttta gccccatttt catttattat actggcttat ccaaccccta gacagagcat 4140
   tggcattttc cctttcctga tcttagaagt ctgatgactc atgaaaccag acagattagt 4200
   tacatacacc acaaatcgag gctgtagctg gggcctcaac actgcagttc ttttataact 4260
   ccttagtaca ctttttgttg atcctttgcc ttgatcctta attttcagtg tctatcacct 4320
   ctcccgtcag tggtgttcca catttgggcc tattctcagt ccagggagtt ttacaacaat 4380
   agatgtattg agaatccaac ctaaagctta actttccact cccatgaatg cctctctcct 4440
   ttttctccat ttataaactg agctattaac cattaatggt tccaggtgga tgtctcctcc 4500
   ccatattacc tgatgtatct tacatattgc caggctgata ttttaagaca ttaaaaggta 4560
   tatttcatta ttgagccaca tggtattgat tactgcttac taaaattttg tcattgtaca 4620
   catctgtaaa aggtggttcc ttttggaatg caaagttcag gtgtttgttg tctttcctga 4680
   cctaaggtct tgtgagcttg tattttttct atttaagcag tgctttctct tggactggct 4740
   tgactcatgg cattctacac gttattgctg gtctaaatgt gattttgcca agcttcttca 4800
   ggacctataa ttttgcttga cttgtagcca aacacaagta aaatgattaa gcaacaaatg 4860
   tatttgtgaa gcttggtttt taggttgttg tgttgtgtgt gcttgtgctc tataataata 4920
   ctatccaggg gctggagagg tggctcggag ttcaagagca cagactgctc ttccagaagt 4980
   cctgagttca attcccagca accacatggt ggctcacaac catctgtaat gggatctgat 5040
   gccctcttct ggtgtgtctg aagaccacaa gtgtattcac attaaataaa taaatcctcc 5100
   ttcttcttct tttttttttt tttaaagaga atactgtctc cagtagaatt tactgaagta 5160
   atgaaatact ttgtgtttgt tccaatatgg tagccaataa tcaaattact ctttaagcac 5220
   tggaaatgtt accaaggaac taatttttat ttgaagtgta actgtggaca gaggagccat 5280
   aactgcagac ttgtgggata cagaagacca atgcagactt taatgtcttt tctcttacac 5340
   taagcaataa agaaataaaa attgaacttc tagtatccta tttgtttaaa ctgctagctt 5400
   tacttaactt ttgtgcttca tctatacaaa gctgaaagct aagtctgcag ccattactaa 5460
   acatgaaagc aagtaatgat aattttggat ttcaaaaatg tagggccaga gtttagccag 5520
   ccagtggtgg tgcttgcctt tatgccttta atcccagcac tctggaggca gagacaggca 5580
   gatctctgag tttgagccca gcctggtcta cacatcaagt tctatctagg atagccagga 5640
   atacacacag aaaccctgtt ggggaggggg gctctgagat ttcataaaat tataattgaa 5700
   gcattcccta atgagccact atggatgtgg ctaaatccgt ctacctttct gatgagattt 5760
   gggtattatt ttttctgtct ctgctgttgg ttgggtcttt tgacactgtg ggctttcttt 5820
   aaagcctcct tcctgccatg tggtctcttg tttgctacta acttcccatg gcttaaatgg 5880
   catggctttt tgccttctaa gggcagctgc tgagatttgc agcctgattt ccagggtggg 5940
   gttgggaaat ctttcaaaca ctaaaattgt cctttaattt tttttttaaa aaatgggtta 6000
   tataataaac ctcataaaat agttatgagg agtgaggtgg actaatatta aatgagtccc 6060
   tcccctataa aagagctatt aaggcttttt gtcttatact taactttttt tttaaatgtg 6120
   gtatctttag aaccaagggt cttagagttt tagtatacag aaactgttgc atcgcttaat 6180
   cagattttct agtttcaaat ccagagaatc caaattcttc acagccaaag tcaaattaag 6240
   aatttctgac ttttaatgtt aatttgctta ctgtgaatat aaaaatgata gcttttcctg 6300
   aggcagggtc tcactatgta tctctgcctg atctgcaaca agatatgtag actaaagttc 6360
   tgcctgcttt tgtctcctga atactaaggt taaaatgtag taatactttt ggaacttgca 6420
   ggtcagattc ttttataggg gacacactaa gggagcttgg gtgatagttg gtaaaatgtg 6480
   tttcaagtga tgaaaacttg aattattatc accgcaacct actttttaaa aaaaaaagcc 6540
   aggcctgtta gagcatgctt aagggatccc taggacttgc tgagcacaca agagtagtta 6600
   cttggcaggc tcctggtgag agcatatttc aaaaaacaag gcagacaacc aagaaactac 6660
   agttaaggtt acctgtcttt aaaccatctg catatacaca gggatattaa aatattccaa 6720
   ataatatttc attcaagttt tcccccatca aattgggaca tggatttctc cggtgaatag 6780
   gcagagttgg aaactaaaca aatgttggtt ttgtgatttg tgaaattgtt ttcaagtgat 6840
   agttaaagcc catgagatac agaacaaagc tgctatttcg aggtctcttg gtttatactc 6900
   agaagcactt ctttgggttt ccctgcacta tcctgatcat gtgctaggcc taccttaggc 6960
   tgattgttgt tcaaataaac ttaagtttcc tgtcaggtga tgtcatatga tttcatatat 7020
   caaggcaaaa catgttatat atgttaaaca tttgtactta atgtgaaagt taggtctttg 7080
   tgggtttgat ttttaatttt caaaacctga gctaaataag tcatttttac atgtcttaca 7140
   tttggtggaa ttgtataatt gtggtttgca ggcaagactc tctgacctag taaccctacc 7200
   tatagagcac tttgctgggt cacaagtcta ggagtcaagc atttcacctt gaagttgaga 7260
   cgttttgtta gtgtatacta gtttatatgt tggaggacat gtttatccag aagatattca 7320
   ggactatttt tgactgggct aaggaattga ttctgattag cactgttagt gagcattgag 7380
   tggcctttag gcttgaattg gagtcacttg tatatctcaa ataatgctgg ccttttttaa 7440
   aaagcccttg ttctttatca ccctgttttc tacataattt ttgttcaaag aaatacttgt 7500
   ttggatctcc ttttgacaac aatagcatgt tttcaagcca tatttttttt cctttttttt 7560
   tttttttttg gtttttcgag acagggtttc tctgtatagc cctggctgtc ctggaactca 7620
   ctttgtagac caggctggcc tcgaactcag aaatccgcct gcctctgcct cctgagtgcc 7680
   gggattaaag gcgtgcacca ccacgcctgg ctaagttgga tattttgtta tataactata 7740
   accaatacta actccactgg gtggattttt aattcagtca gtagtcttaa gtggtcttta 7800
   ttggcccttc attaaaatct actgttcact ctaacagagg ctgttggtac tagtggcact 7860
   taagcaactt cctacggata tactagcaga ttaagggtca gggatagaaa ctagtctagc 7920
   gttttgtata cctaccagct ttatactacc ttgttctgat agaaatattt caggacatct 7980
   agcacccaat tcgccctata gtgagtcgta ttacaattca ctggccgtcg ttttacaacg 8040
   tcgtgactgg gaaaaccctg gcgttaccca acttaatcgc cttgcagcac atcccccttt 8100
   cgccagctgg cgtaatagcg aagaggcccg caccgatcgc ccttcccaac agttgcgcag 8160
   cctgaatggc gaatgggacg cgccctgtag cggcgcatta agcgcggcgg gtgtggtggt 8220
   tacgcgcagc gtgaccgcta cacttgccag cgccctagcg cccgctcctt tcgctttctt 8280
   cccttccttt ctcgccacgt tcgccggctt tccccgtcaa gctctaaatc gggggctccc 8340
   tttagggttc cgatttagtg ctttacggca cctcgacccc aaaaaacttg attagggtga 8400
   tggttcacgt agtgggccat cgccctgata gacggttttt cgccctttga cgttggagtc 8460
   cacgttcttt aatagtggac tcttgttcca aactggaaca acactcaacc ctatctcggt 8520
   ctattctttt gatttataag ggattttgcc gatttcggcc tattggttaa aaaatgagct 8580
   gatttaacaa aaatttaacg cgaattttaa caaaatatta acgcttacaa tttaggtggc 8640
   acttttcggg gaaatgtgcg cggaacccct atttgtttat ttttctaaat acattcaaat 8700
   atgtatccgc tcatgagaca ataaccctga taaatgcttc aataatattg aaaaaggaag 8760
   agtatgagta ttcaacattt ccgtgtcgcc cttattccct tttttgcggc attttgcctt 8820
   cctgtttttg ctcacccaga aacgctggtg aaagtaaaag atgctgaaga tcagttgggt 8880
   gcacgagtgg gttacatcga actggatctc aacagcggta agatccttga gagttttcgc 8940
   cccgaagaac gttttccaat gatgagcact tttaaagttc tgctatgtgg cgcggtatta 9000
   tcccgtattg acgccgggca agagcaactc ggtcgccgca tacactattc tcagaatgac 9060
   ttggttgagt actcaccagt cacagaaaag catcttacgg atggcatgac agtaagagaa 9120
   ttatgcagtg ctgccataac catgagtgat aacactgcgg ccaacttact tctgacaacg 9180
   atcggaggac cgaaggagct aaccgctttt ttgcacaaca tgggggatca tgtaactcgc 9240
   cttgatcgtt gggaaccgga gctgaatgaa gccataccaa acgacgagcg tgacaccacg 9300
   atgcctgtag caatggcaac aacgttgcgc aaactattaa ctggcgaact acttactcta 9360
   gcttcccggc aacaattaat agactggatg gaggcggata aagttgcagg accacttctg 9420
   cgctcggccc ttccggctgg ctggtttatt gctgataaat ctggagccgg tgagcgtggg 9480
   tctcgcggta tcattgcagc actggggcca gatggtaagc cctcccgtat cgtagttatc 9540
   tacacgacgg ggagtcaggc aactatggat gaacgaaata gacagatcgc tgagataggt 9600
   gcctcactga ttaagcattg gtaactgtca gaccaagttt actcatatat actttagatt 9660
   gatttaaaac ttcattttta atttaaaagg atctaggtga agatcctttt tgataatctc 9720
   atgaccaaaa tcccttaacg tgagttttcg ttccactgag cgtcagaccc cgtagaaaag 9780
   atcaaaggat cttcttgaga tccttttttt ctgcgcgtaa tctgctgctt gcaaacaaaa 9840
   aaaccaccgc taccagcggt ggtttgtttg ccggatcaag agctaccaac tctttttccg 9900
   aaggtaactg gcttcagcag agcgcagata ccaaatactg tccttctagt gtagccgtag 9960
   ttaggccacc acttcaagaa ctctgtagca ccgcctacat acctcgctct gctaatcctg 10020
   ttaccagtgg ctgctgccag tggcgataag tcgtgtctta ccgggttgga ctcaagacga 10080
   tagttaccgg ataaggcgca gcggtcgggc tgaacggggg gttcgtgcac acagcccagc 10140
   ttggagcgaa cgacctacac cgaactgaga tacctacagc gtgagctatg agaaagcgcc 10200
   acgcttcccg aagggagaaa ggcggacagg tatccggtaa gcggcagggt cggaacagga 10260
   gagcgcacga gggagcttcc agggggaaac gcctggtatc tttatagtcc tgtcgggttt 10320
   cgccacctct gacttgagcg tcgatttttg tgatgctcgt caggggggcg gagcctatgg 10380
   aaaaacgcca gcaacgcggc ctttttacgg ttcctggcct tttgctggcc ttttgctcac 10440
   atgttctttc ctgcgttatc ccctgattct gtggataacc gtattaccgc c 10491
<210> 11
   <211> 15200
   <212> DNA
   <213> Artificial Sequence
<220>
   <223> Description of Artificial Sequence: Rosa26 targeting vector
<400> 11
   ttacgctagg gataacaggg taatatagcc gcggcaggcc ctccgagcgt ggtggagccg 60
   ttctgtgaga cagccgggta cgagtcgtga cgctggaagg ggcaagcggg tggtgggcag 120
   gaatgcggtc cgccctgcag caaccggagg gggagggaga agggagcgga aaagtctcca 180
   ccggacgcgg ccatggctcg gggggggggg ggcagcggag gagcgcttcc ggccgacgtc 240
   tcgtcgctga ttggcttctt ttcctcccgc cgtgtgtgaa aacacaaatg gcgtgttttg 300
   gttggcgtaa ggcgcctgtc agttaacggc agccggagtg cgcagccgcc ggcagcctcg 360
   ctctgcccac tgggtggggc gggaggtagg tggggtgagg cgagctggac gtgcgggcgc 420
   ggtcggcctc tggcggggcg ggggagggga gggagggtca gcgaaagtag ctcgcgcgcg 480
   agcggccgcc caccctcccc ttcctctggg ggagtcgttt tacccgccgc cggccgggcc 540
   tcgtcgtctg attggctctc ggggcccaga aaactggccc ttgccattgg ctcgtgttcg 600
   tgcaagttga gtccatccgc cggccagcgg gggcggcgag gaggcgctcc caggttccgg 660
   ccctcccctc ggccccgcgc cgcagagtct ggccgcgcgc ccctgcgcaa cgtggcagga 720
   agcgcgcgct gggggcgggg acgggcagta gggctgagcg gctgcggggc gggtgcaagc 780
   acgtttccga cttgagttgc ctcaagaggg gcgtgctgag ccagacctcc atcgcgcact 840
   ccggggagtg gagggaagga gcgagggctc agttgggctg ttttggaggc aggaagcact 900
   tgctctccca aagtcgctct gagttgttat cagtaaggga gctgcagtgg agtaggcggg 960
   gagaaggccg cacccttctc cggagggggg aggggagtgt tgcaatacct ttctgggagt 1020
   tctctgctgc ctcctggctt ctgaggaccg ccctgggcct gggagaatcc cttccccctc 1080
   ttccctcgtg atctgcaact ccagtctttc taggtaaccg atatccctgc aggggtgacc 1140
   tgcacgtcta gggcgcagta gtccagggtt tccttgatga tgtcatactt atcctgtccc 1200
   ttttttttcc acagctcgcg gttgaggaca aactcttcgc ggtctttcca gtactcctgc 1260
   aggtgactga ctgagtcgac gccaccatgg agaagttcct attccgaagt tcctattctt 1320
   caaatagtat aggaacttcg ctcgagctca agcttcgaat tctgcagtcg acggtaccgc 1380
   gggcccggga tccaccggtc gccaccatgg cccagtccaa gcacggcctg accaaggaga 1440
   tgaccatgaa gtaccgcatg gagggctgcg tggacggcca caagttcgtg atcaccggcg 1500
   agggcatcgg ctaccccttc aagggcaagc aggccatcaa cctgtgcgtg gtggagggcg 1560
   gccccttgcc cttcgccgag gacatcttgt ccgccgcctt catgtacggc aaccgcgtgt 1620
   tcaccgagta cccccaggac atcgtcgact acttcaagaa ctcctgcccc gccggctaca 1680
   cctgggaccg ctccttcctg ttcgaggacg gcgccgtgtg catctgcaac gccgacatca 1740
   ccgtgagcgt ggaggagaac tgcatgtacc acgagtccaa gttctacggc gtgaacttcc 1800
   ccgccgacgg ccccgtgatg aagaagatga ccgacaactg ggagccctcc tgcgagaaga 1860
   tcatccccgt gcccaagcag ggcatcttga agggcgacgt gagcatgtac ctgctgctga 1920
   aggacggtgg ccgcttgcgc tgccagttcg acaccgtgta caaggccaag tccgtgcccc 1980
   gcaagatgcc cgactggcac ttcatccagc acaagctgac ccgcgaggac cgcagcgacg 2040
   ccaagaacca gaagtggcac ctgaccgagc acgccatcgc ctccggctcc gccttgccct 2100
   gagcggccga ccggttcgag atccaggcgc ggatcaataa aagatcatta ttttcaatag 2160
   atctgtgtgt tggttttttg tgtgccttgg gggaggggga ggccagaatg aggcgcggcc 2220
   aagggggagg gggaggccag aatgaccttg ggggaggggg aggccagaat gaccttgggg 2280
   gagggggagg ccagaatgag gcgcgtatgc aggtcaattc taccgggtag gggaggcgct 2340
   tttcccaagg cagtctggag catgcgcttt agcagccccg ctggcacttg gcgctacaca 2400
   agtggcctct ggcctcgcac acattccaca tccaccggta gcgccaaccg gctccgttct 2460
   ttggtggccc cttcgcgcca ccttctactc ctcccctagt caggaagttc ccccccgccc 2520
   cgcagctcgc gtcgtgcagg acgtgacaaa tggaagtagc acgtctcact agtctcgtgc 2580
   agatggacag caccgctgag caatggaagc gggtaggcct ttggggcagc ggccaatagc 2640
   agctttgctc cttcgctttc tgggctcaga ggctgggaag gggtgggtcc gggggcgggc 2700
   tcaggggcgg gctcaggggc ggggcgggcg cgaaggtcct cccgaggccc ggcattctcg 2760
   cacgcttcaa aagcgcacgt ctgccgcgct gttctcctct tcctcatctc cgggcctttc 2820
   gacgatccag ccgccaccat gaaaaagcct gaactcaccg cgacgtctgt cgagaagttt 2880
   ctgatcgaaa agttcgacag cgtctccgac ctgatgcagc tctcggaggg cgaagaatct 2940
   cgtgctttca gcttcgatgt aggagggcgt ggatatgtcc tgcgggtaaa tagctgcgcc 3000
   gatggtttct acaaagatcg ttatgtttat cggcactttg catcggccgc gctcccgatt 3060
   ccggaagtgc ttgacattgg ggaattcagc gagagcctga cctattgcat ctcccgccgt 3120
   gcacagggtg tcacgttgca agacctgcct gaaaccgaac tgcccgctgt tctgcagccg 3180
   gtcgcggagg ccatggatgc catcgctgcg gccgatctta gccagacgag cgggttcggc 3240
   ccattcggac cgcaaggaat cggtcaatac actacatggc gtgatttcat atgcgcgatt 3300
   gctgatcccc atgtgtatca ctggcaaact gtgatggacg acaccgtcag tgcgtccgtc 3360
   gcgcaggctc tcgatgagct gatgctttgg gccgaggact gccccgaagt ccggcacctc 3420
   gtgcacgcgg atttcggctc caacaatgtc ctgacggaca atggccgcat aacagcggtc 3480
   attgactgga gcgaggcgat gttcggggat tcccaatacg aggtcgccaa catcttcttc 3540
   tggaggccgt ggttggcttg tatggagcag cagacgcgct acttcgagcg gaggcatccg 3600
   gagcttgcag gatcgccgcg gctccgggcg tatatgctcc gcattggtct tgaccaactc 3660
   tatcagagct tggttgacgg caatttcgat gatgcagctt gggcgcaggg tcgatgcgac 3720
   gcaatcgtcc gatccggagc cgggactgtc gggcgtacac aaatcgcccg cagaagcgcg 3780
   gccgtctgga ccgatggctg tgtagaagta ctcgccgata gtggaaaccg acgccccagc 3840
   actcgtccga gggcaaagga atagtcgatg cagaaattga tgatctatta aacaataaag 3900
   atgtccacta aaatggaagt ttttcctgtc atactttgtt aagaagggtg agaacagagt 3960
   acctacattt tgaatggaag gattggagct acgggggtgg gggtggggtg ggattagata 4020
   aatgcctgct ctttactgaa ggctctttac tattgcttta tgataatgtt tcatagttgg 4080
   atatcataat ttaaacaagc aaaaccaaat taagggccag ctcattcctc ccactcatga 4140
   tctatagatc tatagatctc tcgtgggatc attgtttttc tcttgattcc cactttgtgg 4200
   ttctaagtac tgtggtttcc aaatgtgtca gtttcatagc ctgaagaacg agatcagcag 4260
   cctctgttcc acatacactt cattctcagt attgttttgc caagttctaa ttccatcaga 4320
   agctgactct agatcctgca ttaattaagg ccatagcggc catttgggct agcttgcatg 4380
   cctgcaggtt ttcgacattg attattgact agttattaat agtaatcaat tacggggtca 4440
   ttagttcata gcccatatat ggagttccgc gttacataac ttacggtaaa tggcccgcct 4500
   ggctgaccgc ccaacgaccc ccgcccattg acgtcaataa tgacgtatgt tcccatagta 4560
   acgccaatag ggactttcca ttgacgtcaa tgggtggact atttacggta aactgcccac 4620
   ttggcagtac atcaagtgta tcatatgcca agtacgcccc ctattgacgt caatgacggt 4680
   aaatggcccg cctggcatta tgcccagtac atgaccttat gggactttcc tacttggcag 4740
   tacatctacg tattagtcat cgctattacc atgggtcgag gtgagcccca cgttctgctt 4800
   cactctcccc atctcccccc cctccccacc cccaattttg tatttattta ttttttaatt 4860
   attttgtgca gcgatggggg cggggggggg gggggcgcgc gccaggcggg gcggggcggg 4920
   gcgaggggcg gggcggggcg aggcggagag gtgcggcggc agccaatcag agcggcgcgc 4980
   tccgaaagtt tccttttatg gcgaggcggc ggcggcggcg gccctataaa aagcgaagcg 5040
   cgcggcgggc gggagtcgct gcgttgcctt cgccccgtgc cccgctccgc gccgcctcgc 5100
   gccgcccgcc ccggctctga ctgaccgcgt tactcccaca ggtgagcggg cgggacggcc 5160
   cttctcctcc gggctgtaat tagcgcttgg tttaatgacg gctcgtttct tttctgtggc 5220
   tgcgtgaaag ccttaaaggg ctccgggagg gccctttgtg cgggggggag cggctcgggg 5280
   ggtgcgtgcg tgtgtgtgtg cgtggggagc gccgcgtgcg gcccgcgctg cccggcggct 5340
   gtgagcgctg cgggcgcggc gcggggcttt gtgcgctccg cgtgtgcgcg aggggagcgc 5400
   ggccgggggc ggtgccccgc ggtgcggggg ggctgcgagg ggaacaaagg ctgcgtgcgg 5460
   ggtgtgtgcg tgggggggtg agcagggggt gtgggcgcgg cggtcgggct gtaacccccc 5520
   cctgcacccc cctccccgag ttgctgagca cggcccggct tcgggtgcgg ggctccgtgc 5580
   ggggcgtggc gcggggctcg ccgtgccggg cggggggtgg cggcaggtgg gggtgccggg 5640
   cggggcgggg ccgcctcggg ccggggaggg ctcgggggag ggcgcggcgg gccccggagc 5700
   gccggcggct gtcgaggcgc ggcgagccgc agccattgcc ttttatggta atcgtgcgag 5760
   agggcgcagg gacttccttt gtcccaaatc tggcggagcc gaaatctggg aggcgccgcc 5820
   gcaccccctc tagcgggcgc gggcgaagcg gtgcggcgcc ggcaggaagg aaatgggcgg 5880
   ggagggcctt cgtgcgtcgc cgcgccgccg tccccttctc catctccagc ctcggggctg 5940
   ccgcaggggg acggctgcct tcggggggga cggggcaggg cggggttcgg cttctggcgt 6000
   gtgaccggcg gctctagtaa gcgttggggt gagtactccc tctcaaaagc gggcatgact 6060
   tctgcgctaa gattgtcagt ttccaaaaac gaggaggatt tgatattcac ctggcccgcg 6120
   gtgatgcctt tgagggtggc cgcgtccatc tggtcagaaa agacaatctt tttgttgtca 6180
   agcttgaggt gtggcaggct tgagatctgg ccatacactt gagtgacatt gacatccact 6240
   ttgcctttct ctccacaggt gtccactccc agggcggccc tagagcctct gctaaccatg 6300
   ttcatgcctt cttctttttc ctacagctcc tgggcaacgt gctggttgtt gtgctgtctc 6360
   atcattttgg caaagaatta cccgccgcca ccatggctcc caagaagaag aggaaggtga 6420
   tgagtcaatt tgatatatta tgtaaaacac cacctaaggt cctggttcgt cagtttgtgg 6480
   aaaggtttga aagaccttca ggggaaaaaa tagcatcatg tgctgctgaa ctaacctatt 6540
   tatgttggat gattactcat aacggaacag caatcaagag agccacattc atgagctata 6600
   atactatcat aagcaattcg ctgagtttcg atattgtcaa caaatcactc cagtttaaat 6660
   acaagacgca aaaagcaaca attctggaag cctcattaaa gaaattaatt cctgcttggg 6720
   aatttacaat tattccttac aatggacaaa aacatcaatc tgatatcact gatattgtaa 6780
   gtagtttgca attacagttc gaatcatcgg aagaagcaga taagggaaat agccacagta 6840
   aaaaaatgct taaagcactt ctaagtgagg gtgaaagcat ctgggagatc actgagaaaa 6900
   tactaaattc gtttgagtat acctcgagat ttacaaaaac aaaaacttta taccaattcc 6960
   tcttcctagc tactttcatc aattgtggaa gattcagcga tattaagaac gttgatccga 7020
   aatcatttaa attagtccaa aataagtatc tgggagtaat aatccagtgt ttagtgacag 7080
   agacaaagac aagcgttagt aggcacatat acttctttag cgcaaggggt aggatcgatc 7140
   cacttgtata tttggatgaa tttttgagga attctgaacc agtcctaaaa cgagtaaata 7200
   ggaccggcaa ttcttcaagc aacaaacagg aataccaatt attaaaagat aacttagtca 7260
   gatcgtacaa caaggctttg aagaaaaatg cgccttatcc aatctttgct ataaagaatg 7320
   gcccaaaatc tcacattgga agacatttga tgacctcatt tctgtcaatg aagggcctaa 7380
   cggagttgac taatgttgtg ggaaattgga gcgataagcg tgcttctgcc gtggccagga 7440
   caacgtatac tcatcagata acagcaatac ctgatcacta cttcgcacta gtttctcggt 7500
   actatgcata tgatccaata tcaaaggaaa tgatagcatt gaaggatgag actaatccaa 7560
   ttgaggagtg gcagcatata gaacagctaa agggtagtgc tgaaggaagc atacgatacc 7620
   ccgcatggaa tgggataata tcacaggagg tactagacta cctttcatcc tacataaata 7680
   gacgcatata atgatctaga gtcgagttag gccgcgtcga cctcgagatc caggcgcgga 7740
   tcaataaaag atcattattt tcaatagatc tgtgtgttgg ttttttgtgt gccttggggg 7800
   agggggaggc cagaatgagg cgcggccaag gggagggggg aggccagaat gaccttgggg 7860
   gagggggagg ccagaatgac cttgggggag ggggaggcca gaatgaggcg cgccggtaac 7920
   cgaagttcct atactttcta gagaatagga acttcggaat aggaacttca agcttaagcg 7980
   ctagaagatg ggcgggagtc ttctgggcag gcttaaaggc taacctggtg tgtgggcgtt 8040
   gtcctgcagg ggaattgaac aggtgtaaaa ttggagggac aagacttccc acagattttc 8100
   ggttttgtcg ggaagttttt taataggggc aaataaggaa aatgggagga taggtagtca 8160
   tctggggttt tatgcagcaa aactacaggt tattattgct tgtgatccgc ctcggagtat 8220
   tttccatcga ggtagattaa agacatgctc acccgagttt tatactctcc tgcttgagat 8280
   ccttactaca gtatgaaatt acagtgtcgc gagttagact atgtaagcag aattttaatc 8340
   atttttaaag agcccagtac ttcatatcca tttctcccgc tccttctgca gccttatcaa 8400
   aaggtatttt agaacactca ttttagcccc attttcattt attatactgg cttatccaac 8460
   ccctagacag agcattggca ttttcccttt cctgatctta gaagtctgat gactcatgaa 8520
   accagacaga ttagttacat acaccacaaa tcgaggctgt agctggggcc tcaacactgc 8580
   agttctttta taactcctta gtacactttt tgttgatcct ttgccttgat ccttaatttt 8640
   cagtgtctat cacctctccc gtcagtggtg ttccacattt gggcctattc tcagtccagg 8700
   gagttttaca acaatagatg tattgagaat ccaacctaaa gcttaacttt ccactcccat 8760
   gaatgcctct ctcctttttc tccatttata aactgagcta ttaaccatta atggttccag 8820
   gtggatgtct cctccccata ttacctgatg tatcttacat attgccaggc tgatatttta 8880
   agacattaaa aggtatattt cattattgag ccacatggta ttgattactg cttactaaaa 8940
   ttttgtcatt gtacacatct gtaaaaggtg gttccttttg gaatgcaaag ttcaggtgtt 9000
   tgttgtcttt cctgacctaa ggtcttgtga gcttgtattt tttctattta agcagtgctt 9060
   tctcttggac tggcttgact catggcattc tacacgttat tgctggtcta aatgtgattt 9120
   tgccaagctt cttcaggacc tataattttg cttgacttgt agccaaacac aagtaaaatg 9180
   attaagcaac aaatgtattt gtgaagcttg gtttttaggt tgttgtgttg tgtgtgcttg 9240
   tgctctataa taatactatc caggggctgg agaggtggct cggagttcaa gagcacagac 9300
   tgctcttcca gaagtcctga gttcaattcc cagcaaccac atggtggctc acaaccatct 9360
   gtaatgggat ctgatgccct cttctggtgt gtctgaagac cacaagtgta ttcacattaa 9420
   ataaataaat cctccttctt cttctttttt ttttttttaa agagaatact gtctccagta 9480
   gaatttactg aagtaatgaa atactttgtg tttgttccaa tatggtagcc aataatcaaa 9540
   ttactcttta agcactggaa atgttaccaa ggaactaatt tttatttgaa gtgtaactgt 9600
   ggacagagga gccataactg cagacttgtg ggatacagaa gaccaatgca gactttaatg 9660
   tcttttctct tacactaagc aataaagaaa taaaaattga acttctagta tcctatttgt 9720
   ttaaactgct agctttactt aacttttgtg cttcatctat acaaagctga aagctaagtc 9780
   tgcagccatt actaaacatg aaagcaagta atgataattt tggatttcaa aaatgtaggg 9840
   ccagagttta gccagccagt ggtggtgctt gcctttatgc ctttaatccc agcactctgg 9900
   aggcagagac aggcagatct ctgagtttga gcccagcctg gtctacacat caagttctat 9960
   ctaggatagc caggaataca cacagaaacc ctgttgggga ggggggctct gagatttcat 10020
   aaaattataa ttgaagcatt ccctaatgag ccactatgga tgtggctaaa tccgtctacc 10080
   tttctgatga gatttgggta ttattttttc tgtctctgct gttggttggg tcttttgaca 10140
   ctgtgggctt tctttaaagc ctccttcctg ccatgtggtc tcttgtttgc tactaacttc 10200
   ccatggctta aatggcatgg ctttttgcct tctaagggca gctgctgaga tttgcagcct 10260
   gatttccagg gtggggttgg gaaatctttc aaacactaaa attgtccttt aatttttttt 10320
   ttaaaaaatg ggttatataa taaacctcat aaaatagtta tgaggagtga ggtggactaa 10380
   tattaaatga gtccctcccc tataaaagag ctattaaggc tttttgtctt atacttaact 10440
   ttttttttaa atgtggtatc tttagaacca agggtcttag agttttagta tacagaaact 10500
   gttgcatcgc ttaatcagat tttctagttt caaatccaga gaatccaaat tcttcacagc 10560
   caaagtcaaa ttaagaattt ctgactttta atgttaattt gcttactgtg aatataaaaa 10620
   tgatagcttt tcctgaggca gggtctcact atgtatctct gcctgatctg caacaagata 10680
   tgtagactaa agttctgcct gcttttgtct cctgaatact aaggttaaaa tgtagtaata 10740
   cttttggaac ttgcaggtca gattctttta taggggacac actaagggag cttgggtgat 10800
   agttggtaaa atgtgtttca agtgatgaaa acttgaatta ttatcaccgc aacctacttt 10860
   ttaaaaaaaa aagccaggcc tgttagagca tgcttaaggg atccctagga cttgctgagc 10920
   acacaagagt agttacttgg caggctcctg gtgagagcat atttcaaaaa acaaggcaga 10980
   caaccaagaa actacagtta aggttacctg tctttaaacc atctgcatat acacagggat 11040
   attaaaatat tccaaataat atttcattca agttttcccc catcaaattg ggacatggat 11100
   ttctccggtg aataggcaga gttggaaact aaacaaatgt tggttttgtg atttgtgaaa 11160
   ttgttttcaa gtgatagtta aagcccatga gatacagaac aaagctgcta tttcgaggtc 11220
   tcttggttta tactcagaag cacttctttg ggtttccctg cactatcctg atcatgtgct 11280
   aggcctacct taggctgatt gttgttcaaa taaacttaag tttcctgtca ggtgatgtca 11340
   tatgatttca tatatcaagg caaaacatgt tatatatgtt aaacatttgt acttaatgtg 11400
   aaagttaggt ctttgtgggt ttgattttta attttcaaaa cctgagctaa ataagtcatt 11460
   tttacatgtc ttacatttgg tggaattgta taattgtggt ttgcaggcaa gactctctga 11520
   cctagtaacc ctacctatag agcactttgc tgggtcacaa gtctaggagt caagcatttc 11580
   accttgaagt tgagacgttt tgttagtgta tactagttta tatgttggag gacatgttta 11640
   tccagaagat attcaggact atttttgact gggctaagga attgattctg attagcactg 11700
   ttagtgagca ttgagtggcc tttaggcttg aattggagtc acttgtatat ctcaaataat 11760
   gctggccttt tttaaaaagc ccttgttctt tatcaccctg ttttctacat aatttttgtt 11820
   caaagaaata cttgtttgga tctccttttg acaacaatag catgttttca agccatattt 11880
   tttttccttt tttttttttt ttttggtttt tcgagacagg gtttctctgt atagccctgg 11940
   ctgtcctgga actcactttg tagaccaggc tggcctcgaa ctcagaaatc cgcctgcctc 12000
   tgcctcctga gtgccgggat taaaggcgtg caccaccacg cctggctaag ttggatattt 12060
   tgttatataa ctataaccaa tactaactcc actgggtgga tttttaattc agtcagtagt 12120
   cttaagtggt ctttattggc ccttcattaa aatctactgt tcactctaac agaggctgtt 12180
   ggtactagtg gcacttaagc aacttcctac ggatatacta gcagattaag ggtcagggat 12240
   agaaactagt ctagcgtttt gtatacctac cagctttata ctaccttgtt ctgatagaaa 12300
   tatttcagga catctagcac ccaattcgcc ctatagtgag tcgtattaca attcactggc 12360
   cgtcgtttta caacgtcgtg actgggaaaa ccctggcgtt acccaactta atcgccttgc 12420
   agcacatccc cctttcgcca gctggcgtaa tagcgaagag gcccgcaccg atcgcccttc 12480
   ccaacagttg cgcagcctga atggcgaatg ggacgcgccc tgtagcggcg cattaagcgc 12540
   ggcgggtgtg gtggttacgc gcagcgtgac cgctacactt gccagcgccc tagcgcccgc 12600
   tcctttcgct ttcttccctt cctttctcgc cacgttcgcc ggctttcccc gtcaagctct 12660
   aaatcggggg ctccctttag ggttccgatt tagtgcttta cggcacctcg accccaaaaa 12720
   acttgattag ggtgatggtt cacgtagtgg gccatcgccc tgatagacgg tttttcgccc 12780
   tttgacgttg gagtccacgt tctttaatag tggactcttg ttccaaactg gaacaacact 12840
   caaccctatc tcggtctatt cttttgattt ataagggatt ttgccgattt cggcctattg 12900
   gttaaaaaat gagctgattt aacaaaaatt taacgcgaat tttaacaaaa tattaacgct 12960
   tacaatttag gtggcacttt tcggggaaat gtgcgcggaa cccctatttg tttatttttc 13020
   taaatacatt caaatatgta tccgctcatg agacaataac cctgataaat gcttcaataa 13080
   tattgaaaaa ggaagagtat gagtattcaa catttccgtg tcgcccttat tccctttttt 13140
   gcggcatttt gccttcctgt ttttgctcac ccagaaacgc tggtgaaagt aaaagatgct 13200
   gaagatcagt tgggtgcacg agtgggttac atcgaactgg atctcaacag cggtaagatc 13260
   cttgagagtt ttcgccccga agaacgtttt ccaatgatga gcacttttaa agttctgcta 13320
   tgtggcgcgg tattatcccg tattgacgcc gggcaagagc aactcggtcg ccgcatacac 13380
   tattctcaga atgacttggt tgagtactca ccagtcacag aaaagcatct tacggatggc 13440
   atgacagtaa gagaattatg cagtgctgcc ataaccatga gtgataacac tgcggccaac 13500
   ttacttctga caacgatcgg aggaccgaag gagctaaccg cttttttgca caacatgggg 13560
   gatcatgtaa ctcgccttga tcgttgggaa ccggagctga atgaagccat accaaacgac 13620
   gagcgtgaca ccacgatgcc tgtagcaatg gcaacaacgt tgcgcaaact attaactggc 13680
   gaactactta ctctagcttc ccggcaacaa ttaatagact ggatggaggc ggataaagtt 13740
   gcaggaccac ttctgcgctc ggcccttccg gctggctggt ttattgctga taaatctgga 13800
   gccggtgagc gtgggtctcg cggtatcatt gcagcactgg ggccagatgg taagccctcc 13860
   cgtatcgtag ttatctacac gacggggagt caggcaacta tggatgaacg aaatagacag 13920
   atcgctgaga taggtgcctc actgattaag cattggtaac tgtcagacca agtttactca 13980
   tatatacttt agattgattt aaaacttcat ttttaattta aaaggatcta ggtgaagatc 14040
   ctttttgata atctcatgac caaaatccct taacgtgagt tttcgttcca ctgagcgtca 14100
   gaccccgtag aaaagatcaa aggatcttct tgagatcctt tttttctgcg cgtaatctgc 14160
   tgcttgcaaa caaaaaaacc accgctacca gcggtggttt gtttgccgga tcaagagcta 14220
   ccaactcttt ttccgaaggt aactggcttc agcagagcgc agataccaaa tactgtcctt 14280
   ctagtgtagc cgtagttagg ccaccacttc aagaactctg tagcaccgcc tacatacctc 14340
   gctctgctaa tcctgttacc agtggctgct gccagtggcg ataagtcgtg tcttaccggg 14400
   ttggactcaa gacgatagtt accggataag gcgcagcggt cgggctgaac ggggggttcg 14460
   tgcacacagc ccagcttgga gcgaacgacc tacaccgaac tgagatacct acagcgtgag 14520
   ctatgagaaa gcgccacgct tcccgaaggg agaaaggcgg acaggtatcc ggtaagcggc 14580
   agggtcggaa caggagagcg cacgagggag cttccagggg gaaacgcctg gtatctttat 14640
   agtcctgtcg ggtttcgcca cctctgactt gagcgtcgat ttttgtgatg ctcgtcaggg 14700
   gggcggagcc tatggaaaaa cgccagcaac gcggcctttt tacggttcct ggccttttgc 14760
   tggccttttg ctcacatgtt ctttcctgcg ttatcccctg attctgtgga taaccgtatt 14820
   accgcctttg agtgagctga taccgctcgc cgcagccgaa cgaccgagcg cagcgagtca 14880
   gtgagcgagg aagcggaaga gcgcccaata cgcaaaccgc ctctccccgc gcgttggccg 14940
   attcattaat gcagctggca cgacaggttt cccgactgga aagcgggcag tgagcgcaac 15000
   gcaattaatg tgagttagct cactcattag gcaccccagg ctttacactt tatgcttccg 15060
   gctcgtatgt tgtgtggaat tgtgagcgga taacaatttc acacaggaaa cagctatgac 15120
   catgattacg ccaagcgcgc aattaaccct cactaaaggg aacaaaagct gtcgagatct 15180
   agatatcgat ggccatagag                                             15200
<210> 12
   <211> 5070
   <212> DNA
   <213> Artificial Sequence
<220>
   <223> Description of Artificial Sequence: Exchange vector
<400> 12
   gggtaactaa gtaaggatcg agatccaggc gcggatcaat aaaagatcat tattttcaat 60
   agatctgtgt gttggttttt tgtgtgcctt gggggagggg gaggccagaa tgaggcgcgg 120
   ccaaggggga gggggaggcc agaatgacct tgggggaggg ggaggccaga atgaccttgg 180
   gggaggggga ggccagaatg aggcgcggat ccggagaagt tcctattccg aagttcctat 240
   tcttcaaata gtataggaac ttcgctcgag ggatcggcca ttgaacaaga tggattgcac 300
   gcaggttctc cggccgcttg ggtggagagg ctattcggct atgactgggc acaacagaca 360
   atcggctgct ctgatgccgc cgtgttccgg ctgtcagcgc aggggcgccc ggttcttttt 420
   gtcaagaccg acctgtccgg tgccctgaat gaactgcagg acgaggcagc gcggctatcg 480
   tggctggcca cgacgggcgt tccttgcgca gctgtgctcg acgttgtcac tgaagcggga 540
   agggactggc tgctattggg cgaagtgccg gggcaggatc tcctgtcatc tcaccttgct 600
   cctgccgaga aagtatccat catggctgat gcaatgcggc ggctgcatac gcttgatccg 660
   gctacctgcc cattcgacca ccaagcgaaa catcgcatcg agcgagcacg tactcggatg 720
   gaagccggtc ttgtcgatca ggatgatctg gacgaagagc atcaggggct cgcgccagcc 780
   gaactgttcg ccaggctcaa ggcgcgcatg cccgacggcg aggatctcgt cgtgacccat 840
   ggcgatgcct gcttgccgaa tatcatggtg gaaaatggcc gcttttctgg attcatcgac 900
   tgtggccggc tgggtgtggc ggaccgctat caggacatag cgttggctac ccgtgatatt 960
   gctgaagagc ttggcggcga atgggctgac cgcttcctcg tgctttacgg tatcgccgct 1020
   cccgattcgc agcgcatcgc cttctatcgc cttcttgacg agttcttctg aggggatcga 1080
   tccgctgtaa gtctgcagaa attgatgatc tattaaacaa taaagatgtc cactaaaatg 1140
   gaagtttttc ctgtcatact ttgttaagaa gggtgagaac agagtaccta cattttgaat 1200
   ggaaggattg gagctacggg ggtgggggtg gggtgggatt agataaatgc ctgctcttta 1260
   ctgaaggctc tttactattg ctttatgata atgtttcata gttggatatc ataatttaaa 1320
   caagcaaaac caaattaagg gccagctcat tcctcccact catgatctat agatctatag 1380
   atctctcgtg ggatcattgt ttttctcttg attcccactt tgtggttcta agtactgtgg 1440
   tttccaaatg tgtcagtttc atagcctgaa gaacgagatc agcagcctct gttccacata 1500
   cacttcattc tcagtattgt tttgccaagt tctaattcca tcagaagctg actctagatg 1560
   gcgcgtatgc attaattaag gccatagcgg ccatttatcg gccggccata acttcgtata 1620
   atgtatgcta tacgaagtta tggatcctca cagtaggtgg catcgttcct ttctgactgc 1680
   ccgccccccg catgccgtcc cgcgatattg agctccgaac ctctcgccct gccgccgccg 1740
   gtgctccgtc gccgccgcgc cgccatggaa ttcgaacgct gacgtcatca acccgctcca 1800
   aggaatcgcg ggcccagtgt cactaggcgg gaacacccag cgcgcgtgcg ccctggcagg 1860
   aagatggctg tgagggacag gggagtggcg ccctgcaata tttgcatgtc gctatgtgtt 1920
   ctgggaaatc accataaacg tgaaatgtct ttggatttgg gaatcttata agttctgtat 1980
   gagaccactc tttcccactg gcttctttct tcagtttcct tagcactggg aaattgagga 2040
   aagaagccgg ttttttctag agactcgaga taacttcgta taatgtatgc tatacgaagt 2100
   tatggcgcgt tgacattgat tattgactag aaatcggccg gcctaggcgc gccggtaacc 2160
   gaagttccta tactttctag agaataggaa cttcggaata ggaacttcaa gccggtaccc 2220
   agcttttgtt ccctttagtg agggttaatt tcgagcttgg cgtaatcatg gtcatagctg 2280
   tttcctgtgt gaaattgtta tccgctcaca attccacaca acatacgagc cggaagcata 2340
   aagtgtaaag cctggggtgc ctaatgagtg agctaactca cattaattgc gttgcgctca 2400
   ctgcccgctt tccagtcggg aaacctgtcg tgccagctgc attaatgaat cggccaacgc 2460
   gcggggagag gcggtttgcg tattgggcgc tcttccgctt cctcgctcac tgactcgctg 2520
   cgctcggtcg ttcggctgcg gcgagcggta tcagctcact caaaggcggt aatacggtta 2580
   tccacagaat caggggataa cgcaggaaag aacatgtgag caaaaggcca gcaaaaggcc 2640
   aggaaccgta aaaaggccgc gttgctggcg tttttccata ggctccgccc ccctgacgag 2700
   catcacaaaa atcgacgctc aagtcagagg tggcgaaacc cgacaggact ataaagatac 2760
   caggcgtttc cccctggaag ctccctcgtg cgctctcctg ttccgaccct gccgcttacc 2820
   ggatacctgt ccgcctttct cccttcggga agcgtggcgc tttctcatag ctcacgctgt 2880
   aggtatctca gttcggtgta ggtcgttcgc tccaagctgg gctgtgtgca cgaacccccc 2940
   gttcagcccg accgctgcgc cttatccggt aactatcgtc ttgagtccaa cccggtaaga 3000
   cacgacttat cgccactggc agcagccact ggtaacagga ttagcagagc gaggtatgta 3060
   ggcggtgcta cagagttctt gaagtggtgg cctaactacg gctacactag aaggacagta 3120
   tttggtatct gcgctctgct gaagccagtt accttcggaa aaagagttgg tagctcttga 3180
   tccggcaaac aaaccaccgc tggtagcggt ggtttttttg tttgcaagca gcagattacg 3240
   cgcagaaaaa aaggatctca agaagatcct ttgatctttt ctacggggtc tgacgctcag 3300
   tggaacgaaa actcacgtta agggattttg gtcatgagat tatcaaaaag gatcttcacc 3360
   tagatccttt taaattaaaa atgaagtttt aaatcaatct aaagtatata tgagtaaact 3420
   tggtctgaca gttaccaatg cttaatcagt gaggcaccta tctcagcgat ctgtctattt 3480
   cgttcatcca tagttgcctg actccccgtc gtgtagataa ctacgatacg ggagggctta 3540
   ccatctggcc ccagtgctgc aatgataccg cgagacccac gctcaccggc tccagattta 3600
   tcagcaataa accagccagc cggaagggcc gagcgcagaa gtggtcctgc aactttatcc 3660
   gcctccatcc agtctattaa ttgttgccgg gaagctagag taagtagttc gccagttaat 3720
   agtttgcgca acgttgttgc cattgctaca ggcatcgtgg tgtcacgctc gtcgtttggt 3780
   atggcttcat tcagctccgg ttcccaacga tcaaggcgag ttacatgatc ccccatgttg 3840
   tgcaaaaaag cggttagctc cttcggtcct ccgatcgttg tcagaagtaa gttggccgca 3900
   gtgttatcac tcatggttat ggcagcactg cataattctc ttactgtcat gccatccgta 3960
   agatgctttt ctgtgactgg tgagtactca accaagtcat tctgagaata gtgtatgcgg 4020
   cgaccgagtt gctcttgccc ggcgtcaata cgggataata ccgcgccaca tagcagaact 4080
   ttaaaagtgc tcatcattgg aaaacgttct tcggggcgaa aactctcaag gatcttaccg 4140
   ctgttgagat ccagttcgat gtaacccact cgtgcaccca actgatcttc agcatctttt 4200
   actttcacca gcgtttctgg gtgagcaaaa acaggaaggc aaaatgccgc aaaaaaggga 4260
   ataagggcga cacggaaatg ttgaatactc atactcttcc tttttcaata ttattgaagc 4320
   atttatcagg gttattgtct catgagcgga tacatatttg aatgtattta gaaaaataaa 4380
   caaatagggg ttccgcgcac atttccccga aaagtgccac ctaaattgta agcgttaata 4440
   ttttgttaaa attcgcgtta aatttttgtt aaatcagctc attttttaac caataggccg 4500
   aaatcggcaa aatcccttat aaatcaaaag aatagaccga gatagggttg agtgttgttc 4560
   cagtttggaa caagagtcca ctattaaaga acgtggactc caacgtcaaa gggcgaaaaa 4620
   ccgtctatca gggcgatggc ccactacgtg aaccatcacc ctaatcaagt tttttggggt 4680
   cgaggtgccg taaagcacta aatcggaacc ctaaagggag cccccgattt agagcttgac 4740
   ggggaaagcc ggcgaacgtg gcgagaaagg aagggaagaa agcgaaagga gcgggcgcta 4800
   gggcgctggc aagtgtagcg gtcacgctgc gcgtaaccac cacacccgcc gcgcttaatg 4860
   cgccgctaca gggcgcgtcc cattcgccat tcaggctgcg caactgttgg gaagggcgat 4920
   cggtgcgggc ctcttcgcta ttacgccagc tggcgaaagg gggatgtgct gcaaggcgat 4980
   taagttgggt aacgccaggg ttttcccagt cacgacgttg taaaacgacg gccagtgaat 5040
   tgtaatacga ctcactatag ggcgaattgg                                  5070
<210> 13
   <211> 47
   <212> DNA
   <213> Artificial Sequence
<220>
   <223> Description of Artificial Sequence: DNA encoding an shRNA directed against the CDH-1 gene
<400> 13
   tgagaagtct cccagtcagt tcaagagact gactgggaga cttctca 47
<210> 14
   <211> 47
   <212> DNA
   <213> Artificial Sequence
<220>
   <223> Description of Artificial Sequence: DNA encoding an shRNA directed against the p53 gene
<400> 14
   gactccagtg gtaatctact tcaagagagt agattaccac tggagtc 47
<210> 15
   <211> 47
   <212> DNA
   <213> Artificial Sequence
<220>
   <223> Description of Artificial Sequence: DNA encoding an shRNA directed against the CDC20 gene
<400> 15
   cggcaggact ccgggccgat tcaagagatc ggcccggagt cctgccg 47
<210> 16
   <211> 47
   <212> DNA
   <213> Artificial Sequence
<220>
   <223> Description of Artificial Sequence: DNA encoding an shRNA directed against the CYLD gene
<400> 16
   cctcatgcag ttctctttgt tcaagagaca aagagaactg catgagg 47
<210> 17
   <211> 50
   <212> DNA
   <213> Artificial Sequence
<220>
   <223> Description of Artificial Sequence: DNA encoding an shRNA directed against the RAS-GAP gene
<400> 17
   aagatgaagc cactccctat ttcaagagaa aatagggagt ggcttcatct 50
<210> 18
   <211> 41
   <212> DNA
   <213> Artificial Sequence
<220>
   <223> Description of Artificial Sequence: DNA encoding an shRNA directed against the tubulin gene
<400> 18 gacagagcca agtggactca cagagtccac ttggctctgt c 41
<210> 19
   <211> 42
   <212> DNA
   <213> Artificial Sequence
<220>
   <223> Description of Artificial Sequence: DNA encoding an shRNA directed against the lamin gene
<400> 19
   ctggacttcc agaagaacat tcgtgttctt ctggaagtcc ag 42
<210> 20
   <211> 47
   <212> DNA
   <213> Artificial Sequence
<220>
   <223> Description of Artificial Sequence: DNA encoding an shRNA directed against the gene for the ubiquitin carboxyl-terminal hydrolase 12
<400> 20
   gagattggtc cagaacagtt tcaagagaac tgttctggac caatctc 47
<210> 21
   <211> 47
   <212> DNA
   <213> Artificial Sequence
<220>
   <223> Description of Artificial Sequence: DNA encoding an shRNA directed against the gene for the ubiquitin carboxyl-terminal hydrolase 12
<400> 21
   gcccttccga tcatggtagt tcaagagact accatgatcg gaagggc 47
<210> 22
   <211> 47
   <212> DNA
   <213> Artificial Sequence
<220>
   <223> Description of Artificial Sequence: DNA encoding an shRNA directed against the gene for the ubiquitin carboxyl-terminal hydrolase 12
<400> 22
   tctttagaat tcttaagtat tcaagagata cttaagaatt ctaaaga 47
<210> 23
   <211> 47
   <212> DNA
   <213> Artificial Sequence
<220>
   <223> Description of Artificial Sequence: DNA encoding an shRNA directed against the gene of the ubiquitin carboxyl-terminal hydrolase
<400> 23
   cattagctat atcaacatgt tcaagagaca tgttgatata gctaatg 47
<210> 24
   <211> 47
   <212> DNA
   <213> Artificial Sequence
<220>
   <223> Description of Artificial Sequence: DNA encoding an shRNA directed against the gene for the ubiquitin carboxyl-terminal hydrolase11
<400> 24
   accacaaacg gcggaacgat tcaagagatc gttccgccgt ttgtggt 47
<210> 25
   <211> 47
   <212> DNA
   <213> Artificial Sequence
<220>
   <223> Description of Artificial Sequence: DNA encoding an shRNA directed against the gene for the ubiquitin carboxyl-terminal hydrolase 11
<400> 25
   gagggtcttg gaggtcttct tcaagagaga agacctccaa gaccctc 47
<210> 26
   <211> 47
   <212> DNA
   <213> Artificial Sequence
<220>
   <223> Description of Artificial Sequence: DNA encoding an shRNA directed against the gene for the ubiquitin carboxyl-terminal hydrolase 11
<400> 26
   gtccatgccc agccgtacat tcaagagatg tacggctggg catggac 47
<210> 27
   <211> 47
   <212> DNA
   <213> Artificial Sequence
<220>
   <223> Description of Artificial Sequence: DNA encoding an shRNA directed against the gene for the ubiquitin carboxyl-terminal hydrolase 11
<400> 27
   gctggacacc ctcgtggagt tcaagagact ccacgagggt gtccagc 47
<210> 28
   <211> 47
   <212> DNA
   <213> Artificial Sequence
<220>
   <223> Description of Artificial Sequence: DNA encoding an shRNA directed against the gene for the ubiquitin carboxyl-terminal hydrolase 10
<400> 28
   gaatatcaga gaattgagtt tcaagagaac tcaattctct gatattc 47
<210> 29
   <211> 47
   <212> DNA
   <213> Artificial Sequence
<220>
   <223> Description of Artificial Sequence: DNA encoding an shRNA directed against the gene for the ubiquitin carboxyl-terminal hydrolase 10
<400> 29
   tggacttcat gaggaaatgt tcaagagaca tttcctcatg aagtcca 47
<210> 30
   <211> 47
   <212> DNA
   <213> Artificial Sequence
<220>
   <223> Description of Artificial Sequence: DNA encoding an shRNA directed against the gene for the ubiquitin carboxyl-terminal hydrolase 10
<400> 30
   tattgaatat cctgtggact tcaagagagt ccacaggata ttcaata 47
<210> 31
   <211> 47
   <212> DNA
   <213> Artificial Sequence
<220>
   <223> Description of Artificial Sequence: DNA encoding an shRNA directed against the gene for the ubiquitin carboxyl-terminal hydrolase 10
<400> 31
   ttgtactgag agaaactgct tcaagagagc agtttctctc agtacaa 47
<210> 32
   <211> 47
   <212> DNA
   <213> Artificial Sequence
<220>
   <223> Description of Artificial Sequence: DNA encoding an shRNA directed against the HAUSP gene
<400> 32
   gatcaatgat aggtttgaat tcaagagatt caaacctatc attgatc 47
<210> 33
   <211> 47
   <212> DNA
   <213> Artificial Sequence
<220>
   <223> Description of Artificial Sequence: DNA encoding an shRNA directed against the HAUSP gene
<400> 33
   ggagtttgag aagtttaaat tcaagagatt taaacttctc aaactcc 47
<210> 34
   <211> 47
   <212> DNA
   <213> Artificial Sequence
<220>
   <223> Description of Artificial Sequence: DNA encoding an shRNA directed against the HAUSP gene
<400> 34
   gaactcctcg cttgctgagt tcaagagact cagcaagcga ggagttc 47
<210> 35
   <211> 47
   <212> DNA
   <213> Artificial Sequence
<220>
   <223> Description of Artificial Sequence: DNA encoding an shRNA directed against the HAUSP gene
<400> 35
   ccgaatttaa cagagagaat tcaagagatt ctctctgtta aattcgg 47
<210> 36
   <211> 47
   <212> DNA
   <213> Artificial Sequence
<220>
   <223> Description of Artificial Sequence: DNA encoding an shRNA directed against the gene for the ubiquitin carboxyl-terminal hydrolase 8
<400> 36
   gacagcagaa gaatgcagat tcaagagatc tgcattcttc tgctgtc 47
<210> 37
   <211> 47
   <212> DNA
   <213> Artificial Sequence
<220>
   <223> Description of Artificial Sequence: DNA encoding an shRNA directed against the gene for the ubiquitin carboxyl-terminal hydrolase 8
<400> 37
   ataaagctca acgagaacct tcaagagagg ttctcgttga gctttat 47
<210> 38
   <211> 47
   <212> DNA
   <213> Artificial Sequence
<220>
   <223> Description of Artificial Sequence: DNA encoding an shRNA directed against the gene for the ubiquitin carboxyl-terminal hydrolase 8
<400> 38
   ggtgaagtgg cagaagaatt tcaagagaat tcttctgcca cttcacc 47
<210> 39
   <211> 47
   <212> DNA
   <213> Artificial Sequence
<220>
   <223> Description of Artificial Sequence: DNA encoding an shRNA directed against the gene for the ubiquitin carboxyl-terminal hydrolase 8
<400> 39
   gtattgcagt aatcatcact tcaagagagt gatgattact gcaatac 47
<210> 40
   <211> 47
   <212> DNA
   <213> Artificial Sequence
<220>
   <223> Description of Artificial Sequence: DNA encoding an shRNA directed against the FLJ10785 gene
<400> 40
   gatatggggt tccatgtcat tcaagagatg acatggaacc ccatatc 47
<210> 41
   <211> 47
   <212> DNA
   <213> Artificial Sequence
<220>
   <223> Description of Artificial Sequence: DNA encoding an shRNA directed against the FLJ10785 gene
<400> 41
   ggagacatgg ttcttagtgt tcaagagaca ctaagaacca tgtctcc 47
<210> 42
   <211> 47
   <212> DNA
   <213> Artificial Sequence
<220>
   <223> Description of Artificial Sequence: DNA encoding an shRNA directed against the FLJ10785 gene
<400> 42
   agcaccaagt tcgtctcagt tcaagagact gagacgaact tggtgct 47
<210> 43
   <211> 47
   <212> DNA
   <213> Artificial Sequence
<220>
   <223> Description of Artificial Sequence: DNA encoding an shRNA directed against the FLJ10785 gene
<400> 43
   gatgcaacac tgaaagaact tcaagagagt tctttcagtg ttgcatc 47
<210> 44
   <211> 47
   <212> DNA
   <213> Artificial Sequence
<220>
   <223> Description of Artificial Sequence: DNA encoding an shRNA directed against the KIAA0710 gene
<400> 44
   gtcaatggca gtgatgatat tcaagagata tcatcactgc cattgac 47
<210> 45
   <211> 47
   <212> DNA
   <213> Artificial Sequence
<220>
   <223> Description of Artificial Sequence: DNA encoding an shRNA directed against the KIAA0710 gene
<400> 45
   cctgctagct gcctgtggct tcaagagagc cacaggcagc tagcagg 47
<210> 46
   <211> 47
   <212> DNA
   <213> Artificial Sequence
<220>
   <223> Description of Artificial Sequence: DNA encoding an shRNA directed against the KIAA0710 gene
<400> 46
   ccacctttgc cagaaggagt tcaagagact ccttctggca aaggtgg 47
<210> 47
   <211> 47
   <212> DNA
   <213> Artificial Sequence
<220>
   <223> Description of Artificial Sequence: DNA encoding an shRNA directed against the KIAA0710 gene
<400> 47
   ccctattgag gcaagtgtct tcaagagaga cacttgcctc aataggg 47
<210> 48
   <211> 47
   <212> DNA
   <213> Artificial Sequence
<220>
   <223> Description of Artificial Sequence: DNA encoding an shRNA directed against the genes FLJ12552/FLJ14256
<400> 48
   gaaggaaaac ttgctgacgt tcaagagacg tcagcaagtt ttccttc 47
<210> 49
   <211> 47
   <212> DNA
   <213> Artificial Sequence
<220>
   <223> Description of Artificial Sequence: DNA encoding an shRNA directed against the FLJ12552/FLJ14256 genes
<400> 49
   ctcacctggg tccatgagat tcaagagatc tcatggaccc aggtgag 47
<210> 50
   <211> 47
   <212> DNA
   <213> Artificial Sequence
<220>
   <223> Description of Artificial Sequence: DNA encoding an shRNA directed against the FLJ12552/FLJ14256 genes
<400> 50
   gctgtcttac cgtgtggtct tcaagagaga ccacacggta agacagc 47
<210> 51
   <211> 47
   <212> DNA
   <213> Artificial Sequence
<220>
   <223> Description of Artificial Sequence: DNA encoding an shRNA directed against the FLJ12552/FLJ14256 genes
<400> 51
   cctggaccgc atgtatgact tcaagagagt catacatgcg gtccagg 47
<210> 52
   <211> 47
   <212> DNA
   <213> Artificial Sequence
<220>
   <223> Description of Artificial Sequence: DNA encoding an shRNA directed against the KIAA1203 gene
<400> 52
   gtcaatggca gtgatgatat tcaagagata tcatcactgc cattgac 47
<210> 53
   <211> 47
   <212> DNA
   <213> Artificial Sequence
<220>
   <223> Description of Artificial Sequence: DNA encoding an shRNA directed against the KIAA1203 gene
<400> 53
   cctgctagct gcctgtggct tcaagagagc cacaggcagc tagcagg 47
<210> 54
   <211> 47
   <212> DNA
   <213> Artificial Sequence
<220>
   <223> Description of Artificial Sequence: DNA encoding an shRNA directed against the KIAA1203 gene
<400> 54
   ccacctttgc cagaaggagt tcaagagact ccttctggca aaggtgg 47
<210> 55
   <211> 47
   <212> DNA
   <213> Artificial Sequence
<220>
   <223> Description of Artificial Sequence: DNA encoding an shRNA directed against the KIAA1203 gene
<400> 55
   ccctattgag gcaagtgtct tcaagagaga cacttgcctc aataggg 47
<210> 56
   <211> 47
   <212> DNA
   <213> Artificial Sequence
<220>
   <223> Description of Artificial Sequence: DNA encoding an shRNA directed against the FLJ23277 gene
<400> 56
   ggaaatccga attgcttggt tcaagagacc aagcaattcg gatttcc 47
<210> 57
   <211> 47
   <212> DNA
   <213> Artificial Sequence
<220>
   <223> Description of Artificial Sequence: DNA encoding an shRNA directed against the FLJ23277 gene
<400> 57
   cacatttctt caagtgtggt tcaagagacc acacttgaag aaatgtg 47
<210> 58
   <211> 47
   <212> DNA
   <213> Artificial Sequence
<220>
   <223> Description of Artificial Sequence: DNA encoding an shRNA directed against the FLJ23277 gene
<400> 58
   cagcaggatg ctcaagaatt tcaagagaat tcttgagcat cctgctg 47
<210> 59
   <211> 47
   <212> DNA
   <213> Artificial Sequence
<220>
   <223> Description of Artificial Sequence: DNA encoding an shRNA directed against the FLJ23277 gene
<400> 59
   gctgaatacc tacattggct tcaagagagc caatgtaggt attcagc 47
<210> 60
   <211> 47
   <212> DNA
   <213> Artificial Sequence
<220>
   <223> Description of Artificial Sequence: DNA encoding an shRNA directed against the FLJ14914 (similar to UBP4) gene
<400> 60
   gggcttgtgc ctggccttgt tcaagagaca aggccaggca caagccc 47
<210> 61
   <211> 47
   <212> DNA
   <213> Artificial Sequence
<220>
   <223> Description of Artificial Sequence: DNA encoding an shRNA directed against the FLJ14914 (similar to UBP4) gene
<400> 61
   gccttgtcct gccaagaagt tcaagagact tcttggcagg acaaggc 47
<210> 62
   <211> 47
   <212> DNA
   <213> Artificial Sequence
<220>
   <223> Description of Artificial Sequence: DNA encoding an shRNA directed against the FLJ14914 (similar to UBP4) gene
<400> 62
   gattgaagcc aagggaacgt tcaagagacg ttcccttggc ttcaatc 47
<210> 63
   <211> 47
   <212> DNA
   <213> Artificial Sequence
<220>
   <223> Description of Artificial Sequence: DNA encoding an shRNA directed against the FLJ14914 (similar to UBP4) gene
<400> 63
   tggcgcctgc tccccatctt tcaagagaag atggggagca ggcgcca 47
<210> 64
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L5
<400> 64
   gaaccagcag gctctgtggt tcaagagacc acagagcctg ctggttc 47
<210> 65
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L5
<400> 65
   ggaagcataa ttatctgcct tcaagagagg cagataatta tgcttcc 47
<210> 66
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L5

<400> 66
   agaagaagat gcttttcact tcaagagagt gaaaagcatc ttcttct 47
<210> 67
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L5
<400> 67
   cttgcagagg aggaacccat tcaagagatg ggttcctcct ctgcaag 47
<210> 68
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L3
<400> 68
   gcaaacaatc agcaatgcct tcaagagagg cattgctgat tgtttgc 47
<210> 69
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L3
<400> 69
   ttggactgat tcatgctatt tcaagagaat agcatgaatc agtccaa 47
<210> 70
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L3
<400> 70
   ctggcaattc gttgatgtat tcaagagata catcaacgaa ttgccag 47
<210> 71
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L3
<400> 71
   ttagatgggc ggaagccatt tcaagagaat ggcttccgcc catctaa 47
<210> 72
   <211> 47
   <212> DNA
   <213> Artificial Sequence
<220>
<220>
   <223> Description of Artificial Sequence: DNA encoding an shRNA directed against the gene for the ubiquitin carboxyl-terminal hydrolase isozyme L1
<400> 72
   gaggagtctc tgggctcggt tcaagagacc gagcccagag actcctc 47
<210> 73
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L1
<400> 73
   gagctgaagg gacaagaagt tcaagagact tcttgtccct tcagctc 47
<210> 74
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L1
<400> 74
   tgtcgggtag atgacaaggt tcaagagacc ttgtcatcta cccgaca 47
<210> 75
   <211> 47
   <212> DNA
   <213> Artificial Sequence
<220>
   <223> Description of Artificial Sequence: DNA encoding an shRNA directed against the gene for the ubiquitin carboxyl-terminal hydrolase isozyme L1
<400> 75
   cacagctgtt cttctgttct tcaagagaga acagaagaac agctgtg 47
<210> 76
   <211> 47
   <212> DNA
   <213> Artificial Sequence
<220>
   <223> Description of Artificial Sequence: DNA encoding an shRNA directed against the KIAA1891/FLJ25263 genes
<400> 76
   gtggaagcct ttacagatct tcaagagaga tctgtaaagg cttccac 47
<210> 77
   <211> 47
   <212> DNA
   <213> Artificial Sequence
<220>
   <223> Description of Artificial Sequence: DNA encoding an shRNA directed against the KIAA1891/FLJ25236 genes
<400> 77
   caacagctgc cttcatctgt tcaagagaca gatgaaggca gctgttg 47
<210> 78
   <211> 47
   <212> DNA
   <213> Artificial Sequence
<220>
   <223> Description of Artificial Sequence: DNA encoding an shRNA directed against the KIAA1891/FLJ25263 genes
<400> 78
   ccataggcag tcctcctaat tcaagagatt aggaggactg cctatgg 47
<210> 79
   <211> 47
   <212> DNA
   <213> Artificial Sequence
<220>
   <223> Description of Artificial Sequence: DNA encoding an shRNA directed against the KIAA1891/FLJ25263 genes
<400> 79
   tgtatcactg ccactggttt tcaagagaaa ccagtggcag tgataca 47
<210> 80
   <211> 47
   <212> DNA
   <213> Artificial Sequence
<220>
   <223> Description of Artificial Sequence: DNA encoding an shRNA directed against the FLJ14528 (similar to UBP8) gene
<400> 80
   catgttgggc agctgcagct tcaagagagc tgcagctgcc caacatg 47
<210> 81
   <211> 47
   <212> DNA
   <213> Artificial Sequence
<220>
<220>
   <223> Description of Artificial Sequence: DNA encoding an shRNA directed against the FLJ14528 (similar to UBP8) gene
<400> 81
   cacaactgga gacctgaagt tcaagagact tcaggtctcc agttgtg 47
<210> 82
   <211> 47
   <212> DNA
   <213> Artificial Sequence
<220>
   <223> Description of Artificial Sequence: DNA encoding an shRNA directed against the FLJ14528 (similar to UBP8) gene
<400> 82
   gtatgcctcc aagaaagagt tcaagagact ctttcttgga ggcatac 47
<210> 83
   <211> 47
   <212> DNA
   <213> Artificial Sequence
<220>
   <223> Description of Artificial Sequence: DNA encoding an shRNA directed against the FLJ14528 (similar to UBP8) gene
<400> 83
   cttcacagta catttctctt tcaagagaag agaaatgtac tgtgaag 47
<210> 84
   <211> 47
   <212> DNA
   <213> Artificial Sequence
<220>
   <223> Description of Artificial Sequence: DNA encoding an shRNA directed against the gene for the U4/U6 TRI snRNP 65 kDa protein
<400> 84
   gtactttcaa ggccggggtt tcaagagaac cccggccttg aaagtac 47
<210> 85
   <211> 47
   <212> DNA
   <213> Artificial Sequence
<220>
   <223> Description of Artificial Sequence: DNA encoding an shRNA directed against the gene for the U4/U6 TRI snRNP 65 kDa protein
<400> 85
   cttggacaag caagccaaat tcaagagatt tggcttgctt gtccaag 47
<210> 86
   <211> 47
   <212> DNA
   <213> Artificial Sequence
<220>
   <223> Description of Artificial Sequence: DNA encoding an shRNA directed against the gene for the U4/U6 TRI snRNP 65 kDa protein
<400> 86
   gactattgtg actgatgttt tcaagagaaa catcagtcac aatagtc 47
<210> 87
   <211> 47
   <212> DNA
   <213> Artificial Sequence
<220>
   <223> Description of Artificial Sequence: DNA encoding an shRNA directed against the gene for the U4/U6 TRI snRNP 65kDa protein
<400> 87
   ggagaacttt ctgaagcgct tcaagagagc gcttcagaaa gttctcc 47
<210> 88
   <211> 47
   <212> DNA
   <213> Artificial Sequence
<220>
   <223> Description of Artificial Sequence: DNA encoding an shRNA for the XM_089437 gene
<400> 88
   gacgagagaa accttcacct tcaagagagg tgaaggtttc tctcgtc 47
<210> 89
   <211> 47
   <212> DNA
   <213> Artificial Sequence
<220>
   <223> Description of Artificial Sequence: DNA encoding an shRNA directed against the XM_089437 gene
<400> 89
   acattattct acattctttt tcaagagaaa agaatgtaga ataatgt 47
<210> 90
   <211> 47
   <212> DNA
   <213> Artificial Sequence
<220>
   <223> Description of Artificial Sequence: DNA encoding an shRNA directed against the XM_089437 gene
<400> 90
   agattcgcaa atggatgtat tcaagagata catccatttg cgaatct 47
<210> 91
   <211> 47
   <212> DNA
   <213> Artificial Sequence
<220>
   <223> Description of Artificial Sequence: DNA encoding an shRNA directed against the XM_089437 gene
<400> 91
   cattcccacc atgagtctgt tcaagagaca gactcatggt gggaatg 47
<210> 92
   <211> 47
   <212> DNA
   <213> Artificial Sequence
<220>
   <223> Description of Artificial Sequence: DNA encoding an shRNA directed against the KIAA1453 gene
<400> 92
   gatcgcccga cacttccgct tcaagagagc ggaagtgtcg ggcgatc 47
<210> 93
   <211> 47
   <212> DNA
   <213> Artificial Sequence
<220>
   <223> Description of Artificial Sequence: DNA encoding an shRNA directed against the KIAA1453 gene
<400> 93
   ccagcaggcc tacgtgctgt tcaagagaca gcacgtaggc ctgctgg 47
<210> 94
   <211> 47
   <212> DNA
   <213> Artificial Sequence
<220>
   <223> Description of Artificial Sequence: DNA encoding an shRNA directed against the KIAA1453 gene
<400> 94
   gccagctcct ccacagcact tcaagagagt gctgtggagg agctggc 47
<210> 95
   <211> 47
   <212> DNA
   <213> Artificial Sequence
<220>
   <223> Description of Artificial Sequence: DNA encoding an shRNA directed against the KIAA1453 gene
<400> 95
   cgccgccaag tggagcagat tcaagagatc tgctccactt ggcggcg 47
<210> 96
   <211> 47
   <212> DNA
   <213> Artificial Sequence
<220>
   <223> Description of Artificial Sequence: DNA encoding an shRNA directed against the FLJ12697 gene
<400> 96
   gaagatgccc atgaattcct tcaagagagg aattcatggg catcttc 47
<210> 97
   <211> 47
   <212> DNA
   <213> Artificial Sequence
<220>
   <223> Description of Artificial Sequence: DNA encoding an shRNA directed against the FLJ12697 gene
<400> 97
   caaacaggct gcgccaggct tcaagagagc ctggcgcagc ctgtttg 47
<210> 98
   <211> 47
   <212> DNA
   <213> Artificial Sequence
<220>
   <223> Description of Artificial Sequence: DNA encoding an shRNA directed against the FLJ12697 gene
<400> 98
   acggcctagc gcctgatggt tcaagagacc atcaggcgct aggccgt 47
<210> 99
   <211> 47
   <212> DNA
   <213> Artificial Sequence
<220>
   <223> Description of Artificial Sequence: DNA encoding an shRNA directed against the FLJ12697 gene
<400> 99
   ctgtaacctc tctgatcggt tcaagagacc gatcagagag gttacag 47
<210> 100
   <211> 47
   <212> DNA
   <213> Artificial Sequence
<220>
   <223> Description of Artificial Sequence: DNA encoding an shRNA directed against the gene for the ubiquitin specific protease (USP18)
<400> 100
   tctgtcagtc catcctggct tcaagagagc caggatggac tgacaga 47
<210> 101
   <211> 47
   <212> DNA
   <213> Artificial Sequence
<220>
   <223> Description of Artificial Sequence: DNA encoding an shRNA directed against the gene for the ubiquitin specific protease (USP18)
<400> 101
   tgaagcgaga gtcttgtgat tcaagagatc acaagactct cgcttca 47
<210> 102
   <211> 47
<212> DNA
   <213> Artificial Sequence
<220>
   <223> Description of Artificial Sequence: DNA encoding an shRNA directed against the gene for the ubiquitin specific protease (USP18)
<400> 102
   gatggagtgc taatggaaat tcaagagatt tccattagca ctccatc 47
<210> 103
   <211> 47
   <212> DNA
   <213> Artificial Sequence
<220>
   <223> Description of Artificial Sequence: DNA encoding an shRNA directed against the gene for the ubiquitin specific protease (USP18)
<400> 103
   ccttcagaga ttgacacgct tcaagagagc gtgtcaatct ctgaagg 47
<210> 104
   <211> 47
   <212> DNA
   <213> Artificial Sequence
<220>
   <223> Description of Artificial Sequence: DNA encoding an shRNA directed against the gene for the ubiquitin carboxyl-terminal hydrolase 20
<400> 104
   cctgaccacg ttccgactgt tcaagagaca gtcggaacgt ggtcagg 47
<210> 105
   <211> 47
   <212> DNA
   <213> Artificial Sequence
<220>
   <223> Description of Artificial Sequence: DNA encoding an shRNA directed against the gene for the ubiquitin carboxyl-terminal hydrolase 20
<400> 105
   gagttccttc gctgcctgat tcaagagatc aggcagcgaa ggaactc 47
<210> 106
   <211> 47
   <212> DNA
   <213> Artificial Sequence
<220>
   <223> Description of Artificial Sequence: DNA encoding an shRNA directed against the gene for the ubiquitin carboxyl-terminal hydrolase 20
<400> 106
   gactgccttg ctgccttctt tcaagagaag aaggcagcaa ggcagtc 47
<210> 107
   <211> 47
   <212> DNA
   <213> Artificial Sequence
<220>
   <223> Description of Artificial Sequence: DNA encoding an shRNA directed against the gene for the ubiquitin carboxyl-terminal hydrolase 20
<400> 107
   cgccgagggc tacgtactct tcaagagaga gtacgtagcc ctcggcg 47
<210> 108
   <211> 47
   <212> DNA
   <213> Artificial Sequence
<220>
   <223> Description of Artificial Sequence: DNA encoding an shRNA directed against the gene for the ubiquitin carboxyl-terminal hydrolase 24
<400> 108
   ggcgagaaga aaggactgtt tcaagagaac agtcctttct tctcgcc 47
<210> 109
   <211> 47
   <212> DNA
   <213> Artificial Sequence
<220>
   <223> Description of Artificial Sequence: DNA encoding an shRNA directed against the gene for the ubiquitin carboxyl terminal hydrolase 24
<400> 109
   ggacgagaat tgataaagat tcaagagatc tttatcaatt ctcgtcc 47
<210> 110
   <211> 47
   <212> DNA
   <213> Artificial Sequence
<220>
   <223> Description of Artificial Sequence: DNA encoding an shRNA directed against the gene for the ubiquitin carboxyl-terminal hydrolase 24
<400> 110
   gcacgagaat ttgggaatct tcaagagaga ttcccaaatt ctcgtgc 47
<210> 111
   <211> 47
<212> DNA
   <213> Artificial Sequence
<220>
   <223> Description of Artificial Sequence: DNA encoding an shRNA directed against the gene for the ubiquitin carboxyl-terminal hydrolase 24
<400> 111
   ctacttcatg aaatattggt tcaagagacc aatatttcat gaagtag 47
<210> 112
   <211> 47
   <212> DNA
   <213> Artificial Sequence
<220>
   <223> Description of Artificial Sequence: DNA encoding an shRNA directed against the KIAA1594 gene
<400> 112
   gataacagct tcttgtctat tcaagagata gacaagaagc tgttatc 47
<210> 113
   <211> 47
   <212> DNA
   <213> Artificial Sequence
<220>
   <223> Description of Artificial Sequence: DNA encoding an shRNA directed against the KIAA1594 gene
<400> 113
   gagaatagga catcagggct tcaagagagc cctgatgtcc tattctc 47
<210> 114
   <211> 47
   <212> DNA
   <213> Artificial Sequence
<220>
   <223> Description of Artificial Sequence: DNA encoding an shRNA directed against the KIAA1594 gene
<400> 114
   cttggaagac tgaacctgtt tcaagagaac aggttcagtc ttccaag 47
<210> 115
   <211> 47
   <212> DNA
   <213> Artificial Sequence
<220>
   <223> Description of Artificial Sequence: DNA encoding an shRNA directed against the KIAA1594 gene
<400> 115
   caactccttt gtggatgcat tcaagagatg catccacaaa ggagttg 47
<210> 116
   <211> 47
   <212> DNA
   <213> Artificial Sequence
<220>
   <223> Description of Artificial Sequence: DNA encoding an shRNA directed against the KIAA1350 gene
<400> 116
   gatgttgtct ccaaatgcat tcaagagatg catttggaga caacatc 47
<210> 117
   <211> 47
   <212> DNA
   <213> Artificial Sequence
<220>
   <223> Description of Artificial Sequence: DNA encoding an shRNA directed against the KIAA1350 gene
<400> 117
   cgtggggact gtacctccct tcaagagagg gaggtacagt ccccacg 47
<210> 118
   <211> 47
   <212> DNA
   <213> Artificial Sequence
<220>
   <223> Description of Artificial Sequence: DNA encoding an shRNA direcetd against the KIAA1350 gene
<400> 118
   gtacagcttc agaaccaagt tcaagagact tggttctgaa gctgtac 47
<210> 119
   <211> 47
   <212> DNA
   <213> Artificial Sequence
<220>
   <223> Description of Artificial Sequence: DNA encoding an shRNA directed against the gene for the ubiquitin carboxyl-terminal hydrolase 25
<400> 119
   gatgatcttc agagagcaat tcaagagatt gctctctgaa gatcatc 47
<210> 120
   <211> 47
   <212> DNA
   <213> Artificial Sequence
<220>
   <223> Description of Artificial Sequence: DNA encoding an shRNA directed against the gene for the ubiquitin carboxyl-terminal hydrolase 25
<400> 120
   ggaacatcgg aatttgcctt tcaagagaag gcaaattccg atgttcc 47
<210> 121
   <211> 47
   <212> DNA
   <213> Artificial Sequence
<220>
   <223> Description of Artificial Sequence: DNA encoding an shRNA directed against the gene for the ubiquitin carboxyl-terminal hydrolase 25
<400> 121
   gagctagtga gggactcttt tcaagagaaa gagtccctca ctagctc 47
<210> 122
   <211> 47
   <212> DNA
   <213> Artificial Sequence
<220>
   <223> Description of Artificial Sequence: DNA encoding an shRNA directed against the gene for the ubiquitin carboxyl-terminal hydrolase 25
<400> 122
   gcagggttct ttaaggcaat tcaagagatt gccttaaaga accctgc 47
<210> 123
   <211> 47
   <212> DNA
   <213> Artificial Sequence
<220>
   <223> Description of Artificial Sequence: DNA encoding an shRNA directed against the gene for the ubiquitin carboxyl-terminal hydrolase 16
<400> 123
   tcgatgattc ctctgaaact tcaagagagt ttcagaggaa tcatcga 47
<210> 124
   <211> 47
   <212> DNA
   <213> Artificial Sequence
<220>
   <223> Description of Artificial Sequence: DNA encoding an shRNA directed against the gene for the ubiquitin carboxyl-terminal hydrolase 16
<400> 124
   gataatggaa atattgaact tcaagagagt tcaatatttc cattatc 47
<210> 125
   <211> 47
<212> DNA
   <213> Artificial Sequence
<220>
   <223> Description of Artificial Sequence: DNA encoding an shRNA directed against the gene for the ubiquitin carboxyl-terminal hydrolase 16
<400> 125
   gttcttcatt taaatgatat tcaagagata tcatttaaat gaagaac 47
<210> 126
   <211> 47
   <212> DNA
   <213> Artificial Sequence
<220>
   <223> Description of Artificial Sequence: DNA encoding an shRNA directed against the gene for the ubiquitin carboxyl-terminal hydrolase 16
<400> 126
   gttaacaaac acataaagtt tcaagagaac tttatgtgtt tgttaac 47
<210> 127
   <211> 47
   <212> DNA
   <213> Artificial Sequence
<220>
   <223> Description of Artificial Sequence: DNA encoding an shRNA directed against the USP9X gene
<400> 127
   gttagagaag attcttcgtt tcaagagaac gaagaatctt ctctaac 47
<210> 128
   <211> 47
   <212> DNA
   <213> Artificial Sequence
<220>
   <223> Description of Artificial Sequence: DNA encoding an shRNA directed against the USP9X gene
<400> 128
   gttgattgga caattaaact tcaagagagt ttaattgtcc aatcaac 47
<210> 129
   <211> 47
   <212> DNA
   <213> Artificial Sequence
<220>
   <223> Description of Artificial Sequence: DNA encoding an shRNA directed against the USP9X gene
<400> 129
   ggttgatacc gtaaagcgct tcaagagagc gctttacggt atcaacc 47
<210> 130
   <211> 47
   <212> DNA
   <213> Artificial Sequence
<220>
   <223> Description of Artificial Sequence: DNA encoding an shRNA directed against the USP9X gene
<400> 130
   gcaatgaaac gtccaatggt tcaagagacc attggacgtt tcattgc 47
<210> 131
   <211> 47
   <212> DNA
   <213> Artificial Sequence
<220>
   <223> Description of Artificial Sequence: DNA encoding an shRNA directed against the USP9Y gene
<400> 131
   agctagagaa aattcttcgt tcaagagacg aagaattttc tctagct 47
<210> 132
   <211> 47
   <212> DNA
   <213> Artificial Sequence
<220>
   <223> Description of Artificial Sequence: DNA encoding an shRNA directed against the USP9Y gene
<400> 132
   gatcctatat gatggatgat tcaagagatc atccatcata taggatc 47
<210> 133
   <211> 47
   <212> DNA
   <213> Artificial Sequence
<220>
   <223> Description of Artificial Sequence: DNA encoding an shRNA directed against the USP9Y gene
<400> 133
   gttcttcttg tcagtgaaat tcaagagatt tcactgacaa gaagaac 47
<210> 134
   <211> 47
   <212> DNA
   <213> Artificial Sequence
<220>
   <223> Description of Artificial Sequence: DNA encoding an shRNA directed against the USP9Y gene
<400> 134
   cttgagcttg agtgaccact tcaagagagt ggtcactcaa gctcaag 47
<210> 135
   <211> 47
   <212> DNA
   <213> Artificial Sequence
<220>
   <223> Description of Artificial Sequence: DNA encoding an shRNA direcetd against the gene for the ubiquitin carboxyl-terminal hydrolase 5
<400> 135
   gaccggccag cgagtctact tcaagagagt agactcgctg gccggtc 47
<210> 136
   <211> 47
   <212> DNA
   <213> Artificial Sequence
<220>
   <223> Description of Artificial Sequence: DNA encoding an shRNA directed against the gene for the ubiquitin carboxyl-terminal hydrolase 5
<400> 136
   ggacctgggc tacatctact tcaagagagt agatgtagcc caggtcc 47
<210> 137
   <211> 47
   <212> DNA
   <213> Artificial Sequence
<220>
   <223> Description of Artificial Sequence: DNA encoding an shRNA directed against the gene for the ubiquitin carboxyl-terminal hydrolase 5
<400> 137
   ctctgtggtc caggtgctct tcaagagaga gcacctggac cacagag 47
<210> 138
   <211> 47
   <212> DNA
   <213> Artificial Sequence
<220>
   <223> Description of Artificial Sequence: DNA encoding an shRNA directed against the gene for the ubiquitin carboxyl-terminal hydrolase 5
<400> 138
   gaccacacga tttgcctcat tcaagagatg aggcaaatcg tgtggtc 47
<210> 139
   <211> 47
   <212> DNA
   <213> Artificial Sequence
<220>
   <223> Description of Artificial Sequence: DNA encoding an shRNA directed against the gene for the ubiquitin carboxyl-terminal hydrolase 26
<400> 139
   tggcttgttt attgaaggat tcaagagatc cttcaataaa caagcca 47
<210> 140
   <211> 47
   <212> DNA
   <213> Artificial Sequence
<220>
   <223> Description of Artificial Sequence: DNA encoding an shRNA directed against the gene for the ubiquitin carboxyl-terminal hydrolase 26
<400> 140
   gtgaatttgg ggaagataat tcaagagatt atcttcccca aattcac 47
<210> 141
   <211> 47
   <212> DNA
   <213> Artificial Sequence
<220>
   <223> Description of Artificial Sequence: DNA encoding an shRNA directed against the gene for the ubiquitin carboxyl- terminal hydrolase 26
<400> 141
   cgctatagct tgaatgagtt tcaagagaac tcattcaagc tatagcg 47
<210> 142
   <211> 47
   <212> DNA
   <213> Artificial Sequence
<220>
   <223> Description of Artificial Sequence: DNA encoding an shRNA directed against the gene for the ubiquitin carboxyl- terminal hydrolase 26
<400> 142
   gatatcctgg ctccacacat tcaagagatg tgtggagcca ggatatc 47
<210> 143
   <211> 47
   <212> DNA
   <213> Artificial Sequence
<220>
   <223> Description of Artificial Sequence: DNA encoding an shRNA directed against the KIAA1097 gene
<400> 143
   gagccagtcg gatgtagatt tcaagagaat ctacatccga ctggctc 47
<210> 144
   <211> 47
   <212> DNA
   <213> Artificial Sequence
<220>
   <223> Description of Artificial Sequence: DNA encoding an shRNA directed against the KIAA1097 gene
<400> 144
   gtaaattctg aaggcgaatt tcaagagaat tcgccttcag aatttac 47
<210> 145
   <211> 47
   <212> DNA
   <213> Artificial Sequence
<220>
   <223> Description of Artificial Sequence: DNA encoding an shRNA directed against the KIAA1097 gene
<400> 145
   gccctcctaa atcaggcaat tcaagagatt gcctgattta ggagggc 47
<210> 146
   <211> 47
   <212> DNA
   <213> Artificial Sequence
<220>
   <223> Description of Artificial Sequence: DNA encoding an shRNA directed against the KIAA1097 gene
<400> 146
   gttgagaaat ggagtgaagt tcaagagact tcactccatt tctcaac 47
<210> 147
   <211> 47
   <212> DNA
   <213> Artificial Sequence
<220>
   <223> Description of Artificial Sequence: DNA encoding an shRNA directed against the gene for the ubiquitin specific protease (USP22) gene
<400> 147
   gcttggaaaa tgcaaggcgt tcaagagacg ccttgcattt tccaagc 47
<210> 148
   <211> 47
   <212> DNA
   <213> Artificial Sequence
<220>
   <223> Description of Artificial Sequence: DNA encoding an shRNA directed against the gene for the ubiquitin specific protease (USP22) gene
<400> 148
   ctgcatcata gaccagatct tcaagagaga tctggtctat gatgcag 47
<210> 149
   <211> 47
   <212> DNA
   <213> Artificial Sequence
<220>
   <223> Description of Artificial Sequence: DNA encoding an shRNA directed against the gene for the ubiquitin specific protease (USP22) gene
<400> 149
   gatcaccacg tatgtgtcct tcaagagagg acacatacgt ggtgatc 47
<210> 150
   <211> 47
   <212> DNA
   <213> Artificial Sequence
<220>
   <223> Description of Artificial Sequence: DNA encoding an shRNA directed against the gene for the ubiquitin specific protease 22 (USP22) gene
<400> 150
   tgacaacaag tattccctgt tcaagagaca gggaatactt gttgtca 47
<210> 151
   <211> 47
   <212> DNA
   <213> Artificial Sequence
<220>
   <223> Description of Artificial Sequence: DNA encoding an shRNA directed against the gene for the ubiquitin specific processing protease 29
<400> 151
   gaaatataag acagattcct tcaagagagg aatctgtctt atatttc 47
<210> 152
   <211> 47
   <212> DNA
   <213> Artificial Sequence
<220>
   <223> Description of Artificial Sequence: DNA encoding an shRNA directed against the gene for the ubiquitin specific processing protease 29
<400> 152
   cccatcaagt ttagaggatt tcaagagaat cctctaaact tgatggg 47
<210> 153
   <211> 47
   <212> DNA
   <213> Artificial Sequence
<220>
   <223> Description of Artificial Sequence: DNA encoding an shRNA directed against the gene for the ubiquitin specific processing protease 29
<400> 153
   ggtgtcccat gggaatatat tcaagagata tattcccatg ggacacc 47
<210> 154
   <211> 47
   <212> DNA
   <213> Artificial Sequence
<220>
   <223> Description of Artificial Sequence: DNA encoding an shRNA directed against the gene for the ubiquitin specific processing protease 29
<400> 154
   gaatgccgac ctacaaagat tcaagagatc tttgtaggtc ggcattc 47
<210> 155
   <211> 47
   <212> DNA
   <213> Artificial Sequence
<220>
   <223> Description of Artificial Sequence: DNA encoding an shRNA directed against the CYLD gene
<400> 155
   cagttatatt ctgtgatgtt tcaagagaac atcacagaat ataactg 47
<210> 156
   <211> 47
   <212> DNA
   <213> Artificial Sequence
<220>
   <223> Description of Artificial Sequence: DNA encoding an shRNA directed against the CYLD gene
<400> 156
   gaggtgttgg ggacaaaggt tcaagagacc tttgtcccca acacctc 47
<210> 157
   <211> 47
   <212> DNA
   <213> Artificial Sequence
<220>
   <223> Description of Artificial Sequence: DNA encoding an shRNA directed against the CYLD gene
<400> 157
   gtgggctcat tggctgaagt tcaagagact tcagccaatg agcccac 47
<210> 158
   <211> 47
   <212> DNA
   <213> Artificial Sequence
<220>
   <223> Description of Artificial Sequence: DNA encoding an shRNA directed against the CYLD gene
<400> 158
   gagctactga ggacagaaat tcaagagatt tctgtcctca gtagctc 47
<210> 159
   <211> 47
   <212> DNA
   <213> Artificial Sequence
<220>
   <223> Description of Artificial Sequence: DNA encoding an shRNA directed against the gene for the ubiquitin carboxyl-terminal hydrolase 2
<400> 159
   tcagcaggat gctcaggagt tcaagagact cctgagcatc ctgctga 47
<210> 160
   <211> 47
   <212> DNA
   <213> Artificial Sequence
<220>
   <223> Description of Artificial Sequence: DNA encoding an shRNA directed against the gene for the ubiquitin carboxyl-terminal hydrolase 2
<400> 160
   gaagttctcc atccagaggt tcaagagacc tctggatgga gaacttc 47
<210> 161
   <211> 47
   <212> DNA
   <213> Artificial Sequence
<220>
   <223> Description of Artificial Sequence: DNA encoding an shRNA directed against the gene for the ubiquitin carboxyl-terminal hydrolase 2
<400> 161
   gccggtcccc accagcagct tcaagagagc tgctggtggg gaccggc 47
<210> 162
   <211> 47
   <212> DNA
   <213> Artificial Sequence
<220>
   <223> Description of Artificial Sequence: DNA encoding an shRNA directed against the gene for the ubiquitin carboxyl-terminal hydrolase 2
<400> 162
   cactcgggag ttgagagatt tcaagagaat ctctcaactc ccgagtg 47
<210> 163
   <211> 47
   <212> DNA
   <213> Artificial Sequence
<220>
   <223> Description of Artificial Sequence: DNA encoding an shRNA directed against the gene for the ubiquitin specific protease 3 (USP3)
<400> 163
   gcccttgggt ctgtttgact tcaagagagt caaacagacc caagggc 47
<210> 164
   <211> 47
   <212> DNA
   <213> Artificial Sequence
<220>
   <223> Description of Artificial Sequence: DNA encoding an shRNA directed against the gene for the ubiquitin specific protease 3 (USP3)
<400> 164
   ctcaacacta aacagcaagt tcaagagact tgctgtttag tgttgag 47
<210> 165
   <211> 47
   <212> DNA
   <213> Artificial Sequence
<220>
   <223> Description of Artificial Sequence: DNA encoding an shRNA directed against the gene for the ubiquitin specific protease 3 (USP3)
<400> 165
   gatttcattg gacagcatat tcaagagata tgctgtccaa tgaaatc 47
<210> 166
   <211> 47
   <212> DNA
   <213> Artificial Sequence
<220>
   <223> Description of Artificial Sequence: DNA encoding an shRNA directed against the gene for the ubiquitin specific protease 3 (USP3)
<400> 166
   catggggcac caactaattt tcaagagaaa ttagttggtg ccccatg 47
<210> 167
   <211> 47
   <212> DNA
   <213> Artificial Sequence
<220>
   <223> Description of Artificial Sequence: DNA encoding an shRNA directed against the gene for the ubiquitin carboxyl-terminal hydrolase 23
<400> 167
   ggtgtctctg cgggattgtt tcaagagaac aatcccgcag agacacc 47
<210> 168
   <211> 47
   <212> DNA
   <213> Artificial Sequence
<220>
   <223> Description of Artificial Sequence: DNA encoding an shRNA directed against the gene for the ubiquitin carboxyl-terminal hydrolase 23
<400> 168
   agttcagtag gtgtagactt tcaagagaag tctacaccta ctgaact 47
<210> 169
   <211> 47
   <212> DNA
   <213> Artificial Sequence
<220>
   <223> Description of Artificial Sequence: DNA encoding an shRNA directed against the gene for the ubiquitin carboxyl-terminal hydrolase 23
<400> 169
   gagttcctga agctcctcat tcaagagatg aggagcttca ggaactc 47
<210> 170
   <211> 47
   <212> DNA
   <213> Artificial Sequence
<220>
   <223> Description of Artificial Sequence: DNA encoding an shRNA directed against the gene for the ubiquitin carboxyl-terminal hydrolase 23
<400> 170
   ggatttgctg ggggcaaggt tcaagagacc ttgcccccag caaatcc 47
<210> 171
   <211> 47
   <212> DNA
   <213> Artificial Sequence
<220>
   <223> Description of Artificial Sequence: DNA encoding an shRNA directed against the UBP-32.7 gene
<400> 171
   ctcagaaagc caacattcat tcaagagatg aatgttggct ttctgag 47
<210> 172
   <211> 47
   <212> DNA
   <213> Artificial Sequence
<220>
   <223> Description of Artificial Sequence: DNA encoding an shRNA directed against the UBP-32.7 gene
<400> 172
   cgcattgtaa taagaaggtt tcaagagaac cttcttatta caatgcg 47
<210> 173
   <211> 47
   <212> DNA
   <213> Artificial Sequence
<220>
   <223> Description of Artificial Sequence: DNA encoding an shRNA directed against the UBP-32.7 gene
<400> 173
   gggaggaaaa tgcagaaatt tcaagagaat ttctgcattt tcctccc 47
<210> 174
   <211> 47
   <212> DNA
   <213> Artificial Sequence
<220>
   <223> Description of Artificial Sequence: DNA encoding an shRNA directed against the UBP-32.7 gene
<400> 174
   ttacaaattt aggaaatact tcaagagagt atttcctaaa tttgtaa 47
<210> 175
   <211> 47
   <212> DNA
   <213> Artificial Sequence
<220>
   <223> Description of Artificial Sequence: DNA encoding an shRNA directed against the gene for the Homo sapiens ubiquitin specific protease 13 (isopeptidase T-3)
<400> 175
   gttatgaatt gatatgcagt tcaagagact gcatatcaat tcataac 47
<210> 176
   <211> 47
   <212> DNA
   <213> Artificial Sequence
<220>
   <223> Description of Artificial Sequence: DNA encoding an shRNA directed against the gene for the Homo sapiens ubiquitin specific protease 13 (isopeptidase T-3)
<400> 176
   gtgataacac aactaatggt tcaagagacc attagttgtg ttatcac 47
<210> 177
   <211> 47
   <212> DNA
   <213> Artificial Sequence
<220>
   <223> Description of Artificial Sequence: DNA encoding an shRNA directed against the gene for the Homo sapiens ubiquitin specific protease 13 (isopeptidase T-3)
<400> 177
   gtagaggaga gttctgaaat tcaagagatt tcagaactct cctctac 47
<210> 178
   <211> 47
   <212> DNA
   <213> Artificial Sequence
<220>
   <223> Description of Artificial Sequence: DNA encoding an shRNA directed against the gene for the Homo sapiens ubiquitin specific protease 13 (isopeptidase T-3)
<400> 178
   gcctctaatc ctgataaggt tcaagagacc ttatcaggat tagaggc 47
<210> 179
   <211> 47
   <212> DNA
   <213> Artificial Sequence
<220>
   <223> Description of Artificial Sequence: DNA encoding an shRNA directed against the gene for the ubiquitin carboxyl-terminal hydrolase 28
<400> 179
   gatgatcttc aggctgccat tcaagagatg gcagcctgaa gatcatc 47
<210> 180
   <211> 47
   <212> DNA
   <213> Artificial Sequence
<220>
   <223> Description of Artificial Sequence: DNA encoding an shRNA directed against the gene for the ubiquitin carboxyl-terminal hydrolase 28
<400> 180
   gtatggacaa gagcgttggt tcaagagacc aacgctcttg tccatac 47
<210> 181
   <211> 47
   <212> DNA
   <213> Artificial Sequence
<220>
   <223> Description of Artificial Sequence: DNA encoding an shRNA directed against the gene for the ubiquitin carboxyl-terminal hydrolase 28
<400> 181
   cgaacccttc tggaacagtt tcaagagaac tgttccagaa gggttcg 47
<210> 182
   <211> 47
   <212> DNA
   <213> Artificial Sequence
<220>
   <223> Description of Artificial Sequence: DNA encoding an shRNA directed against the gene for the ubiquitin carboxyl-terminal hydrolase 28
<400> 182
   gtggcatgaa gattatagtt tcaagagaac tataatcttc atgccac 47
<210> 183
   <211> 47
   <212> DNA
   <213> Artificial Sequence
<220>
   <223> Description of Artificial Sequence: DNA encoding an shRNA directed against the ubiquitin carboxyl-terminal hydrolase 14
<400> 183
   ggtgaacaag gacagtatct tcaagagaga tactgtcctt gttcacc 47
<210> 184
   <211> 47
   <212> DNA
   <213> Artificial Sequence
<220>
   <223> Description of Artificial Sequence: DNA encoding an shRNA directed against the gene for the ubiquitin carboxyl-terminal hydrolase 14
<400> 184
   gcaatagagg atgattctgt tcaagagaca gaatcatcct ctattgc 47
<210> 185
   <211> 47
   <212> DNA
   <213> Artificial Sequence
<220>
   <223> Description of Artificial Sequence: DNA encoding an shRNA directed against the gene for the ubiquitin carboxyl-terminal hydrolase 14
<400> 185
   tctgtgaatg ccaaagttct tcaagagaga actttggcat tcacaga 47
<210> 186
   <211> 47
   <212> DNA
   <213> Artificial Sequence
<220>
   <223> Description of Artificial Sequence: DNA encoding an shRNA directed against the gene for the ubiquitin carboxyl-terminal hydrolase 14
<400> 186
   cacaccaggg aaggtctagt tcaagagact agaccttccc tggtgtg 47
<210> 187
   <211> 47
   <212> DNA
   <213> Artificial Sequence
<220>
   <223> Description of Artificial Sequence: DNA encoding an shRNA directed against the DUB1 gene
<400> 187
   gcaggaagat gcccatgaat tcaagagatt catgggcatc ttcctgc 47
<210> 188
   <211> 47
   <212> DNA
   <213> Artificial Sequence
<220>
   <223> Description of Artificial Sequence: DNA encoding an shRNA directed agains the DUB1 gene
<400> 188
   gaatgtgcaa tatcctgagt tcaagagact caggatattg cacattc 47
<210> 189
   <211> 47
   <212> DNA
   <213> Artificial Sequence
<220>
   <223> Description of Artificial Sequence: DNA encoding an shRNA directed against the DUB1 gene
<400> 189
   tggatgatgc caaggtcact tcaagagagt gaccttggca tcatcca 47
<210> 190
   <211> 47
   <212> DNA
   <213> Artificial Sequence
<220>
   <223> Description of Artificial Sequence: DNA encoding an shRNA directed against the DUB1 gene
<400> 190
   gctccgtgct aaacctctct tcaagagaga gaggtttagc acggagc 47
<210> 191
   <211> 47
   <212> DNA
   <213> Artificial Sequence
<220>
   <223> Description of Artificial Sequence: DNA encoding an shRNA directed against the gene for the mouse USP27 homolog
<400> 191
   gcctccacct caacagaggt tcaagagacc tctgttgagg tggaggc 47
<210> 192
   <211> 47
   <212> DNA
   <213> Artificial Sequence
<220>
   <223> Description of Artificial Sequence: DNA encoding an shRNA directed against the gene for the mouse USP27 homolog
<400> 192
   ctgcatcata gaccaaatct tcaagagaga tttggtctat gatgcag 47
<210> 193
   <211> 47
   <212> DNA
   <213> Artificial Sequence
<220>
   <223> Description of Artificial Sequence: DNA encoding an shRNA directed against the gene for the mouse USP27 homolog

<400> 193
   gatcactaca tacatttcct tcaagagagg aaatgtatgt agtgatc 47
<210> 194
   <211> 47
   <212> DNA
   <213> Artificial Sequence
<220>
   <223> Description of Artificial Sequence: DNA encoding an shRNA directed against the gene for the mouse USP27 homolog
<400> 194
   gtaaagagag cagaatgaat tcaagagatt cattctgctc tctttac 47
<210> 195
   <211> 47
   <212> DNA
   <213> Artificial Sequence
<220>
   <223> Description of Artificial Sequence: DNA encoding an shRNA directed against the gene for the ubiquitin carboxyl-terminal hydrolase 4
<400> 195
   cgcggggcgc agtggtatct tcaagagaga taccactgcg ccccgcg 47
<210> 196
   <211> 47
   <212> DNA
   <213> Artificial Sequence
<220>
   <223> Description of Artificial Sequence: DNA encoding an shRNA directed against the gene for the ubiquitin carboxyl-terminal hydrolase 4
<400> 196
   cagaaggcag tggggaagat tcaagagatc ttccccactg ccttctg 47
<210> 197
   <211> 47
   <212> DNA
   <213> Artificial Sequence
<220>
   <223> Description of Artificial Sequence: DNA encoding an shRNA directed against the gene for the ubiquitin carboxyl-terminal hydrolase 4
<400> 197
   gcctgggaga atcacaggtt tcaagagaac ctgtgattct cccaggc 47
<210> 198
   <211> 47
   <212> DNA
   <213> Artificial Sequence
<220>
   <223> Description of Artificial Sequence: DNA encoding an shRNA directed against the gene for the ubiquitin carboxyl-terminal hydrolase 4
<400> 198
   accagacaag gaaataccct tcaagagagg gtatttcctt gtctggt 47
<210> 199
   <211> 47
   <212> DNA
   <213> Artificial Sequence
<220>
   <223> Description of Artificial Sequence: DNA encoding an shRNA directed against the TRE-2 gene
<400> 199
   cacatccacc acatcgacct tcaagagagg tcgatgtggt ggatgtg 47
<210> 200
   <211> 47
   <212> DNA
   <213> Artificial Sequence
<220>
   <223> Description of Artificial Sequence: DNA encoding an shRNA directed against the TRE-2 gene
<400> 200
   gtcacaaccc aagaccatgt tcaagagaca tggtcttggg ttgtgac 47
<210> 201
   <211> 47
   <212> DNA
   <213> Artificial Sequence
<220>
   <223> Description of Artificial Sequence: DNA encoding an shRNA directed against the TRE-2 gene
<400> 201
   ctcaacagga caaatcccat tcaagagatg ggatttgtcc tgttgag 47
<210> 202
   <211> 47
   <212> DNA
   <213> Artificial Sequence
<220>
   <223> Description of Artificial Sequence: DNA encoding an shRNA directed against the TRE-2 gene
<400> 202
   tagatcaatt attgtggatt tcaagagaat ccacaataat tgatcta 47
<210> 203
   <211> 47
   <212> DNA
   <213> Artificial Sequence
<220>
   <223> Description of Artificial Sequence: DNA encoding an shRNA directed against the gene for the ubiquitin carboxyl-terminal hydrolase 15 (UNPH-2)
<400> 203
   ggaacacctt attgatgaat tcaagagatt catcaataag gtgttcc 47
<210> 204
   <211> 47
   <212> DNA
   <213> Artificial Sequence
<220>
   <223> Description of Artificial Sequence: DNA encoding an shRNA directed against the gene for the ubiquitin carboxyl-terminal hydrolase 15 (UNPH-2)
<400> 204
   ctttaacaga aattgtctct tcaagagaga gacaatttct gttaaag 47
<210> 205
   <211> 47
   <212> DNA
   <213> Artificial Sequence
<220>
   <223> Description of Artificial Sequence: DNA encoding an shRNA directed agaainst the gene for the ubiquitin carboxyl-terminal hydrolase 15 (UNPH-2)
<400> 205
   cctatgcagt acaaagtggt tcaagagacc actttgtact gcatagg 47
<210> 206
   <211> 47
   <212> DNA
   <213> Artificial Sequence
<220>
   <223> Description of Artificial Sequence: DNA encoding an shRNA directed against the gene for the ubiquitin carboxyl-terminal hydrolase 15 (UNPH-2)
<400> 206
   gatcttttct tgctttggat tcaagagatc caaagcaaga aaagatc 47
<210> 207
   <211> 47
   <212> DNA
   <213> Artificial Sequence
<220>
   <223> Description of Artificial Sequence: DNA encoding an shRNA directed against the KIAA1372 gene
<400> 207
   cagcatcctt caggccttat tcaagagata aggcctgaag gatgctg 47
<210> 208
   <211> 47
   <212> DNA
   <213> Artificial Sequence
<220>
   <223> Description of Artificial Sequence: DNA encoding an shRNA directed against the KIAA1372 gene
<400> 208
   gatagtgact cggatctgct tcaagagagc agatccgagt cactatc 47
<210> 208
   <211> 47
   <212> DNA
   <213> Artificial Sequence
<220>
   <223> Description of Artificial Sequence: DNA encoding an shRNA directed against the KIAA1372 gene
<400> 209
   gacatcacag cccgggagtt tcaagagaac tcccgggctg tgatgtc 47
<210> 210
   <211> 47
   <212> DNA
   <213> Artificial Sequence
<220>
   <223> Description of Artificial Sequence: DNA encoding an shRNA directed against the KIAA1372 gene
<400> 210
   ggacacagcc tatgtgctgt tcaagagaca gcacataggc tgtgtcc 47
<210> 211
   <211> 47
   <212> DNA
   <213> Artificial Sequence
<220>
   <223> Description of Artificial Sequence: DNA encoding an shRNA directed against the gene for the BRCA1 associated protein-1
<400> 211
   gtggaggaga tctacgacct tcaagagagg tcgtagatct cctccac 47
<210> 212
   <211> 47
   <212> DNA
   <213> Artificial Sequence
<220>
   <223> Description of Artificial Sequence: DNA encoding an shRNA directed against the gene for the BRCA1 associated protein-1
<400> 212
   ctcttgtgca actcatgcct tcaagagagg catgagttgc acaagag 47
<210> 213
   <211> 47
   <212> DNA
   <213> Artificial Sequence
<220>
   <223> Description of Artificial Sequence: DNA encoding an shRNA directed against the gene for the BRCA1 associated protein-1
<400> 213
   acagggcccc tgcagcctct tcaagagaga ggctgcaggg gccctgt 47
<210> 214
   <211> 47
   <212> DNA
   <213> Artificial Sequence
<220>
   <223> Description of Artificial Sequence: DNA encoding an shRNA directed against the gene for the BRCA1 associated protein-1
<400> 214
   gaagacctgg cggcaggtgt tcaagagaca cctgccgcca ggtcttc 47
<210> 215
   <211> 21
   <212> DNA
   <213> Artificial Sequence
<220>
   <223> Description of Artificial Sequence: siRNA
<400> 215
   gcctgtgcct cttcagctac c 21
<210> 216
   <211> 21
   <212> DNA
   <213> Artificial Sequence
<220>
   <223> Description of Artificial Sequence: siRNA
<400> 216
   gcggagacag cgacgaagag c 21
<210> 217
   <211> 19
   <212> DNA
   <213> Artificial Sequence
<220>
   <223> Description of Artificial Sequence: siRNA
<400> 217
   cttattggag agagcacga 19
<210> 218
   <211> 9169
   <212> DNA
   <213> Artificial Sequence
<220>
   <223> Description of Artificial Sequence: RMCE vector with shRNA against LacZ and CAGGS-Fluc
<400> 218
   gggtaactaa gtaaggatcg agatccaggc gcggatcaat aaaagatcat tattttcaat 60
   agatctgtgt gttggttttt tgtgtgcctt gggggagggg gaggccagaa tgaggcgcgg 120
   ccaaggggga gggggaggcc agaatgacct tgggggaggg ggaggccaga atgaccttgg 180
   gggaggggga ggccagaatg aggcgcggat ccggagaagt tcctattccg aagttcctat 240
   tcttcaaata gtataggaac ttcgctcgag ggatcggcca ttgaacaaga tggattgcac 300
   gcaggttctc cggccgcttg ggtggagagg ctattcggct atgactgggc acaacagaca 360
   atcggctgct ctgatgccgc cgtgttccgg ctgtcagcgc aggggcgccc ggttcttttt 420
   gtcaagaccg acctgtccgg tgccctgaat gaactgcagg acgaggcagc gcggctatcg 480
   tggctggcca cgacgggcgt tccttgcgca gctgtgctcg acgttgtcac tgaagcggga 540
   agggactggc tgctattggg cgaagtgccg gggcaggatc tcctgtcatc tcaccttgct 600
   cctgccgaga aagtatccat catggctgat gcaatgcggc ggctgcatac gcttgatccg 660
   gctacctgcc cattcgacca ccaagcgaaa catcgcatcg agcgagcacg tactcggatg 720
   gaagccggtc ttgtcgatca ggatgatctg gacgaagagc atcaggggct cgcgccagcc 780
   gaactgttcg ccaggctcaa ggcgcgcatg cccgacggcg aggatctcgt cgtgacccat 840
   ggcgatgcct gcttgccgaa tatcatggtg gaaaatggcc gcttttctgg attcatcgac 900
   tgtggccggc tgggtgtggc ggaccgctat caggacatag cgttggctac ccgtgatatt 960
   gctgaagagc ttggcggcga atgggctgac cgcttcctcg tgctttacgg tatcgccgct 1020
   cccgattcgc agcgcatcgc cttctatcgc cttcttgacg agttcttctg aggggatcga 1080
   tccgctgtaa gtctgcagaa attgatgatc tattaaacaa taaagatgtc cactaaaatg 1140
   gaagtttttc ctgtcatact ttgttaagaa gggtgagaac agagtaccta cattttgaat 1200
   ggaaggattg gagctacggg ggtgggggtg gggtgggatt agataaatgc ctgctcttta 1260
   ctgaaggctc tttactattg ctttatgata atgtttcata gttggatatc ataatttaaa 1320
   caagcaaaac caaattaagg gccagctcat tcctcccact catgatctat agatctatag 1380
   atctctcgtg ggatcattgt ttttctcttg attcccactt tgtggttcta agtactgtgg 1440
   tttccaaatg tgtcagtttc atagcctgaa gaacgagatc agcagcctct gttccacata 1500
   cacttcattc tcagtattgt tttgccaagt tctaattcca tcagaagctg actctagatg 1560
   gcgcgtatgc attaattaag gccatagcgg ccatttaaat cggccggcca tttaaatagt 1620
   tacgctaggg ataacagggt aatatagtta attaatctag aactagtgga tccagtggaa 1680
   agacgcgcag gcaaaacgca ccacgtgacg gagcgtgacc gcgcgccgag cccaaggtcg 1740
   ggcaggaaga gggcctattt cccatgattc cttcatattt gcatatacga tacaaggctg 1800
   ttagagagat aattagaatt aatttgactg taaacacaaa gatattagta caaaatacgt 1860
   gacgtagaaa gtaataattt cttgggtagt ttgcagtttt aaaattatgt tttaaaatgg 1920
   actatcatat gcttaccgta acttgaaagt atttcgattt cttggcttta tatatcttgt 1980
   ggaaaggacg aaacaccgtg gatggagccg atattggatt caagagattc aatattggct 2040
   tcatccactt tttggcgcgt gctagcccgg gctagcttgc atgcctgcag gttttcgaca 2100
   ttgattattg actagttatt aatagtaatc aattacgggg tcattagttc atagcccata 2160
   tatggagttc cgcgttacat aacttacggt aaatggcccg cctggctgac cgcccaacga 2220
   cccccgccca ttgacgtcaa taatgacgta tgttcccata gtaacgccaa tagggacttt 2280
   ccattgacgt caatgggtgg actatttacg gtaaactgcc cacttggcag tacatcaagt 2340
   gtatcatatg ccaagtacgc cccctattga cgtcaatgac ggtaaatggc ccgcctggca 2400
   ttatgcccag tacatgacct tatgggactt tcctacttgg cagtacatct acgtattagt 2460
   catcgctatt accatgggtc gaggtgagcc ccacgttctg cttcactctc cccatctccc 2520
   ccccctcccc acccccaatt ttgtatttat ttatttttta attattttgt gcagcgatgg 2580
   gggcgggggg ggggggggcg cgcgccaggc ggggcggggc ggggcgaggg gcggggcggg 2640
   gcgaggcgga gaggtgcggc ggcagccaat cagagcggcg cgctccgaaa gtttcctttt 2700
   atggcgaggc ggcggcggcg gcggccctat aaaaagcgaa gcgcgcggcg ggcgggagtc 2760
   gctgcgttgc cttcgccccg tgccccgctc cgcgccgcct cgcgccgccc gccccggctc 2820
   tgactgaccg cgttactccc acaggtgagc gggcgggacg gcccttctcc tccgggctgt 2880
   aattagcgct tggtttaatg acggctcgtt tcttttctgt ggctgcgtga aagccttaaa 2940
   gggctccggg agggcccttt gtgcgggggg gagcggctcg gggggtgcgt gcgtgtgtgt 3000
   gtgcgtgggg agcgccgcgt gcggcccgcg ctgcccggcg gctgtgagcg ctgcgggcgc 3060
   ggcgcggggc tttgtgcgct ccgcgtgtgc gcgaggggag cgcggccggg ggcggtgccc 3120
   cgcggtgcgg gggggctgcg aggggaacaa aggctgcgtg cggggtgtgt gcgtgggggg 3180
   gtgagcaggg ggtgtgggcg cggcggtcgg gctgtaaccc ccccctgcac ccccctcccc 3240
   gagttgctga gcacggcccg gcttcgggtg cggggctccg tgcggggcgt ggcgcggggc 3300
   tcgccgtgcc gggcgggggg tggcggcagg tgggggtgcc gggcggggcg gggccgcctc 3360
   gggccgggga gggctcgggg gaggggcgcg gcggccccgg agcgccggcg gctgtcgagg 3420
   cgcggcgagc cgcagccatt gccttttatg gtaatcgtgc gagagggcgc agggacttcc 3480
   tttgtcccaa atctggcgga gccgaaatct gggaggcgcc gccgcacccc ctctagcggg 3540
   cgcgggcgaa gcggtgcggc gccggcagga aggaaatggg cggggagggc cttcgtgcgt 3600
   cgccgcgccg ccgtcccctt ctccatctcc agcctcgggg ctgccgcagg gggacggctg 3660
   ccttcggggg ggacggggca gggcggggtt cggcttctgg cgtgtgaccg gcggctctag 3720
   aagcgttggg gtgagtactc cctctcaaaa gcgggcatga cttctgcgct aagattgtca 3780
   gtttccaaaa acgaggagga tttgatattc acctggcccg cggtgatgcc tttgagggtg 3840
   gccgcgtcca tctggtcaga aaagacaatc tttttgttgt caagcttgag gtgtggcagg 3900
   cttgagatct ggccatacac ttgagtgaca ttgacatcca ctttgccttt ctctccacag 3960
   gtgtccactc ccagggcggc ctccggagcg atcgccggtc cgcctaggca attgtttaaa 4020
   ctcgaggatc tgcgatctaa gtaagcttgg cattccggta ctgttggtaa agccaccatg 4080
   gaagacgcca aaaacataaa gaaaggcccg gcgccattct atccgctgga agatggaacc 4140
   gctggagagc aactgcataa ggctatgaag agatacgccc tggttcctgg aacaattgct 4200
   tttacagatg cacatatcga ggtggacatc acttacgctg agtacttcga aatgtccgtt 4260
   cggttggcag aagctatgaa acgatatggg ctgaatacaa atcacagaat cgtcgtatgc 4320
   agtgaaaact ctcttcaatt ctttatgccg gtgttgggcg cgttatttat cggagttgca 4380
   gttgcgcccg cgaacgacat ttataatgaa cgtgaattgc tcaacagtat gggcatttcg 4440
   cagcctaccg tggtgttcgt ttccaaaaag gggttgcaaa aaattttgaa cgtgcaaaaa 4500
   aagctcccaa tcatccaaaa aattattatc atggattcta aaacggatta ccagggattt 4560
   cagtcgatgt acacgttcgt cacatctcat ctacctcccg gttttaatga atacgatttt 4620
   gtgccagagt ccttcgatag ggacaagaca attgcactga tcatgaactc ctctggatct 4680
   actggtctgc ctaaaggtgt cgctctgcct catagaactg cctgcgtgag attctcgcat 4740
   gccagagatc ctatttttgg caatcaaatc attccggata ctgcgatttt aagtgttgtt 4800
   ccattccatc acggttttgg aatgtttact acactcggat atttgatatg tggatttcga 4860
   gtcgtcttaa tgtatagatt tgaagaagag ctgtttctga ggagccttca ggattacaag 4920
   attcaaagtg cgctgctggt gccaacccta ttctccttct tcgccaaaag cactctgatt 4980
   gacaaatacg atttatctaa tttacacgaa attgcttctg gtggcgctcc cctctctaag 5040
   gaagtcgggg aagcggttgc caagaggttc catctgccag gtatcaggca aggatatggg 5100
   ctcactgaga ctacatcagc tattctgatt acacccgagg gggatgataa accgggcgcg 5160
   gtcggtaaag ttgttccatt ttttgaagcg aaggttgtgg atctggatac cgggaaaacg 5220
   ctgggcgtta atcaaagagg cgaactgtgt gtgagaggtc ctatgattat gtccggttat 5280
   gtaaacaatc cggaagcgac caacgccttg attgacaagg atggatggct acattctgga 5340
   gacatagctt actgggacga agacgaacac ttcttcatcg ttgaccgcct gaagtctctg 5400
   attaagtaca aaggctatca ggtggctccc gctgaattgg aatccatctt gctccaacac 5460
   cccaacatct tcgacgcagg tgtcgcaggt cttcccgacg atgacgccgg tgaacttccc 5520
   gccgccgttg ttgttttgga gcacggaaag acgatgacgg aaaaagagat cgtggattac 5580
   gtcgccagtc aagtaacaac cgcgaaaaag ttgcgcggag gagttgtgtt tgtggacgaa 5640
   gtaccgaaag gtcttaccgg aaaactcgac gcaagaaaaa tcagagagat cctcataaag 5700
   gccaagaagg gcggaaagat cgccgtgtaa ttctagagtc ggggcggccg gccgcttcga 5760
   gcagacatga taagatacat tgatgagttt ggacaaacca caactagaat gcagtgaaaa 5820
   aaatgcttta tttgtgaaat ttgtgatgct attgctttat ttgtaaccat tataagctgc 5880
   aataaacaag ttaacaacaa caattgcatt cattttatgt ttcaggttca gggggaggtg 5940
   tgggaggttt tttaaagcaa gtaaaacctc tacaaatgtg gtaaaatcga taaggatctg 6000
   aacgatggag cggagaatgg gcggaactgg gcggagttag gggcgggatg ggcggagtta 6060
   ggggcgggac tatggttgct gactaattga gatgcatgct ttgcatactt ctgcctgctg 6120
   gggagcctgg ggactttcca cacctggttg ctgactaatt gagatgcatg ctttgcatac 6180
   ttctgcctgc tggggagcct ggggactttc cacaccctaa ctgacacaca ttccacagcg 6240
   gatccacgcg ccggtaaccg aagttcctat actttctaga gaataggaac ttcggaatag 6300
   gaacttcaag ccggtaccca gcttttgttc cctttagtga gggttaattt cgagcttggc 6360
   gtaatcatgg tcatagctgt ttcctgtgtg aaattgttat ccgctcacaa ttccacacaa 6420
   catacgagcc ggaagcataa agtgtaaagc ctggggtgcc taatgagtga gctaactcac 6480
   attaattgcg ttgcgctcac tgcccgcttt ccagtcggga aacctgtcgt gccagctgca 6540
   ttaatgaatc ggccaacgcg cggggagagg cggtttgcgt attgggcgct cttccgcttc 6600
   ctcgctcact gactcgctgc gctcggtcgt tcggctgcgg cgagcggtat cagctcactc 6660
   aaaggcggta atacggttat ccacagaatc aggggataac gcaggaaaga acatgtgagc 6720
   aaaaggccag caaaaggcca ggaaccgtaa aaaggccgcg ttgctggcgt ttttccatag 6780
   gctccgcccc cctgacgagc atcacaaaaa tcgacgctca agtcagaggt ggcgaaaccc 6840
   gacaggacta taaagatacc aggcgtttcc ccctggaagc tccctcgtgc gctctcctgt 6900
   tccgaccctg ccgcttaccg gatacctgtc cgcctttctc ccttcgggaa gcgtggcgct 6960
   ttctcatagc tcacgctgta ggtatctcag ttcggtgtag gtcgttcgct ccaagctggg 7020
   ctgtgtgcac gaaccccccg ttcagcccga ccgctgcgcc ttatccggta actatcgtct 7080
   tgagtccaac ccggtaagac acgacttatc gccactggca gcagccactg gtaacaggat 7140
   tagcagagcg aggtatgtag gcggtgctac agagttcttg aagtggtggc ctaactacgg 7200
   ctacactaga aggacagtat ttggtatctg cgctctgctg aagccagtta ccttcggaaa 7260
   aagagttggt agctcttgat ccggcaaaca aaccaccgct ggtagcggtg gtttttttgt 7320
   ttgcaagcag cagattacgc gcagaaaaaa aggatctcaa gaagatcctt tgatcttttc 7380
   tacggggtct gacgctcagt ggaacgaaaa ctcacgttaa gggattttgg tcatgagatt 7440
   atcaaaaagg atcttcacct agatcctttt aaattaaaaa tgaagtttta aatcaatcta 7500
   aagtatatat gagtaaactt ggtctgacag ttaccaatgc ttaatcagtg aggcacctat 7560
   ctcagcgatc tgtctatttc gttcatccat agttgcctga ctccccgtcg tgtagataac 7620
   tacgatacgg gagggcttac catctggccc cagtgctgca atgataccgc gagacccacg 7680
   ctcaccggct ccagatttat cagcaataaa ccagccagcc ggaagggccg agcgcagaag 7740
   tggtcctgca actttatccg cctccatcca gtctattaat tgttgccggg aagctagagt 7800
   aagtagttcg ccagttaata gtttgcgcaa cgttgttgcc attgctacag gcatcgtggt 7860
   gtcacgctcg tcgtttggta tggcttcatt cagctccggt tcccaacgat caaggcgagt 7920
   tacatgatcc cccatgttgt gcaaaaaagc ggttagctcc ttcggtcctc cgatcgttgt 7980
   cagaagtaag ttggccgcag tgttatcact catggttatg gcagcactgc ataattctct 8040
   tactgtcatg ccatccgtaa gatgcttttc tgtgactggt gagtactcaa ccaagtcatt 8100
   ctgagaatag tgtatgcggc gaccgagttg ctcttgcccg gcgtcaatac gggataatac 8160
   cgcgccacat agcagaactt taaaagtgct catcattgga aaacgttctt cggggcgaaa 8220
   actctcaagg atcttaccgc tgttgagatc cagttcgatg taacccactc gtgcacccaa 8280
   ctgatcttca gcatctttta ctttcaccag cgtttctggg tgagcaaaaa caggaaggca 8340
   aaatgccgca aaaaagggaa taagggcgac acggaaatgt tgaatactca tactcttcct 8400
   ttttcaatat tattgaagca tttatcaggg ttattgtctc atgagcggat acatatttga 8460
   atgtatttag aaaaataaac aaataggggt tccgcgcaca tttccccgaa aagtgccacc 8520
   taaattgtaa gcgttaatat tttgttaaaa ttcgcgttaa atttttgtta aatcagctca 8580
   ttttttaacc aataggccga aatcggcaaa atcccttata aatcaaaaga atagaccgag 8640
   atagggttga gtgttgttcc agtttggaac aagagtccac tattaaagaa cgtggactcc 8700
   aacgtcaaag ggcgaaaaac cgtctatcag ggcgatggcc cactacgtga accatcaccc 8760
   taatcaagtt ttttggggtc gaggtgccgt aaagcactaa atcggaaccc taaagggagc 8820
   ccccgattta gagcttgacg gggaaagccg gcgaacgtgg cgagaaagga agggaagaaa 8880
   gcgaaaggag cgggcgctag ggcgctggca agtgtagcgg tcacgctgcg cgtaaccacc 8940
   acacccgccg cgcttaatgc gccgctacag ggcgcgtccc attcgccatt caggctgcgc 9000
   aactgttggg aagggcgatc ggtgcgggcc tcttcgctat tacgccagct ggcgaaaggg 9060
   ggatgtgctg caaggcgatt aagttgggta acgccagggt tttcccagtc acgacgttgt 9120
   aaaacgacgg ccagtgaatt gtaatacgac tcactatagg gcgaattgg 9169
<210> 219
   <211> 5778
   <212> DNA
   <213> Artificial Sequence
<220>
   <223> Description of Artificial Sequence: Targeted Rosa26 locus with CAGGS-Fluc and LacZ site
<400> 219
   cctaggtaac cgatatccct gcaggttttc gacattgatt attgactagt tattaatagt 60
   aatcaattac ggggtcatta gttcatagcc catatatgga gttccgcgtt acataactta 120
   cggtaaatgg cccgcctggc tgaccgccca acgacccccg cccattgacg tcaataatga 180
   cgtatgttcc catagtaacg ccaataggga ctttccattg acgtcaatgg gtggactatt 240
   tacggtaaac tgcccacttg gcagtacatc aagtgtatca tatgccaagt acgcccccta 300
   ttgacgtcaa tgacggtaaa tggcccgcct ggcattatgc ccagtacatg accttatggg 360
   actttcctac ttggcagtac atctacgtat tagtcatcgc tattaccatg ggtcgaggtg 420
   agccccacgt tctgcttcac tctccccatc tcccccccct ccccaccccc aattttgtat 480
   ttatttattt tttaattatt ttgtgcagcg atgggggcgg gggggggggg ggcgcgcgcc 540
   aggcggggcg gggcggggcg aggggcgggg cggggcgagg cggagaggtg cggcggcagc 600
   caatcagagc ggcgcgctcc gaaagtttcc ttttatggcg aggcggcggc ggcggcggcc 660
   ctataaaaag cgaagcgcgc ggcgggcggg agtcgctgcg ttgccttcgc cccgtgcccc 720
   gctccgcgcc gcctcgcgcc gcccgccccg gctctgactg accgcgttac tcccacaggt 780
   gagcgggcgg gacggccctt ctcctccggg ctgtaattag cgcttggttt aatgacggct 840
   cgtttctttt ctgtggctgc gtgaaagcct taaagggctc cgggagggcc ctttgtgcgg 900
   gggggagcgg ctcggggggt gcgtgcgtgt gtgtgtgcgt ggggagcgcc gcgtgcggcc 960
   cgcgctgccc ggcggctgtg agcgctgcgg gcgcggcgcg gggctttgtg cgctccgcgt 1020
   gtgcgcgagg ggagcgcggc cgggggcggt gccccgcggt gcgggggggc tgcgagggga 1080
   acaaaggctg cgtgcggggt gtgtgcgtgg gggggtgagc agggggtgtg ggcgcggcgg 1140
   tcgggctgta acccccccct gcacccccct ccccgagttg ctgagcacgg cccggcttcg 1200
   ggtgcggggc tccgtgcggg gcgtggcgcg gggctcgccg tgccgggcgg ggggtggcgg 1260
   caggtggggg tgccgggcgg ggcggggccg cctcgggccg gggagggctc gggggagggg 1320
   cgcggcggcc ccggagcgcc ggcggctgtc gaggcgcggc gagccgcagc cattgccttt 1380
   tatggtaatc gtgcgagagg gcgcagggac ttcctttgtc ccaaatctgg cggagccgaa 1440
   atctgggagg cgccgccgca ccccctctag cgggcgcggg cgaagcggtg cggcgccggc 1500
   aggaaggaaa tgggcgggga gggccttcgt gcgtcgccgc gccgccgtcc ccttctccat 1560
   ctccagcctc ggggctgccg cagggggacg gctgccttcg ggggggacgg ggcagggcgg 1620
   ggttcggctt ctggcgtgtg accggcggct ctagaagcgt tggggtgagt actccctctc 1680
   aaaagcgggc atgacttctg cgctaagatt gtcagtttcc aaaaacgagg aggatttgat 1740
   attcacctgg cccgcggtga tgcctttgag ggtggccgcg tccatctggt cagaaaagac 1800
   aatctttttg ttgtcaagct tgaggtgtgg caggcttgag atctggccat acacttgagt 1860
   gacattgaca tccactttgc ctttctctcc acaggtgtcc actcccaggg cggcctccgg 1920
   agcgatcgcc ggtccgccta ggctagccgg ccgcgtcgac ttaattaagg ccatagcggc 1980
   cattttcgac gacactgcag agacctactt cactaacaac cggtacagtt cggtggagta 2040
   cgcgcccggg gagcccaagg gcacgccctg gcacccgcac cgcggcgaat aacttcgtat 2100
   agcatacatt atacgaagtt atacggcgcg ccatgcctaa gaagaagagg aaggttgtcg 2160
   cgagtagctt ggcactggcc gtcgttttac aacgtcgtga ctgggaaaac cctggcgtta 2220
   cccaacttaa tcgccttgca gcacatcccc ctttcgccag ctggcgtaat agcgaagagg 2280
   cccgcaccga tcgcccttcc caacagttgc gcagcctgaa tggcgaatgg cgctttgcct 2340
   ggtttccggc accagaagcg gtgccggaaa gctggctgga gtgcgatctt cctgaggccg 2400
   atactgtcgt cgtcccctca aactggcaga tgcacggtta cgatgcgccc atctacacca 2460
   acgtaaccta tcccattacg gtcaatccgc cgtttgttcc cacggagaat ccgacgggtt 2520
   gttactcgct cacatttaat gttgatgaaa gctggctaca ggaaggccag acgcgaatta 2580
   tttttgatgg cgttaactcg gcgtttcatc tgtggtgcaa cgggcgctgg gtcggttacg 2640
   gccaggacag tcgtttgccg tctgaatttg acctgagcgc atttttacgc gccggagaaa 2700
   accgcctcgc ggtgatggtg ctgcgttgga gtgacggcag ttatctggaa gatcaggata 2760
   tgtggcggat gagcggcatt ttccgtgacg tctcgttgct gcataaaccg actacacaaa 2820
   tcagcgattt ccatgttgcc actcgcttta atgatgattt cagccgcgct gtactggagg 2880
   ctgaagttca gatgtgcggc gagttgcgtg actacctacg ggtaacagtt tctttatggc 2940
   agggtgaaac gcaggtcgcc agcggcaccg cgcctttcgg cggtgaaatt atcgatgagc 3000
   gtggtggtta tgccgatcgc gtcacactac gtctgaacgt cgaaaacccg aaactgtgga 3060
   gcgccgaaat cccgaatctc tatcgtgcgg tggttgaact gcacaccgcc gacggcacgc 3120
   tgattgaagc agaagcctgc gatgtcggtt tccgcgaggt gcggattgaa aatggtctgc 3180
   tgctgctgaa cggcaagccg ttgctgattc gaggcgttaa ccgtcacgag catcatcctc 3240
   tgcatggtca ggtcatggat gagcagacga tggtgcagga tatcctgctg atgaagcaga 3300
   acaactttaa cgccgtgcgc tgttcgcatt atccgaacca tccgctgtgg tacacgctgt 3360
   gcgaccgcta cggcctgtat gtggtggatg aagccaatat tgaaacccac ggcatggtgc 3420
   caatgaatcg tctgaccgat gatccgcgct ggctaccggc gatgagcgaa cgcgtaacgc 3480
   gaatggtgca gcgcgatcgt aatcacccga gtgtgatcat ctggtcgctg gggaatgaat 3540
   caggccacgg cgctaatcac gacgcgctgt atcgctggat caaatctgtc gatccttccc 3600
   gcccggtgca gtatgaaggc ggcggagccg acaccacggc caccgatatt atttgcccga 3660
   tgtacgcgcg cgtggatgaa gaccagccct tcccggctgt gccgaaatgg tccatcaaaa 3720
   aatggctttc gctacctgga gagacgcgcc cgctgatcct ttgcgaatac gcccacgcga 3780
   tgggtaacag tcttggcggt ttcgctaaat actggcaggc gtttcgtcag tatccccgtt 3840
   tacagggcgg cttcgtctgg gactgggtgg atcagtcgct gattaaatat gatgaaaacg 3900
   gcaacccgtg gtcggcttac ggcggtgatt ttggcgatac gccgaacgat cgccagttct 3960
   gtatgaacgg tctggtcttt gccgaccgca cgccgcatcc agcgctgacg gaagcaaaac 4020
   accagcagca gtttttccag ttccgtttat ccgggcaaac catcgaagtg accagcgaat 4080
   acctgttccg tcatagcgat aacgagctcc tgcactggat ggtggcgctg gatggtaagc 4140
   cgctggcaag cggtgaagtg cctctggatg tcgctccaca aggtaaacag ttgattgaac 4200
   tgcctgaact accgcagccg gagagcgccg ggcaactctg gctcacagta cgcgtagtgc 4260
   aaccgaacgc gaccgcatgg tcagaagccg ggcacatcag cgcctggcag cagtggcgtc 4320
   tggcggaaaa cctcagtgtg acgctccccg ccgcgtccca cgccatcccg catctgacca 4380
   ccagcgaaat ggatttttgc atcgagctgg gtaataagcg ttggcaattt aaccgccagt 4440
   caggctttct ttcacagatg tggattggcg ataaaaaaca actgctgacg ccgctgcgcg 4500
   atcagttcac ccgtgcaccg ctggataacg acattggcgt aagtgaagcg acccgcattg 4560
   accctaacgc ctgggtcgaa cgctggaagg cggcgggcca ttaccaggcc gaagcagcgt 4620
   tgttgcagtg cacggcagat acacttgctg atgcggtgct gattacgacc gctcacgcgt 4680
   ggcagcatca ggggaaaacc ttatttatca gccggaaaac ctaccggatt gatggtagtg 4740
   gtcaaatggc gattaccgtt gatgttgaag tggcgagcga tacaccgcat ccggcgcgga 4800
   ttggcctgaa ctgccagctg gcgcaggtag cagagcgggt aaactggctc ggattagggc 4860
   cgcaagaaaa ctatcccgac cgccttactg ccgcctgttt tgaccgctgg gatctgccat 4920
   tgtcagacat gtataccccg tacgtcttcc cgagcgaaaa cggtctgcgc tgcgggacgc 4980
   gcgaattgaa ttatggccca caccagtggc gcggcgactt ccagttcaac atcagccgct 5040
   acagtcaaca gcaactgatg gaaaccagcc atcgccatct gctgcacgcg gaagaaggca 5100
   catggctgaa tatcgacggt ttccatatgg ggattggtgg cgacgactcc tggagcccgt 5160
   cagtatcggc ggaattccag ctgagcgccg gtcgctacca ttaccagttg gtctggtgtc 5220
   aaaaataata ataaccgggc aggggggatc tttgtgaagg aaccttactt ctgtggtgtg 5280
   acataattgg acaaactacc tacagagatt taaagctcta aggtaaatat aaaattttta 5340
   agtgtataat gtgttaaact actgattcta attgtttgtg tattttagat tccaacctat 5400
   ggaactgatg aatgggagca gtggtggaat gccagatcca gacatgataa gatacattga 5460
   tgagtttgga caaaccacaa ctagaatgca gtgaaaaaaa tgctttattt gtgaaatttg 5520
   tgatgctatt gctttatttg taaccattat aagctgcaat aaacaagtta acaacaacaa 5580
   ttgcattcat tttatgtttc aggttcaggg ggaggtgtgg gaggtttttt aaagcaagta 5640
   aaacctctac aaatgtggta tggctgatta tgatctgcgg ccaaatcggc cggcctaggc 5700
   gcgccggtaa ccgaagttcc tatactttct agagaatagg aacttcggaa taggaacttc 5760
   aagcttaagc gctagcac                                               5778

## Description

### Introduction

The invention provides a method for targeted transgenesis of short hairpin RNA expression cassettes using recombinase mediated cassette exchange. Suitable nucleotide acid sequences and vectors for the targeted transgenesis and recombinase mediated transgenesis are provided.

### Background of the Invention

The generation of transgenic mice by nuclear injection of purified DNA into fertilized eggs is a widely used approach for studying gene or promoter function *in vivo.* However, the level and pattern of expression often varies strongly depending on copy number, configuration, and integration site of the transgene. In addition, founder mice occasionally do not transmit the transgene. Thus, a number of different founders need to be generated and tested in order to identify a useful strain, which is a laborious and time-consuming undertaking (Bradley et. al., Nature Genet., 14:121-123 (1996); Jasin et al., Proc. Natl. Acad. Sci. USA, 93:8804-8808 (1996); Dobie et al., Trends Genet., 13:127-130 (1997); Garrick et al., Nature Genet., 18:56-59 (1998), Al-Shawi et al., Mol. Cell. Boil. 10:1192-1198 (1990)).

To overcome these limitations, homologous recombination in embryonic stem cells has been used to produce mice carrying a single copy of the transgene integrated into a predetermined site of the genome (Shaw-White et al., Transgenic Res.; (1):1-13 (1993); Bronson et al., Proc. Natl. Acad. Sci. USA, 93(17:9067-72 (1996); Hatada et al., J. Biol., Chem., 274(2):948-55 (1999); Vivian et al., Biotechniques, 27(1): 154-62 (1999); Evans et al., Physiol. Genomics, Mar. 13, 2(2):67-75 (2000); Cvetkovic et al., J. boil. Chem., 275(2):1073-8 (2000); Guillot et al., Physiol. Genomics, Mar. 13, (2):77-83 (2000); Magness et al., Blood, 95(11):3568-77 (2000); Misra et al., BMC Biotechnol., 1(1):12 (2001); Minami et al., Blood, 100(12):4019-25 (2002); Tang et al., Genesis, 32(3): 199-202 (2002)). In these studies, the ubiquitous Hprt locus was more or less successfully used for 'targeted transgenesis'. Insertion of a lacZ gene under the control of the polyoma enhancer/HSV thymidine kinase promoter into the third exon of Hprt resulted in variable (ß-galactosidase expression that was both orientation and cell-type dependent (Shaw-White et al., Transgenic Res.; (1):1-13 (1993)). Although transgenes under the control of the human and the chicken 6-action gene promoter resulted in widespread expression when inserted into the Hprt locus, the level of transcripts varied strongly in different tissues (Bronson et al., Proc. Natl. Acad. Sci. USA, 93(17:9067-72 (1996)). Unexpectedly, expression of these transgenes, but not of the endogenous Hprt gene appeared to be low or undetectable in kidney and liver (Bronson et al., Proc. Natl. Acad. Sci. USA, 93(17:9067-72 (1996)). Hatada et al. demonstrated that the HPRT locus suppresses the activity of both, the haptoglobin gene promoter as well as the herpes simplex thymidine kinase promoter in several tissues of mice (Hatada et al., J. Biol., Chem., 274(2):948-55 (1999)). Likewise, a human eNOS promoter-LacZ reporter gene placed in the Hprt locus was found to be inactive in hepatic vessels that otherwise express the endogenous eNOS gene (Guillot et al., Physiol. Genomics, Mar. 13, (2):77-83 (2000). Finally, since the HPRT gene is on the X chromosome, transgene expression at this locus is subjected to random X-inactivation. The expression of the transgene in all cells of the female, therefore, requires the generation of homozygotes.

WO 04/63381 reports on a particular autosomal locus, namely Rosa 26 that allows strong and predictable expression of transgenes inserted through homologous recombination. This chromosomal locus was found useful in the context of the "targeted transgenesis" approach for the efficient generation of transgenic organisms (such as mice) with a predictable transgene expression pattern. The "targeted transgenesis" method provided in said application comprises consecutive experimental steps. A gene expression cassette comprising a suitable promoter (e.g. a ubiquitous or tissue specific promoter, either inducible or constitutive) functionally linked to a gene of interest is created; subsequently a vector for the targeted insertion of the above mentioned gene expression cassette into the Rosa26 locus is generated; the insertion of the above mentioned gene expression cassette into the Rosa26 locus through homologous recombination or site specific recombination in embryonic stem cells follows; finally transgenic mice are generated by the injection of such genetically modified ES cells into blastocysts.

Previously, he rosa26 locus had been identified by random insertion of retroviral sequences and a ß-galactosidase-neomycin resistance fusion gene into the genome of mouse embryonic stem cells (Zambrowicz et al., Proc. Natl. Acad. Sci. USA, 94, 3789-94 (1997)). The rosa26 promoter appeared to mediate ubiquitous expression of promoter-less genes both in embryos and adult mice (Kisseberth et al., Dev. Biol., 214:128-138 (1999); Zambrowicz et al., Proc. Natl. Acad. Sci. USA, 94, 3789-94 (1997)), albeit at different levels in different organs (Vooijs et al., EMBO reports, 21:292-297 (2001)).

Moreover, WO 99/53017 describes a process for making transgenic animals which ubiquitously express a heterlogous gene, wherein the heterologous gene is under the control of a ubiquitously expressed endogenous promoter, e.g. that of the mouse Rosa26 locus. R. Dacquin et al., Dev. Dynamics 224 :245-251 (2002) and K. A. Moses et al., Genesis 31:176-180 (2001) utilize the transgenic mouse strain R26R obtained according to WO 99/53017 for the expression of heterlogous genes. WO 02/098217 describes a method of targeting promoter-less selection cassettes into transcriptionally active loci, such as the Rosa26 locus.

Finally, WO 03/020743 describes the expression of transgenes *In vivo* by targeting protected transgene cassettes into predetermined loci (e.g. the Rosa26 locus), such that the introduced tissue specific exogenous promoter has at least some tissue specific activity. The protected transgene cassette contains (from 5' to 3' direction) a transcriptional stop signal, the exogenous tissue specific promoter and the gene of interest. The presence of a transcriptional stop signal is vital for the method of WO 03/020743 as therewith the expression pattern is determined primarily by the nature of the tissue specific exogenous promoter.

RNA interference (RNAi) has been discovered some years ago as a tool for inhibition of gene expression (Fire, A. et al., Nature 391, 806-811 (1998)). It based on the introduction of double stranded RNA (dsRNA) molecules into cells, whereby one strand is complementary to the coding region of a target gene. Through pairing of the specific mRNA with the introduced RNA molecule, the mRNA is degraded by a cellular mechanism. Since long dsRNA provokes an interferon response in mammalian cells, the technology was initially restricted to organisms or cells showing no interferon response (Bass, B.L., Nature 411, 428-429 (2001)). The finding that *short* (<30 bp) *interfering RNAs* (siRNA) circumvent the interferon response extended the application to mammalian cells (Elbashir, S.M. et al., Nature 411, 494-498 (2001)).

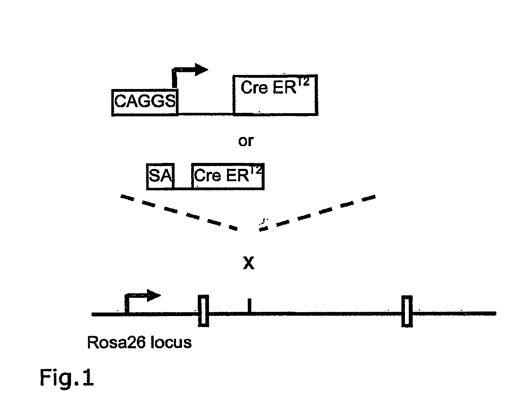

Although RNAi in mice has been in principle demonstrated, the current technology does not allow performing systematic gene function analysis *in vivo.* So far the inhibition of gene expression has been achieved by injection of *purified* siRNA into the tail vain of mice (McCaffrey, A.P. et al., Nature 418, 38-39 (2002); Lewis, D.H. et al., Nature Genet. 32, 107-108 (2002)). Using this approach, gene inhibition is restricted to specific organs and persists only a few days. A further improvement of the siRNA technology is based on the intracellular transcription of short hairpin RNA (shRNA) molecules using gene expression vectors (see Fig. 1; Brummelkamp, T.R. et al., Science 296, 550-553 (2002); Paddison, P.J. et al, Genes Dev. 16, 948-958 (2002); Yu, J.Y. et al., Proc. Natl. Acad. Sci. USA 99, 6047-6052 (2002); Sui, G. et al., Proc. Natl. Acad. Sci. USA 99, 5515-5520 (2002); Paul, C.P. et al., Nature Biotechnol. 20, 505-508 (2002); Xia, H. et al., Nat. Biotechnol. 10, 1006-10 (2002); Jacque, J.M. et al., Nature 418(6896):435-8 (2002)). The activity of shRNA in mice has been demonstrated by McCaffrey et al., 2002 through injection of shRNA expression vectors into the tail vain. Again, gene inhibition was temporally and spatially restricted. Although these results demonstrate that the mechanism of shRNA mediated gene silencing is functional in mice, they do not clarify whether constitutive RNAi can be achieved in transgenic animals. Brummelkamp, T.R. et al., Science 296, 550-553 (2002), Paddison, P.J. et al., Genes Dev. 16, 948-958 (2002), Hemann, M.T. et al., Nat. Genet. 33(3):396-400 (2003); and Devroe, E. et al., BMC Biotechnol. 2(1):15 (2002) have shown the long-term inhibition of gene expression through stable integration of shRNA vectors in cultivated cell lines. These experiments included random integration of shRNA transgenes and screening for clones with appropriate siRNA expression, which is not applicable for testing of a large number of different shRNA transgenes in mice. Finally, several reports have demonstrated shRNA-mediated gene silencing in transgenic mice and rats (Hasuwa, H. et al., FEBS Lett. 532(1-2):227-30 (2002); Carmell, M.A. et al., Nat. Struct. Biol. 10(2):91-2 (2003); Rubinson, D.A. et al., Nat. Genet. 33(3):401-6 (2003); Kunath, T. et al., Nat. Biotechnol. (2003)). However, these experiments again included random integration of shRNA transgenes resulting in variable levels and patterns of shRNA expression. Thus, testing of ES cell clones or mouse lines with appropriate shRNA expression had been required, which is a laborious and time-consuming undertaking.

The *in vivo* validation of genes by RNAi mediated gene repression in a large scale setting requires the expression of siRNA at sufficiently high levels and with a predictable pattern in multiple organs. Targeted transgenesis provides the only approach to achieve reproducible expression of transgenes in the living organism (e.g. mammalians such as mice). WO 04/035782 discloses for the first time that a single copy of a siRNA expression vector integrated into a defined locus of the genome can provide sufficiently high levels of siRNA for efficient RNAi-mediated gene inhibition in multiple organs of the living organism.

Two types of procedures have been described for targeted integration of transgenes into defined loci of the embryonic stem (ES) cell genome. One is based on homologous recombination (HR) in embryonic stem cells, and the other on site-specific recombination. In the first case, the efficiency is limited by the low frequency of HR. In contrast, site-specific recombination has emerged as a powerful tool for the targeted insertion of transgenes into the eukaryotic genome.

Site-specific recombinases such as Flp and Cre mediate recombination between two copies of their target sequence termed FRT and loxP, respectively. The use of two incompatible target sequences, for example FRT in combination with F3 (Schlake & Bode, Biochemistry, 33(43):12746-51 (1994)) as well as inverted recognition target sites (Feng et al., J. Mol. Biol. 292(4):779-85 (1999)) allows the insertion of DNA segments into a predefined chromosomal locus carrying target sequences in a similar configuration. This exchange system is called recombinase mediated cassette exchange (RMCE; Bode & Baer, Curr Opin Biotechnol. 12(5):473-80 (2001), and Bode et al., Biol. Chem. 381:801-813 (2000)). In contrast to approaches using a single recombination site the targeting product is stable even under the permanent influence of the recombinase unless it is exposed to an exchange plasmid (Seibler & Bode (1997) Biochemistry 36, 1740- 1747 (1997)).

So far, only few examples of successful RMCE in ES have been described (Feng et al., J Mol Biol.;292(4):779-85 (1999); Seibler et al., Biochemistry.;37(18):6229-34 (1998); Kolb, Anal Biochem.;290(2):260-71 (2001); Belteki et al., Nat Biotechnol.;21(3):321-4. (2003); Cesari et al., Genesis 38:87-92 (2004)). In these experiments, random integration of the exchange vector as well as incomplete recombination frequently produced unwanted transgene configurations. The efficiency of RMCE appeared to vary strongly depending on the choice of recombination sites, the selection strategy, and the chromosomal target. The criteria for efficient RMCE at a given locus are therefore not defined and unpredictable for a person skilled in the art.

The only example of efficient (>90%) RMCE at a defined locus used the tissue-specific ß-casein gene as chromosomal target (Kolb, Anal Biochem. 2001 Mar15;290(2):260-71). However, a HPRT gene was required to exclude random integration or incomplete recombination of the exchange vector. The application of this strategy is therefore limited to HPRT-negative ES cells. In addition, the cell type specific activity of the (ß-Casein locus may not be suitable for the expression of transgenes in multiple tissues. Taken together, a general strategy for efficient RMCE at a ubiquitously active locus has never been achieved.

### Summary of the Invention

It was surprisingly found that RMCE can be effectively be performed at ubiquitously active loci with high efficiency. The invention provides:
(1) an *in vitro* method for generating transgenic mammalian cells other than human embryonic cells, which method comprises the introduction by recombinase-mediated cassette exchange (RMCE) of an expression cassette for a short hairpin RNA (shRNA) into an ubiquitous locus of said cells, characterized in that:
   (i) the ubiquitous locus comprises an acceptor DNA which comprises two mutually incompatible first recombinase recognition sites (RRSs); and
   (ii) the exchange vector for RMCE comprises a donor DNA and a promoter-less positive selection marker, wherein
   (iii) the donor DNA comprises an expression cassette that includes a DNA encoding a shRNA operably linked to a promoter, said cassette being flanked by the same two mutually incompatible first RRSs present in the acceptor DNA;
(2) the method of (1) above, wherein the acceptor DNA has been introduced in the ubiquitous locus Rosa26 by homologous recombination with a vector that comprises said acceptor DNA flanked by DNA sequences homologous to the ubiquitous locus.;
(3) the method of (1) or (2) above, wherein the transgenic mammalian cells are derived from mouse and the ubiquitous locus is a Rosa26 locus, and
   (i) the DNA sequences homologous to the Rosa26 locus are derived from the 5' and 3' flanking arm of the mouse Rosa26 locus, preferably said homologous DNA sequences having the sequences shown in SEQ ID NO:4 and 5, respectively, and/or
   (ii) the RRSs of the targeting and exchange vectors are F3/Frt and the targeting vectors encodes the recombinase Flp or a mutant thereof, preferably Flp^{e}; and/or
   (iii) the targeting vector comprises a negative selection marker; and/or
   (iv) the exchange vector comprises a promoter-less positive selection marker; and/or
   (v) the promoter of the expression cassette is a H1 or H6;
      most preferably the targeting vector has the sequence shown In SEQ ID NO:11 and the exchange vector has the sequence shown in SEQ ID NO:12 or a variant thereof with modification in the short hairpin RNA construct;
(4) an exchange vector comprising a donor DNA and a promoter-less positive selection marker, wherein the donor DNA comprises an expression cassette that includes a DNA encoding a shRNA operably linked to a promoter, said cassette being flanked by to mutually incompatible RSS, said exchange vector being suitable for carrying out the method as defined in (1) to (3) above;
(5) a mammalian cell having a ubiquitous locus carrying an expression cassette for a short hairpin RNA which is introduced into said locus by a recombinantly mediated cassette exchange according to the method of (1), (2) and (3) above;
(6) a method for preparing a non-human transgenic mammal having a modified ubiquitous locus, which method comprises the steps of
   (i) obtaining isolated transfected ES cells of a non-human mammal according to the method as defined in (1) to (3) above, and
   (ii) injecting the ES cells obtained in step (i) into non-human blastocysts;
(7) a method for preparing a non-human transgenic mammal having a modified ubiquitous locus, comprising the step of injecting an exchange vector according to (4) above into an early stage embryo of a non-human mammal having corresponding RMCE target sites;
(8) a transgenic non-human mammal or a tissue culture derived therefrom, which are obtainable by the above defined methods (6) and (7), respectively and have an operably functional gene expression cassette for a short hairpin RNA integrated into at least one of its loci;
(9) the use of an exchange vector of (4) above for preparing an agent for constitutive and/or inducible gene knock down in a non-human mammal, or in tissue culture or cells of a cell culture derived from the non-human mammal; or
(10) the use of an exchange vector of (4) above for preparing an agent for injection into cells of an eight cell stage embryo of non-human mammals; and
(11) a method for constitutive and/or inducible gene knock down in a tissue culture or cells of a cell culture derived from a non-human mammal, which comprises stably integrating an exchange vector as defined in (3) above into the genome of the tissue culture or of the cells of the cell culture;
(12) the use of the mammalian cell of (5) above, the transgenic non-human mammal or tissue culture thereof of (8) above for gene function studies, drug development or as disease model.

The method of the invention offers several advantages over the current technology of pronuclear injection. In particular, the targeting vector allows insertion of a single copy of a gene expression cassette, thus avoiding modulation of transgene expression by the arrangement of multiple copies. By choosing the autosomal Rosa26 locus as insertion site, the expression pattern of the inserted transgene in the non-human animal is predictable; random X-inactivation and/or modulation by chromosomal position effects are avoided. This also eliminates the need to generate and analyse multiple transgenic strains for any given transgene. Finally, the Rosa26 targeting vector for the site-specific integration can be used for multiple gene expression cassettes. Moreover, the RMCE strategy provides for more flexibility for consitutive and inducible gene knock-down, RNA mediated gene silencing in transgene animals and living organs.

### Description of the Figures

Figure 1: Targeted insertion of CreER and CAGGS-Cre-ER into the Rosa26 locus. A cassette comprising a Cre-ER operationally linked to a CAGGS promoter or a cassette comprising a splice acceptor site (SA) linked to a Cre-ER are inserted into the Rosa26 locus via homologous recombination. A perpendicular dash marks the insertion point within the Rosa26 locus and the rectangular boxes delinate the starting and end points of the Rosa26 transcript.

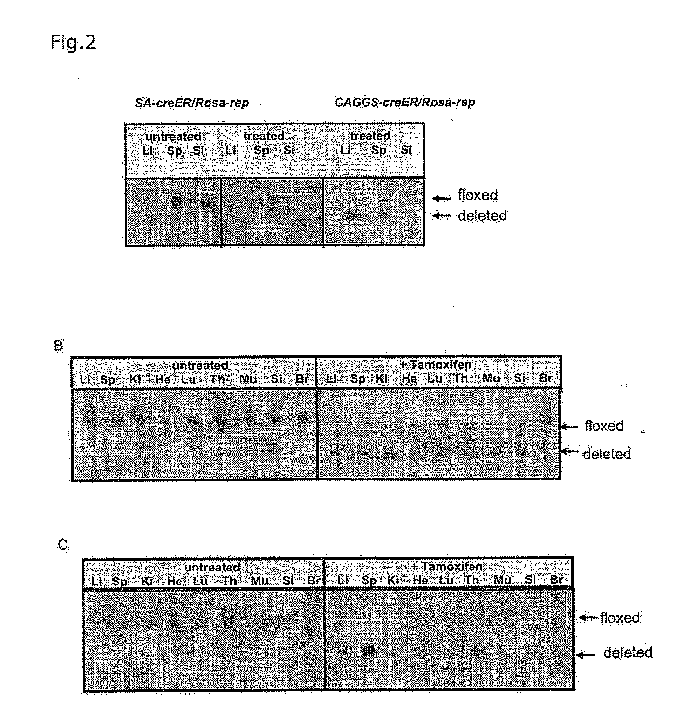

Figure 2: Southern Blot analysis of the inducible recombination of the Rosa (reporter). (A) Genomic DNA was isolated from liver (Li) spleen (Sp) and small intestine (Si) of transgenic mice carrying the SA-creER/Rosa-rep insert or the CAGGS-creER/Rosa-rep insert. To induce the Cre-ER recombinase the mice were treated with Tamoxifen (treated). As a control, a group of mice with the SA-creER/Rosa-rep insert was left untreated (untreated). Presence of the reporter band (floxed) and deletion (deleted) of it upon an induced recombination event are indicated. (B) Transgenic mice carrying at one Rosa26 locus a IoxP flanked DNA polymerase p gene segment (pol*^{ßflox}*) and at the other a SA-creER/Rosa-rep were treated with Tamoxifen (treated). A control group of mice was left untreated (untreated). Genomic DNA from liver (Li), spleen (Sp), kidney (Ki). heart (He), lung (Lu), thymus (Th), muscle (Mu), small intestine (Si) and brain (Br) was analysed for presence of pol^{ß*flox*}. In a non-recombination event the pol^{ß*flox*} band remained (floxed), in a recombination event deletion occurred (deleted). (C) As (B), but mice carried instead of the SA-creER/Rosa-rep the CAGGS-creER/Rosa-rep insert.

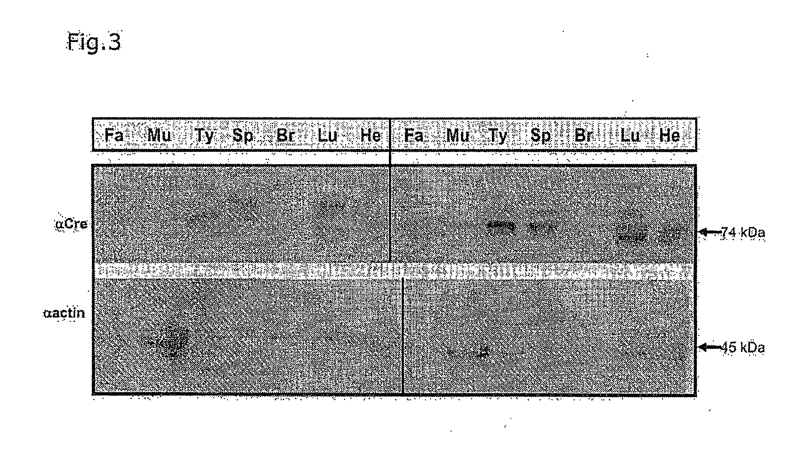

Figure 3: Western Blot analysis of recombinase and α-actin expression. Proteins were extracted from rosa(SA-CreER^{T2}) and rosa (CAGGS-CreER^{T2}) mice and analyzed as described in the "Materials and Method" section. The positions of bands representing CreER^{T2} and actin are indicated. FA: fat tissue, Ty: Thymus; Sp: spleen, Br: Brain, Lu: lung, He: heart.

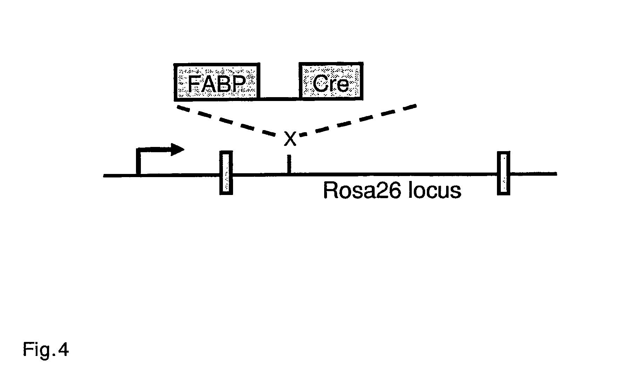

Figure 4: Fabp-Cre targeting vector. An expression cassette, in which the Cre recombinase is expressed under the control of the Fabpl^{4x at -132} promoter is inserted into the Rosa26 targeting vector. This vector was used to insert the Fabp-Cre cassette into the Rosa26 locus by homologous recombination in ES cells.

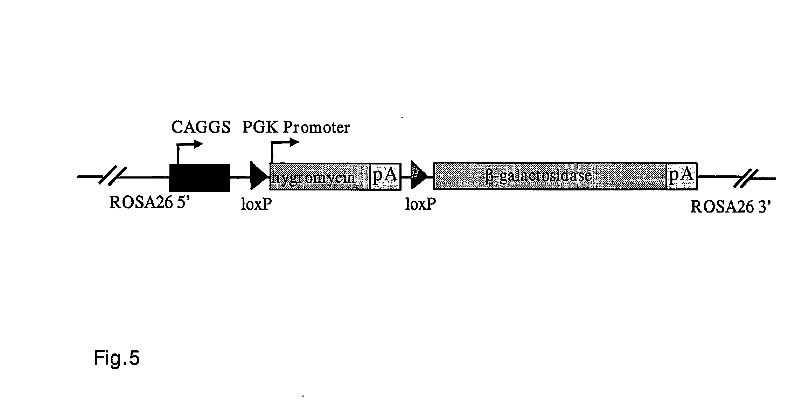

Figure 5: ROSA26 locus of the Cre reporter mice carrying a Cre substrate reporter construct. A recombination substrate (Seq ID NO:9) has been inserted in the ROSA26 locus. The substrate consists of a CAGGS promoter followed by a cassette consisting of the hygromycin resistance gene driven by a PGK promoter and flanked by IoxP recombination sites. This cassette is followed by the coding region for beta-galactosidase, which is only expressed when the hygromycin resistance gene has been deleted by recombination.

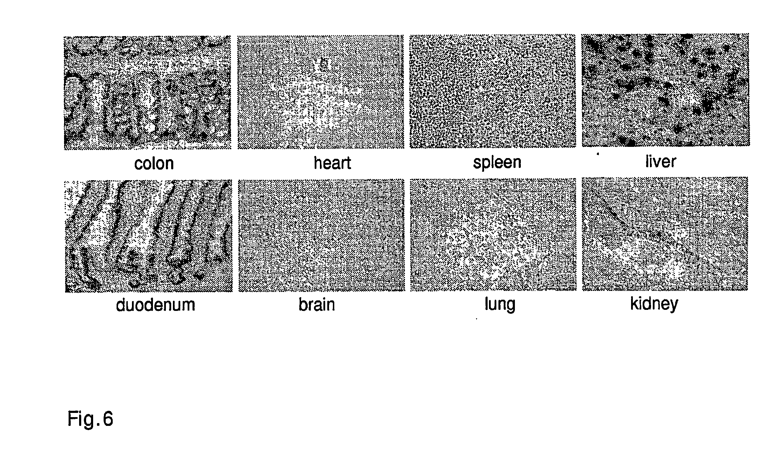

Figure 6: *In situ* detection of beta-galactosidase in cryosections of different tissues of Fabp-Cre/reporter substrate double transgenic mice. Mouse tissues were embedded in OCT, frozen and cut into microsections. The sections were stained for beta-galactosidase activity (indicated by the blue color) by X-gal staining, counterstained with Nuclear Fast Red Solution, dehydrated, mounted and photographed.

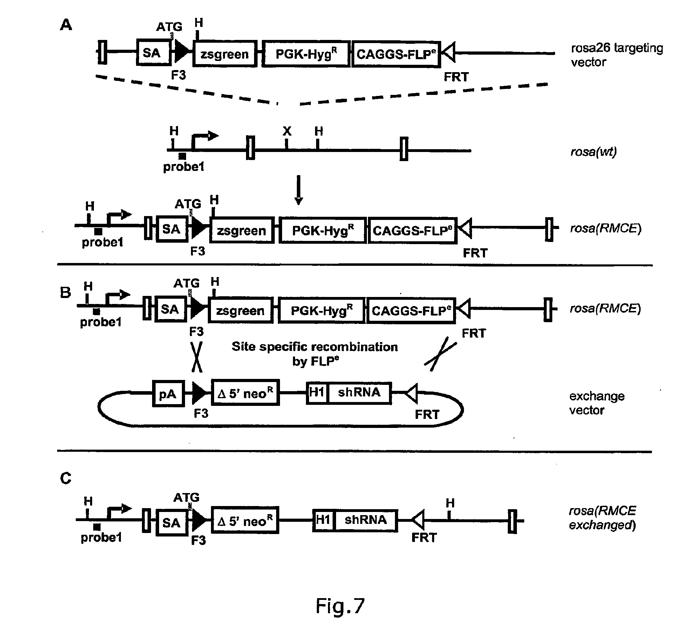

Figure 7: RMCE targeting system for rosa26. A) Insertion of the RMCE target into the rosa26 locus. A cassette comprising zsgreen, PGK-Hyg, and CAGGS-FLP is inserted into the Rosa26 locus via homologous recombination in ES cells. The FRT and F3 sites are oriented in opposite direction to each other. A perpendicular dash with 'X' marks the insertion point within the rosa26 locus. B) Exchange vector carrying FRT and F3 sites together with a truncated neo^{R} gene for positive selection of RMCE and a shRNA expression cassette under the control of the U6 promoter for targeted integration into the Rosa26 locus. The polyA signal is included to prevent expression of the truncated neo^{R} gene at sites of random integration. C) Configuration of the targeted Rosa26 locus following. X: XbaI, H: HindIII.

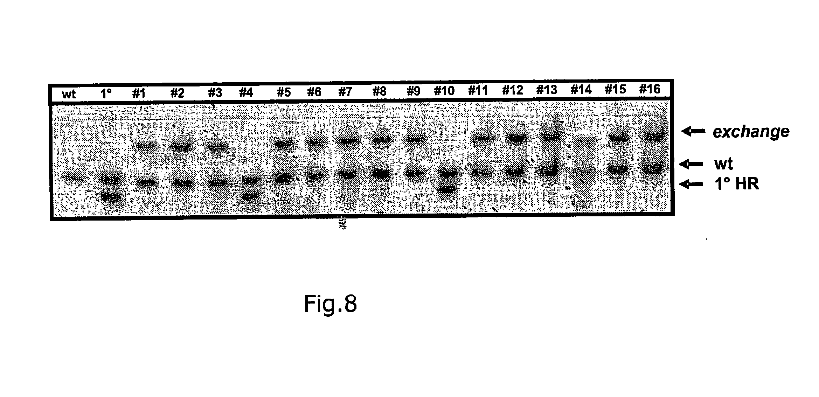

Figure 8: Southern blot analysis of genomic DNA from rosa(RMCE) targeted ES cells transfected with the exchange vector. *rosa(RMCE exchanged)* alleles. The sizes of the wt, Rosa26 targeted (1° HR) and RMCE alleles (exchange) are 4.4 kb, 3.9 kb and 5.8 kb, respectively. In clones #1 - 3, 5 - 9, and 11 - 16 successful RMCE had occurred. Genomic DNA was digested with HindIII and analyzed using probe 1.

### Detailed Descripion of the Invention

The term "mammals" according to the present invention relates to non-human animals such as rodents including mice and rats; and humans. Most preferred mammals are mice.

"Mammalian cells" and "starting mammalian cells" according to the present invention include cells isolated (derived) from the above defined mammals and cultured *in vitro.* These cells can be transformed (immortalized) or untransformed. (directly derived from living mammals; primary cell culture).

It is preferred in the method (1) of the present invention that the mammalian cells are derived from rodents such as mouse and rat.

In the method (1) of the invention it is preferred that the functional DNA sequence comprises a gene encoding a protein/peptide of interest (i.e. is a expressible and translatable DNA sequence), more preferably said functional DNA sequence is a gene expression cassette (a) comprising a gene of interest operatively linked to a promoter, or (b) is a DNA sequence which can be converted into such gene expression cassette (i.e. into an operatively linked "promoter-gene of interest" construct, e.g. by subsequent modification reactions after its integration). The gene of interest within the gene expression cassette can be any gene coding for a certain protein/peptide of interest, including, but not limited to, recombinases, reporter genes, receptors, signaling molecules, transcription factors, pharmaceutically active proteins and peptides, drug target candidates, disease causing gene products, toxins, etc.

The promoter of the gene expression cassette (which is a heterologous promoter relative to the Rosa26 locus) preferably is a ubiquitous or tissue specific promoter, either constitutive or inducible. The ubiquitous promoter in the vector according to the invention is preferably selected from polymerases I, II and III dependent promoters, preferably is a polymerase II or III dependent promoter including, but not limited to, a CMV promoter, a CAGGS promoter, a snRNA promoter such as U6, a RNAse P RNA promoter such as H1, a tRNA promoter, a 7SL RNA promoter, a 5 S rRNA promoter, etc. Particularly preferred ubiquitous promoters are CAGGS, hCMV, PGK. Preferred tissue specific promoters are FABP (Saam & Gordon, J. Biol. Chem., 274:38071-38082 (1999)), Lck (Orban et al., Proc. Natl. Acad. Sci. USA, 89:6861-5 (1992)), CamKII (Tsien et al., Cell 87: 1317-1326 (1996)), CD19 (Rickert et al., Nucleic Acids Res. 25:1317-1318 (1997)), Keratin (Li et al., Development, 128:675-88 (201)), Albumin (Postic & Magnuson, Genesis, 26:149-150 (2000)), aP2 (Barlow et al., Nucleic Acids Res., 25 (1997)), Insulin (Ray et al., Int. J. Pancreatol. 25:157-63 (1999)), MCK (Brüning et al., Molecular Cell 2:559-569 (1998)), MyHC (Agak et al., J. Clin. Invest., 100:169-179 (1997), WAP (Utomo et al., Nat. Biotechnol. 17:1091-1096 (1999)), Col2A (Ovchinnikov et al., Genesis, 26:145-146 (2000)); preferred inducible promoter systes are Mx (Kühn et al. Scinence, 269:1427-1429 (1995)), tet (Urlinger et al., Proc. Natl. Acad. Sci. USA, 97:7963-8 (2000)), Trex (Feng and Erikson, Human Gene Therapy, 10:419-27). Suitable inducible promoters are the above-mentioned promoters containing an operator sequence including, but not limited to, tet, Gal4, lac, etc.

The targeting vector, recombination vector, functional DNA sequence or gene expression cassette may further comprises one ore more additional functional sequences including but not limited to (selectable) marker genes (such as the neomycin phosphotransferase gene of *E*. *coli* transposon, etc.), recombinase recognition sites (which in case of the recombination vector differ from the first recombinase recognition sites and which include IoxP, FRT, variants thereof, etc.), poly A signals (such as synthetic polyadenylation sites, or the polyadenylation site of human growth hormones, etc.), splice acceptor sequences (such as a splice acceptor of adenovirus, etc.), introns, tags for protein detection, enhancers, selection markers, etc.

In a preferred embodiment methods (1) to (3) of the invention comprise homologous recombination. It is then preferred that the DNA sequences homologous to the Rosa26 locus are 0.2 to 20 kB, preferably 1 to 10 kB long. In a particularly preferred embodiment of the method (2) the eukaryotic cells are derived from mouse, the DNA sequences homologous to the Rosa26 locus are derived from the 5' and 3' flanking arm of the mouse Rosa26 locus, preferably said homologous DNA sequences having the sequences shown in SEQ ID NO:4 and 5, respectively, and the promoter is a CAGGS-promoter, most preferably the targeting vector has the sequence shown in SEQ ID NO:7.

As set forth above, methods (1) to (3) of the invention comprise recombinase mediated cassette exchange (RMCE). The insertion of transgenes or DNA segments into the genome can be mediated by site specific recombination (Fukushige & Sauer, Proc. Natl. Acad. Sci. USA 89(17):7905-9 (1992)). A site specific recombinase like cre or FLP recombines two recognition target sites like IoxP or FRT, respectively. The use of two incompatible recognition target sites (F3 or F5, Schlake & Bode, Biochemistry, 1994 Nov. 1, 33(43): 12746-51) or inverted recognition target sites (Feng et al., J. Mol. Biol. 292(4):779-85 (1999)) allows the insertion of DNA segments flanked by two incompatible or inverted target sites. This exchange system has been called recombinase mediated cassette exchange (RMCE). In a preferred embodiment a FLP based RMCE system is inserted into the Rosa26 locus. Said recombinase mediated recombination preferably comprises the steps:
(a1) introducing into the starting cells an acceptor DNA which integrates into the genome of the starting cell, the acceptor DNA comprising two mutally incompatible first RRSs, and introducing into the therewith obtained cell
(a2) a donor DNA comprising the same two mutually incompatible first RRSs contained in the acceptor DNA by utilizing a recombination vector as defined above; and
(a3) the recombinase which catalyzes recombination between the RRSs of the acceptor and donor

In said recombinase mediated recombination method it is preferred that
(i) the RRS are IoxP or FRT sites or variants thereof (such as single mutant recognition sited lox66 and lox71 (Albert et al., The Plant J. 7:649-659 (1995)); and/or
(ii) the acceptor DNA comprises a negatively selectable marker (e.g. herpes simplex virus thymidin kinase gene, etc.) and or
(iii) the donor DNA comprises an inactive positive selection marker (e.g. neomycin phosphotransferase, etc.).

For further selectable markers it is referred to U.S. Patents Nos. 5,487,932 and 5,464,763 which are hereby incorporated in their entirety.

The ubiquitous promoter in the vector according to the invention is preferably selected from polymerase I, II and III dependent promoters, preferably is a polymerase II or III dependent promoter including, but not limited to, a CMV promoter, a CAGGS promoter, a snRNA promoter such as U6, a RNAse P RNA promoter such as H1, a tRNA promoter, a 7SL RNA promoter, a 5 S rRNA promoter, etc.

The ubiquitous promoter can be a constitutive promoter, or can be an inducible promoter. Suitable inducible promoters are the above-mentioned polymerase I, II and III dependent promoters containing an operator sequence including, but not limited to, tet, Gal4, lac, etc.

The expression vector of the invention is suitable for the following particularly preferred approaches (for constitutive and inducible expression):
A. a Pol III dependent promoter (constitutive U6, H1 or the like) driven shRNA construct (to be integrated into a ubiquitously active Pol II dependent locus (see Fig. 2);
B. a Pol III dependent promoter (inducible U6, H1 or the like) driven shRNA construct (to be integrated into a ubiquitously active Pol II dependent locus (Fig 3 and 4)); or
C. a polymerase II (Pol II) dependent promoter (inducible CMV or the like) driven shRNA construct (to be integrated into a ubiquitously active Pol II dependent locus (Fig. 5 and 6)).

The short hairpin RNA construct or inactive precursor thereof of the expression cassette comprises at least one segment corresponding to a short hairpin RNA (shRNA) or to complementary short interfering RNA (siRNA) strands. In case shRNA segments are utilized within the expression cassette, said cassette preferably comprises at least one shRNA segment having a nucleotide (e.g. DNA) sequence of the structure A-B-C or C-B-A. In case siRNA segments are utilized within the expression cassette, said cassette preferably comprises at least least two DNA segments A and C or C and A, wherein each of said at least two segments is under the control of a separate promoter as defined above (such as the Pol III promoter including inducible U6, H1 or the like). In the above segments
A is a 15 to 35, preferably a 19 to 29 bp DNA sequence being at least 90%, preferably 100% complementary to the gene to be knocked down (e.g. firefly luciferase, p53, etc.);
B is a spacer DNA sequence having 5 to 9 bp forming the loop of the expressed RNA hairpin molecule, and
C is a 15 to 35, preferably a 19 to 29 bp DNA sequence being at least 85% complementary to the sequence A.

The above shRNA and siRNA segments may further comprise stop and/or polyadenylation sequences.

Suitable shRNA sequences for the knock down of a given target gene are well known in the art (see e.g. the particular shRNA sequences mentioned in Tables 1 and 2 below) or can readily be determined by the skilled artesian.

**Table 1:**

| target gene | shRNA sequence /SEQ ID NO | Reference |
|---|---|---|
| CDH-1 p53 CDC20 | TgagaagtctcccagtcagTTCAAGAGActgactgggagacttctca (13) | Brummelkam p et al., Science, 296: 550-3 (2002). |
| | GactccagtggtaatctacTTCAAGAGAgtagattaccactggagtc (14) | |
| | CggcaggactccgggccgaTTCAAGAGAtcggcccggagtcctgccg (15) | |
| CYLD | CctcatgcagttctctttgTTCAAGAGAcaaagagaactgcatgagg (16) | Kovalenko et al, Nature, 424:801-5 (2003). |
| Ras-Gap | AagatgaagccactccctatttCAAGAGAaaatagggagtggcttcatctt (17) | Kunath et al., Nature Biotechnology , 21:559-561 (2003). |
| tubulin | GacagagccaagtggactcACAgagtccacttggctctgtc (18) | Yu et al., PNAS, 99: 6047-52 (2002) |
| lamin | Ctggacttccagaagaacattcgtgttcttctggaagtccag (19) | Paul et al., Nature Biotechnology, 20:505-8 (2002). |

**Table 2 shRNA sequences known from Brummelkamp et al., Nature, 424:797-801 (2003):**

| **Target Gene** | **shRNA Sequence / SEQ ID NO** |
|---|---|
| UBIQUITIN CARBOXYL- TERMINAL HYDROLASE 12 | GAGATTGGTCCAGAACAGTTTCAAGAGAACTGTTCTGGACCAATCTC (20) |
| | GCCCTTCCGATCATGGTAGTTCAAGAGACTACCATGATCGGAAGGGC (21) |
| | TCTTTAGAATTCTTAAGTATTCAAGAGATACTTAAGAATTCTAAAGA (22) |
| | CATTAGCTATATCAACATGTTCAAGAGACATGTTGATATAGCTAATG (23) |
| | |
| UBIQUITIN CARBOXYL- TERMINAL HYDROLASE 11 | ACCACAAACGGCGGAACGATTCAAGAGATCGTTCCGCCGTTTGTGGT (24) |
| | GAGGGTCTTGGAGGTCTTCTTCAAGAGAGAAGACCTCCAAGACCCTC (25) |
| | GTCCATGCCCAGCCGTACATTCAAGAGATGTACGGCTGGGCATGGAC (26) |
| | GCTGGACACCCTCGTGGAGTTCAAGAGACTCCACGAGGGTGTCCAGC (27) |
| | |
| UBIQUTTIN CARBOXYL- TERMINAL HYDROLASE 10 | GAATATCAGAGAATTGAGTTTCAAGAGAACTCAATTCTCTGATATTC (28) |
| | TGGACTTCATGAGGAAATGTTCAAGAGACATTTCCTCATGAAGTCCA (29) |
| | TATTGAATATCCTGTGGACTTCAAGAGAGTCCACAGGATATTCAATA (30) |
| | TTGTACTGAGAGAAACTGCTTCAAGAGAGCAGTTTCTCTCAGTACAA (31) |
| | |
| HAUSP | GATCAATGATAGGTTTGAATTCAAGAGATTCAAACCTATCATTGATC (32) |
| | GGAGTTTGAGAAGTTTAAATTCAAGAGATTTAAACTTCTCAAACTCC (33) |
| | GAACTCCTCGCTTGCTGAGTTCAAGAGACTCAGCAAGCGAGGAGTTC (34) |
| | CCGAATTTAACAGAGAGAATTCAAGAGATTCTCTCTGTTAAATTCGG (35) |
| | |
| UBIQUITIN CARBOXYL-TERMINAL HYDROLASE 8 | GACAGCAGAAGAATGCAGATTCAAGAGATCTGCATTCTTCTGCTGTC (36) |
| | ATAAAGCTCAACGAGAACCTTCAAGAGAGGTTCTCGTTGAGCTTTAT (37) |
| | GGTGAAGTGGCAGAAGAATTTCAAGAGAATTCTTCTGCCACTTCACC (38) |
| | GTATTGCAGTAATCATCACTTCAAGAGAGTGATGATTACTGCAATAC (39) |
| | |
| FLJ10785 | GATATGGGGTTCCATGTCATTCAAGAGATGACATGGAACCCCATATC (40) |
| | GGAGACATGGTTCTTAGTGTTCAAGAGACACTAAGAACCATGTCTCC (41) |
| | AGCACCAAGTTCGTCTCAGTTCAAGAGACTGAGACGAACTTGGTGCT (42) |
| | GATGCAACACTGAAAGAACTTCAAGAGAGTTCTTTCAGTGTTGCATC (43) |
| | |
| KIAA0710 | GTCAATGGCAGTGATGATATTCAAGAGATATCATCACTGCCATTGAC (44) |
| | CCTGCTAGCTGCCTGTGGCTTCAAGAGAGCCACAGGCAGCTAGCAGG (45) |
| | CCACCTTTGCCAGAAGGAGTTCAAGAGACTCCTTCTGGCAAAGGTGG (46) |
| | CCCTATTGAGGCAAGTGTCTTCAAGAGAGACACTTGCCTCAATAGGG (47) |
| | |
| FLJ12552/ FLJ14256 | GAAGGAAAACTTGCTGACGTTCAAGAGACGTCAGCAAGTTTTCCTTC (48) |
| | CTCACCTGGGTCCATGAGATTCAAGAGATCTCATGGACCCAGGTGAG (49) |
| | GCTGTCTTACCGTGTGGTCTTCAAGAGAGACCACACGGTAAGACAGC (50) |
| | CCTGGACCGCATGTATGACTTCAAGAGAGTCATACATGCGGTCCAGG (51) |
| | |
| KIAA1203 | GTCAATGGCAGTGATGATATTCAAGAGATATCATCACTGCCATTGAC (52) |
| | CCTGCTAGCTGCCTGTGGCTTCAAGAGAGCCACAGGCAGCTAGCAGG (53) |
| | CCACCTTTGCCAGAAGGAGTTCAAGAGACTCCTTCTGGCAAAGGTGG (54) |
| | CCCTATTGAGGCAAGTGTCTTCAAGAGAGACACTTGCCTCAATAGGG (55) |
| FLJ23277 | GGAAATCCGAATTGCTTGGTTCAAGAGACCAAGCAATTCGGATTTCC (56) |
| | CACATTTCTTCAAGTGTGGTTCAAGAGACCACACTTGAAGAAATGTG (57) |
| | CAGCAGGATGCTCAAGAATTTCAAGAGAATTCTTGAGCATCCTGCTG (58) |
| | GCTGAATACCTACATTGGCTTCAAGAGAGCCAATGTAGGTATTCAGC (59) |
| | |
| FLJ14914 (similar to UBP4) | GGGCTTGTGCCTGGCCTTGTTCAAGAGACAAGGCCAGGCACAAGCCC (60) |
| | GCCTTGTCCTGCCAAGAAGTTCAAGAGACTTCTTGGCAGGACAAGGC (61) |
| | GATTGAAGCCAAGGGAACGTTCAAGAGACGTTCCCTTGGCTTCAATC (62) |
| | TGGCGCCTGCTCCCCATCTTTCAAGAGAAGATGGGGAGCAGGCGCCA (63) |
| | |
| UBIQUITIN CARBOXYL-TERMINAL HYDROLASE ISOZYME L5 | GAACCAGCAGGCTCTGTGGTTCAAGAGACCACAGAGCCTGCTGGTTC (64) |
| | GGAAGCATAATTATCTGCCTTCAAGAGAGGCAGATAATTATGCTTCC (65) |
| | AGAAGAAGATGCTTTTTCACTTCAAGAGAGTGAAAAGCATCTTCTTCT (66) |
| | CTTGCAGAGGAGGAACCCATTCAAGAGATGGGTTCCTCCTCTGCAAG (67) |
| | |
| UBIQUITIN CARBOXYL-TERMINAL HYDROLASE ISOZYME L3 | GCAAACAATCAGCAATGCCTTCAAGAGAGGCATTGCTGATTGTTTGC (68) |
| | TTGGACTGATTCATGCTATTTCAAGAGAATAGCATGAATCAGTCCAA (69) |
| | CTGGCAATTCGTTGATGTATTCAAGAGATACATCAACGAATTGCCAG (70) |
| | TTAGATGGGCGGAAGCCATTTCAAGAGAATGGCTTCCGCCCATCTAA (71) |
| | |
| UBIQUITIN CAR-BOXYL-TERMINAL HYDROLASE ISOZYME L1 | GAGGAGTCTCTGGGCTCGGTTCAAGAGACCGAGCCCAGAGACTCCTC (72) |
| | GAGCTGAAGGGACAAGAAGTTCAAGAGACTTCTTGTCCCTTCAGCTC (73) |
| | TGTCGGGTAGATGACAAGGTTCAAGAGACCTTGTCATCTACCCGACA (74) |
| | CACAGCTGTTCTTCTGTTCTTCAAGAGAGAACAGAAGAACAGCTGTG (75) |
| | |
| KIAA1891 / FLJ25263 | GTGGAAGCCTTTACAGATCTTCAAGAGAGATCTGTAAAGGCTTCCAC (76) |
| | CAACAGCTGCCTTCATCTGTTCAAGAGACAGATGAAGGCAGCTGTTG (77) |
| | CCATAGGCAGTCCTCCTAATTCAAGAGATTAGGAGGACTGCCTATGG (78) |
| | TGTATCACTGCCACTGGTTTTCAAGAGAAACCAGTGGCAGTGATACA (79) |
| | |
| FLJ14528 (similar to UBP8) | CATGTTGGGCAGCTGCAGCTTCAAGAGAGCTGCAGCTGCCCAACATG (80) |
| | CACAACTGGAGACCTGAAGTTCAAGAGACTTCAGGTCTCCAGTTGTG (81) |
| | GTATGCCTCCAAGAAAGAGTTCAAGAGACTCTTTCTTGGAGGCATAC (82) |
| | CTTCACAGTACATTTCTCTTTCAAGAGAAGAGAAATGTACTGTGAAG (83) |
| | |
| U4/U6 TRI SNRNP 65 kDa protein | GTACTTTCAAGGCCGGGGTTTCAAGAGAACCCCGGCCTTGAAAGTAC (84) |
| | CTTGGACAAGCAAGCCAAATTCAAGAGATTTGGCTTGCTTGTCCAAG (85) |
| | GACTATTGTGACTGATGTTTTCAAGAGAAACATCAGTCACAATAGTC (86) |
| | GGAGAACTTTCTGAAGCGCTTCAAGAGAGCGCTTCAGAAAGTTCTCC (87) |
| | |
| XM_089437 | GACGAGAGAAACCTTCACCTTCAAGAGAGGTGAAGGTTTCTCTCGTC (88) |
| | ACATTATTCTACATTCTTTTTCAAGAGAAAAGAATGTAGAATAATGT (89) |
| | AGATTCGCAAATGGATGTATTCAAGAGATACATCCATTTGCGAATCT (90) |
| | CATTCCCACCATGAGTCTGTTCAAGAGACAGACTCATGGTGGGAATG (91) |
| | |
| KIAA1453 | GATCGCCCGACACTTCCGCTTCAAGAGAGCGGAAGTGTCGGGCGATC (92) |
| | CCAGCAGGCCTACGTGCTGTTCAAGAGACAGCACGTAGGCCTGCTGG (93) |
| | GCCAGCTCCTCCACAGCACTTCAAGAGAGTGCTGTGGAGGAGCTGGC (94) |
| | CGCCGCCAAGTGGAGCAGATTCAAGAGATCTGCTCCACTTGGCGGCG (95) |
| FLJ12697 | GAAGATGCCCATGAATTCCTTCAAGAGAGGAATTCATGGGCATCTTC (96) |
| | CAAACAGGCTGCGCCAGGCTTCAAGAGAGCCTGGCGCAGCCTGTTTG (97) |
| | ACGGCCTAGCGCCTGATGGTTCAAGAGACCATCAGGCGCTAGGCCGT (98) |
| | CTGTAACCTCTCTGATCGGTTCAAGAGACCGATCAGAGAGGTTACAG (99) |
| | |
| UBIQUITIN SPECIFIC PROTEASE 18 (USP18) | TCTGTCAGTCCATCCTGGCTTCAAGAGAGCCAGGATGGACTGACAGA (100) |
| | TGAAGCGAGAGTCTTGTGATTCAAGAGATCACAAGACTCTCGCTTCA (101) |
| | GATGGAGTGCTAATGGAAATTCAAGAGATTTCCATTAGCACTCCATC (102) |
| | CCTTCAGAGATTGACACGCTTCAAGAGAGCGTGTCAATCTCTGAAGG (103) |
| | |
| UBIQUITIN CARBOXYL-TERMINAL HYDROLASE 20 | CCTGACCACGTTCCGACTGTTCAAGAGACAGTCGGAACGTGGTCAGG (104) |
| | GAGTTCCTTCGCTGCCTGATTCAAGAGATCAGGCAGCGAAGGAACTC (105) |
| | GACTGCCTTGCTGCCTTCTTTCAAGAGAAGAAGGCAGCAAGGCAGTC (106) |
| | CGCCGAGGGCTACGTACTCTTCAAGAGAGAGTACGTAGCCCTCGGCG (107) |
| | |
| UBIQUITIN CARBOXYL-TERMINAL HYDROLASE 24 | GGCGAGAAGAAAGGACTGTTTCAAGAGAACAGTCCTTTCTTCTCGCC (108) |
| | GGACGAGAATTGATAAAGATTCAAGAGATCTTTATCAATTCTCGTCC (109) |
| | GCACGAGAATTTGGGAATCTTCAAGAGAGATTCCCAAATTCTCGTGC (110) |
| | CTACTTCATGAAATATTGGTTCAAGAGACCAATATTTCATGAAGTAG (111) |
| | |
| KIAA1594 | GATAACAGCTTCTTGTCTATTCAAGAGATAGACAAGAAGCTGTTATC (112) |
| | GAGAATAGGACATCAGGGCTTCAAGAGAGCCCTGATGTCCTATTCTC (113) |
| | CTTGGAAGACTGAACCTGTTTCAAGAGAACAGGTTCAGTCTTCCAAG (114) |
| | CAACTCCTTTGTGGATGCATTCAAGAGATGCATCCACAAAGGAGTTG (115) |
| | |
| KIAA1350 | GATGTTGTCTCCAAATGCATTCAAGAGATGCATTTGGAGACAACATC (116) |
| | CGTGGGGACTGTACCTCCCTTCAAGAGAGGGAGGTACAGTCCCCACG (117) |
| | GTACAGCTTCAGAACCAAGTTCAAGAGACTTGGTTCTGAAGCTGTAC (118) |
| | |
| UBIQUITIN CARBOXYL-TERMINAL HYDROLASE 25 | GATGATCTTCAGAGAGCAATTCAAGAGATTGCTCTCTGAAGATCATC (119) |
| | GGAACATCGGAATTTGCCTTTCAAGAGAAGGCAAATTCCGATGTTCC (120) |
| | GAGCTAGTGAGGGACTCTTTTCAAGAGAAAGAGTCCCTCACTAGCTC (121) |
| | GCAGGGTTCTTTAAGGCAATTCAAGAGATTGCCTTAAAGAACCCTGC (122) |
| | |
| UBIQUITIN CARBOXYL-TERMINAL HYDROLASE 16 | TCGATGATTCCTCTGAAACTTCAAGAGAGTTTCAGAGGAATCATCGA (123) |
| | GATAATGGAAATATTGAACTTCAAGAGAGTTCAATATTTCCATTATC (124) |
| | GTTCTTCATTTAAATGATATTCAAGAGATATCATTTAAATGAAGAAC (125) |
| | GTTAACAAACACATAAAGTTTCAAGAGAACTTTATGTGTTTGTTAAC (126) |
| | |
| USP9X | GTTAGAGAAGATTCTTCGTTTCAAGAGAACGAAGAATCTTCTCTAAC (127) |
| | GTTGATTGGACAATTAAACTTCAAGAGAGTTTAATTGTCCAATCAAC (128) |
| | GGTTGATACCGTAAAGCGCTTCAAGAGAGCGCTTTACGGTATCAACC (129) |
| | GCAATGAAACGTCCAATGGTTCAAGAGACCATTGGACGTTTCATTGC (130) |
| | |
| USP9Y | AGCTAGAGAAAATTCTTCGTTCAAGAGACGAAGAATTTTCTCTAGCT (131) |
| | GATCCTATATGATGGATGATTCAAGAGATCATCCATCATATAGGATC (132) |
| | GTTCTTCTTGTCAGTGAAATTCAAGAGATTTCACTGACAAGAAGAAC (133) |
| | CTTGAGCTTGAGTGACCACTTCAAGAGAGTGGTCACTCAAGCTCAAG (134) |
| UBIQUITIN CARBOXYL-TERMINAL HYDROLASE 5 | GACCGGCCAGCGAGTCTACTTCAAGAGAGTAGACTCGCTGGCCGGTC (135) |
| | GGACCTGGGCTACATCTACTTCAAGAGAGTAGATGTAGCCCAGGTCC (136) |
| | CTCTGTGGTCCAGGTGCTCTTCAAGAGAGAGCACCTGGACCACAGAG (137) |
| | GACCACACGATTTGCCTCATTCAAGAGATGAGGCAAATCGTGTGGTC (138) |
| | |
| UBIQUITIN CARBOXYL-TERMINAL HYDROLASE 26 | TGGCTTGTTTATTGAAGGATTCAAGAGATCCTTCAATAAACAAGCCA (139) |
| | GTGAATTTGGGGAAGATAATTCAAGAGATTATCTTCCCCAAATTCAC (140) |
| | CGCTATAGCTTGAATGAGTTTCAAGAGAACTCATTCAAGCTATAGCG (141) |
| | GATATCCTGGCTCCACACATTCAAGAGATGTGTGGAGCCAGGATATC (142) |
| | |
| KIAA1097 | GAGCCAGTCGGATGTAGATTTCAAGAGAATCTACATCCGACTGGCTC (143) |
| | GTAAATTCTGAAGGCGAATTTCAAGAGAATTCGCCTTCAGAATTTAC (144) |
| | GCCCTCCTAAATCAGGCAATTCAAGAGATTGCCTGATTTAGGAGGGC (145) |
| | GTTGAGAAATGGAGTGAAGTTCAAGAGACTTCACTCCATTTCTCAAC (146) |
| | |
| UBIQUITIN SPECIFIC PROTEASE 22 (USP22) | GCTTGGAAAATGCAAGGCGTTCAAGAGACGCCTTGCATTTTCCAAGC (147) |
| | CTGCATCATAGACCAGATCTTCAAGAGAGATCTGGTCTATGATGCAG (148) |
| | GATCACCACGTATGTGTCCTTCAAGAGAGGACACATACGTGGTGATC (149) |
| | TGACAACAAGTATTCCCTGTTCAAGAGACAGGGAATACTTGTTGTCA (150) |
| | |
| UBIQUITIN-SPECIFIC | GAAATATAAGACAGATTCCTTCAAGAGAGGAATCTGTCTTATATTTC (151) |
| | CCCATCAAGTTTAGAGGATTTCAAGAGAATCCTCTAAACTTGATGGG (152) |
| PROCESSING PROTEASE 29 | GGTGTCCCATGGGAATATATTCAAGAGATATATTCCCATGGGACACC (153) |
| | GAATGCCGACCTACAAAGATTCAAGAGATCTTTGTAGGTCGGCATTC (154) |
| | |
| CYLD | CAGTTATATTCTGTGATGTTTCAAGAGAACATCACAGAATATAACTG (155) |
| | GAGGTGTTGGGGACAAAGGTTCAAGAGACCTTTGTCCCCAACACCTC (156) |
| | GTGGGCTCATTGGCTGAAGTTCAAGAGACTTCAGCCAATGAGCCCAC (157) |
| | GAGCTACTGAGGACAGAAATTCAAGAGATTTCTGTCCTCAGTAGCTC (158) |
| | |
| UBIQUITIN CARBOXYL-TERMINAL HYDROLASE 2 | TCAGCAGGATGCTCAGGAGTTCAAGAGACTCCTGAGCATCCTGCTGA (159) |
| | GAAGTTCTCCATCCAGAGGTTCAAGAGACCTCTGGATGGAGAACTTC (160) |
| | GCCGGTCCCCACCAGCAGCTTCAAGAGAGCTGCTGGTGGGGACCGGC (161) |
| | CACTCGGGAGTTGAGAGATTTCAAGAGAATCTCTCAACTCCCGAGTG (162) |
| | |
| UBIQUITIN SPECIFIC PROTEASE 3 (USP3) | GCCCTTGGGTCTGTTTGACTTCAAGAGAGTCAAACAGACCCAAGGGC (163) |
| | CTCAACACTAAACAGCAAGTTCAAGAGACTTGCTGTTTAGTGTTGAG (164) |
| | GATTTCATTGGACAGCATATTCAAGAGATATGCTGTCCAATGAAATC (165) |
| | CATGGGGCACCAACTAATTTTCAAGAGAAATTAGTTGGTGCCCCATG (166) |
| | |
| UBIQUITIN CARBOXYL-TERMINAL HYDROLASE 23 | GGTGTCTCTGCGGGATTGTTTCAAGAGAACAATCCCGCAGAGACACC (167) |
| | AGTTCAGTAGGTGTAGACTTTCAAGAGAAGTCTACACCTACTGAACT (168) |
| | GAGTTCCTGAAGCTCCTCATTCAAGAGATGAGGAGCTTCAGGAACTC (169) |
| | GGATTTGCTGGGGGCAAGGTTCAAGAGACCTTGCCCCCAGCAAATCC (170) |
| | |
| UBP-32.7 | CTCAGAAAGCCAACATTCATTCAAGAGATGAATGTTGGCTTTCTGAG (171) |
| | CGCATTGTAATAAGAAGGTTTCAAGAGAACCTTCTTATTACAATGCG (172) |
| | GGGAGGAAAATGCAGAAATTTCAAGAGAATTTCTGCATTTTCCTCCC (173) |
| | TTACAAATTTAGGAAATACTTCAAGAGAGTATTTCCTAAATTTGTAA (174) |
| HOMO SAPIENS UBIQUITIN SPECIFIC PROTEASE 13 (ISOPEP-TIDASE T-3) | GTTATGAATTGATATGCAGTTCAAGAGACTGCATATCAATTCATAAC (175) |
| | GTGATAACACAACTAATGGTTCAAGAGACCATTAGTTGTGTTATCAC (176) |
| | GTAGAGGAGAGTTCTGAAATTCAAGAGATTTCAGAACTCCTCTAC (177) |
| | GCCTCTAATCCTGATAAGGTTCAAGAGACCTTATCAGGATTAGAGGC (178) |
| | |
| UBIQUITIN CARBOXYL-TERMINAL HYDROLASE 28 | GATGATCTTCAGGCTGCCATTCAAGAGATGGCAGCCTGAAGATCATC (179) |
| | GTATGGACAAGAGCGTTGGTTCAAGAGACCAACGCTCTTGTCCATAC (180) |
| | CGAACCCTTCTGGAACAGTTTCAAGAGAACTGTTCCAGAAGGGTTCG (181) |
| | GTGGCATGAAGATTATAGTTTCAAGAGAACTATAATCTTCATGCCAC (182) |
| | |
| UBIQUITIN CARBOXYL-TERMINAL HYDROLASE 14 | GGTGAACAAGGACAGTATCTTCAAGAGAGATACTGTCCTTGTTCACC (183) |
| | GCAATAGAGGATGATTCTGTTCAAGAGACAGAATCATCCTCTATTGC (184) |
| | TCTGTGAATGCCAAAGTTCTTCAAGAGAGAACTTTGGCATTCACAGA (185) |
| | CACACCAGGGAAGGTCTAGTTCAAGAGACTAGACCTTCCCTGGTGTG (186) |
| | |
| DUB1 | GCAGGAAGATGCCCATGAATTCAAGAGATTCATGGGCATCTTCCTGC (187) |
| | GAATGTGCAATATCCTGAGTTCAAGAGACTCAGGATATTGCACATTC (188) |
| | TGGATGATGCCAAGGTCACTTCAAGAGAGTGACCTTGGCATCATCCA (189) |
| | GCTCCGTGCTAAACCTCTCTTCAAGAGAGAGAGGTTTAGCACGGAGC (190) |
| | |
| MOUSE USP27 HOMOLOG | GCCTCCACCTCAACAGAGGTTCAAGAGACCTCTGTTGAGGTGGAGGC (191) |
| | CTGCATCATAGACCAAATCTTCAAGAGAGATTTGGTCTATGATGCAG (192) |
| | GATCACTACATACATTTCCTTCAAGAGAGGAAATGTATGTAGTGATC (193) |
| | GTAAAGAGAGCAGAATGAATTCAAGAGATTCATTCTGCTCTCTTTAC (194) |
| | |
| UBIQUITIN CARBOXYL-TERMINAL HYDROLASE 4 | CGCGGGGCGCAGTGGTATCTTCAAGAGAGATACCACTGCGCCCCGCG (195) |
| | CAGAAGGCAGTGGGGAAGATTCAAGAGATCTTCCCCACTGCCTTCTG (196) |
| | GCCTGGGAGAATCACAGGTTTCAAGAGAACCTGTGATTCTCCCAGGC (197) |
| | ACCAGACAAGGAAATACCCTTCAAGAGAGGGTATTTCCTTGTCTGGT (198) |
| | |
| TRE-2 | CACATCCACCACATCGACCTTCAAGAGAGGTCGATGTGGTGGATGTG (199) |
| | GTCACAACCCAAGACCATGTTCAAGAGACATGGTCTTGGGTTGTGAC (200) |
| | CTCAACAGGACAAATCCCATTCAAGAGATGGGATTTGTCCTGTTGAG (201) |
| | TAGATCAATTATTGTGGATTTCAAGAGAATCCACAATAATTGATCTA (202) |
| | |
| UBIQUITIN CARBOXYL-TERMINAL HYDROLASE 15 (UNPH-2). | GGAACACCTTATTGATGAATTCAAGAGATTCATCAATAAGGTGTTCC (203) |
| | CTTTAACAGAAATTGTCTCTTCAAGAGAGAGACAATTTCTGTTAAAG (204) |
| | CCTATGCAGTACAAAGTGGTTCAAGAGACCACTTTGTACTGCATAGG (205) |
| | GATCTTTTCTTGCTTTGGATTCAAGAGATCCAAAGCAAGAAAAGATC (206) |
| | |
| KIAA1372 | CAGCATCCTTCAGGCCTTATTCAAGAGATAAGGCCTGAAGGATGCTG (207) |
| | GATAGTGACTCGGATCTGCTTCAAGAGAGCAGATCCGAGTCACTATC (208) |
| | GACATCACAGCCCGGGAGTTTCAAGAGAACTCCCGGGCTGTGATGTC (209) |
| | GGACACAGCCTATGTGCTGTTCAAGAGACAGCACATAGGCTGTGTCC (210) |
| | |
| BRCA1 ASSOCIATED PROTEIN-1 | GTGGAGGAGATCTACGACCTTCAAGAGAGGTCGTAGATCTCCTCCAC (211) |
| | CTCTTGTGCAACTCATGCCTTCAAGAGAGGCATGAGTTGCACAAGAG (212) |
| | ACAGGGCCCCTGCAGCCTCTTCAAGAGAGAGGCTGCAGGGGCCCTGT (213) |
| | GAAGACCTGGCGGCAGGTGTTCAAGAGACACCTGCCGCCAGGTCTTC (214) |

Suitable siRNA sequences for the knockdown of a given target gene are well known in the art ( e.g. the particular siRNA sequences mentioned in Lee N.S. et al., J. Nat. Biotechnol. 20(5):500-5 (2002) gcctgtgcctcttcagctacc (SEQ ID NO:215) and gcggagacagcgacgaagagc (SEQ ID NO:216) and in Du, Q. et al., Nucl. Acids Res. 21; 33(5):1671-7 (2005) cttattggagagagcacga (SEQ ID NO:217)) or can readily be determined by the skilled artisan.

A preferred embodiment of the method (1) or (2) of the invention concerns the following steps:
1. Generation of the short hairpin DNA containing the antisense- and sense-strand of the coding region of a gene (e.g. firefly luciferase; p53). Antisense and sense-strand are separated by a spacer of 5 to 9 bp.
2. Generation of constructs for the expression of the above mentioned shRNA under the control of a constitutive or inducible promoter (Pol II or Pol III dependent).
3. Insertion of the mentioned expression constructs into an exchange vector and subsequent insertion of the exchange vector into a ubiquitously expressed locus in ES cells by RMCE.
4. Analysis of the constitutive and inducible inhibition of gene expression (e.g. firefly luciferase; p53) in ES cells (e.g. through Western blot analysis).
5. Generation of mice using the mentioned ES cells and analysis of the inhibition of gene expression in several tissues (e.g. firefly luciferase; p53; e.g. through Western blot analysis).

The vector according to embodiment (4) of the invention is suitable for stable or transient integration. Said vector is suitable for gene transfer.

The technology of the present application provides for the following advantages:
(i) A stable and body wide inhibition of gene expression by generating transgenic animals (such as mice).
(ii) A reversible inhibition of gene expression using the inducible constructs.

We showed that high efficient (>90%) RMCE at the ubiquitously expressed Rosa26 locus. The following features where combined in the RMCE strategy of the invention:
1. We utilized Flp mediated RMCE using a wild type Flp target site (FRT) in combination with an inverted F3 site. The F3 sequence was generated by systematic mutagenesis of the 8 bp spacer localized between the Flp binding elements (Schlake & Bode (1994) Biochemistry 33, 12746- 12751.). The F3/F3 couple is recombined by FLP with the same efficiency as two wild type recombinase recognition sites (RRS) whereas recombination of a FRT/F3 pair is not catalyzed (Seibler & Bode (1997) Biochemistry 36, 1740- 1747.). This characteristic contrasts other pairs of wild type and mutant RRS such as loxp/lox511 that exhibit a residual recombination capacity (Lauth et al., 2002, Nucleic Acids Res. 30:e115).
2. We included a constitutive FLP^{e} expression cassette on the targeting vector to provide sufficient recombinase activity until successful RMCE of the exchange vector. Thus, incomplete recombination intermediates should be avoided.
3. The positive selection marker along with a splice acceptor site on the exchange vector lacks a functional promoter. Thus, expression of the selection marker should only be mediated by the endogenous Rosa26 promoter following successful RMCE, but not through random integration of the exchange vector.
4. The fluorescent protein expression cassette on the targeting vector should allow for the detection of RMCE in early embryos, avoiding long term culture in medium containing antibiotics.

The methods (1) to (3) may further (besides step (a) and (b) defined above) comprise one or more of the steps (c) isolating the mammalian cells, preferably the ES cells having the desired fuctional exchange cassette integrated into the Rosa26 locus; and/or (d) modifying the integrated precursor of the exchange cassette and isolating (ES) cells having the desired modified functional exchange cassette.

The steps (a) and (b) of the methods (1) to (3) are preferably performed *in vitro.* The step (c) may be performed *in vitro* and *in vivo.*

The invention also provides a method for preparing a transgenenic mammal having a modified Rosa26 locus which comprises utilizing the method as defined in (1) to (3) above. This includes a method for preparing a non-human mammal comprising modifying starting ES cells according to steps (a) to (c). The ES cells may subsequently processed according one or more of the following steps:
(d) the ES cells obtained in steps (b) or (c) are injected into blastocysts; and/or
(e) transgenic non-human animals carrying one or more functional genes of interest at the Rosa26 locus are generated (viz. by well known breeding procedures).

The transgenic non-human mammals obtainable by the method (6) and (7), preferably have an operatively functional gene expression cassette (as defined above) integrated into its Rosa26 locus. Such

transgenic non-human mammals are suitable for gene function studies, drug development or as disease model animals.

The invention is further explained by the following examples and the attached figures.

### Examples

### Materials and Methods

Cell culture: Culture and targeted mutagenesis of ES cells were carried out as described in Hogan et al., (Cold Spring Harbor Laboratory Press, Cold Spring Harbor NY.), pp. 253-289 with ES cell lines derived from both inbred and F1 embryos (Examples 1 and 2). In Example 3 Art4.12 ES cells (Seibler et al., Nucl. Acid Res., 31(4):e12 (2003) were used.

Mice: All mice were kept in the animal facility at Artemis Pharmaceuticals GmbH in microisolator cages (Tecniplast Sealsave). B6D2F1 Mice for the generation of tetraploid blastocysts were obtained from Janvier. The *polb^{flox}*/*rosa(CreER^{T2})* and *ect2^{flox}*/*rosa(CreER^{T2})* mice were generated by breeding of *rosa(CreER^{T2})* ES mice with ßT14 (Gu et al., Science, 265, 103-106.), respectively.

Production of ES mice by tetraploid embryo complementation: The production of mice by tetraploid embryo complementation was essentially performed as described (Eggan et al., Proc Natl Acad Sci USA, 98, 6209-6214.).

Ligand administration: 100 mg Tamoxifen-free base (Sigma, T5648) was suspended in 100 µl Ethanol and solved in 1 ml sunflower oil (Sigma). This 10 mg/100 µl tamoxifen solution was sonicated for 1-2 minutes and then stored at - 20°C. For p.o. administration the solution was thawed at 55°C and administrated to 4-8 week old mice by a feeding needle (FST Fine Science Tools GmbH, 18061-20).

Western blot analysis: Western blot analysis was performed using SDS-PAGE (NuPAGE, Invitrogen) and the Breeze Immunodetection System (Invitrogen) according to the manufacturer protocols. Immunodetection was done using sc-543 (HC-20, Santa Cruz Biotechnology, Inc.) against ER, PRB-106C against cre, actin sc-1616 Actin (I-19) against actin and rabbit polyclonal IgG (Santa Cruz Biotechnology, Inc.) antibodies.

X-Gal staining on tissue sections: To detect beta-galactosidase activity, tissues were embedded in Tissue Tec OCT (Sakura Finetek Europe B.V., The Netherlands), frozen on dry ice and cut into microsections. The sections were mounted onto slides and dried for 1-4 hours at room temperature. Sections were fixed for 5 min at room temperature in fixing solution (0,2% glutaraldehyde, 5 mM EGTA, 2mM MgCl₂ in 0.1 M PB ((0.1 M K₂HPO₄, pH 7.3)) and washed three times for 15 min at room temperature in washing buffer (2 mM MgCl₂, 0.02% Nonidet-40 in 0.1 M PB). Subsequently, tissues were stained for beta-galactosidase activity over night at 37°C using X-Gal solution (0.6 mg/ml X-Gal (predissolved in DMSO), 5 mM potassium hexacyanoferrat III, 5 mM potassium hexacyanoferrat II, in washing buffer). Sections were washed twice for 5 min at room temperature in PBS, counterstained with Nuclear Fast Red Solution for 10 min, rinsed shortly in aqua dest., dehydrated through a graded ethanol series and mounted in Eukitt (Sigma, Germany).

### Example 1 (Reference Example)

CreER Rosa-targeting vector: A 129 SV/EV-BAC library (Incyte Genomics) was screened with a probe against *exon2* of the *Rosa26* locus (amplified from mouse genomic DNA using Rscreen1s (GACAGGACAGTGCTTGTTTAAGG) (SEQ ID NO:1) and Rscreen1as (TGACTACACAATATTGCTCGCAC) (SEQ ID NO:2)). Out of the identified BACclone a 11 kb EcoRV subfragment was inserted into the HindII site of pBS. Two fragments (a 1 kb SacII/XbaI- and a 4 kb XbaI-fragment, SEQ ID Nos:4 and 5, respectively) were used as homology arms and inserted into a vector containing a FRT-flanked neomycin resistance gene (unpublished) to generate the basic Rosa26 targeting vector. The CAGGS-promoter (SEQ ID NO:6, nucleotides 1-1616) or a splice acceptor site (SA) from adenovirus (Friedrich G., Soriano P., Genes Dev., 5: 1513-23 (1991)) were inserted between the 5' arm and the FRT flanked neomycin resistance gene. The CreER^{T2} and a polyadenylation site (pA; SEQ ID NO:6, nucleotides 3921-4099) were cloned 3' of the SA or the CAGGS-promoter. The vector is free of a transcriptional stop sequence 5' to the CAGGS-promoter

A CreER^{T2} gene (Fell et al., (1997) Biochem Biophys Res Commun., 237, 752-757) under the control of the CAGGS-promoter (Okabe, Fabs Letters 407:313-19 (1997)) was inserted into the rosa26 locus by homologous recombination in ES cells by utilizing the CreER Rosa-targeting vector as described above (Fig. 1). In addition to the *CreER^{T2}* gene a splice acceptor sequence (Friedrich and Soziano (1991), Genes Dev., 9, 1513-1523) was introduced as a control for the endogenous activity of the rosa26 gene promoter (Fig. 1). A *IoxP*-flanked hygromycin resistance gene was introduced into the second allele of *rosa26* to provide test substrate for Cre *ER^{T2}* (Seibler et al., Nucl. Acids. Res. Feb. 15, 2003, 31(4):(12) (2003)), in press). ES cells modified at both rosa26 alleles were injected into tetraploid blastocysts and completely ES cell derived mice were generated (Eggan et al., (2001). PNAS, 98, 6209-6214). *Rosa(SA-CreER^{T2}*/*reporter)* and *Rosa(CAGGS-CreER^{T2}*/*reporter)* mice were fed with daily 5 mg Tamoxifen for 5 days and recombination of the reporter was analyzed 3 days after the last administration. Southern analysis of genomic DNA from different organs showed up to 50% recombination in the *Rosa(SA-CreER^{T2}*/*reporter)* mice and up to 90% recombination in the *rosa(CAGGS-CreER^{T2}*/*reporter)* mice, respectively (Fig. 2A). As the second substrate, we used the IoxP flanked DNA *polymerase B* gene segment *(polB^{flox})* (Gu et al., (1994). Science, 265, 103-106). The *polB^{flox}*/*rosa(SA-CreER^{T2})* and *polB^{flox}*/*rosa(CAGGS-CreER^{T2}*mice were fed with 5 mg tamoxifen per day for 5 days and analyzed 3 days later. Southern blot analysis revealed that the *IoxP*-flanked *polymerase ß* gene segment was excised in more than 90% of cells in all organs except brain in the *rosa(SA-CreER^{T2}*/*reporter)* mice (Fig. 2B). In contrast, the degree of inducible recombination was significantly higher in *rosa(CAGGS-CreER^{T2}*/*reporter)* mice, reaching 100% efficiency in most organs and up to 70% in brain.

To investigate the pattern and level of *CreER^{T2}* expression in *rosa(SA-CreER^{T2})* and *rosa(CAGGS- CreER^{T2})* mice, we performed Western analysis using antibodies specific for Cre. The 74 kDa band corresponding to the CreER^{T2} fusion protein was detectable in all organs of *rosa(CAGGS- CreER^{T2})* mice, including brain (Fig. 3). In contrast, the CreER^{T2} expression level in *rosa(SA-CreER^{T2})* mice was significantly lower compared to the *rosa(CAGGS-CreER^{T2})* strain and appeared to be undetectable in brain (Fig. 3).

### Example 2 (Reference Example)

FABP-Cre Rosa-targeting vector (SEQ ID NO:8): The splice acceptor site from adenovirus (SEQ ID NO:8, nucleotides 18569-18689) was inserted into the basic Rosa26 targeting vector described in 1. above. Into the SwaI and AscI restriction sites of the resulting plasmid was inserted a 3195 bp Xba_{blunt}/AscI DNA fragment comprising in 5' to 3' order the polyadenylation signal from the human growth hormone gene (SEQ ID NO:8, nucleotides 18760-688; Bond et al, Science 289:1942-1946 (2000)), a modified Fabpl promoter (SEQ ID NO:8, nucleotides 702-1481; Fabpl^{4x at-132}; Simon et al., J. Biol. Chem. 272:10652-10663 (1997)), a synthetic intron (SEQ ID NO:8, nucleotides 1521-1758), the Cre coding sequence (SEQ ID NO:8, nucleotides 1778-2830) and a synthetic polyA signal (SEQ ID NO:8, nucleotides 2888-3066).

A Cre gene under the control of the Fabpl^{4x at-132}-promoter (SEQ ID NO:8; Fig. 4) was inserted into the Rosa26 locus by homologous recombination in F1 ES cells carrying a Cre reporter substrate in the second Rosa26 allele. LacZ expression from the reporter construct (SEQ ID NO:9; Fig. 5) is activated upon Cre-mediated recombination. Targeted ES cells were injected into tetraploid blastocysts to generate FABP-Cre/reporter-substrate double transgenic ES mice. The Cre recombination pattern in these mice was examined by analyzing beta-galactosidase activity in tissues sections (Fig. 6). Cre-mediated recombination in these mice was restricted to the intestinal epithelium, liver and part of the cells in the epithelium of the tubuli in the kidney, thus exactly reflecting the expression pattern of the endogenous Fabpl gene (Simon et al., J. Biol. Chem., 272:10652-10663 (1997)).

### Example 3

### Rosa targeting and exchange vectors

*Rosa26 RMCE targeting vector* (SEQ ID NO:11): A 129 SV/EV-BAC library (Incyte Genomics) was screened with a probe against *exon2* of the *Rosa26* locus (SEQ ID NO:3). The *exon2* probe was amplified from mouse genomic DNA using primers Rscreen1s (GACAGGACAGTGCTTGTTTAAGG; SEQ ID NO:1) and Rscreen1as (TGACTACACAATATTGCTCGCAC; SEQ ID NO:2). A 11 kb EcoRV fragment isolated from the identified BAC clone a was inserted into the HindII site of pBS. Two subfragments from the 11 kb EcoRV fragment, the 1 kb SacII/XbaI- (SEQ ID NO:4) and the 4 kb XbaI-fragment (SEQ ID NO:5), were used as homology arms and inserted into a vector containing a FRT-flanked neomycin resistance gene (unpublished) to generate the basic Rosa26 targeting vector (SEQ ID NO:10). A splice acceptor site (SA) from adenovirus (Friedrich G., Soriano P., Genes Dev., 5:1513-23 (1991)) was inserted between the 5' arm and the FRT flanked neomycin resistance gene. The neomycin was deleted by Flp-mediated deletion in bacteria (Buchholz et al., Nucleic Acids Res. 1996, 24:3118-9). The final Rosa(RMCE) targeting vector (SEQ ID NO:11, Fig. 7A) was generated by standard cloning procedures and has the following order in 5' to 3' direction: a ATG start codon, a F3 site (Schlake & Bode (1994) Biochemistry 33, 12746- 12751; (SEQ ID NO:11, nucleotides 1292-1339)), a zsgreen ORF (Clontech; SEQ ID NO:11, nucleotides 1407-2099), a synthetic polyA signal (SEQ ID NO:11, nucleotides 2121-2299), a PGK-hygro resistance gene (SEQ ID NO:11, nucleotides 2314-4335), a CAGGS-promoter (SEQ ID NO:11, nucleotides 4397-6012), a Flp^{e}-recombinase gene (Buchholz et al., Nat Biotechnol. 1998, 16:657-62.), a synthetic polyA signal (SEQ ID NO:11, nucleotides 7728-7906), and a FRT site (SEQ ID NO:11, nucleotides 7922-7969) 5' of the 3' homology arm.

*Exchange vector* (SEQ ID NO:12): The vector contains the F3 site and the FRT site in the same configuration as in the Rosa26 targeting vector described above. The vector was generated using standard cloning procedures and has the following order in 5' to 3' direction: a synthetic polyA signal (SEQ ID NO:12, nucleotides 23-201), a F3-site (SEQ ID NO:12, nucleotides 216-263), a neomycin-resistance gene lacking the start ATG (SEQ ID NO:12, nucleotides 271-1559), a H1-promoter (SEQ ID NO:12, nucleotides 1763-1996), a hairpin sequence (SEQ ID NO:12, nucleotides 1997-2051), and a FRT site (SEQ ID NO:12, nucleotides 2161-2208).

Cell culture: ES cell culture and homologous recombination were carried out as previously described (Hogan et al., (Cold Spring Harbor Laboratory Press, Cold Spring Harbor NY.), pp. 253-289.)

*Transfection of cells with the exchange vector:* 1 day before transfection, 2 × 10⁵ ES cells were plated on a 3 cm dish in 2 ml medium. Before transfection 2 ml fresh medium was given to the cells. 3 µl Fugene6 Reagent (Roche; Cat No. 1 814 443) was mixed with 100 µl serum free medium (OptiMEM 1 with Glutamax-I Invitrogen; Cat.No. Cat.No. 51985-035) and incubated for 5 min. 100 µl of the Fugene/OptiMEM solution was added to 2 µg circular DNA (c = 0.33 µg/µl) and incubated for 15 min. This transfection complex was added drop wise to the medium and mixed by a circuiting movement. Fresh medium was added to the transfected cells the following day. From day 2, the medium was changed daily replaced by medium containing 250 µg/ml G418 (Geneticin; Invitrogen; Cat.No. 10131-019). 7 days after transfection, single clones were isolated by standard procedures as described (Hogan et al., (Cold Spring Harbor Laboratory Press, Cold Spring Harbor NY.), pp. 253-289.).

The targeting vector to prepare the Rosa26 locus for RMCE is depicted in figure 7A. The vector carries a FLP^{e} expression cassette to provide the recombinase for RMCE. The hygromycine resistance gene was used for positive selection of homologous recombinant clones. In addition, a zsGreen gene was placed between the FRT and F3 sites to allow for the identification of recombinant clones that have not undergone RMCE following secondary transfection of the exchange vector. The splice acceptor site (SA) and the ATG start codon should facilitate expression of the truncated neomycine resistance gene (Δ5'neo^{R}) on the exchange vector by employing the endogenous rosa26 promoter following RMCE.

The hybrid ES cell line ART4.12 ([C57BL/6 x 129S6/SvEvTac] F1) was used for homologous recombination, since these lines are capable to derive completely ES cell derived mice (ES mice) through tetraploid blastocyst complementation with high efficiency (Seibler et al., Nucl. Acid Res., 31(4):e12 (2003). ART4.12 cells where transfected with the rosa26 targeting vector and incubated in cell culture medium containing hygromycin B. Independent recombinant Rosa(RMCE) ES cell clones were obtained at a frequency of 2 % as verified by Southern blot analysis (Fig. 8, first and second lane.

The exchange vector (Fig.7B) carries the FRT and F3 sites together with a truncated neo^{R} gene for positive selection of RMCE. The shRNA expression cassette served as a test transgene for targeted integration into the Rosa26 locus. The upstream polyA signal was included to prevent expression of the truncated neo^{R} gene in ES cells carrying randomly integrated vectors. The configuration of the targeted Rosa26 locus following RMCE is depicted in Fig. 7C.

Rosa(RMCE) ES cells where transfected with the exchange vector and selected in medium containing G418. Southern blot analysis of G418 resistant colonies revealed that successful RMCE had occurred in >90 % of clones (Fig. 8). This is the first demonstration of efficient RMCE for targeted transgenesis at a ubiquitously expressed locus.

ShRNA transgenic ES cells were injected into tetraploid blastocysts and ES cell derived mice were obtained three weeks later at a frequency of 3 %. Real time PCR analysis of 15 week old mice indicated a >80 % reduction of leptin receptor mRNA in most organs, indicating that the shRNA transgene is ubiquitously expressed (Seibler et al. 2005, Nucl Acids Res 33(7):e67).

### Example 4

lacZ specific shRNA (nucleotide 1998-2055, SEQ ID NO:218) under the control of the human U6 promoter through RMCE was introduced into ART4.12/*rosa26*(RMCE) ES cells (Seibler et al. 2005, Nucl Acids Res 33(7):e67). Southern blot analysis of G418 resistant clones revealed that successful RMCE had occurred in >90 % of clones. Recombinant ES cells were injected into tetraploid blastocysts and ES cell derived mice were derived. A highly expressed β-galactosidase gene was provided through breeding using a mouse strain carrying lacZ (nucleotide 2161-5678, SEQ ID NO:219) under the control of the ubiquitous CAGGS promoter, that had been placed into the Rosa26 promoter. X-Gal staining on tissue sections revealed a strong, uniform expression of lacZ under the control of the CAGGS promoter in every single cell, whereas the presence of the shRNA construct resulted in marked reduction of ß-galactosidase activity in the vast majority of cells (Seibler et al. 2005, Nucl Acids Res 33(7):e67). These results indicate that the lacZ specific shRNA is sufficiently expressed to mediate efficient RNAi in all organs.

### Example 5

A CAGGS-Fluc (nucleotide 2100-5983, SEQ ID NO:218) expression cassette was inserted into the ES cell genome using RMCE at the *rosa26* locus (Seibler et al. 2005, Nucl Acids Res 33(7):e67). Again, successful RMCE had occurred in >90 % of clones as confirmed by Southern blot analysis. Recombinant ES cells were injected into blastocysts and mice were obtained upon transfer of blastocysts into pseudopregnant females. The Fluc-specific shRNA gene under the control of the human U6 promoter and the CAGGS-Fluc transgene were combined through breeding of mice. Measurement of luciferase activity in protein extracts from various organs revealed a strong reduction of luciferase activity in the presence, but not in the absence of the shRNA, indicating that both transgenes are ubiquitously expressed (Seibler et al. 2005, Nucl Acids Res 33(7):e67).

## Claims

1. An *in vitro* method for generating transgenic mammalian cells other than human embryonic cells, which method comprises the introduction by recombinase-mediated cassette exchange (RMCE) of an expression cassette for a short hairpin RNA (shRNA) into an ubiquitous locus of said cells, **characterized in that**:
(i) the ubiquitous locus comprises an acceptor DNA which comprises two mutually incompatible first recombinase recognition sites (RRSs); and
(ii) the exchange vector for RMCE comprises a donor DNA and a promoter-less positive selection marker, wherein:
(iii) the donor DNA comprises an expression cassette that includes a DNA encoding a shRNA operably linked to a promoter, said cassette being flanked by the same two mutually incompatible first RRSs present in the acceptor DNA;
(iv) said donor DNA and selection marker are flanked by the same two mutually incompatible first RRSs present in the acceptor DNA.

2. The method of claim 1, wherein the acceptor DNA has been introduced in the ubiquitous locus Rosa26 by homologous recombination with a targeting vector that comprises said acceptor DNA flanked by DNA sequences homologous to the ubiquitous locus.

3. The method of claim 1, wherein
(i) the mammalian cells are human cells or non-human mammalian cells, including rodent cells such as mouse cells and rat cells, provided that the cells are not human embryonic cells; and/or
(ii) the mammalian cells are primary cells or immortalized cells, preferably embryonic stem (ES) cells, provided that the cells are not human embryonic cells; and/or
(iii) the ubiquitous locus is selected from Rosa26, Collagen, ß-Actin, HPRT, U6, H1, tRNA and 7SL RNA, preferably is a Rosa26 locus.

4. The method according to any one of claims 1 to 3, wherein
(i) the promoter of the expression cassette is a heterologous promoter and preferably is a ubiquitous or tissue specific promoter, either constitutive or inducible; and/or
(ii) the targeting vector, the exchange vector and/or the expression cassette further comprises one or more additional functional sequences including but not limited to marker genes, second recombinase recognition sites differing from the first recombinase recognition sites, poly A signal and introns; and/or
(iii) the targeting vector and the exchange vector further comprises tags for protein detection, enhancers and selection markers; and/or
(iv) the targeting vector further comprises a gene coding for the recombinase which catalyses recombination between acceptor and donor DNA; and/or
(v) the DNA sequences homologous to the ubiquitous locus are 0.2 to 20 kB, preferably 1 to 10 kB long; and/or
(vi) the mutually incompatible RRS are selected from pairs of mutually incompatible loxP, FRT, and Att sites or variants thereof, preferably are selected from the following group of mutually incompatible RRS pairs: F3/FRT, F5/FRT, F5/F3, lox/lox511, lox/lox2722, lox66/lox71 and AttB/AttP; and/or
(vii) the recombinase, which may be added to the cell or may be expressed by the cell, is selected from recombinases suitable for cassette exchange of the first RSSs present in the acceptor/donor DNA, such as Cre, Flp, ΦC31 or mutants thereof; and/or
(viii) the short hairpin RNA expression cassette comprises a stop and/or polyadenylation sequence.

5. The method according to claim 4, wherein
(i) the ubiquitous promoter is selected from polymerase I, II and III dependent promoters, preferably is a polymerase II or III dependent promoter, most preferably is a CMV promoter, a CAGGS promoter, a Mx promoter, a PGK promoter, a snRNA promoter such as U6, a RNAse P RNA promoter such as H1, a tRNA promoter, a 7SL RNA promoter, and a 5 S rRNA promoter, and the tissue specific promoter is FABP, Lck, CamKII, CD19, Keratin, Albumin, aP2, Insulin, MCK, MyHC, WAP and Col2A promoter; and/or
(ii) the ubiquitous promoter is a constitutive promoter, or is an inducible promoter, preferably a promoter containing an operator sequence selected from tet, Gal4, lac, or RRSs for recombinase mediated control.

6. The method of claim 4, wherein the promoter is
(i) a Pol III dependent promoter, preferably constitutive H1 or U6, driven shRNA construct suitable to be integrated into a ubiquitously active Pol II dependent locus; or
(ii) a Pol III dependent promoter, preferably inducible U6 or H1, driven shRNA construct suitable to be integrated into a ubiquitously active Pol II dependent locus; or
(iii) a Pol II dependent promoter, preferably inducible CMV, driven shRNA construct suitable to be integrated into a ubiquitously active Pol II dependent locus.

7. The method according to any one of claims 1 to 6, wherein the short hairpin RNA construct or inactive precursor thereof comprises at least one segment corresponding to a short hairpin RNA (shRNA) or two complementary short interfering RNA (siRNA) strands, preferably comprises at least one shRNA segment having a DNA sequence A-B-C or C-B-A, or comprises at least two siRNA segments A and C or C and A, each of said at least two siRNA segments being under the control of a separate promoter, wherein
A is a 15 to 35, preferably 19 to 29 bp DNA sequence with at least 95%, preferably 100% complementarily to the gene to be knocked down;
B is a spacer DNA sequence having 5 to 9 bp forming the loop of the expressed RNA hair pin molecule, and
C is a 15 to 35, preferably 19 to 29 bp DNA sequence with at least 85% complementarily to the sequence A.

8. The method of any one of claims 3 to 7, wherein the transgenic mammalian cells are derived from mouse and the ubiquitous locus is a Rosa26 locus, and
(i) the DNA sequences homologous to the Rosa26 locus are derived from the 5' and 3' flanking arm of the mouse Rosa26 locus, preferably said homologous DNA sequences having the sequences shown in SEQ ID NO:4 and 5, respectively, and/or
(ii) the RRSs of the targeting and exchange vectors are F3/Frt and the targeting vector encodes the recombinase Flp or a mutant thereof, preferably Flp^{e}; and/or
(iii) the targeting vector comprises a negative selection marker; and/or
(iv) the promoter of the expression cassette is a H1 or H6;
most preferably the targeting vector has the sequence shown in SEQ ID NO:11 and the exchange vector has the sequence shown in SEQ ID NO:12 or a variant thereof with modification in the short hairpin RNA construct.

9. The method according to any one of claims 2 to 8, which further comprises one or more of the steps
isolating the mammalian cells, preferably the ES cells, having the desired functional exchange cassette or the inactive precursor integrated into the ubiquitous locus; and
optionally modifying the integrated precursor of the expression cassette to activate the precursor and isolating ES cells having the desired modified functional exchange cassette.

10. An exchange vector for RMCE which comprises a donor DNA and a promoter-less positive selection marker, wherein the donor DNA comprises an expression cassette that includes a DNA encoding a shRNA operably linked to a promoter, said donor DNA and selection marker being flanked by two mutually incompatible RRS, said exchange vector being suitable for carrying out the method of claims 1 to 9.

11. A mammalian cell having a ubiquitous locus carrying an expression cassette for a short hairpin RNA which is introduced into said locus by a recombinantly mediated cassette exchange according to the method of claims 1 to 9.

12. A method for preparing a non-human transgenic mammal having a modified ubiquitous locus, which method comprises the steps of
(i) obtaining isolated transfected ES cells of a non-human mammal according to the method of claim 9; and
(ii) injecting the ES cells obtained in step (i) into non-human blastocysts.

13. A method for preparing a non-human transgenic mammal having a modified ubiquitous locus, comprising the step of injecting an exchange vector according to claim 10 into an early stage embryo of a non-human mammal having corresponding RMCE target sites.

14. The method of claims 12 or 13, wherein said ubiquitous locus is a Rosa26 locus.

15. A transgenic non-human mammal or a tissue culture derived therefrom, which are obtainable by the method of claims 12 to 14, respectively, and have an operatively functional gene expression cassette for a short hairpin RNA integrated into at least one of its loci.

16. Use of an exchange vector of claim 10 for preparing an agent for constitutive and/or inducible gene knock down in a non-human mammal, or in tissue culture or cells of a cell culture derived from the non-human mammal.

17. Use of an exchange vector of claim 10 for preparing an agent suitable for injection into cells of an eight cell stage embryo of non-human mammals.

18. A method for constitutive and/or inducible gene knock down in a tissue culture or cells of a cell culture derived from a non-human mammal, which comprises stably integrating an exchange vector of claim 10 into the genome of the tissue culture or of the cells of the cell culture.

19. The method of claim 18, wherein the exchange vector is integrated at the Rosa26 locus of the tissue culture or cell culture.

20. Use of the mammalian cell of claim 11, the transgenic non-human mammal or tissue culture of the non-human mammal of claim 15 for gene function studies, drug development or as disease model animals.

## Patentansprüche

1. In-vitro-Verfahren zur Erzeugung von transgenen Säugerzellen mit Ausnahme von humanen embryonalen Zellen, wobei das Verfahren die Einführung einer Expressionscassette für eine kurze Haarnadel-RNA (shRNA) durch Rekombinase-vermittelten Cassettenaustausch (RMCE) in einen ubiquitären Locus dieser Zellen umfasst, **dadurch gekennzeichnet, dass**:
(i) der ubiquitäre Locus eine Akzeptor-DNA umfasst, die zwei miteinander unverträgliche erste Rekombinase-Erkennungsstellen (RRS) umfasst; und
(ii) der Austauschvektor für den RMCE eine Donor-DNA und einen promotorlosen positiven Selektionsmarker umfasst, wobei
(iii) die Donor-DNA eine Expressionscassette umfasst, welche eine DNA umfasst, die eine shRNA codiert und funktionell mit einem Promotor verknüpft ist;
(iv) wobei die Donor-DNA und der Selektionsmarker durch dieselben beiden miteinander unverträglichen ersten RRS, die in der Akzeptor-DNA vorhanden sind, flankiert sind.

2. Verfahren gemäß Anspruch 1, wobei die Akzeptor-DNA in den ubiquitären Locus Rosa26 durch homologe Rekombination mit einem Zielsteuerungsvektor eingeführt wurde, der die Akzeptor-DNA umfasst, die durch zu dem ubiquitären Locus homologe DNA-Sequenzen flankiert ist.

3. Verfahren gemäß Anspruch 1, wobei
(i) die Säugerzellen humane Zellen oder nichthumane Säugerzellen einschließlich Nagerzellen, wie Mäusezellen und Rattenzellen, sind, mit der Maßgabe, dass die Zellen keine humanen embryonalen Zellen sind; und/oder
(ii) die Säugerzellen primäre Zellen oder immortalisierte Zellen, vorzugsweise embryonale Stammzellen (ES-Zellen), sind, mit der Maßgabe, dass die Zellen keine humanen embryonalen Zellen sind; und/oder
(iii) der ubiquitäre Locus aus Rosa26, Collagen, β-Actin, HPRT, U6, H1, tRNA und 7SL-RNA ausgewählt ist und vorzugsweise ein Rosa26-Locus ist.

4. Verfahren gemäß einem der Ansprüche 1 bis 3, wobei
(i) der Promotor der Expressionscassette ein heterologer Promotor ist und vorzugsweise ein ubiquitärer oder gewebespezifischer Promotor ist, entweder konstitutiv oder induzierbar; und/oder
(ii) der Zielsteuerungsvektor, der Austauschvektor und/oder die Expressionscassette weiterhin eine oder mehrere zusätzliche funktionelle Sequenzen einschließlich, aber nicht beschränkt auf Marker-Gene, zweite Rekombinase-Erkennungsstellen, die sich von den ersten Rekombinase-Erkennungsstellen unterscheiden, Poly-A-Signal und Introns umfasst; und/oder
(iii) der Zielsteuerungsvektor und der Austauschvektor weiterhin Tags für den Proteinnachweis, Enhancer und Selektionsmarker umfassen; und/oder
(iv) der Zielsteuerungsvektor weiterhin ein Gen umfasst, das für die Recombinase codiert, die die Rekombination zwischen Akzeptor- und Donor-DNA katalysiert; und/oder
(v) in Schritt (a) die zu dem ubiquitären Locus homologen DNA-Sequenzen eine Länge von 0,2 bis 20 kB, vorzugsweise 1 bis 10 kB, aufweisen; und/oder
(vi) die miteinander unverträglichen RRS aus Paaren von miteinander unverträglichen loxP-, FRT- und Att-Stellen oder Varianten davon ausgewählt sind und vorzugsweise aus der folgenden Gruppe von miteinander unverträglichen RRS-Paaren ausgewählt sind: F3/FRT, F5/FRT, F5/F3, lox/lox511, lox/lox2722, lox66/lox71 und AttB/AttP; und/oder
(vii) die Recombinase, die zu der Zelle hinzugefügt oder von der Zelle exprimiert werden kann, aus Rekombinasen ausgewählt ist, die für einen Cassettenaustausch der ersten RSS, die in der Akzeptor-/ Donor-DNA vorhanden sind, wie Cre, Flp, ΦC31 oder Mutanten davon, geeignet ist; und/oder
(viii) die Expressionscassette für die kurze Haarnadel-RNA ein Stopcodon und/oder eine Polyadenylierungssequenz umfasst.

5. Verfahren gemäß Anspruch 4, wobei
(i) der ubiquitäre Promotor aus Polymerase-I-, -II- und -III-abhängigen Promotoren ausgewählt ist, vorzugsweise ein Polymerase-II- oder -III-abhängiger Promotor ist, am meisten bevorzugt ein CMV-Promotor, ein CAGGS-Promotor, ein Mx-Promotor, ein PGK-Promotor, ein snRNA-Promotor, wie U6, ein RNase-P-RNA-Promotor, wie H1, ein tRNA-Promotor, ein 7SL-RNA-Promotor und ein -S-rRNA-Promotor ist und der gewebespezifische Promotor ein FABP-, Lck-, CamKII-, CD19-, Keratin-, Albumin-, aP2-, Insulin-, MCK-, MyHC-, WAP- und Col2A-Promotor ist; und/oder
(ii) der ubiquitäre Promotor ein konstitutiver Promotor oder ein induzierbarer Promotor ist, vorzugsweise ein Promotor, der eine Operatorsequenz, die aus tet, Gal4, lac oder RRSs ausgewählt ist, für die Rekombinase-vermittelte Steuerung enthält.

6. Verfahren gemäß Anspruch 4, wobei der Promotor
(i) ein von einem Pol-III-abhängigen Promotor, vorzugsweise konstitutivem H1 oder U6, reguliertes shRNA-Konstrukt ist, das in einen ubiquitär aktiven Pol-II-abhängigen Locus integriert werden kann; oder
(ii) ein von einem Pol-III-abhängigen Promotor, vorzugsweise induzierbarem U6 oder H1, reguliertes shRNA-Konstrukt ist, das in einen ubiquitär aktiven Pol-II-abhängigen Locus integriert werden kann; oder
(iii) ein von einem Pol-II-abhängigen Promotor, vorzugsweise induzierbarem CMV, reguliertes shRNA-Konstrukt ist, das in einen ubiquitär aktiven Pol-II-abhängigen Locus integriert werden kann.

7. Verfahren gemäß einem der Ansprüche 1 bis 6, wobei das shRNA-Konstrukt oder ein inaktiver Vorläufer davon wenigstens ein Segment umfasst, das einer kurzen Haarnadel-RNA (shRNA) oder zwei komplementären kurzen interferierenden RNA-Strängen (siRNA) entspricht, vorzugsweise wenigstens ein shRNA-Segment umfasst, das eine DNA-Sequenz A-B-C oder C-B-A aufweist, oder wenigstens zwei siRNA-Segmente A und C oder C und A umfasst, wobei jedes der wenigstens zwei siRNA-Segmente unter der Kontrolle eines getrennten Promotors steht, wobei
A eine DNA-Sequenz von 15 bis 35 bp, vorzugsweise 19 bis 29 bp, mit wenigstens 95%, vorzugsweise 100%, Komplementarität zu dem abzuschaltenden Gen ist;
B eine Spacer-DNA-Sequenz mit 5 bis 9 bp ist, die die Schleife des exprimierten RNA-Haarnadelmoleküls bildet; und
C eine DNA-Sequenz von 15 bis 35 bp, vorzugsweise 19 bis 29 bp, mit wenigstens 85% Komplementarität zur Sequenz A ist.

8. Verfahren gemäß einem der Ansprüche 3 bis 7, wobei die transgenen Säugerzellen von der Maus stammen und der ubiquitäre Locus ein Rosa26-Locus ist; und
(i) die zu dem Rosa26-Locus homologen DNA-Sequenzen aus dem 5'- und 3'-flankierenden Arm des Maus-Rosa26-Locus stammen, die homologen DNA-Sequenzen vorzugsweise die jeweils in SEQ ID Nr. 4 und 5 gezeigten Sequenzen aufweisen; und/oder
(ii) es sich bei den RRS des Zielsteuerungs- und des Austauschvektors um F3/Frt handelt und der Zielsteuerungsvektor die Recombinase Flp oder eine Mutante davon, vorzugsweise Flp^{e}, codiert; und/oder
(iii) der Zielsteuerungsvektor einen negativen Selektionsmarker umfasst; und/oder
(iv) es sich bei dem Promotor der Expressionscassette um H1 oder H6 handelt;
am meisten bevorzugt der Zielsteuerungsvektor die in SEQ ID Nr. 11 gezeigte Sequenz aufweist und der Austauschvektor die in SEQ ID Nr. 12 gezeigte Sequenz oder eine Variante davon mit einer Modifikation im kurzen Haarnadel-RNA-Konstrukt aufweist.

9. Verfahren gemäß einem der Ansprüche 2 bis 8, das weiterhin einen oder mehrere der folgenden Schritte umfasst:
Isolieren der Säugerzellen, vorzugsweise der ES-Zellen, mit der gewünschten funktionellen Austauschcassette oder dem inaktiven Vorläufer, die oder der in den ubiquitären Locus integriert ist; und
gegebenenfalls Modifizieren des integrierten Vorläufers der Expressionscassette in einer solchen Weise, dass der Vorläufer aktiviert wird, und Isolieren von ES-Zellen mit der gewünschten modifizierten funktionellen Austauschcassette.

10. Austauschvektor für RMCE, der eine Donor-DNA und einen promotorlosen positiven Selektionsmarker umfasst, wobei die Donor-DNA eine Expressionscassette umfasst, welche eine DNA umfasst, die eine shRNA codiert und funktionell mit einem Promotor verknüpft ist, wobei die Donor-DNA und der Selektionsmarker durch zwei miteinander unverträgliche RRS flankiert sind, wobei der Austauschvektor für die Durchführung des Verfahrens gemäß den Ansprüchen 1 bis 9 geeignet ist.

11. Säugerzelle, die einen ubiquitären Locus aufweist, der eine Expressionscassette für eine kurze Haarnadel-RNA trägt, die durch einen rekombinant vermittelten Cassettenaustausch nach dem Verfahren gemäß den Ansprüchen 1 bis 9 in den Locus eingeführt ist.

12. Verfahren zur Herstellung eines nichthumanen transgenen Säugers, der einen modifizierten ubiquitären Locus aufweist, wobei das Verfahren die folgenden Schritte umfasst:
(i) Gewinnen isolierter transfizierter ES-Zellen eines nichthumanen Säugers nach dem Verfahren gemäß Anspruch 9; und
(ii) Injizieren der in Schritt (i) gewonnenen ES-Zellen in nichthumane Blastocysten.

13. Verfahren zur Herstellung eines nichthumanen transgenen Säugers, der einen modifizierten ubiquitären Locus aufweist, umfassend den Schritt des Injizierens eines Austauschvektors gemäß Anspruch 10 in einen im frühen Stadium befindlichen Embryo eines nichthumanen Säugers, der entsprechende RMCE-Zielstellen aufweist.

14. Verfahren gemäß Anspruch 12 oder 13, wobei der ubiquitäre Locus ein Rosa26-Locus ist.

15. Transgener nichthumaner Säuger oder davon abgeleitete Gewebekultur, die jeweils nach dem Verfahren gemäß Anspruch 12 bis 14 erhältlich sind und eine funktionell funktionsfähige Genexpressionscassette für eine kurze Haarnadel-RNA aufweisen, die in wenigstens einen seiner Loci integriert ist.

16. Verwendung eines Austauschvektors gemäß Anspruch 10 zur Herstellung eines Mittels zur konstitutiven und/oder induzierbaren Gen-Abschaltung in einem nichthumanen Säuger oder in Gewebekultur oder in Zellen einer Zellkultur, die von dem nichthumanen Säuger stammen.

17. Verwendung eines Austauschvektors gemäß Anspruch 10 zur Herstellung eines Mittels, das für die Injektion in Zellen eines im Acht-Zell-Stadium befindlichen Embryos von nichthumanen Säugern geeignet ist.

18. Verfahren zur konstitutiven und/oder induzierbaren Gen-Abschaltung in einer Gewebekultur oder in Zellen einer Zellkultur, die von einem nichthumanen Säuger stammen, umfassend das stabile Integrieren eines Austauschvektors gemäß Anspruch 10 in das Genom der Gewebekultur oder der Zellen der Zellkultur.

19. Verfahren gemäß Anspruch 18, wobei der Austauschvektor am Rosa26-Locus der Gewebekultur oder Zellkultur integriert ist.

20. Verwendung der Säugerzelle gemäß Anspruch 11, des transgenen nichthumanen Säugers oder der Gewebekultur des nichthumanen Säugers gemäß Anspruch 15 für Genfunktionsstudien, die Wirkstoffentwicklung oder als Krankheitsmodelltiere.

## Revendications

1. Procédé *in vitro* permettant de générer des cellules de mammifères transgéniques autres que des cellules embryonnaires humaines, lequel procédé comprend l'introduction, par échange de cassette médié par la recombinase (RMCE), d'une cassette d'expression d'un petit ARN en épingle à cheveu (ARNsh) dans un locus ubiquitaire desdites cellules, **caractérisé en ce que** :
(i) le locus ubiquitaire comprend un ADN accepteur qui comprend deux premiers sites de reconnaissance de la recombinase mutuellement incompatibles (RRS) ; et
(ii) le vecteur d'échange pour le RMCE comprend un ADN donneur et un marqueur de sélection positive sans promoteur, dans lequel :
(iii)l'ADN donneur comprend une cassette d'expression qui comprend un ADN codant pour un ARNsh lié de manière fonctionnelle à un promoteur ;
(iv) lesdits ADN donneur et marqueur de sélection sont flanqués par les mêmes deux premiers RSS mutuellement incompatibles présents dans l'ADN accepteur.

2. Procédé selon la revendication 1, dans lequel l'ADN accepteur a été introduit dans le locus ubiquitaire Rosa26 par recombinaison homologue avec un vecteur de ciblage qui comprend ledit ADN accepteur flanqué par des séquences d'ADN homologues au locus ubiquitaire.

3. Procédé selon la revendication 1, dans lequel
(i) les cellules de mammifères sont des cellules humaines ou des cellules de mammifères non humaines, parmi lesquelles des cellules de rongeurs telles que des cellules de souris et des cellules de rats, à la condition que les cellules ne soient pas des cellules embryonnaires humaines ; et/ou
(ii) les cellules de mammifères sont des cellules primaires ou des cellules immortalisées, de préférence des cellules souches embryonnaires (SE), à la condition que les cellules ne soient pas des cellules embryonnaires humaines ; et/ou
(iii) le locus ubiquitaire est sélectionné parmi Rosa26, le collagène, la β-actine, HPRT, U6, H1, l'ARNt et l'ARN 7SL, de préférence le locus Rosa26.

4. Procédé selon l'une quelconque des revendications 1 à 3, dans lequel
(i) le promoteur de la cassette d'expression est un promoteur hétérologue et est de préférence un promoteur ubiquitaire ou tissu-spécifique, soit constitutif soit inductible ; et/ou
(ii) le vecteur de ciblage, le vecteur d'échange et/ou la cassette d'expression comprennent en outre une ou plusieurs séquences fonctionnelles supplémentaires comprenant mais sans s'y limiter les gènes marqueurs, les seconds sites de reconnaissance de la recombinase différant des premiers sites de reconnaissance de la recombinase, le signal poly A et des introns ; et/ou
(iii) le vecteur de ciblage et le vecteur d'échange comprennent en outre des marqueurs pour la détection des protéines, des amplificateurs et des marqueurs de sélection ;
(iv) le vecteur de ciblage comprend en outre un gène codant pour la recombinase qui catalyse la recombinaison entre l'ADN accepteur et donneur ; et/ou
(v) à l'étape (a), les séquences d'ADN homologues au locus ubiquitaire ont une longueur de 0,2 à 20 kB, de préférence de 1 à 10 kB ; et/ou
(vi) les RRS mutuellement incompatibles sont sélectionnés parmi des paires de sites loxP, FRT et Att mutuellement incompatibles ou de variants de ceux-ci, sont de préférence sélectionnés dans le groupe suivant de paires de RRS mutuellement incompatibles: F3/FRT, F5/FRT, F5/F3, lox/lox511, lox/lox2722, lox66/lox71 et AttB/AttP ; et/ou
(vii) la recombinase, qui peut être ajoutée à la cellule ou peut être exprimée par la cellule, est sélectionnée parmi des recombinases appropriées pour un échange de cassette des premiers RRS présents dans l'ADN accepteur/donneur, telles que Cre, Flp, ΦC31 ou des mutants de celles-ci ;
et/ou
(viii) la cassette d'expression de petit ARN en épingle à cheveu comprend une séquence d'arrêt et/ou de polyadénylation.

5. Procédé selon la revendication 4, dans lequel
(i) le promoteur ubiquitaire est sélectionné parmi les promoteurs dépendants de la polymérase I, II et III, est de préférence un promoteur dépendant de la polymérase II ou III, est le plus préférablement un promoteur CMV, un promoteur CAGGS, un promoteur Mx, un promoteur PGK, un promoteur ARNsn tel que U6, un promoteur ARN de l'ARNase P tel que H1, un promoteur ARNt, un promoteur ARN 7SL et un promoteur ARNr 5S, et le promoteur tissu-spécifique est FABP, Lck, CamKII, CD19, la kératine, l'albumine, aP2, l'insuline, MCK, MyHC, WAP et le promoteur Col2A ; et/ou
(ii) le promoteur ubiquitaire est un promoteur constitutif, ou est un promoteur inductible, de préférence un promoteur contenant une séquence opératrice sélectionnée parmi tet, Gal4, lac, ou les RRS pour un contrôle médié par la recombinase.

6. Procédé selon la revendication 4, dans lequel le promoteur est (i) une construction d'ARNsh sous le contrôle d'un promoteur dépendant de Pol III, de préférence le promoteur constitutif H1 ou U6, appropriée pour être intégrée dans un locus dépendant de Pol II ubiquitairement actif ; ou (ii) une construction d'ARNsh sous le contrôle d'un promoteur dépendant de Pol III, de préférence le promoteur inductible U6 ou H1, appropriée pour être intégrée dans un locus dépendant de Pol II ubiquitairement actif ; ou (iii) une construction d'ARNsh sous le contrôle d'un promoteur dépendant de Pol II, de préférence le promoteur inductible CMV, appropriée pour être intégrée dans un locus dépendant de Pol II ubiquitairement actif.

7. Procédé selon l'une quelconque des revendications 1 à 6, dans lequel la construction de petit ARN en épingle à cheveu ou le précurseur inactif de celle-ci comprend au moins un segment correspondant à un petit ARN en épingle à cheveu (ARNsh) ou deux brins de petit ARN interférent (ARNsi) complémentaires, comprend de préférence au moins un segment d'ARNsh ayant une séquence d'ADN A-B-C ou C-B-A, ou comprend au moins deux segments d'ARNsi A et C ou C et A, chacun desdits au moins deux segments d'ARNsi étant sous le contrôle d'un promoteur séparé, où
A est une séquence d'ADN de 15 à 35, de préférence 19 à 29 pb, avec au moins 95 %, de préférence 100 % de complémentarité avec le gène à inactiver ;
B est une séquence d'ADN espaceur de 5 à 9 pb formant la boucle de la molécule en épingle à cheveu d'ARN exprimée, et
C est une séquence d'ADN de 15 à 35, de préférence 19 à 29 pb, avec au moins 85 % de complémentarité avec la séquence A.

8. Procédé selon l'une quelconque des revendications 3 à 7, dans lequel les cellules de mammifères transgéniques sont dérivées de la souris et le locus ubiquitaire est un locus Rosa26, et
(i) les séquences d'ADN homologues au locus Rosa26 sont dérivées du bras flanquant 5' et 3' du locus Rosa26 de la souris, lesdites séquences d'ADN homologues ayant de préférence les séquences montrées dans les SEQ ID n° 4 et 5, respectivement, et/ou
(ii) les RRS des vecteurs de ciblage et d'échange sont F3/FRT et le vecteur de ciblage code pour la recombinase Flp ou un mutant de celle-ci, de préférence Flp^{e} ; et/ou
(iii) le vecteur de ciblage comprend un marqueur de sélection négative ; et/ou
(iv) le promoteur de la cassette d'expression est un promoteur H1 ou H6 ; le plus préférablement le vecteur de ciblage a la séquence montrée dans le SEQ ID n° 11 et le vecteur d'échange a la séquence montrée dans le SEQ ID n° 12 ou un variant de celle-ci avec une modification de la construction de petit ARN en épingle à cheveu.

9. Procédé selon l'une quelconque des revendications 2 à 8, qui comprend en outre une ou plusieurs des étapes consistant à isoler les cellules de mammifères, de préférence les cellules SE, ayant la cassette d'échange fonctionnelle souhaitée ou le précurseur inactif intégré(e) dans le locus ubiquitaire ; et
modifier facultativement le précurseur intégré de la cassette d'expression pour activer le précurseur et isoler les cellules SE ayant la cassette d'échange fonctionnelle modifiée souhaitée.

10. Vecteur d'échange pour RMCE qui comprend un ADN donneur et un marqueur de sélection positive sans promoteur, dans lequel l'ADN donneur comprend une cassette d'expression qui comprend un ADN codant pour un ARNsh lié de manière fonctionnelle à un promoteur, lesdits ADN donneur et marqueur de sélection étant flanqués par deux RRS mutuellement incompatibles, ledit vecteur d'échange étant approprié pour réaliser le procédé selon les revendications 1 à 9.

11. Cellule de mammifère ayant un locus ubiquitaire portant une cassette d'expression d'un petit ARN en épingle à cheveu qui est introduite dans ledit locus par un échange de cassette médié de manière recombinante conformément au procédé selon les revendications 1 à 9.

12. Procédé permettant de préparer un mammifère transgénique non humain ayant un locus ubiquitaire modifié, lequel procédé comprend les étapes consistant à
(i) obtenir des cellules SE transfectées isolées d'un mammifère non humain conformément au procédé selon la revendication 9 ; et
(ii) injecter les cellules SE obtenues à l'étape (i) dans des blastocystes non humains.

13. Procédé permettant de préparer un mammifère transgénique non humain ayant un locus ubiquitaire modifié, comprenant l'étape consistant à injecter un vecteur d'échange selon la revendication 10 dans un embryon à un stade précoce d'un mammifère non humain ayant des sites cibles RMCE correspondants.

14. Procédé selon les revendications 12 ou 13, dans lequel ledit locus ubiquitaire est un locus Rosa26.

15. Mammifère transgénique non humain ou culture de tissus dérivée de celui-ci, qui peut être obtenu(e) par le procédé selon les revendications 12 à 14, respectivement, et a une cassette d'expression de gènes fonctionnelle d'un petit ARN en épingle à cheveu intégré dans au moins un de ses loci.

16. Utilisation d'un vecteur d'échange selon la revendication 10 pour préparer un agent destiné à l'inactivation d'un gène constitutif et/ou inductible chez un mammifère non humain, ou dans une culture de tissus ou des cellules d'une culture de cellules dérivée du mammifère non humain.

17. Utilisation d'un vecteur d'échange selon la revendication 10 pour préparer un agent approprié pour une injection dans des cellules d'un embryon, au stade de huit cellules, de mammifères non humains.

18. Procédé permettant d'inactiver un gène constitutif et/ou inductible dans une culture de tissus ou des cellules d'une culture de cellules dérivée d'un mammifère non humain, qui comprend l'intégration stable d'un vecteur d'échange selon la revendication 10 dans le génome de la culture de tissus ou des cellules de la culture de cellules.

19. Procédé selon la revendication 18, dans lequel le vecteur d'échange est intégré au niveau du locus Rosa26 de la culture de tissus ou de la culture de cellules.

20. Utilisation de la cellule de mammifère selon la revendication 11, du mammifère non humain transgénique ou de la culture de tissus du mammifère non humain selon la revendication 15 dans le cadre de l'étude des fonctions des gènes, du développement de médicaments ou en tant que modèles animaux de maladies.
